# EUROPEAN PATENT APPLICATION

(11) **EP 2 913 333 A1**
(43) Date of publication of application: **02.09.2015**
(21) Application number: 14305283.5
(22) Date of filing: 27.02.2014
(51) Int. Cl.: C07D 487/04, C07D 495/04, A61K 31/53, A61K 31/519, A61P 31/04

(54) **3H-thieno[3,4]pyrimidin-4-one and pyrrolopyrimidone as gram-positive antibacterial agents**

(71) Applicant: LABORATOIRE BIODIM, 75007 Paris (FR)
(72) Inventor: Atamanyuk, Dmytro, 77500 Chelles (FR); Chevreuil, Francis, 60500 Chantilly (FR); Faivre, Fabien, 93700 Drancy (FR); Gerusz, Vincent, 75017 Paris (FR); Gold, Johan, 74500 Publier (FR); Moreau, Francois, 91400 Orsay (FR); Simon, Christophe, 94550 Chevilly La Rue (FR)
(74) Representative: Hirsch & Associés

(57) **Abstract**

The invention relates to novel heteroaromatic derivatives of formula (I) and their addition salts thereof with acids and bases; their preparation, their use as drugs and pharmaceutical compositions and associations containing them. 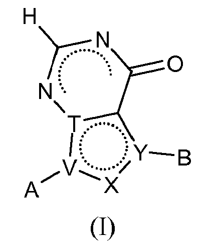 wherein A, B, T, V, X and Y represent organic substituents as defined herein,

The compounds of formula (I) are able to inhibit the activity of the Gram-positive bacterial DltA enzyme and they are used to prevent and/or treat Gram-positive bacterial infections in humans or animals, alone or in association with antibacterials, antivirulence agents or drugs reinforcing the host innate immunity,

## Description

The invention relates to novel heteroaromatic derivatives, their preparation and intermediates, their use as drugs and pharmaceutical compositions containing them.

The invention particularly relates to new compounds capable of inhibiting the activity of the Gram-positive bacterial DltA enzyme and their use to prevent and/or treat Gram-positive bacterial infections in humans or animals.

The emergence of antibiotic-resistant pathogens has become a serious worldwide healthcare problem. Indeed, some infections are now caused by multi-drug resistant organisms that are no longer responsive to currently available treatments. This issue has led to the search for innovative antibacterial approaches with novel modes of action. Among them the inhibition of virulence targets, a concept called antivirulence, is aiming at lessening or inhibiting the pathogenicity of a bacterium to its host (Curr. Opin. Chem. Biol. 2008, 12, 1). Potential advantages of this strategy over classical treatments lay in the preservation of the commensal flora as well as a selection pressure confined to the host tissues where the targeted virulence factor is essential for bacterial survival.

It is known that the extracellular bacteria responsible for serious infections are capable of growth in the extra cellular fluids and are resistant to the bactericidal action of the host innate immunity. This resistance of bacteria to the innate immunity components allows dissemination of the infection, via the blood, to the various tissues. The components of the innate immunity are either circulating molecules such as the complement factors, or the antibacterial peptides such as defensins, which could have bactericidal effects by direct interactions with the bacterium cell wall, or either circulating cells such as the polymorphonuclear leukocytes (PMNs) able to kill invading bacteria.

Among the many virulence factors described to be important for bacteria to resist the innate immunity components are the mechanisms involved in resistance to cationic antimicrobial peptides (CAMP). CAMP play a fundamental role in innate immune defenses, both through direct antimicrobial activity and through immunomodulatory effects (Boman et al., J Intern Med. 2003, 254(3), 197). The CAMP dominating targets are bacterial membranes and CAMP interact with negatively charged bacterial surface. One of the main mechanisms present in bacteria to decrease the negative charge of the cell wall is the addition of positively charged amino acid on structural element of the cell surface.

The dlt operon is responsible for D-alanine modification of cell wall teichoic acids, and its suppression has been linked to attenuated virulence in mice (Peschel et al., J. Infect. Dis. 2002, 186, 214). Among the different proteins expressed by this operon, DltA is a D-alanine:D-alanyl carrier protein ligase acting as the first enzyme controlling the D-alanylation of the teichoic acids as major components of Gram-positive cell wall: the lipoteichoic acids (LTA) and the wall teichoic acids (WTA). Mutant lacking the *dltA* gene resulted in the absence of D-Ala ester in the LTA (Poyart et al., J Bacteriol. 2001, 183, 6324). As a consequence, D-Ala deficient bacteria displayed a modification in the net charge of their cell surface and an increased susceptibility to various bactericidal compounds including cationic antimicrobial peptides and Vancomycin (FEBS Journal 2005, 272, 299). Resistance to Daptomycin was shown to correlate with higher dltA expression, and higher level of wall teichoic acids D-Alanylation (Bertsche et al., Antimicrob Agents Chemother. 2011 55, 3922).

Because the *dlt* operon is conserved in many Gram positive bacteria of medical relevance, such inhibitors would be useful in rendering invading bacteria sensitive to killing by the innate immunity mechanism of the host, allowing eradication or prevention of the infection by a new mechanism of action when compared to current antibiotic treatment.

As antivirulent target, DltA is still largely unexploited at this time since there are no drugs on market nor on advanced clinical phases. Only very few derivatives that display a selective spectrum of activity on species containing DltA have been reported.

The novel compounds of the present invention are based on innovative heteroaromatic bicyclic systems and unexpectedly display an especially interesting spectrum of activity on Gram-positive bacteria.

The prior art is essentially constituted by derivatives as those described in WO2012172043A1, Khimiya Geterotsiklicheskikh Soedinenii (1975), (7), 914-17, WO2008024978, Journal of Medicinal Chemistry (2013), 56(20), 8073-8088, US7557113, WO2005019219A1, WO9635689A1 and Journal of Organic Chemistry (1979), 44(22), 3826-9. To the knowledge of the applicant, only the purine derivatives described in WO2012172043A1 has been reported in the therapeutic domain of the invention.With respect to these purine derivatives, the level of activity of the compounds of the present invention is significantly higher.

One of the purposes of the present invention is therefore to provide novel compounds active on DltA and related targets. Among other properties, these compounds advantageously modify the surface charge of the Gram-positive cell wall by inhibiting the D-Alanylation of the teichoic acids. As a consequence they can display synergistic effects with components of the innate immune system like CAMP such as alpha-defensins, beta-defensins or peptides of the cathelicidin family. As bacterial cell wall modifiers, they could also be associated and synergize with drugs aiming at treating bacterial infections such as compounds reinforcing the innate immunity of the host, or antibacterials and more specifically with compounds targeting the bacterial cell wall and/or membrane, or for which the bacterial cell wall and/or membrane are a limiting step for penetration. Such compounds include any antibiotics, and more specifically glycopeptides like vancomycin, lipopeptides like daptomycin, antimicrobial peptides like polymixins, cathelicidins, defensins or any synthetic or natural peptide derived from the above listed ones. The association of a DltA inhibitor and an antibacterial could, by lowering the active dose of the antibacterial, expand its therapeutic window.

The invention relates to compounds having the general formula (I) 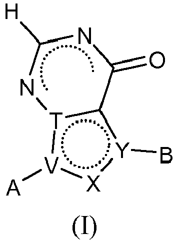 wherein:
the dotted line in the 6-membered heterocycle indicates a double bond in either of the possible positions between one C and one N;
the dotted line in the 5-membered cycle indicates a cycle with an aromatic character;
TisCorN,
VisCorN,
Y is C or N,
X is CH or S with the proviso that when X is S then T is C, V is C and Y is C,
A is selected from the group consisting of phenyl, naphthyl and (5-11) membered monocyclic or bicyclic unsaturated cycle or heterocycle linked by a carbon atom and containing 1-4 heteroatoms selected from N, O and S, all the above members of the group representing A being possibly substituted on carbon or on nitrogen atoms by 1-4 identical or different groups R₁; and
A can also comprise either a further (4-7) membered heterocycle formed with R₁, said heterocyle being a monocycle fused, saturated or unsaturated, and closed with R₁ by a simple bond or a double bond, or a further (4-7) membered monocycle formed by two substituents R₁ one with the other, said monocycle being then fused, saturated or unsaturated, and the two R₁ being linked together by a simple bond or a double bond, the polycyclic system representing A with R₁ then comprising up to 14 members and up to 5 heteroatoms selected from N, O and S;
B is selected from the group consisting of Halogen, NR₂R₃, SO₂NRₐR_{b}, (C₂-C₆)Alkyl, (C₂-C₆)Alkenyl, (C₂-C₆)Alkynyl, (C₃-C₁₂)Cycloalkyl, (C₅-C₈)Cycloalkenyl, O-(C₁-C₆)Alkyl, O-(C₃-C₁₂)Cycloalkyl, S-(C₁-C₆)Alkyl and S-(C₃-C₁₂)Cycloalkyl, the Alkyl, Alkenyl, Cycloalkenyl, Alkynyl and Cycloalkyl groups being possibly substituted by 1 to 3 identical or different substituents selected from the groups consisting of (C₁-C₆)Alkyl, (C₁-C₆)Alkyl-O-Rₐ, (C₁-c₆)Alkyl-S-Rₐ, (C₁-C₆)Alkyl-NRₐR_{b}, C(O)-O-Rₐ, C(O)-NRₐR_{b}, O-Rₐ, NR₂R₃, O-(C₇-C₁₂)Aralkyl and NRₐR_{b};
or B is a (4-10) membered monocyclic or bicyclic saturated or unsaturated heterocycle containing 1 to 3 heteroatoms selected from N, O and S, and possibly substituted by 1 to 3 identical or different substituents R₄;
R₁ is selected from the group consisting of Hydrogen, Halogen, CN, C(Halogen)ₙ with n an integer equal to 1, 2 or 3, NO₂ (C₁-C₆)Alkyl, (C₃-C₁₂)Cycloalkyl, =O, =N-O-(C₁-C₆)Alkyl, =N-O-(C₃-C₁₂)Cycloalkyl, C(O)-NRₐR_{b}, O-Rₐ, NR₂R₃ and NRₐC(O)R_{b}; the Alkyl and Cycloalkyl groups being possibly substituted by 1 to 3 identical or different substituents selected from the group consisting of Halogen, (C₁-C₆)Alkyl, (C₃-C₁₂)Cycloalkyl, O-Rₐ and NR₂R₃;
or R₁ is a (4-8) membered monocyclic saturated or unsaturated heterocycle containing 1-3 heteroatoms selected from N, O and S, and possibly substituted by 1 to 3 identical or different substituents selected from the group consisting of Halogen, (C₁-C₆)Alkyl, (C₃C₁₂)Cycloalkyl, O-Rₐ and NR₂R₃;
or R₁ forms with A, or forms within A with another R₁, a cycle defined above;
R₂ and R₃, identical or different, are selected from the group consisting of Hydrogen, (C₁-C₆)Alkyl, (C₃-C₁₂)Cycloalkyl, C(O)-Rₐ, C(O)-NRₐR_{b}, C(O)-ORₐ, SO₂(C₁-C₆)Alkyl, SO₂(C₃-C₁₂)Cycloalkyl and O-Rₐ, the Alkyl and Cycloalkyl groups being possibly substituted by 1 to 3 identical or different substituents selected from the group consisting of Halogen, O-Rₐ, NRₐR_{b}, NRₐC(O)-R_{b}, C(O)-Rₐ, SO₂(C₁-C₆)Alkyl, SO₂(C₃-C₁₂)Cycloalkyl, C(O)-O-Rₐ and C(O)-NRₐR_{b};
or R₂ and R₃ are selected from the group consisting of (C₁-C₆)Alkyl and (C₃-C₁₂)Cycloalkyl, which are substituted by a (3-10) membered monocyclic or bicyclic saturated or unsaturated heterocycle possibly containing 1-3 heteroatoms selected from N, O and S, and possibly substituted by 1 to 3 identical or different substituents selected from the group consisting of (C₁-C₆)Alkyl, (C₃-C₁₂)Cycloalkyl and C(O)Rₐ;
or R₂ and R₃ form together with N a (4-10) membered saturated or unsaturated monocyclic or bicyclic heterocycle possibly containing another heteroatom selected from N, O, and S and possibly substituted by 1 to 3 identical or different groups R₄;
R₄ is selected from the group consisting of Halogen, =O, CN, (C₁-C₆)Alkyl, (C₃-C₁₂)Cycloalkyl, C(O)-Rₐ, C(O)-O-Rₐ, C(O)-NRₐR_{b}, O-Rₐ, N(Rₐ)-C(O)-R_{b} and NRₐR_{b}, the Alkyl and Cycloalkyl groups being possibly substituted by 1 to 3 identical or different substituents selected from the group consisting of O-Rₐ and NRₐR_{b};
Rₐ and R_{b}, identical or different, are selected from the group consisting of Hydrogen, (C₁-C₆)Alkyl and (C₃-C₁₂)Cycloalkyl, the Alkyl and Cycloalkyl groups being possibly substituted by 1 to 3 substituents selected from the group consiting of OH, O-(C₁-C₆)Alkyl and O-(C₃-C₁₂)Cycloalkyl.

Also included in the invention are the addition salts of the compounds of formula (I) with acids and bases, the tautomeric forms of the compounds of formula (I), as well as the racemic mixtures, the pure enantiomers and the non-racemic (scalemic) mixture of enantiomers and diastereoisomers, in case the compounds of formula (I) have one or more chiral centers, both the cis (Z) and trans (E) isomers as well as their mixtures in case the compounds of formula (I) have unsaturated carbon carbon double bonds.

According to the conditions of the medium, the compounds of formula (I) exist under either of the tautomeric forms presented by the following formulae (Ia), (Ib) and (Ic) 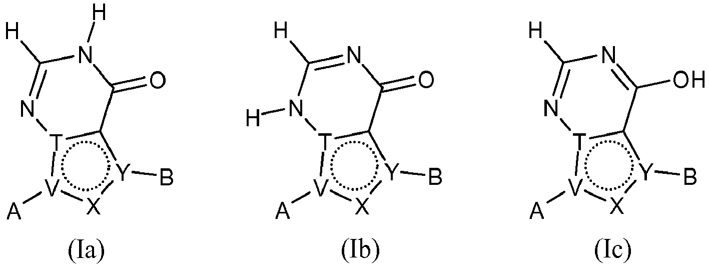 wherein A and B are defined as above.

Hereafter in the application, by commodity, the compounds prepared in the examples have all been represented under formula (Ia).

Among the acid salts of the compounds of formula (I), there may be cited, among others, those formed with mineral acids, such as hydrochloric, hydrobromic, hydroiodic, sulfuric or phosphoric acid or with organic acids, such as formic, acetic, trifluoroacetic, propionic, benzoic, maleic, fumaric, succinic, tartaric, citric, oxalic, glyoxylic, aspartic, alkanesulfonic acids, such as methanesulfonic and ethanesulfonic acids, arylsulfonic acids such as benzenesulfonic and paratoluenesulfonic acids.

Among the base salts of the compounds of formula (I), there may be cited, among others, those formed with mineral alkalis such as, for example, sodium, potassium, lithium, calcium, magnesium or ammonium or organic bases such as, for example, methylamine, ethylamine, propylamine, trimethylamine, diethylamine, triethylamine, N,N-dimethylethanolamine, tris(hydroxymethyl)aminomethane, ethanolamine, pyridine, piperidine, piperazine, picoline, dicyclohexylamine, morpholine, benzylamine, procaine, lysine, arginine, histidine, N-methylglucamine.

In the general formula (I), as applied herein and wherever they appear:
"(CᵢCⱼ)Alkyl" means any linear, i.e-straight-chain, or branched hydrocarbon groups having i to j carbon atoms. This also applies if an "(Cᵢ-Cⱼ)Alkyl" group occurs as a substituent on another group, for example in alkoxy group "O-(C₁-C₆)Alkyl", etc. Preferred (C₁-C₆)Alkyl are selected from the group consisting of methyl, ethyl, propyl, butyl, pentyl or hexyl, the n-isomers of all these groups, isopropyl, isobutyl, 1-methylbutyl, isopentyl, neopentyl, 2,2-dimethylbutyl, 2-methylpentyl, 3-methylpentyl, isohexyl, sec-butyl, tert-butyl and tert-pentyl. Preferred (C₂-C₆)Alkyl are selected from the group consisting of ethyl, propyl, butyl, pentyl or hexyl, the n-isomers of all these groups, isopropyl, isobutyl, 1-methylbutyl, isopentyl, neopentyl, 2,2-dimethylbutyl, 2-methylpentyl, 3-methylpentyl, isohexyl, sec-butyl, tert-butyl and tert-pentyl.
"(C₂-C₆)Alkenyl" as applied herein means any linear, i.e-straight-chain, branched hydrocarbon groups of 2 to 6 carbon atoms having at least one double bond. Preferred (C₂-C₆)Alkenyl are selected from the group consisting of ethenyl (= vinyl), 1-propenyl, 2-propenyl (= allyl), 2-butenyl, 3-butenyl, 2- methyl-2-butenyl, 3-methyl-2-butenyl, 5-hexenyl and 1,3-pentadienyl.
"(C₂-C₆)Alkynyl" as applied herein means any linear, i.e-straight-chain, branched hydrocarbon groups of 2 to 6 carbon atoms having at least one triple bond. Preferred "(C₂-C₆)Alkynyl" are selected from the group consisting of ethynyl, 1-propynyl, 2-propynyl (= propargyl) and 2-butynyl.
"(C₃-C₁₂)Cycloalkyl" as applied herein means hydrocarbon rings containing from three to twelve carbon atoms. These cycloalkyls may be saturated ring systems. This also applies if an "(C₃-C₁₂)Cycloalkyl " group occurs as a substituent on another group, for example in alkoxy group "O-(C₃-C₁₂)Cycloalkyl". Preferred cycloalkyl include but are not limited to cyclopropyl, cyclobutyl, cyclopentyl, cyclohexyl, cycloheptanyl and tetrahydronaphthyl.
"(C₅-C₈)Cycloalkenyl" as applied herein means hydrocarbon ring containing from 5 to 8 carbon atoms having at least one double bond. Prefered "(C₅-C₈)Cycloalkenyl" are selected from the group consisting of cyclopentenyl, cyclohexenyl, cycloheptenyl and cyclooctenyl.
"(C₇-C₁₂)Aralkyl" as applied herein means means any linear or branched hydrocarbon group substituted by phenyl or naphthyl. Preferred (C₇-C₁₂)Aralkyl are benzyl or phenethyl.

The term "Halogen" or "Hal" as applied herein means bromine, chlorine, fluorine or iodine, preferably fluorine and chlorine. The definitions "partially or fully halogenated"; partially or fully fluorinated; "substituted by one or more halogen atoms", includes for example, mono-, di- or trihalo derivatives on one or more carbon atoms. For alkyl, a nonlimiting example would be C(Hal)₃, CF₃ etc.

"(5-11) membered monocyclic or bicyclic unsaturated cycle or heterocycle containing 1-4 heteroatoms selected from N, O and S" means any possible cyclic unsaturated, including aromatic, system containing 1, 2, 3 or 4 heteroatoms selected from N, O and S.

Illustrative (5-11) membered monocyclic or bicyclic unsaturated heterocycle containing 1-4 heteroatoms selected from N, O and S as mentioned in the definition of A in formula (I) are for example those listed hereafter: azaindolyl, benzimidazolyl, benzodioxanyl, benzodioxolyl, benzofuranyl, benzoisothiazolyl, benzoisoxazolyl, benzopyranyl, benzothiazolyl, benzothiophenyl, benzoxazolyl, benzoxazinyl, cinnolinyl, coumarinyl, dihydropyranopyrimidinyl, dioxinopyridinyl, furanopyridinyl, furanyl, imidazolyl, imidazopyridinyl, imidazopyrimidinyl, imidazoquinolinyl, imidazoquinoxalinyl, indanyl, indazolyl, indolinyl, indolyl, isoindolyl, isoquinolinyl, isothiazolidinyl, isothiazolopyridinyl, isothiazolopyrimidinyl, isothiazolyl, isoxazolopyridinyl, isoxazolopyrimidinyl, isoxazolyl, naphtyridinyl, oxadiazabenzocycloheptenyl, oxadiazaacenaphtylenyl, oxadiazolyl, oxazolopyridinyl, oxazolopyrimidinyl, oxazolopyrazinyl, oxazolopyridazinyl, oxazolyl, oxoazabenzocycloheptenyl, oxodiazabenzocycloheptenyl, phtalazinyl, pteridinyl, purinyl, pyranopyridazinyl, pyranopyrazinyl, pyranopyridinyl, pyranopyrimidinyl, pyrazinyl, pyrazolopyrazinyl, pyrazolopyridinyl, pyrazolopyrimidinyl, pyrazolotriazinyl, pyrazolyl, pyridazinyl, pyridinyl, pyrimidinyl, pyridoazepinyl, pyridooxazinyl, pyrrolopyridinyl, pyrrolyl, quinolinyl, quinoxalinyl, tetrahydronaphtyl, tetrazolyl, thiadiazolyl, thiatriazaindenyl, thiazolopyridinyl, thiazolopyrimidinyl, thiazolopyrazinyl, thiazolopyridazinyl, thiazolyl, thienopyridinyl, thienopyrimidinyl, thiophenyl, thiopyranyl, triazolyl, triazinyl, triazolopyridinyl, triazolopyrimidinyl, and, if appropriate, their partially saturated analogs.

Illustrative "(4-7) membered monocyclic saturated or unsaturated heterocycle" are for example those listed hereafter: azepanyl, azetidinyl, beta-lactamyl, diazepanyl, dihydrofuranonyl, dioxanonyl, dioxanyl, dioxolanyl, 1,1-dioxidethiophenyl, furanonyl, furanyl, imidazolidinonyl, imidazolidinyl, imidazolyl, imidazolonyl, isothiazolidinonyl, isothiazolidinyl, isothiazolonyl, isothiazolyl, isoxazolonyl, isoxazolyl, morpholinyl, oxazolidinonyl, oxazolidinyl, oxazolonyl, oxazolyl, oxepanyl, oxetanyl, perhydroazepinyl, piperazinonyl, piperazinyl, piperidinonyl, piperidinyl, pyranonyl, pyranyl, pyrazinyl, pyrazolonyl, pyrazolyl, pyridazinonyl, pyridazinyl, pyridinonyl, pyridinyl, pyrimidinonyl, pyrimidinyl, pyrrolidinedionyl, pyrrolidinonyl, pyrrolidinyl, pyrrolyl, tetrahydrofuranyl, tetrahydropyranonyl, tetrahydropyranyl, thiazolidinonyl, thiazolidinyl, thiazolonyl, thiazolyl, thiophenyl, thiopyranyl, triazolyl, and if appropriate, their partially saturated analogs.

"Polycyclic system representing A with R₁, comprising up to 14 members and up to 5 heteroatoms" or A with two groups R₁ means for example: diazabenzazulenyl, imidazoquinolinyl, imidazoquinoxalinyl, oxadiazabenzazulenyl, oxadiazaacenaphtylenyl, triazabenzazulenyl, and, if appropriate, their partially saturated analogs.

"(4-10) membered monocyclic or bicyclic saturated or unsaturated heterocycle" in the definition of B means for example: azabicyclo[3.2.0]heptanyl, azabicyclo[3.1.0]hexanyl, azabicyclo[3.3.0]octanyl, azepinyl, azetidinyl, azocinyl, bis-pyrrolidinyl, diazocinyl, diazepinyl, dioxanyl, morpholinyl, oxepanyl, oxetanyl, piperazinonyl, piperazinyl, piperidinyl, pyranyl, pyrrolidinyl, tetrahydrofuranyl, thiomorpholinyl, and, if appropriate, their partially unsaturated analogs.

"(4-8) membered monocyclic saturated or unsaturated heterocycle containing 1-3 heteroatoms selected from N, O and S" in the definition of R₁ means for example: oxepanyl, oxetanyl, oxocanyl, pyranyl, furanyl.

"(3-10) membered monocyclic or bicyclic saturated or unsaturated heterocycle" as mentioned in the definition of R₂ and R₃ means for example: azepinyl, azetidinyl, benzimidazolyl, benzofuranyl, benzothiazolyl, benzothiophenyl, benzoxazolyl, cyclobutyl, cyclohexyl, cyclopentyl, cyclopropyl, dioxanyl, furanyl, imidazolyl, indolyl, isoindolyl, isoquinolinyl, isothiazolyl, isoxazolyl, morpholinonyl, morpholinyl, oxazolyl, oxetanyl, piperazinyl, pyranyl, pyrazinyl, pyridazinyl, piperidinonyl, piperidinyl, pyrazinyl, pyrazolyl, pyridinonyl, pyridinyl, pyrimidinyl, pyrrolidinyl, pyrrolyl, quinolinyl, tetrahydrofuranyl, tetrazolyl, thiazolyl, thiomorpholinyl, thiophenyl, triazolyl, and, if appropriate, their partially saturated or unsaturated analogs.

"(4-10) membered saturated or unsaturated monocyclic or bicyclic heterocycle" as formed by R₂ and R₃ together with N means for example azabicyclo[3.2.0]heptanyl, azabicyclo[3.1.0]hexanyl, azabicyclo[3.3.0]octanyl azetidinyl, bis-pyrrolidinyl, morpholinyl, piperazinonyl, piperazinyl, piperidinyl and pyrrolidinyl.

According to a particular embodiment, the invention relates to compounds of general formula (I) as defined above, wherein T is C; V is C or N; Y is C or N; and X is S or CH with the proviso that when X is S then T is C, V is C and Y is C; A is defined as above; B is defined as above.

According to another particular embodiment, the invention relates to compounds of general formula (I) as defined above wherein T is C; V is C; Y is C or N; and X is S or CH with the proviso that when X is S then T is C, V is C and Y is C; A is defined as above; B is defined as above.

Among the compounds of formula (I), compounds of formula (Id) are preferred: 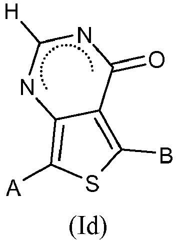 wherein A and B are defined as above.

According to a particular embodiment, the invention relates to compounds of general formula (Id) as defined above, wherein A is defined as above and B is selected from the group consisting of NR₂R₃, (C₂-C₆)Alkyl, (C₂-C₆)Alkenyl, (C₂-C₆)Alkynyl, (C₃-C₁₂)Cycloalkyl, the Alkyl, Alkenyl, Alkynyl and Cycloalkyl groups being possibly substituted by 1 to 3 identical or different substituents selected from the groups consisting of O-Rₐ, NR₂R₃, (C₁-C₆)Alkyl, O-(C₇-C₁₂)Aralkyl, (C₁-C₆)Alkyl-O-Rₐ and (C₁-C₆)Alkyl-NRₐR_{b}; or B is a (4-10) membered monocyclic or bicyclic saturated or unsaturated heterocycle containing 1 to 3 heteroatoms selected from N, O and S, and possibly substituted by 1 to 3 identical or different substituents R₄; with R₂, R₃, R₄, Rₐ and R_{b} being defined as above.

More preferably, the invention relates to compounds of general formula (Id) as defined above, wherein A is defined as above and B is NR₂R₃ or B is a (4-10) membered monocyclic or bicyclic saturated or unsaturated heterocycle containing 1 to 3 heteroatoms selected from N, O and S, and possibly substituted by 1 to 3 identical or different substituents R₄; with R₂, R₃ and R₄ being defined as above.

Among the compounds of formula (I), the compounds of formula (Ie) are preferred: 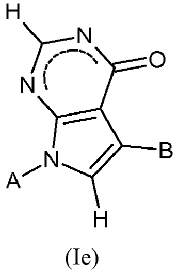 wherein A and B are defined as above.

Among the compounds of formula (I), the compounds of formula (If) are preferred: 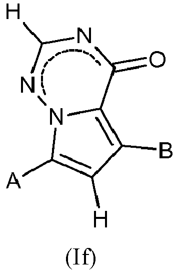 wherein A and B are defined as above.

Among the compounds of formula (I), the compounds of formula (Ig) are preferred: 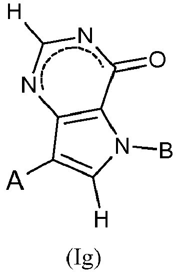 wherein A and B are defined as above.

According to a particular embodiment, the invention relates to compounds of general formula (Ig) as defined above, wherein A is as defined as above and B is selected from the group consisting of SO₂NRₐR_{b}, (C₂-C₆)Alkyl, (C₃-C₁₂)Cycloalkyl; the Alkyl and Cycloalkyl groups being possibly substituted by 1 to 3 identical or different substituents selected from the groups consisting of O-Rₐ, NR₂R₃,(C₁-C₆)Alkyl, O-(C₇-C₁₂)Aralkyl, (C₁-C₆)Alkyl-ORₐ and (C₁-C₆)Alkyl-NRₐR_{b}; or B is a (4-10) membered monocyclic or bicyclic saturated or unsaturated heterocycle containing 1 to 3 heteroatoms selected from N, O and S, and possibly substituted by 1 to 3 identical or different substituents R₄; with R₂, R₃, R₄, Rₐ and R_{b} being defined as above.

According to a particular embodiment, the invention relates to compounds of general formula (I), preferably compounds of formulae (Id), (Ie), (If) and (Ig), as defined above, wherein A is selected from the group consisting of phenyl and (5-11) membered monocyclic or bicyclic unsaturated cycle or heterocycle linked by a carbon atom and containing 1-4 heteroatoms selected from N, O and S, all the above members of the group representing A being possibly substituted on carbon or on nitrogen atoms by 1-4 identical or different groups R₁, with R₁ as defined above.

Among the compounds of formula (I) of the invention, preferably compounds of formula (Id), (Ie), (If) and (Ig), there may be cited the following compounds and their salts, in particular hydrochlorides:
- 7-(1-Cyclohexyl-1H-benzoimidazol-5-yl)-5-((S)-3-hydroxymethyl-pyrrolidin-1-yl)-3H-thieno[3,4-d]pyrimidin-4-one,
- 5-(3,3-Bis-hydroxymethyl-pyrrolidin-1-yl)-7-(1-cyclohexyl-1H-benzoimidazol-5-yl)-3H-thieno[3,4-d]pyrimidin-4-one,
- 7-(1-Cyclohexyl-1H-benzoimidazol-5-yl)-5-[(3-dimethylamino-propyl)-methyl-amino]-3H-thieno[3,4-d]pyrimidin-4-one,
- 7-(1-Cyclohexyl-1H-benzoimidazol-5-yl)-5-(hexahydro-pyrrolo[3,4-c]pyrrol-2-yl)-3H-thieno[3,4-d]pyrimidin-4-one hydrochloride,
- 7-(1-cyclohexyl-1H-benzoimidazol-5-yl)-5-piperazin-1-yl-3H-thieno[3,4-d]pyrimidin-4-one hydrochloride,
- 7-(1-cyclohexyl-1H-benzoimidazol-5-yl)-5-hydroxymethyl-hexahydro-pyrrolo[3,4-c]pyrrol-2-yl)-3H-thieno[3,4-d]pyrimidin-4-one hydrochloride,
- 5-((*3R,4R*)-3,4-Bis-hydroxymethyl-pyrrolidin-1-yl)-7-(1-cyclohexyl-1*H*-benzoimidazol-5-yl)-3*H-*thieno[3,4-d]pyrimidin-4-one,
- 7-(1-cyclohexyl-1H-benzoimidazol-5-yl)-5-(1,2,3,6-tetrahydro-pyridin-4-yl)-3H-thieno[3,4-d]pyrimidin-4-one hydrochloride,
- 7-(1-cyclohexyl-1H-benzoimidazol-5-yl)-5-(1,2,3,6-tetrahydro-pyridin-4-yl)-3H-thieno[3,4-d]pyrimidin-4-one hydrochloride,
- 7-(1-ethyl-1H-benzoimidazol-5-yl)-5-((S)-3-hydroxymethyl-pyrrolidin-1-yl)-3H-thieno[3,4-d]pyrimidin-4-one,
- 5-((6)-3-hydroxymethyl-pyrrolidin-1-yl)-7-[1-(2-methoxy-ethyl)-1H-benzoimidazol-5-yl]-3H-thieno[3,4-d]pyrimidin-4-one,
- 7-[1-((*1R,2R*)-2-fluoro-cyclohexyl)-1H-benzoimidazol-5-yl]-5-((*S*)-3-hydroxymethyl-pyrrolidin-1-yl)-3H-thieno[3,4-d]pyrimidin-4-one,
- 5-((*S*)-3-Hydroxymethyl-pyrrolidin-1-yl)-7-[1-(*trans*-3-ethyl-cyclohexyl)-1H-benzoimidazol-5-yl]-3H-thieno[3,4-d]pyrimidin-4-one,
- 5-((*S*)-3-hydroxymethyl-pyrrolidin-1-yl)-7-[1-(tetrahydro-pyran-3-yl)-1-H-benzoimidazol-5-yl]-3H-thieno[3,4-d]pyrimidin-4-one,
- 7-(1-Cyclohexyl-1H-benzoimidazol-5-yl)-5-(3-hydroxy-propyl)-3,7-dihydro-pyrrolo[2,3-d]pyrimidin-4-one,
- 7-(1-cyclohexyl-1*H*-benzoimidazol-5-yl)-5-(1,2,3,6-tetrahydro-pyridin-4-yl)-3,7-dihydro-pyrrolo[2,3-d]pyrimidin-4-one hydrochloride,
- 7-(1-cyclohexyl-1*H*-benzoimidazol-5-yl)-5-(1-methyl-1,2,3,6-tetrahydro-pyridin-4-yl)-3,7-dihydro-pyrrolo[2,3-d]pyrimidin-4-one,
- 7-(1-Cyclohexyl-1H-benzoimidazol-5-yl)-5-((S)-3-hydroxymethyl-pyrrolidin-1-yl)-3,7-dihydro-pyrrolo[2,3-d]pyrimidin-4-one,
- 7-(1-Cyclohexyl-1H-benzoimidazol-5-yl)-5-(3-hydroxymethyl-cyclopentyl)-3,7-dihydro-pyrrolo[2,3-d]pyrimidin-4-one,
- 7-(1-Cyclohexyl-1H-benzoimidazol-5-yl)-5-((S)-3-hydroxymethyl-pyrrolidin-1-yl)-3H-pyrrolo[2,1-f] [1,2,4]triazin-4-one,
- 7-(1-Cyclohexyl-1H-benzimidazol-5-yl)-5-(3-hydroxy-prop-1-ynyl)-3H-pyrrolo[2,1-f] [1,2,4]triazin-4-one,
- 7-(1-Cyclohexyl-1H-benzimidazol-5-yl)-5-(3-hydroxy-propyl)-3,5-dihydro-pyrrolo[3,2-d]pyrimidin-4-one,
- 7-(1-Cyclohexyl-1H-benzimidazol-5-yl)-5-(5-hydroxy-pentyl)-3,5-dihydro-pyrrolo[3,2-d]pyrimidin-4-one,
- 7-(1-Cyclohexyl-1H-benzimidazol-5-yl)-5-(1-methyl-piperidin-4-yl)-3,5-dihydro-pyrrolo[3,2-d]pyrimidin-4-one,
- 7-(1-Cyclohexyl-1H-benzimidazol-5-yl)-5-piperidin-4-yl-3,5-dihydro-pyrrolo[3,2-d]pyrimidin-4-one,
- 7-(1-Cyclohexyl-1H-benzimidazol-5-yl)-5-[*cis*-(4-hydroxymethyl-cyclohexyl)]-3,5-dihydro-pyrrolo[3,2-d]pyrimidin-4-one,
- 7-(1-Cyclohexyl-1H-benzimidazol-5-yl)-5-(S)-piperidin-3-yl-3,5-dihydro-pyrrolo[3,2-d]pyrimidin-4-one,
- 7-(1-Cyclohexyl-1H-benzimidazol-5-yl)-5-(3-dimethylamino-propyl)-3,5-dihydro-pyrrolo[3,2-d]pyrimidin-4-one,
- 7-(1-Cyclohexyl-1H-benzimidazol-5-yl)-5-(tetrahydro-pyran-4-yl)-3,5-dihydro-pyrrolo[3,2-d]pyrimidin-4-one,
- 7-(1-Cyclohexyl-1H-benzimidazol-5-yl)-5-(1-isopropyl-piperidin-4-yl)-3,5-dihydro-pyrrolo[3,2-d]pyrimidin-4-one,

As indicated above, the compounds of formula (I), as defined above, are active on DltA and related targets which makes them useful as drugs.

In particular, the compounds of formula (Id), (Ie), (If) and (Ig), as defined above, are active on DltA and related targets which makes them useful as drugs.

Another object of the invention is the use of the compounds of formula (I), as defined above, as drugs for preventing or treating human or animal infections by microbial pathogens. In one embodiment of the invention, an object of the invention is the use of the compounds of formula (Id), (Ie), (If) and (Ig), as defined above, as drugs for preventing or treating human or animal infections by microbial pathogens.

The drugs of the invention are useful as antibacterial agents for the prevention and therapeutical treatment of severe infections due to microbial pathogens, in particular Gram-positive bacteria growing under aerobic or anaerobic conditions. Such drugs are useful against bacteria of the genus *Staphylococcus*, more specifically *S. aureus* and coagulase-negative staphylococci like *S. epidermidis* and *S. saprophyticus* (including multiresistant strains such as methicillin-resistant staphylococci, vancomycin intermediate and vancomycin resistant *Staphylococcus aureus*), *Bacillus* (including *B. anthracis* and *B. cereus*), *Listeria* (including *L. monocytogenes*), *Enterococcus* (including *E. faecalis* and *E. faecium* including vancomycin resistant isolates), *Streptococcus* (including *S. pneumonia, S. agalactiae, S. pyogenes,* and *streptococci* of the viridans group) and, *Corynebacterium* (including *C. diphtheriae, C. amicolatum, C. striatum, C. jeikeium, C. urealyticum,* and *C. xerosis*).

The drugs according to the invention are also for use in preventing and treating human or animal bacterial infections, in association with one or more drug(s) and more specifically with one or more antibacterial agent(s) or with one or more antivirulence agent(s) or with one or more drug(s) reinforcing the host innate immunity and this use of the compounds of formula (I) in association constitutes another object of the invention.

The invention relates in particular to the use of the compounds of formula (I) as defined above, preferably the use of compounds of formula (Id), (Ie), (If) and (Ig) as defined above, in preventing or treating human or animal bacterial infections in association with one or more antibacterial agent(s) targeting the bacterial cell wall and/or membrane.

The invention relates in particular to the use of the compounds of formula (I) as defined above, preferably the use of compounds of formula (Id), (Ie), (If) and (Ig) as defined above, in preventing or treating human or animal bacterial infections, in association with one or more antibacterial agent(s) of the CAMP type such as, as non-limiting examples, polymyxins, cathelicidins, defensins or any synthethic or natural peptide derived from the above listed ones.

The invention relates in particular to the use of the compounds of formula (I) as defined above, preferably the use of compounds of formula (Id), (Ie), (If) and (Ig) as defined above, in preventing or treating human or animal bacterial infections, in association with one or more antibacterial agent(s) of the glycopeptides type such as, as non-limiting examples, Vancomycin or Teicoplanin.

The invention relates in particular to the use of the compounds, of formula (I) as defined above, preferably the use of compounds of formula (Id), (Ie), (If) and (Ig) as defined above, in preventing or treating human or animal bacterial infections, in association with one or more antibacterial agent(s) of the lipopeptides type such as, as non-limiting examples, Daptomycin.

The invention relates in particular to the use of the compounds, of formula (I) as defined above, preferably the use of compounds of formula (Id), (Ie), (If) and (Ig) as defined above, in preventing or treating human or animal bacterial infections, in association with one or more drug(s) reinforcing the host innate immunity such as, as non-limiting examples, immunomodulatory peptides.

The invention relates in particular to the use of the compounds, of formula (I), as defined above, preferably the use of compounds of formula (Id), (Ie), (If) and (Ig) as defined above, in preventing or treating human or animal bacterial infections, in association with GM-CSF (Granulocyte Macrophage Colony-Stimulating Factor), an approved white blood cell growth factor.

A further object of the invention is therefore the pharmaceutical mixtures or associations of the compounds of formula (I) as defined above, preferably of the compounds of formula (Id), (Ie), (If) and (Ig) as defined above, with the above defined antibacterials and/or antivirulence agents and/or drugs reinforcing the host innate immunity.

The invention also relates to pharmaceutical compositions comprising, as active principle, a therapeutically effective amount of at least one compound of formula (I) such as above defined, preferably of the compounds of formula (Id), (Ie), (If) and (Ig) as defined above, as well as pharmaceutical compositions comprising , as active principles, a therapeutically effective amount of at least one compound of formula (I) as defined above, preferably of the compounds of formula (Id), (Ie), (If) and (Ig) as defined above, in association with at least another therapeutically active compound defined above. In the composition of the invention, the active principles are in association with a pharmaceutically acceptable carrier.

Said pharmaceutical compositions are advantageously formulated to be administered under oral, topical, transdermal, sublingual, rectal, parenteral including intravenous, intramuscular, intraperitoneal and subcutaneous routes, with individual doses appropriate for the patient to be treated. The injectable routes are preferred.

The compositions according to the invention can be solid, liquid including solutions, emulsions or suspensions, or in the form of a gel/cream and be presented in the pharmaceutical forms commonly used in human medicine, such as for example, plain or sugar-coated tablets, gelatin capsules, granules, suppositories, injectable preparations, ointments, creams, gels; they are prepared according to the customary methods. The active ingredient/s can be incorporated using excipients which are customarily used in these pharmaceutical compositions, such as talc, gum arabic, lactose, starch, magnesium stearate, aqueous or non-aqueous vehicles, fatty substances of animal or vegetable origin, paraffin derivatives, glycols, various wetting agents, dispersants or emulsifiers, preservatives. These compositions can in particular be presented in the form of a powder intended to be dissolved or suspended extemporaneously in an appropriate vehicle, for example, non-pyrogenic sterile water.

The dose of the compound of formula (I) as defined above, preferably the dose of the compounds of formula (Id), (Ie), (If) and (Ig) as defined above, administered varies according to the condition treated, the patient in question, the administration route and the compound envisaged. It can, for example, be comprised between 0.01 g and 10 g per day in humans. When the compound is administered in association, the dose of the associated active principle is the dose normally prescribed for such compound. For example, the compound of formula (I) as defined above, preferably the compounds of formula (Id), (Ie), (If) and (Ig) as defined above, can be administered in association with vancomycin at doses of 0.5 to 3g per day in human (intravenous administration) or colistin at doses of 0.1 to 0.3g per day in human (intravenous administration).

### Preparation of the molecules

The compounds of formula (I) and their salts may be prepared by processes illustrated hereafter in the experimental part and more generally by processes known to the skilled chemist to be applicable for preparing chemically related compounds. Such processes use known starting materials or intermediates which may be obtained by standard procedures of organic chemistry. The following processes provide a variety of non-limiting routes for the production of the compounds of formula (I) and their intermediates. These processes constitute further features of the present invention.

In the text herein below, the term "leaving group" means a group that can be readily cleaved from a molecule by breaking a heterolytic bond, with loss of an electron pair. This group may thus be readily replaced by another group during a substitution reaction, for example.

In the text herein below, the term "protecting group" (PG) means a group that can be momentarily incorporated into a chemical structure for the purpose of temporarily inactivating a part of the molecule during a reaction, and which may be readily removed in a subsequent synthetic step.

According to the invention, when T is C, V is C, Y is C, X is S, compounds of the formula (I) are represented by the formula (Id). According to this embodiment, compounds of formula (Id) may be prepared by a reaction of cyclization of compounds of formula (II) according to the following scheme 1: 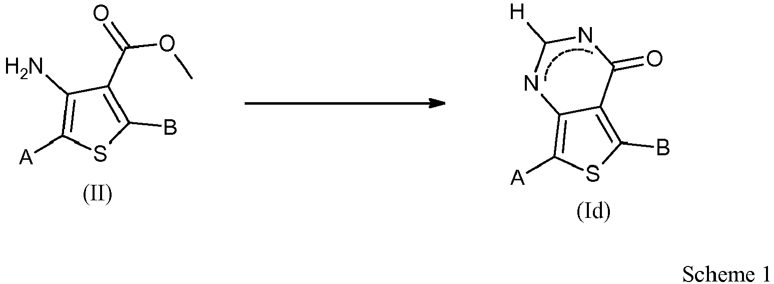 wherein
- A is defined above; and
- B is defined as above;
in the presence of suitable cyclization conditions known to the skilled in the art, non-limiting examples comprising the use of a solvent such as ethanol and a cyclization reactant such as formamidine acetate.

Compounds of formula (II), as defined above, may be prepared by a reaction of reduction of compounds of formula (III) according to the following scheme 2: 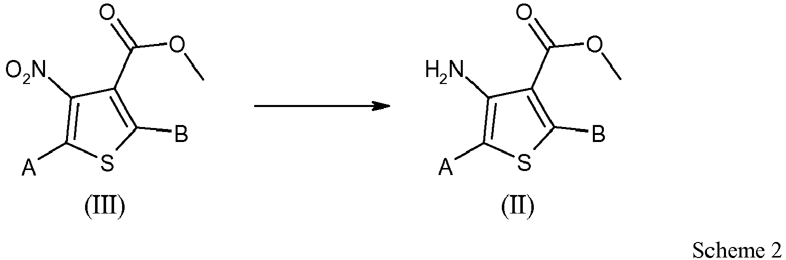 wherein;
- A is defined above; and
- B is defined as above,
in the presence of suitable reduction conditions known to the skilled in the art, non-limiting examples comprising the use of a solvent such as methanol, ethanol, ethyl acetate, tetrahydrofuran, a catalyst such as Palladium or Platinum and a reducing agent as hydrogen gas.

Compounds of formula (III), as defined above, may be prepared by reacting compounds of formula (V) with a compound of formula (IV) according to the following scheme 3: 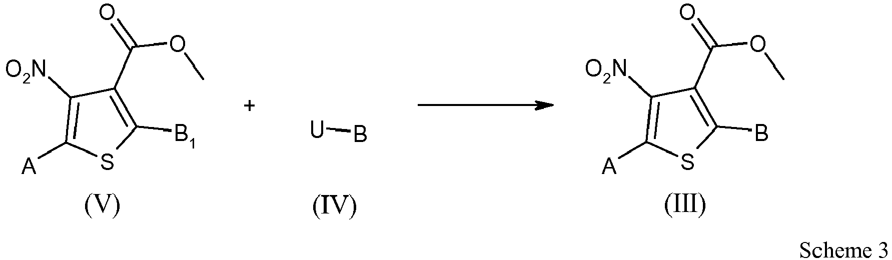 wherein:
- A is defined above; and
- B is defined as above,
- B₁ represents a suitable leaving group; preferably selected from the group consisting of halogen, triflate, boron and a substituted boron, and
- U is selected from the group consisting of an hydrogen atom, a halogen atom, triflate, boron and substituted boron;
in the presence of suitable aromatic nucleophilic substitution conditions known to the skilled in the art, non-limiting examples comprising the use of a base such as triethylamine, diisopropylethylamine, 1,8-diazabicyclo[5.4.0]undec-7-ene, sodium, potassium or cesium carbonate or hydroxide, in a suitable solvant such as tetrahydrofuran, acetonitrile, dimethylformamide, methanol, ethanol, propanol, butanol, 1,4-dioxane or dimethylsulfoxide,
or in the presence of suitable transition-metal catalyzed coupling conditions known to the skilled in the art, non-limiting examples comprising the use of palladium catalyst such as tris(dibenzylideneacetone)dipalladium, Palladium(II) acetate, Tetrakis(triphenylphosphine)palladium, or Dichloro[1,1'-bis(diphenylphosphino)ferrocene]palladium, optionnaly an appropriate ligand such as Xantphos or tricyclohexylphosphine and an appropriate base such as sodium or potassium carbonate in an appropriate solvent such as 1,4-dioxane, tetrahydrofuran, dimethylformamide or toluene.

Compounds of formula (V), as defined above, may be prepared by reacting compounds of formula (VI) with a compound of formula (VII) according to the following scheme 4: 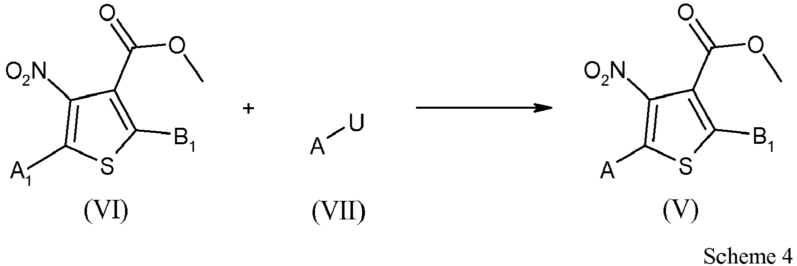 wherein
- A is defined as above;
- B₁ is defined above;
- A₁ represents a suitable leaving group; preferably selected from the group consisting of halogen, triflate, boron and a substituted boron,
- U is defined above;
in the presence of suitable transition-metal catalyzed coupling conditions known to the skilled in the art, non-limiting examples comprising the use of palladium catalyst such as tris(dibenzylideneacetone)dipalladium, Palladium(II) acetate, Tetrakis(triphenylphosphine)palladium, or Dichloro[1,1'-bis(diphenylphosphino)ferrocene]palladium, optionnaly an appropriate ligand such as Xantphos, or tricyclohexylphosphine and an appropriate base such as sodium or potassium carbonate in an appropriate solvent such as 1,4-dioxane, tetrahydrofuran, dimethylformamide or toluene.

According to the invention, when T is C, V is N, Y is C, X is C, compounds of the formula (I) are represented by the formula (Ie). According to this embodiment, compounds of formula (Ie) may be prepared by a reaction of deprotection of compounds of formula (VIII) according to the following scheme 5: 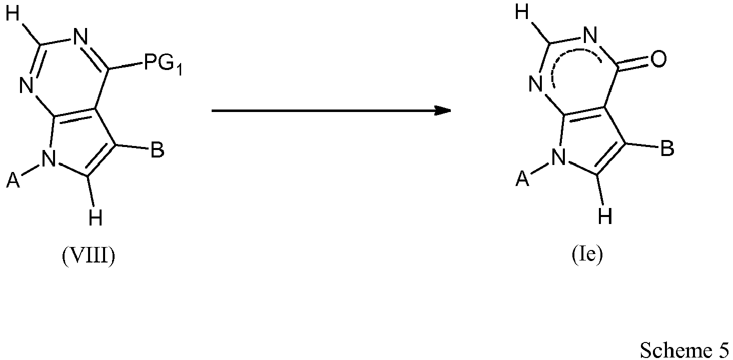 wherein:
- A is defined above;
- B is defined as above;
- PG₁ is a protecting group, preferably selected from the group consisting of O-Benzyle, O-*p*-methoxybenzyle and tetrahydropyranyle;
in the presence of suitable hydrogenolysis conditions known to the skilled in the art, non-limiting examples comprising the use of Palladium catalyst such as Palladium hydroxide or Palladium on charcoal and a reducing agent as hydrogen gas,
or in the presence of suitable hydrolysis conditions known to the skilled in the art, non-limiting examples comprising the use of a strong acid such as hydrochloric acid or trifluoroacetic acid.

Compounds of formula (VIII), as defined above, may be prepared by reacting compounds of formula (IX) with compound of formula (IV) according to the following scheme 6: 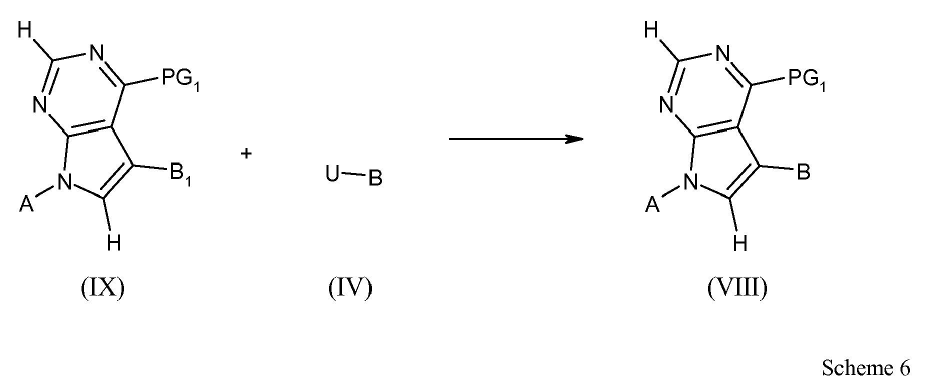 wherein
- A is defined above;
- B is defined as above;
- U is defined as above; and
- B₁ represents a suitable leaving group, preferably selected from the group consisting of halogen, triflate, boron and substituted boron;
in the presence of suitable transition-metal catalyzed coupling conditions known to the skilled in the art, non limiting examples comprising the use of palladium catalyst such as tris(dibenzylideneacetone)dipalladium, Palladium(II) acetate, Tetrakis(triphenylphosphine)palladium, or Dichloro[1,1'-bis(diphenylphosphino)ferrocene]palladium or copper catalyst such as copper iodide or copper acetate, optionnaly an appropriate ligand such as Xantphos, tricyclohexylphosphine or L-proline and an appropriate base such as sodium or potassium carbonate in an appropriate solvent such as 1,4-dioxane, tetrahydrofuran, dimethylformamide, dimethylsulfoxide or toluene.

Compounds of formula (IX), as defined above, may be prepared by reacting compounds of formula (X) with compound of formula (IV) according to the following scheme 7: 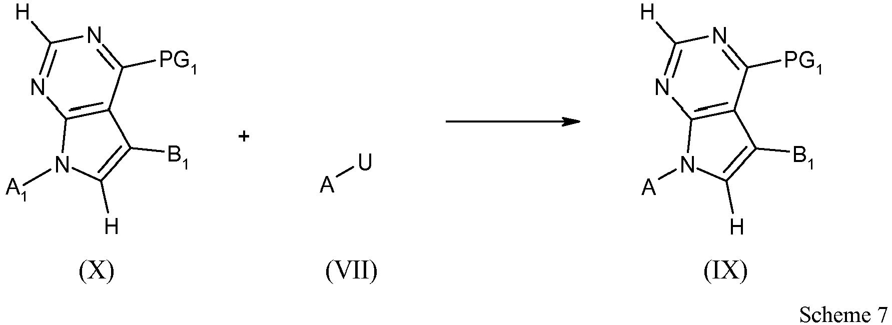 wherein
- PG₁ is defined above;
- B₁ is defined above;
- A₁ is defined above;
- A is defined above; and
- U is defined above;
in the presence of suitable transition-metal catalyzed coupling conditions known to the skilled in the art, non-limiting examples comprising the use of copper catalyst such as copper iodide or copper acetate and an appropriate base such as dimethylethylenediamine in an appropriate solvent such as 1,4-dioxane, tetrahydrofuran, dimethylformamide or toluene.

According to the invention, when T is N, V is C, Y is C, X is C, compounds of the formula (I) are represented by the formula (If). According to this embodiment, compounds of formula (If) may be prepared by reacting compounds of formula (XI) with compounds of formula (IV) according to the following scheme 8: 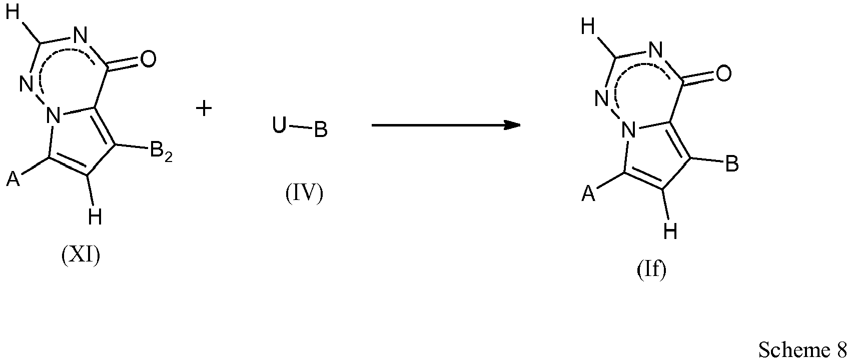 wherein
- A is defined above;
- B₂ is halogen;
- B is defined above; and
- U is defined above;
in the presence of suitable transition-metal catalyzed coupling conditions known to the skilled in the art, non-limiting examples comprising the use of palladium catalyst such as tris(dibenzylideneacetone)dipalladium, Palladium(II) acetate, Tetrakis(triphenylphosphine)palladium, or Dichloro[1,1'-bis(diphenylphosphino)ferrocene]palladium or copper catalyst such as copper iodide or copper acetate, optionnaly an appropriate ligand such as Xantphos, tricyclohexylphosphine or L-proline and an appropriate base such as sodium or potassium carbonate or triethylamine in an appropriate solvent such as 1,4-dioxane, tetrahydrofuran, dimethylformamide, dimethylsulfoxide or toluene.

Compounds of formula (XI), as defined above, may be prepared by a reaction of halogenations of compounds of formula (XII) according to the following scheme 9: 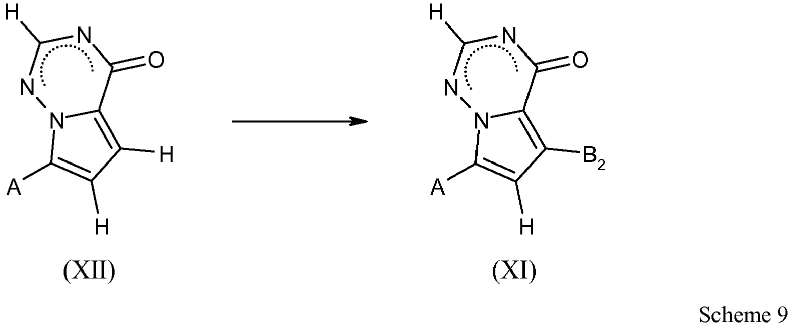 wherein
- A is defined above; and
- B₂ is halogen;
in the presence of suitable halogenations conditions known to the skilled in the art, non-limiting examples comprising the use of halogenating reagent such as N-chlorosuccinimide, N-bromosuccinimide or N-iodosuccinimide in an appropriate solvent such as dichloromethane, chloroform or dimethylformamide.

Compounds of formula (XII), as defined above, may be prepared by reacting compounds of formula (XIII) with compounds of formula (VII) according to the following scheme 10: 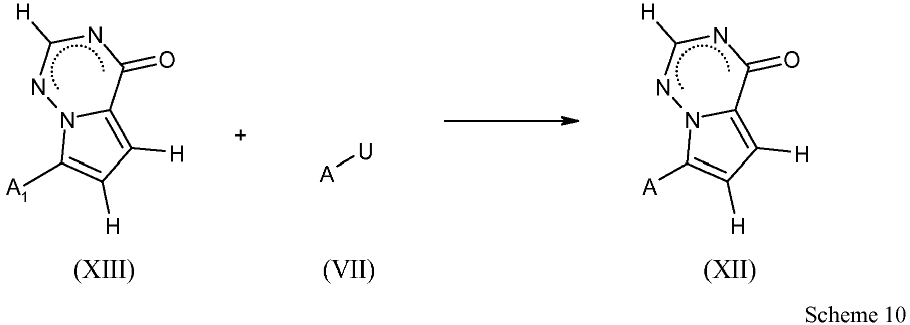 wherein
- A is defined above;
- A₁ is defined above ; and
- U is defined as above;
in the presence of suitable transition-metal catalyzed coupling conditions known to the skilled in the art, non-limiting examples comprising the use of palladium catalyst such as tris(dibenzylideneacetone)dipalladium, Palladium(II) acetate, Tetrakis(triphenylphosphine)palladium, Dichlorobis(di-tert-butylphenylphosphine)palladium (II) or Dichloro[1,1'-bis(diphenylphosphino)ferrocene]palladium, optionnaly an appropriate ligand such as Xantphos or tricyclohexylphosphine and an appropriate base such as sodium or potassium carbonate or potassium phosphate tribasic in an appropriate solvent such as 1,4-dioxane, tetrahydrofuran, dimethylformamide, dimethylsulfoxide or toluene.

According to the invention, when T is C, V is C, Y is N, X is C, compounds of the formula (I) are represented by the formula (Ig). According to this embodiment, compounds of formula (Ig) may be prepared by a reaction of deprotection of compounds of formula (XIV) according to the following scheme 11: 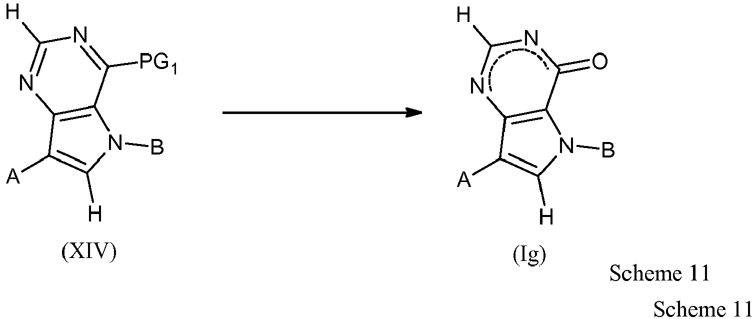 wherein
- A is defined above;
- B is defined above; and
- PG₁ is defined above;
in the presence of suitable hydrogenolysis conditions known to the skilled in the art, non-limiting examples comprising the use of Palladium catalyst such as Palladium on charcoal in appropriate solvent such as methanol or dichloromethane and a reducing agent as hydrogen gas,
or in the presence of suitable hydrolysis conditions known to the skilled in the art, non-limiting examples comprising the use of a strong acid such as hydrochloric acid or trifluoroacetic acid.

Compounds of formula (XIV), as defined above, may be prepared by reacting compounds of formula (XV) with a compound of formula (IV) according to the following scheme 12: 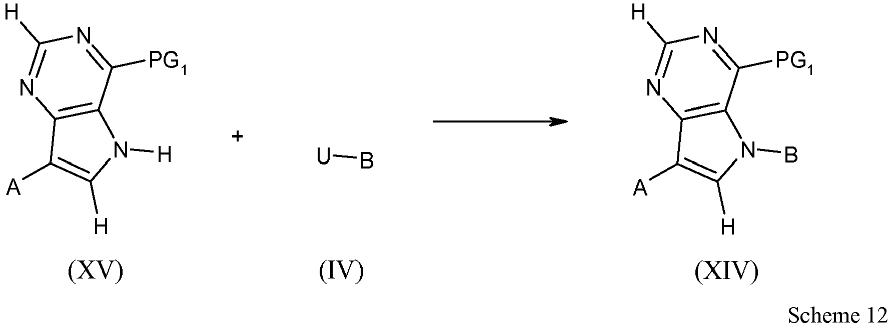 wherein
- A is defined above;
- B is defined above;
- PG₁ is defined above; and
- U is defined as above;
in the presence of suitable nucleophilic substitution conditions known to the skilled in the art, non-limiting examples comprising the use of a base such as sodium hydride in appropriate solvent such as tetrahydrofuran or dimethylformamide,
or in the presence of suitable Michael conditions known to the skilled in the art, non-limiting examples comprising the use of a base such as triethylamine or cesium carbonate in appropriate solvent such as tetrahydrofuran or dimethylformamide,
or in the presence of suitable Mitsunobu conditions known to the skilled in the art, non-limiting examples comprising the use of Mitsunobu reagents such as ditertbutylazodicarboxylate and triphenylphosphine,
or in the presence of suitable transition-metal catalyzed coupling conditions known to the skilled in the art, non-limiting examples comprising the use of palladium catalyst such as Tetrakis(triphenylphosphine)palladium or copper catalyst such as copper acetate and an appropriate base such as sodium or potassium carbonate or triethylamine in an appropriate solvent such as 1,4-dioxane, tetrahydrofuran, dimethylformamide or toluene.

Compounds of formula (XV), as defined above, may be prepared by a reaction of deprotection of compounds of formula (XVI) according to the following scheme 13: 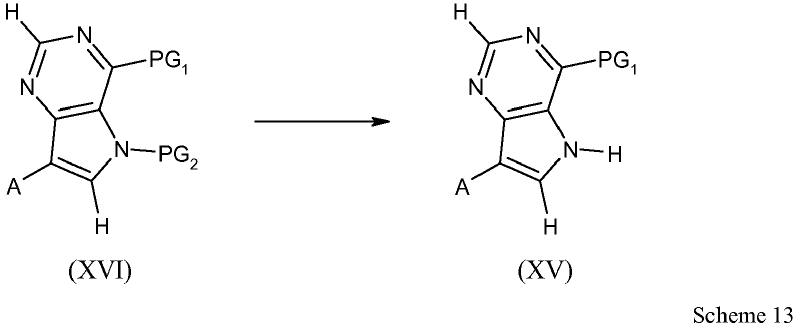 wherein
- A is defined as above;
- PG₁ is defined above and
- PG₂ is a protecting group, preferably selected from the group consisting of S(O)₂NMe₂, tert-butylcarboxylate and benzylcarboxylate;
in the presence of suitable deprotection conditions known to the skilled in the art, non-limiting examples comprising the use of deprotective reagent such as tetrabutylammonium fluoride or hydrochloric acid in an appropriate solvent such as tetrahydrofuran.

Compounds of formula (XVI), as defined above, may be prepared by reacting compounds of formula (XVII) with compounds of formula (VII) according to the following scheme 14: 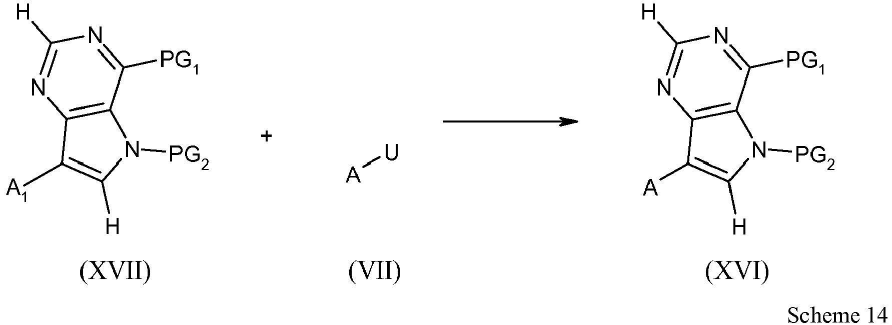 wherein
- A is defined as above;
- A₁ is defined as above;
- PG₁ is defined above ;
- PG₂ is defined as above;
- U is defined as above
in the presence of suitable transition-metal catalyzed coupling conditions known to the skilled in the art, non-limiting examples comprising the use of palladium catalyst such as Bis(triphenylphosphine) palladium(II) dichloride and an appropriate base such as sodium or potassium carbonate in an appropriate solvent such as 1,4-dioxane, tetrahydrofuran, dimethylformamide or toluene.

If appropriate, the reactions previously are followed or preceded by one or more reactions known to the skilled of the art and are performed in an appropriate order to achieve the requisite substitutions on A or/and B as defined above to afford other compounds of formula (I), in particular to afford other compounds of formula (Id), (Ie), (If) and (Ig). Such reactions are described hereafter in the experimental part. Non-limiting examples of such reactions include:
protection of reactive functions,
deprotection of reactive functions,
condensation reactions,
carbon chain elongation such as Wittig, Grignard, Petasis, Tebbe, Peterson, base-promoted substitution reactions,
halogenation,
dehalogenation,
dealkylation,
dihydroxylation,
epoxydation,
alkylation of amine, aniline, alcohol and phenol,
Mitsunobu reaction on hydroxyl groups,
Cyclization and cycloaddition reactions on appropriate groups,
reduction of nitro, esters, cyano, aldehydes, carboxylic acids, amides or unsaturated bonds, transition metal-catalyzed coupling reactions,
acylation,
sulfonylation/introduction of sulfonyl groups,
nitration/introduction of nitro groups,
saponification/hydrolysis of esters groups,
amidification or transesterification of ester groups,
amidification or esterification of carboxylic groups,
halogen exchange,
nucleophilic substitution with amine, thiol or alcohol,
reductive amination,
oxime formation on carbonyl and hydroxylamine groups,
oxidation reactions on appropriate groups,
salification.

All these reactions and the conditions for performing them are classical and well known to the skilled chemist. General and more specific references can be cited, including, as the general references, Michael B. Smith, Jerry March, March's Advanced Organic Chemistry: Reactions, Mechanisms, and Structure, 6th Edition, Wiley, 2007; Science of Synthesis: Houben-Weyl Methods of Molecular Transformations, Thieme, 2010 and as more specific references illustrating conditions for the above reactions, in particular those listed hereafter:
Protection and deprotection of reactive functions: Peter G. M. Wuts, Theodora W. Greene, Greene's Protective Groups in Organic Synthesis, 4th Edition, Wiley, 2006);
Condensation reactions: Condensations leading to double bonds, in The Chemistry of Carbonyl Group: Volume 1 (1966) (ed S. Patai), Interscience Publishers; J. Alvarez-Builla, J. Jose Vaquero, J. Barluenga, Modern Heterocyclic Chemistry, Wiley, 2011;
Carbon chain elongation such as Wittig, Grignard, Petasis, Tebbe, Peterson, base-promoted substitution reactions: Li, J. J. (ed) Name Reactions for Homologations, John Wiley & Sons, Inc., 2009;
Halogenation reactions: Sasson, Y. Formation of Carbon-Halogen Bonds (Cl, Br, I). In PATAI'S Chemistry of Functional Groups, John Wiley & Sons, Ltd: 2009.

Epoxide formation and opening: Crotti, P. and Pineschi, M. (2006) Epoxides in Complex Molecule Synthesis, in Aziridines and Epoxides in Organic Synthesis (ed A. K. Yudin), Wiley-VCH Verlag GmbH & Co. KGaA, Weinheim, FRG ; Rosowsky, A. (2008) Ethylene Oxides, in Chemistry of Heterocyclic Compounds: Heterocyclic Compounds with Three- and Four-Membered Rings, Volume 19 (ed A. Weissberger), John Wiley & Sons, Inc., Hoboken, NJ, USA

Dihydroxylation reaction of carbon-carbon double bonds: Noe, M. C., Letavic, M. A., Snow, S. L. 2005. Asymmetric dihydroxylation of alkenes: Organic Reactions. 109-625;
Transition metal-catalyzed reactions: Matthias Beller, Carsten Bolm, Transition Metals for Organic Synthesis, Wiley, 2004; Chem. Rev. 2002, 102, 1359 or Tetrahedron 2005, 61, 2245;
Cyclization and cycloaddition reactions on appropriate groups: Cycloaddition Reactions in Organic Synthesis (eds S. Kobayashi and K. A. Jorgensen), Wiley-VCH Verlag GmbH, Weinheim, FRG, 2001.

The conditions for performing the above various reactions are well known to the skilled in the art and several examples are illustrated below in the experimental part.

### Experimental part

This part represents the preparation of the examples (intermediates and final compounds). The following examples are provided for the purpose of illustrating the present invention and by no means should be interpreted to limit the scope of the present invention.

### Materials and procedures

Proton nuclear magnetic resonance (¹H NMR) spectra were recorded on either a 300 or 400 MHz Brüker instrument, and chemical shifts are reported in parts per million downfield from the internal standard tetramethylsilane (TMS). Abbreviations for NMR data are as follows: s = singlet, d = doublet, t = triplet, q = quartet, quin = quintet, sex = sextet, sep = septet, m = multiplet, dd = doublet doublet, dt = doublet of triplet, td = triplet of doublet, tt = triplet of triplet, ddd = doublet of doublet of doublet, bs = broad singlet, bt = broad triplet. J indicates the NMR coupling constant measured in Hertz. CDCl₃ is deuteriochloroform, DMSO-d₆ is hexadeuteriodimethylsulfoxide, and CD₃OD is tetradeuteriomethanol. Mass spectra were obtained using electrospray ionization (ESI) techniques on an Agilent 1100 Series LCMS and Waters Acquity UPLC-MS. In the context of mass spectrometry, M refers to the molecular weight, MS refers to mass spectrometer. Analtech Silica Gel GF and E. Merck Silica Gel 60 F-254 thin layer plates were used for thin layer chromatography. Flash chromatography was carried out on Flashsmart Pack cartridge irregular silica 40-60µm or spherical silica 20-40µm. Preparative thin layer chromatography was carried out on Analtech Silica Gel GF 1000 µm 20x20 cm.

### Abbreviations

Ac refers to acetyl, ACN or CH₃CN refers to acetonitrile, AIBN refers to 2,2'-Azobis(2-methylpropionitrile), Bn refers to benzyl, Boc refers to tert-butylcarboxylate, *n*-BuOH refers to *n-*Butanol, *n*-BuLi refers to *n*-butyl lithium, BTEAC refers to benzyltriethylammonium chloride, Bu refers to butyl, CDI refers to 1,1'-carbonyldiimidazole, Cbz refers to benzyl carboxylate, DBU refers to 1,8-diazabicyclo[5.4.0]undec-7-ene, DCM refers to dichloromethane, DIAD refers to diisopropyl azodicarboxylate, DIBAL-H refers to diisobutylaluminum hydride, DIPA refers to *N,N-*diisopropylamine, DIPEA refers to *N,N*-diisopropylethylamine, DMAP refers to 4-(dimethylamino)pyridine, DME refers to 1,2-dimethoxyethane, DMEDA refers to N,N'-dimethylethylenediamine, DMF refers to *N,N*-dimethylformamide, DMSO refers to dimethylsulfoxide, DTAD refers to ditertbutyl azodicarboxylate, dppf refers to 1,1'-bis(diphenylphosphino)ferrocene, EDC, EDCI or EDAC refers *N*-(3-dimethylaminopropyl)-*N'*-ethylcarbodiimide hydrochloride, Et refers to ethyl, Et₂O refers to diethyl ether, EtOH refers to ethanol, LDA refers to lithium diisopropylamide, EtOAc refers to ethyl acetate, HATU refers to O-(7-azabenzotriazol-1-yl)-N,N,N',N'-tetramethyluronium hexafluorophosphate, HOBt refers to 1-hydroxybenzotriazole, Me refers to methyl, MeOH refers to methanol, MePhos refers to 2-dicyclohexylphosphino-2'-methylbiphenyl, NBS refers to N-bromosuccinimide, NMO refers to N-methylmorpholine N-oxide, OBn refers to benzyloxy, Pd(PPh₃)₄ refers to tetrakis(triphenylphosphine)palladium (0), pH refers to potential of hydrogen, Ph refers to phenyl, PTSA refers to *para*-toluenesulfonic acid, TBAF refers to tetrabutylammonium fluoride, tBDMS refers to tertbutyldimethylsilyl, tBDPS refers to tertbutyldiphenylsilyl, TEA refers to triethylamine, TFA refers to trifluoroacetic acid, THF refers to tetrahydrofuran, thp refers to tetrahydropyranyl, TMEDA refers to N,N,N',N'-tetramethylethylenediamine, TMS refers to trimethylsilyl, TMSOK refers to potassium trimethylsilanolate, TLC refers to Thin Layer Chromatography, xantphos refers to 4,5-bis(diphenyphosphino)-9,9-dimethylxanthene.

### Synthetic examples

### Example 1: Synthesis of 7-(1-Cyclohexyl-1H-benzoimidazol-5-yl)-5-((3R,4R)-3-hydroxy-4-hydroxymethyl- piperidin-1-yl)-3H-thieno[3,4-d]pyrimidin-4-one

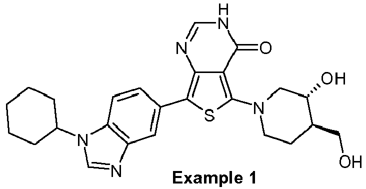

### Step 1: 2,5-Dichloro-4-nitro-thiophene-3-carboxylic acid (1a)

At -10°C, 2,5-Dichloro-thiophene-3-carboxylic acid (1 g, 5 mmol) was added to a mixture of HN0₃cc (10 mL) and H₂SO₄cc (6 mL). The middle was afforded to return at room temperature and was stirred for 3 hours. The mixture was poured on ice and obtained solid was filtered and dried under vacuum to afford compound **(1a)** as a beige solid (800 mg, 3.32 mmol, 66%) without further purification. MS *m*/*z* ([M+H]⁺) 196 (M-COOH).

### Step 2: methyl 2,5-Dichloro-4-nitro-thiophene-3-carboxylate (1b)

Compound **(1a)** (700 mg, 2.89 mmol) was dissolved in MeOH (5mL). H₂SO₄cc (0.5 mL) was added and the mixture was heated at reflux for 24 hours. The middle was concentrated, poured on ice and neutralized by addition of a solution of sodium carbonate. The middle was extracted with DCM. Organic layer was dried over sodium sulfate, filtered and concentrated under reduced pressure. The crude material was purified by chromatography on silica gel (cyclohexane to cyclohexane/EtOAc 9/1) to give compound **(1b)** (560 mg, 2.18 mmol, 76%) as an oil which crystallized. MS *m*/*z* ([M+H]⁺) 256.

### Step 3: Methyl 2-Chloro-5-(1-cyclohexyl-1H-benzoimidazol-5-yl)-4-nitro-thiophene-3-carboxylate (1c)

Under argon atmosphere, compound (**1b**) (100 mg, 0.39 mmol), 1-Cyclohexyl-5-(4,4,5,5-tetramethyl-1,3,2-dioxaborolan-2-yl)-1H-benzimidazole (127 mg, 0.39 mmol), potassium carbonate (161 mg, 1.17 mmol) were dissolved in a mixture of THF (3 mL) and water (0.6 mL). The solution was degassed under argon for 5 minutes and Pd(PPh₃)₄ (23 mg, 0,019 mmol) was added. The reaction was heated at 90°C overnight. The middle was diluted with water and ethyl acetate. The organic layer was washed with brine, dried over sodium sulfate, filtered and concentrated under reduced pressure. The crude material was purified by preparative TLC on silica gel (DCM/MeOH 95/5). Obtained gum was precipitated in Et₂O filtered and dried to give compound **(1c)** (81 mg, 0.19 mmol, 50%). ¹H NMR (400 MHz, CDCl₃) δ 1.28-1.38 (m, 1H), 1.46-1.57 (m, 2H), 1.76-1.86 (m, 3H), 1.97-2.01 (m, 2H), 2.20-2.23 (m, 2H), 3.91 (s, 3H), 4.17-4.25 (m, 1H), 7.34 (dd, J = 1.5/8.5 Hz, 1H), 7.48 (d, J = 8.5 Hz, 1H), 7.89 (s, 1H), 8.11 (s, 1H). MS *m*/*z* ([M+H]⁺) 420.

### Step 4: Methyl 5-(1-Cyclohexyl-1H-benzoimidazol-5-yl)-2-((3R,4R)-3-hydroxy-4-hydroxymethyl-piperidin-1-yl)-4-nitro-thiophene-3-carboxylate (1d)

Compound **(1c)** (50 mg, 0.119 mmol) was dissolved in *n*-BuOH (2 mL). DIPEA (62 µL, 0.357 mmol) and (3R,4R)-4-Hydroxymethyl-piperidin-3-ol (24 mg, 0.179 mmol) were added and the mixture was heated at 110°C for 20 hours. The middle was concentrated and purified by preparative TLC on silica gel (DCM/MeOH 92/8) to give compound **(1d)** (37 mg, 0.072 mmol, 60%) as an orange solid. ¹H NMR (400 MHz, CDCl₃) δ 1.24-1.38 (m, 1H), 1.44-1.60 (m, 3H), 1.69-1.83 (m, 5H), 1.95-1.99 (m, 2H), 2.18-2.22 (m, 2H), 2.79 (t, J = 10.1 Hz, 1H), 2.85-2.95 (m, 1H), 3.54-3.61 (m, 1H), 3.69-3.73 (m, 1H), 3.75 (s, 3H), 3.76-3.86 (m, 2H), 3.88-3.96 (m, 1H), 4.14-4.22 (m, 1H), 7.31 (dd, J = 1.5/8.5 Hz, 1H), 7.43 (d, J = 8.5 Hz, 1H), 7.83 (bs, 1H), 8.07 (bs, 1H). MS *m*/*z* ([M+H]⁺) 515.

### Step 5: Methyl 4-Amino-5-(1-cyclohexyl-1H-benzoimidazol-5-yl)-2-((3R,4R)-3-hydroxy-4-hydroxymethyl-piperidin-1-yl)-thiophene-3-carboxylate (1e)

Compound **(1d)** (37 mg, 0.072 mmol) was dissolved in MeOH (2 mL). Pd(C) (8 mg, 20% mass) was added and the mixture was stirred under hydrogen atmosphere overnight. The middle was filtered on celite and concentrated to give compound **(1e)** (35 mg, 0.072 mmol, 100%) as a yellow residue without further purification. MS *m*/*z* ([M+H]⁺) 485.

### Step 6: 7-(1-Cyclohexyl-1H-benzoimidazol-5-yl)-5-((3R,4R)-3-hydroxy-4-hydroxymethyl-piperidin-1-yl)-3H-thieno[3,4-d]pyrimidin-4-one (Example 1)

Compound **(2e)** (35 mg, 0.072 mmol) and formamidine acetate (38 mg, 0.36 mmol) were dissolved in EtOH (2 mL). The mixture was heated at reflux for 2 hours. Starting material was not totally consumed, so formamidine acetate (19 mg, 0.18 mmol) was added again and mixture was heated at reflux for additional 2 hours. The middle was concentrated. The residue was solubilized with DCM, water and a few drops of MeOH. Organic layer was extracted, dried over sodium sulfate, filtered and concentrated under reduced pressure. The crude material was purified by chromatography on silica gel (DCM/MeOH 85/15) to give **(Example 1)** (5 mg, 0.01 mmol, 15%) as a yellow solid. ¹H NMR (400 MHz, DMSO-*d₆*) δ 1.22-1.35 (m, 1H), 1.40-1.58 (m, 4H), 1.69-1.76 (m, 1H), 1.80-1.90 (m, 5H), 2.02-2.08 (m, 2H), 2.59 (t, J = 10.5 Hz, 1H), 2.76-2.84 (m, 1H), 3.40-3.48 (m, 1H), 3.50-3.60 (m, 1H), 3.65-3.71 (m, 1H), 3.83-3.96 (m, 2H), 4.30-4.40 (m, 1H), 4.45 (t, J = 5.1 Hz, 1H), 4.92 (d, J = 5.3 Hz, 1H), 7.64 (d, 8.5 Hz, 1H), 7.68 (d, J = 3.3Hz, 1H), 7.74 (dd, J = 1.6/8.5 Hz, 1H), 8.23 (d, J = 1.3 Hz, 1H), 8.31 (s, 1H), 11.22 (d, 3.3 Hz, 1H). MS *m*/*z* ([M+H]⁺) 480.

### Example 2: Synthesis of 7-(1-Cyclohexyl-1H-benzoimidazol-5-yl)-5-(4-hydroxymethyl-piperidin-1-yl)-3H-thieno[3,4-d]pyrimidin-4-one

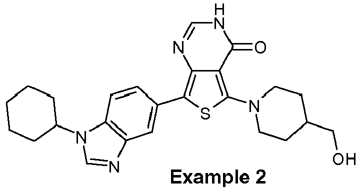

### Step 1: Methyl 5-(1-Cyclohexyl-1H-benzoimidazol-5-yl)-2-(4-hydroxymethyl-piperidin-1-yl)-4-nitro-thiophene-3-carboxylate (2a)

According to the procedure described in example 1, step 4, compound **(1c)** (50 mg, 0.119 mmol) was converted, by reaction with piperidin-4-yl-methanol (21 mg, 0.179 mmol) and after purification by preparative TLC on silica gel (DCM/MeOH 92/8), to compound **(2a)** (36 mg, 0.072 mmol, 61%) as an orange solid. MS *m*/*z* ([M+H]⁺) 499.

### Step 2: Methyl 4-Amino-5-(1-cyclohexyl-1H -benzoimdazol-5-yl)-2-(4-hydroxymethyl-piperidin-1-yl)-thiophene-3-carboxylate (2b)

According to the procedure described in example 1, step 5, compound **(2a)** (36 mg, 0.072 mmol) was converted to compound **(2b)** (34 mg, 0.072 mmol, 100%) as a yellow residue without further purification. MS *m*/*z* ([M+H]⁺) 469.

### Step 3: 7-(1-Cyclohexyl-1H-benzoimidazol-5-yl)-5-(4-hydroxymethyl-piperidin-1-yl)-3H-thieno[3,4-d]pyrimidin-4-one (Example 2)

According to the procedure described in example 1, step 6, compound **(2b)** (34 mg, 0.072 mmol) was converted, after purification by preparative TLC on silica gel (DCM/MeOH 9/1), to **(Example 2)** (6.5 mg, 0.014 mmol, 20%) as a yellow solid. ¹H NMR (400 MHz, DMSO-*d₆*) δ 1.22-1.32 (m, 1H), 1.38-1.56 (m, 5H), 1.68-1.81 (m, 4H), 1.81-1.91 (m, 4H), 2.03-2.10 (m, 2H), 2.80-2.88 (m, 2H), 3.30-3.38 (m, 1H), 3.91 (d, J = 11.7 Hz, 2H), 4.31-4.41 (m, 1H), 4.48-4.56 (bs, 1H), 7.66 (d, J = 8.5 Hz, 1H), 7.69 (d, J = 3.1 Hz, 1H), 7.77 (dd, J = 1.6/8.5 Hz, 1H), 8.25 (d, J = 1.4 Hz, 1H), 8.41 (bs, 1H), 11.22 (d, J = 3.2 Hz, 1H). MS *m*/*z* ([M+H]⁺) 464.

### Example 3: Synthesis of 7-(1-Cyclohexyl-1H-benzoimidazol-5-yl)-5-(3-hydroxymethyl-piperidin-1-yl)-3H-thieno[3,4-d]pyrimidin-4-one

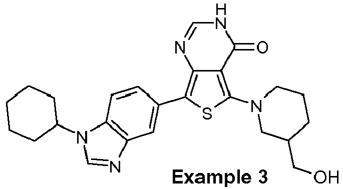

### Step 1: Methyl 5-(1-Cyclohexyl-1H-benzoimidazol-5-yl)-2-(3-hydroxymethyl-piperidin-1-yl)-4-nitro-thiophene-3-carboxylate (3a)

According to the procedure described in example 1, step 4, compound **(1c)** (50 mg, 0.119 mmol) was converted, by reaction with piperidin-3-yl-methanol (21 mg, 0.179 mmol) and after purification by preparative TLC on silica gel (DCM/MeOH 92/8), to compound **(3a)** (40 mg, 0.080 mmol, 68%) as an orange gum. MS *m*/*z* ([M+H]⁺) 499.

### Step 2: Methyl 4-Amino-5-(1-cyclohexyl-1H-benzoimidazol-5-yl)-2-(3-hydroxymethyl-piperidin-1-yl)-thiophene-3-carboxylate (3b)

According to the procedure described in example 1, step 5, compound **(3a)** (40 mg, 0.080 mmol) was converted to compound **(3b)** (37 mg, 0.080 mmol, 100%) as a yellow residue without further purification. MS *m*/*z* ([M+H]⁺) 469.

### Step 3: 7-(1-Cyclohexyl-1H-benzoimidazol-5-yl)-5-(3-hydroxymethyl-piperidin-1-yl)-3H-thieno[3,4-d]pyrimidin-4-one (Example 3)

According to the procedure described in example 1, step 6, compound **(3b)** (37 mg, 0.080 mmol) was converted, after purification by preparative TLC on silica gel (DCM/MeOH 9/1), to **(Example 3)** (6 mg, 0.013 mmol, 19%) as a yellow solid. ¹H NMR (400 MHz, DMSO-*d₆*) δ 1.10-1.35 (m, 2H), 1.45-1.55 (m, 2H), 1.68-1.80 (m, 4H), 1.80-1.92 (m, 5H), 2.05-2.15 (m, 2H), 2.73 (t, J = 10.5 Hz, 1H), 2.84-2.92 (m, 1H), 3.30-3.40 (m, 2H), 3.76-3.82 (m, 1H), 3.85-3.92 (m, 1H), 4.42-4.51 (m, 1H), 4.51-4.61 (bs, 1H), 7.72 (d, J = 3.1 Hz, 1H), 7.80 (d, J = 8.7 Hz, 1H), 7.85 (d, J = 8.7 Hz, 1H), 8.35 (bs, 1H), 8.79 (bs, 1H), 11.28 (bs, 1H). MS *m*/*z* ([M+H]⁺) 464.

### Example 4: Synthesis of 7-(1-Cyclohexyl-1H-benzoimidazol-5-yl)-5-(4-hydroxy-piperidin-1-yl)-3H-thieno[3,4-d]pyrimidin-4-one

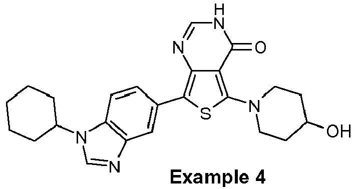

### Step 1: Methyl 5-(1-Cyclohexyl-1H-benzoimidazol-5-yl)-2-(4-hydroxy-piperidin-1-yl)-4-nitrothiophene-3-carboxylate (4a)

According to the procedure described in example 1, step 4, compound **(1c)** (50 mg, 0.119 mmol) was converted, by reaction with piperidin-4-ol (18 mg, 0.179 mmol) and after purification by preparative TLC on silica gel (DCM/MeOH 92/8), to compound **(4a)** (37 mg, 0.076 mmol, 65%) as an orange gum. MS *m*/*z* ([M+H]⁺) 485.

### Step 2: Methyl 4-Amino-5-(1-cyclohexyl-1H-benzoimidazol-5-yl)-2-(4-hydroxy-piperidin-1-yl)-thiophene-3-carboxylate (4b)

According to the procedure described in example 1, step 5, compound **(4a)** (37 mg, 0.076 mmol) was converted to compound **(4b)** (34 mg, 0.076 mmol, 100%) as a yellow residue without further purification. MS *m*/*z* ([M+H]⁺) 455.

### Step 3: 7-(1-Cyclohexyl-1H-benzoimidazol-5-yl)-5-(4-hydroxy-piperidin-1-yl)-3H-thieno[3,4-d]pyrimidin-4-one (Example 4)

According to the procedure described in example 1, step 6, compound **(4b)** (34 mg, 0.076 mmol) was converted, after purification by preparative TLC on silica gel (DCM/MeOH 9/1), to **(Example 4)** (10 mg, 0.022 mmol, 30%) as a yellow solid. ¹H NMR (400 MHz, DMSO-d₆) δ 1.22-1.36 (m, 1H), 1.42-1.56 (m, 2H), 1.58-1.67 (m, 2H), 1.68-1.76 (m, 1H), 1.78-1.94 (m, 6H), 2.02-2.10 (m, 2H), 3.08-3.16 (m, 2H), 3.62-3.74 (m, 3H), 4.30-4.40 (m, 1H), 4.75 (d, J = 4.2 Hz, 1H), 7.64 (d, J = 8.5 Hz, 1H), 7.68 (d, J = 3.3 Hz, 1H), 7.75 (dd, J = 1.6/8.5 Hz, 1H), 8.22 (d, J = 1.6 Hz, 1H), 8.31 (s, 1H), 11.21 (d, J = 3.3 Hz, 1H). MS *m*/*z* ([M+H]⁺) 450.

### Example 5: Synthesis of 7-(1-Cyclohexyl-1H-benzoimidazol-5-yl)-5-(3-hydroxy-piperidin-1-yl)-3H-thieno[3,4-d]pyrimidin-4-one

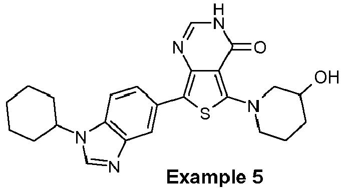

### Step 1: Methyl 5-(1-Cyclohexyl-1H-benzoimidazol-5-yl)-2-(3-hydroxy-piperidin-1-yl)-4-nitrothiophene-3-carboxylate (5a)

According to the procedure described in example 1, step 4, compound **(1c)** (50 mg, 0.119 mmol) was converted, by reaction with piperidin-3-ol (18 mg, 0.179 mmol) and after purification by preparative TLC on silica gel (DCM/MeOH 92/8), to compound **(5a)** (30 mg, 0.062 mmol, 53%) as an orange gum. MS *m*/*z* ([M+H]⁺) 485.

### Step 2: Methyl 4-Amino-5-(1-cyclohexyl-1H-benzoimidazol-5-yl)-2-(3-hydroxy-piperidin-1-yl)-thiophene-3-carboxylate (5b)

According to the procedure described in example 2, step 5, compound **(5a)** (30 mg, 0.062 mmol) was converted to compound **(5b)** (28 mg, 0.062 mmol, 100%) as a yellow residue without further purification. MS *m*/*z* ([M+H]⁺) 455.

### Step 3: 7-(1-Cyclohexyl-1H-benzoimidazol-5-yl)-5-(3-hydroxy-piperidin-1-yl)-3H-thieno[3,4-d]pyrimidin-4-one (Example 5)

According to the procedure described in example 1, step 6, compound **(5b)** (28 mg, 0.062 mmol) was converted, after purification by preparative TLC on silica gel (DCM/MeOH 9/1), to **(Example 5)** (6 mg, 0.013 mmol, 18%) as a yellow solid. ¹H NMR (400 MHz, DMSO-d₆) δ 1.24-1.38 (m, 2H), 1.43-1.56 (m, 2H), 1.62-1.76 (m, 2H), 1.78-1.92 (m, 6H), 2.01-2.09 (m, 2H), 2.71-2.78 (m, 1H), 2.87-2.96 (m, 1H), 3.63-3.82 (m, 3H), 4.30-4.40 (m, 1H), 4.93 (d, J = 4.3 Hz, 1H), 7.64 (d, J = 8.5 Hz, 1H), 7.68 (d, J = 3.3 Hz, 1H), 7.74 (dd, J = 1.6/8.5 Hz, 1H), 8.22 (d, J = 1.4 Hz, 1H), 8.31 (s, 1H), 11.21 (d, J = 3.2 Hz, 1H). MS *m*/*z* ([M+H]⁺) 450.

### Example 6: Synthesis of 7-(1-Cyclohexyl-1H-benzoimidazol-5-yl)-5-(3-hydroxymethyl-pyrrolidin-1-yl)-3H-thieno[3,4-d]pyrimidin-4-one

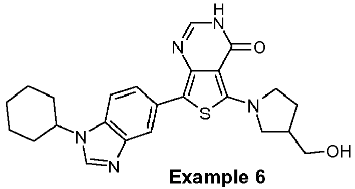

### Step 1: Methyl 5-(1-Cyclohexyl-1H-benzoimidazol-5-yl)-2-(3-hydroxymethyl-pyrrolidin-1-yl)-4-nitro-thiophene-3-carboxylate (6a)

According to the procedure described in example 1, step 4, compound **(1c)** (50 mg, 0.119 mmol) was converted, by reaction with pyrrolidin-3-yl-methanol (18 mg, 0.179 mmol) and after purification by preparative TLC on silica gel (DCM/MeOH 92/8), to compound **(6a)** (35 mg, 0.072 mmol, 61%) as an orange gum. MS *m*/*z* ([M+H]⁺) 485.

### Step 2: Methyl 4-Amino-5-(1-cyclohexyl-1H-benzoimidazol-5-yl)-2-(3-hydroxymethyl-pyrrolidin-1-yl)-thiophene-3-carboxylate (6b)

According to the procedure described in example 2, step 5, compound **(6a)** (35 mg, 0.072 mmol) was converted to compound **(6b)** (33 mg, 0.072 mmol, 100%) without further purification. MS *m*/*z* ([M+H]⁺) 455.

### Step 3: 7-(1-Cyclohexyl-1H-benzoimidazol-5-yl)-5-(3-hydroxymethyl-pyrrolidin-1-yl)-3H-thieno[3,4-d]pyrimidin-4-one (Example 6)

According to the procedure described in example 1, step 6, compound **(6b)** (33 mg, 0.072 mmol) was converted, after purification by preparative TLC on silica gel (DCM/MeOH 9/1), to **(Example 6)** (5 mg, 0.013 mmol, 15%) as a yellow solid. ¹H NMR (400 MHz, DMSO-*d₆*) δ 1.24-1.36 (m, 1H), 1.44-1.56 (m, 2H), 1.69-1.76 (m, 1H), 1.77-1.91 (m, 5H), 2.01-2.10 (m, 3H), 2.42-2.48 (m, 1H), 3.36-3.50 (m, 3H), 3.56-3.69 (m, 3H), 4.28-4.38 (m, 1H), 4.74 (t, J = 5.1 Hz, 1H), 7.61 (d, J = 8.5 Hz, 1H), 7.63 (d, J = 3.3 Hz, 1H), 7.74 (dd, J = 1.6/8.5 Hz, 1H), 8.19 (d, J = 1.6 Hz, 1H), 8.28 (s, 1H), 10.98 (d, J = 3.2 Hz, 1H). MS *m*/*z* ([M+H]⁺) 450.

### Example 7: Synthesis of 7-(1-Cyclohexyl-1H -benzoimidazol-5-yl)-5-((S)-3-hydroxymethyl-pyrrolidin-1-yl)-3H-thieno[3,4-d]pyrimidin-4-one

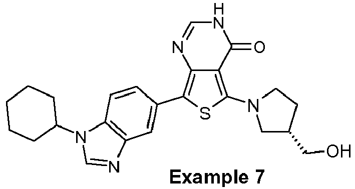

### Step 1: Methyl 5-(1-Cyclohexyl-1H-benzoimidazol-5-yl)-2-((S)-3-hydroxymethyl-pyrrolidin-1-yl)-4-nitro-thiophene-3-carboxylate (7a)

Compound (1c) (170 mg, 0.405 mmol) was dissolved in CH₃CN (4 mL). DIPEA (0.21 mL, 1.21 mmol) and (S)-1-Pyrrolidin-3-yl-methanol (62 mg, 0.608 mmol) were added and the mixture was heated at 80°C for 24 hours. The middle was concentrated and purified by preparative TLC on silica gel (DCM/MeOH 9/1) to give compound **(7a)** (135 mg, 0.278 mmol, 69%) as an orange gum. ¹H NMR (400 MHz, CDCl₃) δ 1.30-1.45 (m, 2H), 1.45-1.65 (m, 2H), 1.80-2.00 (m, 4H), 2.00-2.10 (m, 2H), 2.12-2.22 (m, 1H), 2.25-2.36 (m, 2H), 2.58-2.70 (m, 1H), 3.28-3.36 (m, 1H), 3.41-3.54 (m, 3H), 3.63-3.75 (m, 2H), 3.77 (s, 3H), 4.35-4.48 (m, 1H), 7.54 (dd, J = 1.5/8.7 Hz, 1H), 7.63 (d, J = 8.7 Hz, 1H), 8.03 (d, J = 1.3 Hz, 1H), 9.32 (s, 1H). MS *m*/*z* ([M+H]⁺) 485.

### Step 2: Methyl 4-Amino-5-(1-cyclohexyl-1H -benzoimidazol-5-yl)-2-((S)-3-hydroxymethyl-pyrrolidin-1-yl)-thiophene-3-carboxylate (7b)

According to the procedure described in example 1, step 5, compound **(7a)** (130 mg, 0.268 mmol) was converted to compound **(7b)** (118 mg, 0.259 mmol, 97%) without further purification. MS m/z ([M+H]⁺) 455.

### Step 3: 7-(1-Cyclohexyl-1H-benzoimidazol-5-yl)-5-((S)-3-hydroxymethyl-pyrrolidin-1-yl)-3H-thieno[3,4-d]pyrimidin-4-one (Example 7)

According to the procedure described in example 1, step 6, compound **(7b)** (118 mg, 0.259 mmol) was converted, after purification by preparative TLC on silica gel (DCM/MeOH 9/1), to **(Example 7)** (28 mg, 0.062 mmol, 24%) as a yellow solid. ¹H NMR (400 MHz, DMSO-d₆) δ 1.23-1.36 (m, 1H), 1.44-1.56 (m, 2H), 1.68-1.76 (m, 1H), 1.77-1.90 (m, 6H), 2.01-2.10 (m, 3H), 3.36-3.48 (m, 3H), 3.58-3.69 (m, 3H), 4.30-4.40 (m, 1H), 4.74 (t, J = 5.3 Hz, 1H), 7.61-7.65 (m, 2H), 7.75 (dd, J = 1.6/8.5 Hz, 1H), 8.19(d,J=1.5Hz, 1H),8.31 (s, 1H), 10.98 (d, J=2.8 Hz, 1H). MS *m*/*z* ([M+H]⁺) 450.

### Example 8: Synthesis of 7-(1-Cyclohexyl-1H-benzoimidazol-5-yl)-5-((R)-3-hydroxymethyl-pyrrolidin-1-yl)-3H-thieno[3,4-d]pyrimidin-4-one

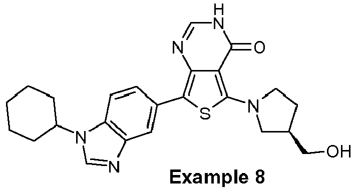

### Step 1: Methyl 5-(1-Cyclohexyl-1H-benzoimidazol-5-yl)-2-((R)-3-hydroxymethyl-pyrrolidin-1-yl)-4-nitro-thiophene-3-carboxylate (8a)

According to the procedure described in example 7, step 1, compound **(1c)** (50 mg, 0.119 mmol) was converted, by reaction with (R)-1-Pyrrolidin-3-yl-methanol (18 mg, 0.179 mmol) and after purification by preparative TLC on silica gel (DCM/MeOH 92/8), to compound **(8a)** (49 mg, 0.101 mmol, 86%) as an orange solid. MS *m*/*z* ([M+H]⁺) 485.

### Step 2: Methyl 4-Amino-5-(1-cyclohexyl-1H-benzoimidazol-5-yl)-2-((R)-3-hydroxymethyl-pyrrolidin-1-yl)-thiophene-3-carboxylate (8b)

According to the procedure described in example 1, step 5, compound **(8a)** (49 mg, 0.101 mmol) was converted to compound **(8b)** (46 mg, 0.101 mmol, 100%) as a yellow residue without further purification. MS *m*/*z* ([M+H]⁺) 455.

### Step 3: 7-(1-Cyclohexyl-1H-benzoimidazol-5-yl)-5-((R)-3-hydroxymethyl-pyrrolidin-1-yl)-3H-thieno[3,4-d]pyrimidin-4-one (Example 8)

According to the procedure described in example 1, step 6, compound **(8b)** (46 mg, 0.101 mmol) was converted, after purification by preparative TLC on silica gel (DCM/MeOH 9/1), to **(Example 8)** (5 mg, 0.011 mmol, 12%) as a yellow solid. ¹H NMR (400 MHz, DMSO-*d₆*) δ 1.24-1.36 (m, 1H), 1.43-1.56 (m, 2H), 1.68-1.76 (m, 1H), 1.76-1.90 (m, 6H), 2.01-2.10 (m, 3H), 3.36-3.50 (m, 3H), 3.58-3.69 (m, 3H), 4.28-4.37 (m, 1H), 4.72-4.76 (t, J = 5.2 Hz, 1H), 7.61 (d, J = 8.5 Hz, 1H), 7.62 (d, J = 3.2 Hz, 1H), 7.74 (dd, J = 1.6/8.5 Hz, 1H), 8.18 (d, J = 1.4 Hz, 1H), 8.28 (s, 1H), 10.97 (d, J = 3.2 Hz, 1H). MS *m*/*z* ([M+H]⁺) 450.

### Example 9: Synthesis of 7-(1-Cyclohexyl-1H-benzoimidazol-5-yl)-5-[2-(2-hydroxy-ethyl)-pyrrolidin-1-yl]-3H-thieno[3,4-d]pyrimidin-4-one

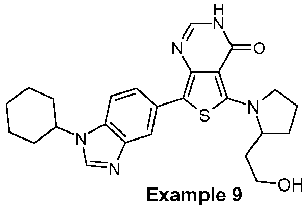

### Step 1: Methyl 5-(1-Cyclohexyl-1H-benzoimidazol-5-yl)-2-[2-(2-hydroxy-ethyl)-pyrrolidin-1-yl]-4-nitro-thiophene-3-carboxylate (9a)

According to the procedure described in example 7, step 1, compound **(1c)** (50 mg, 0.119 mmol) was converted, by reaction with 2-Pyrrolidin-2-yl-ethanol (21 mg, 0.179 mmol) and after purification by preparative TLC on silica gel (DCM/MeOH 92/8), to compound **(9a)** (53 mg, 0.106 mmol, 90%) as an orange gum. MS *m*/*z* ([M+H]⁺) 499.

### Step 2: Methyl 4-Amino-5-(1-cyclohexyl-1H-benzoimidazol-5-yl)-2-[2-(2-hydroxy-ethyl)-pyrrolidin-1-yl]-thiophene-3-carboxylate (9b)

According to the procedure described in example 1, step 5, compound **(9a)** (53 mg, 0.106 mmol) was converted to compound **(9b)** (50 mg, 0.106 mmol, 100%) as a yellow residue without further purification. MS **m/z** ([M+H]⁺) 469.

### Step 3: 7-(1-Cyclohexyl-1H-benzoimidazol-5-yl)-5-[2-(2-hydroxy-ethyl)-pyrrolidin-1-yl]-3H-thieno[3,4-d]pyrimidin-4-one (Example 9)

According to the procedure described in example 1, step 6, compound **(9b)** (50 mg, 0.106 mmol) was converted, after purification by preparative TLC on silica gel (DCM/MeOH 9/1), to **(Example 9)** (11 mg, 0.023 mmol, 23%) as a yellow solid. ¹H NMR (400 MHz, DMSO-*d₆*) δ 1.24-1.36 (m, 1H), 1.44-1.54 (m, 3H), 1.70-1.76 (m, 1H), 1.78-1.90 (m, 7H), 1.93-2.00 (m, 1H), 2.05-2.10 (m, 2H), 2.16-2.24 (m, 1H), 3.22-3.28 (m, 1H), 3.44-3.52 (m, 2H), 3.96-4.06 (m, 1H), 4.12-4.20 (m, 1H), 4.30-4.39 (m, 1H), 4.48 (t, J = 5.2 Hz, 1H), 7.61 (d, J = 8.6 Hz, 1H), 7.64 (bs, 1H), 7.75 (dd, J = 1.5/8.5 Hz, 1H), 8.18 (d, J = 1.4 Hz, 1H), 8.29 (s, 1H), 11.01 (bs, 1H). MS *m*/*z* ([M+H]⁺) 464.

### Example 10: Synthesis of 7-(1-Cyclohexyl-1H-benzoimidazol-5-yl)-5-(3-hydroxy-pyrrolidin-1-yl)-3H-thieno[3,4-d]pyrimidin-4-one

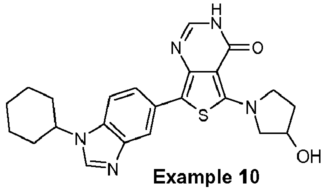

### Step 1: Methyl 5-(1-Cyclohexyl-1H-benzoimidazol-5-yl)-2-(3-hydroxy-pyrrolidin-1-yl)-4-nitrothiophene-3-carboxylate (10a)

According to the procedure described in example 7, step 1, compound **(1c)** (50 mg, 0.119 mmol) was converted, by reaction with Pyrrolidin-3-ol (16 mg, 0.179 mmol) and after purification by preparative TLC on silica gel (DCM/MeOH 92/8), to compound **(10a)** (50 mg, 0.106 mmol, 90%) as an orange solid. MS *m*/*z* ([M+H]⁺) 471.

### Step 2: Methyl 4-Amino-5-(1-cyclohexyl-1H-benzoimidazol-5-yl)-2-(3-hydroxy-pyrrolidin-1-yl)-thiophene-3-carboxylate (10b)

According to the procedure described in example 1, step 5, compound **(10a)** (50 mg, 0.106 mmol) was converted to compound **(10b)** (47 mg, 0.106 mmol, 100%) as a yellow residue without further purification. MS *m*/*z* ([M+H]⁺) 441.

### Step 3: 7-(1-Cyclohexyl-1H-benzoimidazol-5-yl)-5-(3-hydroxy-pyrrolidin-1-yl)-3H-thieno[3,4-d]pyrimidin-4-one (Example 10)

According to the procedure described in example 1, step 6, compound **(10b)** (47 mg, 0.106 mmol) was converted, after purification by preparative TLC on silica gel (DCM/MeOH 9/1), to **(Example 10)** (7 mg, 0.016 mmol, 15%) as a yellow solid. ¹H NMR (400 MHz, DMSO-*d₆*) δ 1.24-1.36 (m, 1H), 1.44-1.56 (m, 2H), 1.68-1.76 (m, 1H), 1.78-1.98 (m, 5H), 2.02-2.16 (m, 3H), 3.37 (d, J = 11.8 Hz, 1H), 3.51-3.58 (m, 1H), 3.64-3.72 (m, 1H), 3.88 (dd, J = 4.3/11.6 Hz, 1H), 4.30-4.44 (m, 2H), 5.04 (d, J = 2.6 Hz, 1H), 7.61-7.65 (m, 2H), 7.76 (dd, J = 1.5/8.5 Hz, 1H), 8.20 (d, J = 1.4 Hz, 1H), 8.34 (s, 1H), 10.98 (d, J = 2.9 Hz, 1H). (MS *m*/*z* ([M+H]⁺) 436.

### Example 11: Synthesis of 7-(1-Cyclohexyl-1H-benzoimidazol-5-yl)-5-[3-(2-hydroxy-ethyl)-pyrrolidin-1-yl]-3H-thieno[3,4-d]pyrimidin-4-one

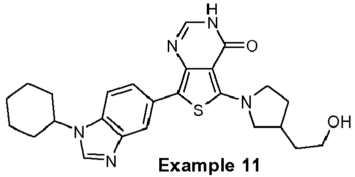

### Step 1: Methyl 5-(1-Cyclohexyl-1H-benzoimidazol-5-yl)-2-[3-(2-hydroxy-ethyl)-pyrrolidin-1-yl]-4-nitro-thiophene-3-carboxylate (11a)

According to the procedure described in example 7, step 1, compound **(1c)** (50 mg, 0.119 mmol) was converted, by reaction with 2-Pyrrolidin-3-yl-ethanol (21 mg, 0.179 mmol) and after purification by preparative TLC on silica gel (DCM/MeOH 92/8), to compound **(11a)** (57 mg, 0.114 mmol, 97%) as an orange gum. MS *m*/*z* ([M+H]⁺) 499.

### Step 2: Methyl 4-Amino-5-(1-cyclohexyl-1H-benzoimidazol-5-yl)-2-[3-(2-hydroxy-ethyl)-pyrrolidin-1-yl]-thiophene-3-carboxylate (11b)

According to the procedure described in example 1, step 5, compound **(11a)** (57 mg, 0.114 mmol) was converted to compound **(11b)** (53 mg, 0.114 mmol, 100%) as a yellow residue without further purification. MS *m*/*z* ([M+H]⁺) 469.

### Step 3: 7-(1-Cyclohexyl-1H-benzoimidazol-5-yl)-5-[3-(2-hydroxy-ethyl)-pyrrolidin-1-yl]-3H-thieno[3,4-d]pyrimidin-4-one (Example 11)

According to the procedure described in example 1, step 6, compound **(11b)** (53 mg, 0.114 mmol) was converted, after purification by preparative TLC on silica gel (DCM/MeOH 9/1), to **(Example 11)** (6.5 mg, 0.014 mmol, 13%) as a yellow solid. ¹H NMR (400 MHz, DMSO-d₆) δ 1.22-1.36 (m, 1H), 1.43-1.56 (m, 2H), 1.56-1.63 (m, 2H), 1.64-1.76 (m, 2H), 1.78-1.91 (m, 4H), 2.01-2.09 (m, 2H), 2.10-2.20 (m, 1H), 2.34-2.44 (m, 1H), 3.26-3.32 (m, 1H), 3.48 (q, J = 5.7 Hz, 2H), 3.53-3.60 (m, 1H), 3.60-3.70 (m, 2H), 4.28-4.38 (m, 1H), 4.48 (t, J = 5.0 Hz, 1H), 7.61 (d, J = 8.5 Hz, 1H), 7.63 (d, J = 3.2 Hz, 1H), 7.74 (d, J = 8.5 Hz, 1H), 8.19 (s, 1H), 8.28 (s, 1H), 10.95 (d, J = 2.9 Hz, 1H). MS m/z ([M+H]⁺) 464.

### Example 12: Synthesis of 7-(1-Cyclohexyl-1H-benzoimidazol-5-yl)-5-(hexahydro-pyrrolo[3,4-b]pyrrol-1-yl)-3H-thieno[3,4-d]pyrimidin-4-one

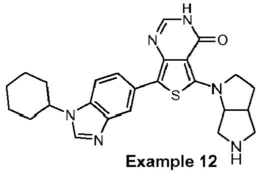

### Step 1: tert-butyl 1-[5-(1-Cyclohexyl-1H-benzoimidazol-5-yl)-3-methoxycarbonyl-4-nitro-thiophen-2-yl]-hexahydro-pyrrolo[3,4-b]pyrrole-5-carboxylate (12a)

According to the procedure described in example 7, step 1, compound **(1c)** (50 mg, 0.119 mmol) was converted, by reaction with tert-butyl Hexahydro-pyrrolo[3,4-b]pyrrole-5-carboxylate (38 mg, 0.179 mmol) and after purification by preparative TLC on silica gel (DCM/MeOH 96/4), to compound **(12a)** (53 mg, 0.089 mmol, 76%) as an orange solid. MS *m*/*z* ([M+H]⁺) 596.

### Step 2: tert-butyl 1-[4-Amino-5-(1-cyclohexyl-1H-benzoimidazol-5-yl)-3-methoxycarbonyl-thiophen-2-yl]-hexahydro-pyrrolo[3,4-b]pyrrole-5-carboxylate (12b)

According to the procedure described in example 1, step 5, compound **(12a)** (53 mg, 0.089 mmol) was converted to compound **(12b)** (50 mg, 0.089 mmol, 100%) as a yellow residue without further purification. MS *m*/*z* ([M+H]⁺) 566.

### Step 3: tert-butyl 1-[7-(1-Cyclohexyl-1H-benzoimidazol-5-yl)-4-oxo-3,4-dihydro-thieno[3,4-d]pyrimidin-5-yl]-hexahydro-pyrrolo[3,4-b]pyrrole-5-carboxylate (12c)

According to the procedure described in example 1, step 6, compound **(12b)** (50 mg, 0.089 mmol) was converted, after purification by preparative TLC on silica gel (DCM/MeOH 9/1), to compound **(12c)** (25 mg, 0.044 mmol, 50%) as a yellow solid. MS *m*/*z* ([M+H]⁺) 561.

### Step 4: 7-(1-Cyclohexyl-1H-benzoimidazol-5-yl)-5-(hexahydro-pyrrolo[3,4-b]pyrrol-1-yl)-3H-thieno[3,4-d]pyrimidin-4-one (Example 12)

Compound **(12c)** (25 mg, 0.044 mmol) was dissolved in MeOH (2 mL). HCl 4N in dioxane (0.22 mL, 0.89 mmol) was added and the mixture was stirred at room temperature for 2 hours. The middle was concentrated and the residue was purified by chromatography on silica gel (DCM/MeOH 9/1+ 5% NH3 7M in MeOH). The residue was triturated in water. Obtained solid was filtered, dissolved in MeOH and then concentrated. The residue was precipitated in Et₂O, filtered and dried under vacuum to give **(Example 12)** (6.5 mg, 0.014, 33%) as a yellow solid. ¹H NMR (400 MHz, CD₃OD) δ 1.32-1.42 (m, 1H), 1.52-1.64 (m, 2H), 1.76-2.00 (m, 7H), 2.14-2.20 (m, 2H), 2.20-2.30 (m, 1H), 2.84-2.90 (m, 1H), 2.91-2.99 (m, 1H), 3.02-3.14 (m, 3H), 3.33-3.38 (m, 1H), 4.02-4.10 (m, 1H), 4.30-4.40 (m, 1H), 7.56-7.62 (m, 2H), 7.80 (d, J = 8.5 Hz, 1H), 8.13 (s, 1H), 8.24 (s, 1H). MS *m*/*z* ([M+H]⁺) 461.

### Example 13: Synthesis of 5-(3,3-Bis-hydroxymethyl-pyrrolidin-1-yl)-7-(1-cyclohexyl-1H-benzoimidazol-5-yl)-3H-thieno[3,4-d]pyrimidin-4-one

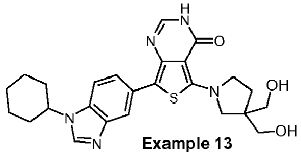

### Step 1: dimethyl 1-Benzyl-pyrrolidine-3,3-dicarboxylate (13a)

To the solution of LDA (2M in THF, 61 mL, 123 mmol) in dry THF (98 mL) at -70°C, a solution of methyl 1-Benzyl-pyrrolidine-3-carboxylate (9 g, 41 mmol) in dry THF (65 ml) was added dropwise. The mixture was stirred at -70°C for 90 minutes, then at -40°C for 2 hours. The mixture was cooled to -70°C and methyl chloroformate (9.54 mL, 123 mmol) in THF (65 mL) was added dropwise. The reaction was stirred at -70°C for 30 minutes. The mixture was allowed to warm to room temperature and stirred overnight. The reaction was quenched with saturated NH₄Cl solution at 0°C. Layers were separated and the aqueous phase was washed with EtOAc. Mixed organic layers were washed with brine, dried (MgSO₄) and concentrated. The crude product was purified by column chromatography on silica gel (EtOAc/hexane 4/6) to afford compound **(13a)** (7.19 g, 26 mmol, 63%). ¹H NMR (300 MHz, CD₃OD) δ 2.41 (t, J = 6.6 Hz, 2H), 2.66 (t, J = 6.6 Hz, 2H), 3.06 (s, 2H), 3.63 (s, 2H), 3.71 (s, 6H), 7.22-7.32 (m, 5H).

### Step 2: (1-Benzyl-3-hydroxymethyl-pyrrolidin-3-yl)-methanol (13b)

To a stirred solution of compound **(13a)** (7.19 g, 25.9 mmol) in dry THF (72 mL) at -5°C under Argon, LiAlH₄ (1.97 g, 51.9 mmol) was added in portions. Reaction was completed after 15 minutes. Reaction was quenched carefully with saturated NH₄Cl aqueous solution at 0°C-10°C. Inorganic salts were filtered through a Celite pad. The filter cake was washed with EtOAc fine. Layers were separated and the aqueous phase was washed with EtOAc. The mixed organic layers were washed with brine, dried (MgSO₄) and concentrated to obtain compound **(13b)** product as pale yellow oil (5.54 g, 25.1 mmol, 96%). ¹H NMR (300 MHz, CD₃OD) δ 1.61 (t, J = 6.8 Hz, 2H), 2.43 (s, 2H), 2.61 (t, J = 6.8 Hz, 2H), 3.51 (s, 4H), 3.60 (s, 2H), 7.22-7.34 (m, 5H). MS *m*/*z* ([M+H]⁺) 222.

### Step 3: (3-Hydroxymethyl-pyrrolidin-3-yl)-methanol (13c)

Compound **(13b)** (5.54 g, 25.1 mmol) was dissolved in MeOH (55 mL). Pd(OH)₂/C (20%, 1.39 g, 0.25 wt) was added and the mixture was stirred under hydrogen atmosphere overnight in Parr hydrogenation apparatus for 2 days. Another portion of Pd(OH)₂/C (20%, 1.39 g, 0.25 wt) was added and reaction was further continued overnight. On TLC only product was observed. Catalyst was filtrated through a Celite pad and the filter cake was washed with MeOH. A filtrate was concentrated and drying under reduced pressure to give compound **(13c)** as a yellow residue without further purification (3.27 g, 25 mmol, 99%). ¹H NMR (300 MHz, CD₃OD) δ 3.51 (d, J = 1.4 Hz, 4H), 2.88 (t, J = 7.1 Hz, 2H), 2.71 (s, 2H), 1.59 (t, J = 7.1Hz, 2H). MS *m*/*z* ([M+H]⁺) 132.

### Step 4: Methyl 2-(3,3-Bis-hydroxymethyl-pyrrolidin-1-yl)-5-(1-cyclohexyl-1H-benzoimidazol-5-yl)-4-nitro-thiophene-3-carboxylate (13d)

Compound **(1c)** (100 mg, 0.238 mmol) was dissolved in CH₃CN (2 mL). DIPEA (0.12 mL, 0.71 mmol), compound **(13c)** (47 mg, 0.358 mmol) and a few drops of DMSO were added and the mixture was heated at 75°C overnight. The middle was concentrated and the residue was solubilized with EtOAc and water. The organic layer was washed with brine, dried over sodium sulfate, filtered and concentrated under reduced pressure. Crude product was purified by preparative TLC on silica gel (DCM/MeOH 9/1) to give compound **(13d)** (92 mg, 0.179 mmol, 76%) as an orange solid. ¹H NMR (400 MHz, CD₃OD) δ 1.39 (qt, J = 12.9/3.7 Hz, 1H), 1.54-1.66 (m, 2H), 1.78-1.84 (m, 1H), 1.85-1.94 (m, 2H), 1.94-2.02 (m, 4H), 2.15-2.21 (m, 2H), 3.34 (s, 2H), 3.50 (t, J = 7.2 Hz, 2H), 3.56-3.62 (m, 4H), 3.74 (s, 3H), 4.39 (tt, J = 11.8/3.7 Hz, 1H), 7.33 (dd, J = 8.5/1.7 Hz, 1H), 7.66 (d, J = 8.5 Hz, 1H), 7.70 (d, J = 1.3 Hz, 1H), 8.34 (s, 1H). MS *m*/*z* ([M+H]⁺) 515.

### Step 5: Methyl 4-Amino-2-(3,3-bis-hydroxymethyl-pyrrolidin-1-yl)-5-(1-cyclohexyl-1H-benzoimidazol-5-yl)-thiophene-3-carboxylate (13e)

According to the procedure described in example 1, step 5, compound **(13d)** (90 mg, 0.175 mmol) was converted to compound **(13e)** (60mg, 0.124 mmol, 72%) as a yellow residue without further purification. MS *m*/*z* ([M+H]⁺) 485.

### Step 6: 5-(3,3-Bis-hydroxymethyl-pyrrolidin-1-yl)-7-(1-cyclohexyl-1H-benzoimidazol-5-yl)-3H-thieno[3,4-d]pyrimidin-4-one (Example 13)

According to the procedure described in example 1, step 6, compound **(13e)** (60mg, 0.124 mmol) was converted, after purification by preparative TLC on silica gel (DCM/MeOH 85/15+2%NH3 7M in MeOH) and trituration with CH₃CN, to **(Example 13)** (20 mg, 0.041 mmol, 34%) as a yellow solid. ¹H NMR (400 MHz, CD₃OD) δ 1.34-1.47 (m, 1H), 1.54-1.66 (m, 2H), 1.78-1.84 (m, 1H), 1.85-1.95 (m, 2H), 1.95-2.02 (m, 4H), 2.16-2.24 (m, 2H), 3.56 (s, 2H), 3.63 (s, 4H), 3.74 (t, J = 7.1 Hz, 2H), 4.33-4.43 (m, 1H), 7.57 (s, 1H), 7.60 (d, J = 8.3 Hz, 1H), 7.81 (d, J = 8.2 Hz, 1H), 8.11 (s, 1H), 8.27 (s, 1H). (MS *m*/*z* ([M+H]⁺) 480.

### Example 14: Synthesis of 7-(1-Cyclohexyl-1H-benzoimidazol-5-yl)-5-(3-oxo-piperazin-1-yl)-3H-thieno[3,4-d]pyrimidin-4-one

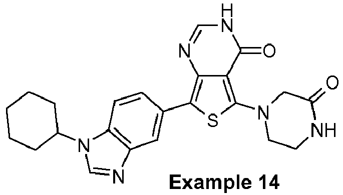

### Step 1: Methyl 5-(1-Cyclohexyl-1H-benzoimidazol-5-yl)-4-nitro-2-(3-oxo-piperazin-1-yl)-thiophene-3-carboxylate (14a)

According to the procedure described in example 13, step 4, compound **(1c)** (100 mg, 0.238 mmol) was converted, by reaction with Piperazin-2-one (47 mg, 0.358 mmol) and after purification by preparative TLC on silica gel (DCM/MeOH 92/8), to compound **(14a)** (73 mg, 0.151 mmol, 64%) as an orange solid. ¹H NMR (400 MHz, CDCl₃) δ 1.28-1.39 (m, 1H), 1.45-1.56 (m, 2H), 1.74-1.88 (m, 3H), 1.96-2.04 (m, 2H), 2.19-2.26 (m, 2H), 3.61 (s, 4H), 3.81 (s, 3H), 3.94 (s, 2H), 4.20 (tt, J = 11.8/4.1Hz, 1H), 6.04 (s, 1H), 7.34 (d, J = 8.5 Hz, 1H), 7.45 (d, J = 8.5 Hz, 1H), 7.86 (s, 1H), 8.04 (s, 1H). MS *m*/*z* ([M+H]⁺) 484.

### Step 2: Methyl 4-Amino-5-(1-cyclohexyl-1H-benzoimidazol-5-yl)-2-(3-oxo-piperidin-1-yl)-thiophene-3-carboxylate (14b)

According to the procedure described in example 1, step 5, compound **(14a)** (71 mg, 0.147 mmol) was converted to compound **(14b)** (53mg, 0.117 mmol, 81%) as a yellow residue without further purification. MS *m*/*z* ([M+H]⁺) 454.

### Step 3: 7-(1-Cyclohexyl-1H-benzoimidazol-5-yl)-5-(3-oxo-piperazin-1-yl)-3H-thieno[3,4-d]pyrimidin-4-one (Example 14)

According to the procedure described in example 1, step 6, compound **(14b)** (53mg, 0.117 mmol) was converted, after purification by preparative TLC on silica gel (DCM/MeOH 9/1+2%NH3 7M in MeOH) and trituration with CH₃CN, to **(Example 14)** (15 mg, 0.033 mmol, 29%) as a yellow solid. ¹H NMR (400 MHz, DMSO-*d₆*) δ 1.24-1.36 (m, 1H), 1.44-1.56 (m, 2H), 1.69-1.77 (m, 1H), 1.79-1.92 (m, 4H), 2.02-2.10 (m, 2H), 3.37-3.42 (m, 2H), 3.62-3.67 (m, 2H), 3.99 (s, 2H), 4.35 (tt, J = 11.6/3.8 Hz, 1H), 7.66 (d, J = 8.5 Hz, 1H), 7.72 (d, J = 3.4 Hz, 1H), 7.75 (dd, J = 8.5/1.7 Hz, 1H), 8.14 (bs, 1H), 8.25 (d, J = 1.5 Hz, 1H), 8.32 (s, 1H), 11.36 (d, J = 3.2 Hz, 1H). (MS *m*/*z* ([M+H]⁺) 449.

### Example 15: Synthesis of 1-[7-(1-Cyclohexyl-1H-benzoimidazol-5-yl)-4-oxo-3,4-dihydro-thieno[3,4-d]pyrimidin-5-yl]-pyrrolidine-3-carbonitrile

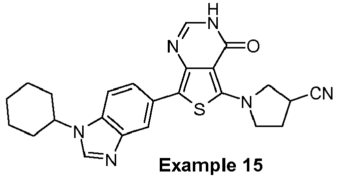

### Step 1: Methyl 2-(3-Cyano-pyrrolidin-1-yl)-5-(1-cyclohexyl-1H-benzoimidazol-5-yl)-4-nitrothiophene-3-carboxylate (15a)

Compound **(1c)** (200 mg, 0.47 mmol) was dissolved in DMSO (1 mL). DIPEA (0.25 mL, 1.41 mmol) and Pyrrolidine-3-carbonitrile (93 mg, 0.71 mmol) were added and the mixture was heated at 80°C overnight. After cooling, EtOAc and water were added. The organic layer was washed with brine, dried over sodium sulfate, filtered and concentrated under reduced pressure and purified by preparative TLC on silica gel (DCM/MeOH 95/5) to give compound **(15a)** (137 mg, 0.286 mmol, 61%) as an orange solid. ¹H NMR (400 MHz, CDCl₃) δ 1.32 (qt, J = 13.0/3.5 Hz, 1H), 1.45-1.56 (m, 2H), 1.74-1.87 (m, 3H), 1.95-2.03 (m, 2H), 2.18-2.25 (m, 2H), 2.42-2.57 (m, 2H), 3.28 (quin, J = 6.6 Hz, 1H), 3.54-3.63 (m, 2H), 3.64-3.70 (m, 1H), 3.79 (s, 3H), 3.80-3.85 (m, 1H), 4.19 (t, J = 12.0/3.6 Hz, 1H), 7.32 (dd, J = 8.5/1.7 Hz, 1H), 7.43 (d, J = 8.5 Hz, 1H), 7.85 (d, J = 1.4 Hz, 1H), 8.03 (s, 1H). MS *m*/*z* ([M+H]⁺) 480.

### Step 2: Methyl 4-Amino-2-(3-cyano-pyrrolidin-1-yl)-5-(1-cyclohexyl-1H-benzoimidazol-5-yl)-thiophene-3-carboxylate (15b)

According to the procedure described in example 1, step 5, compound **(15a)** (136 mg, 0.284 mmol) was converted to compound **(15b)** (127 mg, 0.284 mmol, 100%) as a yellow residue without further purification. MS *m*/*z* ([M+H]⁺) 450.

### Step 3: 1-[7-(1-Cyclohexyl-1H-benzoimidazol-5-yl)-4-oxo-3,4-dihydro-thieno[3,4-d]pyrimidin-5-yl]-pyrrolidine-3-carbonitrile (Example 15)

According to the procedure described in example 1, step 6, compound **(15b)** (127 mg, 0.284 mmol) was converted, after purification by preparative TLC on silica gel (DCM/MeOH 9/1+2%NH₃ 7M in MeOH) and trituration with CH₃CN, to **(Example 15)** (45 mg, 0.101 mmol, 35%) as a yellow solid. ¹H NMR (400 MHz, DMSO-*d₆*) δ 1.24-1.36 (m, 1H), 1.42-1.57 (m, 2H), 1.69-1.76 (m, 1H), 1.80-1.91 (m, 4H), 2.01-2.09 (m, 2H), 2.27-2.36 (m, 1H), 2.39-2.48 (m, 1H), 3.54-3.63 (m, 2H), 3.64-3.72 (m, 1H), 3.92 (d, J = 6.5 Hz, 2H), 4.34 (tt, J = 11.5/3.5 Hz, 1H), 7.63 (d, J = 8.5 Hz, 1H), 7.68 (s, 1H), 7.75 (dd, J = 8.5/1.7 Hz, 1H), 8.21 (d, J = 1.5 Hz, 1H), 8.30 (s, 1H), 11.15 (s, 1H). MS *m*/*z* ([M+H]⁺) 445.

### Example 16: Synthesis of 5-Amino-7-(1-cyclohexyl-1H-benzoimidazol-5-yl)-3H-thieno[3,4-d]pyrimidin-4-one

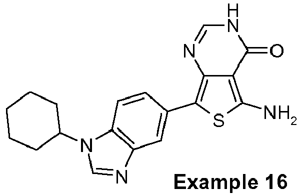

**(Example 16)** was purified and isolated (16mg, 0.043 mmol, 16%) as a byproduct of **(Example 15),** step 3. ¹H NMR (400 MHz, DMSO-*d₆*) δ 1.22-1.36 (m, 1H), 1.40-1.58 (m, 2H), 1.68-1.78 (m, 1H), 1.79-1.92 (m, 4H), 2.00-2.10 (m, 2H), 4.26-4.40 (m, 1H), 7.45 (s, 2H), 7.56-7.62 (m, 2H), 7.72 (dd, J = 8.5/1.7 Hz, 1H), 8.08 (d, J = 1.4 Hz, 1H), 8.27 (s, 1H), 11.00 (d, J = 3.2 Hz, 1H). MS *m*/*z* ([M+H]⁺) 366.

### Example 17: Synthesis of 7-(1-Cyclohexyl-1H-benzoimidazol-5-yl)-5-(6-hydroxy-3-aza-bicyclo[3.1.01]hex-3-yl)-3H-thieno[3,4-d]pyrimidin-4-one

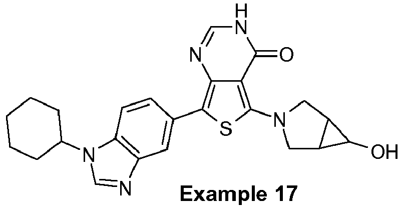

### Step 1: Benzyl 6,6-Dibromo-3-aza-bicyclo[3.1.0]hexane-3-carboxylate (17a)

To the mixture of Benzyl 2,5-Dihydro-pyrrole-1-carboxylate (30 g, 148 mmol), BTEAC (3.36 g, 14.8 mmol), 15-crown-5 (0.325 g, 1.5 mmol), DCM (450 mL) and 50% NaOH (450 ml), a solution of CHBr₃ (56 g, 221 mmol) in DCM (97 mL) was dropped over 5 hours. The mixture was stirring for 4 hours. To the mixture CHBr₃ (36.31 g, 12.9 mL, 148 mmol) was added and reaction was stirring overnight. The mixture was diluted with water and filtered through a Celite pad. A filter cake was washed with water and DCM. The layers were separated and the aqueous phase was washed with DCM. Mixed organic layer were washed with saturated NH₄Cl solution, brine, dried (MgSO₄) and concentrated. The crude was purified by flash chromatography on silica gel (DCM/EtOAc 99/1) to afford compound **(17a)** (29.75 g, 79 mmol, 54%). ¹H NMR (300 MHz, CDCl₃) δ 2.41-2.45 (m, 2H), 3.63-3.65 (m, 2H), 3.66-3.70 (m, 2H), 5.11 (s, 2H), 7.31-7.36 (m, 5H).

### Step 2: Benzyl 6-Hydroxy-3-aza-bicyclo[3.1.0]hexane-3-carboxylate (17b)

A *n*-BuLi solution (1.6 M in hexanes, 32.1 mL, 51.3 mmol) was added dropwise over 1 hour to the cooled (>-100°C) solution of compound **(17a)** (16.75 g, 44.7 mmol) in dry THF (385 mL) under Argon. After 10 minutes, a solution of catechol borane (10.7 g, 89.3 mmol) in THF (89 mL) was added dropwise over 1 hour. The reaction mixture allowed to warm slowly to room temperature and then heated at 50°C overnight. After cooling to 0°C, the reaction was treated with 50% H₂O₂ (11.22 mL) and 2.5M NaOH (53 mL) and stirred overnight. The reaction was quenched with saturated Na₂S₂O₃ and 2.5% NaHCO₃. THF was removed under reduced pressure and the aqueous solution was extracted with EtOAc. The combined organic layers were washed with saturated Na₂S₂O₃, brine, then dried (MgSO₄) and concentrated. The crude was purified via flash chromatography (EtOAc/hexane 1/1) to obtain compound **(17b)** (2.51 g, 10.8 mmol, 24%) as a pale yellow oil. ¹H NMR (300 MHz, CDCl₃) δ 1.70-1.72 (m, 2H), 2.30 (bs, 1H), 3.19 (bs, 1H), 3.41-3.48 (m, 2H), 3.56-3.66 (t, J = 11.1 Hz, 2H), 5.09 (d, J =1.3 Hz, 2H), 7.28-7.38 (m, 5H).

### Step 3: 3-Aza-bicyclo[3.1.0]hexan-6-ol (17c)

Pd(OH)₂/C (20%, 1.62 g, 0.5wt) was added to a solution of compound **(17b)** (3.85 g, 16.5 mmol) in MeOH (38 mL) and the reaction was performed under H₂ balloon overnight. The mixture was filtrated through a Celite pad and the filtrate was concentrated. The product was purified by column chromatography on silica gel (EtOAc/MeOH 8/2) to give compound **(17c)** (1.63 g, 16.5 mmol, 100%). ¹H NMR (300 MHz, DMSO-*d₆*) δ 1.31 (m, 2H), 2.61 (dt, J = 11.0/1.5 Hz, 2H), 2.81 (d, J = 11.0 Hz, 2H), 2.96 (t, J=1.5 Hz, 1H). MS *m*/*z* ([M+H]⁺) 100.

### Step 4: Methyl 5-(1-Cyclohexyl-1H-benzoimidazol-5-yl)-2-(6-hydroxy-3-aza-bicyclo[3.1.0]hex-3-yl)-4-nitro-thiophene-3-carboxylate (17d)

According to the procedure described in example 15, step 1, compound **(1c)** (100 mg, 0.238 mmol) was converted, by reaction with compound **(17c)** (47 mg, 0.358 mmol) and after purification by preparative TLC on silica gel (DCM/MeOH 9/1), to compound **(17d)** (77 mg, 0.159 mmol, 68%) as an orange solid. ¹H NMR (400 MHz, CDCl₃) δ 1.26-1.38 (m, 1H), 1.44-1.55 (m, 2H), 1.74-1.86 (m, 3H), 1.91 (bs, 1H), 1.95-2.03 (m, 2H), 2.12 (bs, 1H), 2.18-2.25 (m, 2H), 3.30-3.32 (m, 1H), 3.53-3.60 (m, 4H), 3.78 (s, 3H), 4.18 (tt, J = 12.1/3.7 Hz, 1H), 7.30 (dd, J = 8.5/1.7 Hz, 1H), 7.41 (d, J = 8.5 Hz, 1H), 7.82 (d, J = 1.4 Hz, 1H), 8.02 (s, 1H). MS *m*/*z* ([M+H]⁺) 483.

### Step 5: Methyl 4-Amino-5-(1-cyclohexyl-1H-benzoimidazol-5-yl)-2-(6-hydroxy-3-aza-bicyclo[3.1.0]hex-3-yl)-thiophene-3-carboxylate (17e)

According to the procedure described in example 1, step 5, compound **(17d)** (76 mg, 0.157 mmol) was converted to compound **(17e)** (60 mg, 0.132 mmol, 85%) as a yellow residue without further purification. MS *m*/*z* ([M+H]⁺) 453.

### Step 6: 7-(1-Cyclohexyl-1H-benzoimidazol-5-yl)-5-(6-hydroxy-3-aza-bicyclo[3.1.0]hex-3-yl)-3H-thieno[3,4-d]pyrimidin-4-one (Example 17)

According to the procedure described in example 1, step 6, compound **(17e)** (60 mg, 0.132 mmol) was converted, after purification by preparative TLC on silica gel (DCM/MeOH 9/1+2%NH₃ 7M in MeOH) and trituration with CH₃CN, to **(Example 17)** (21 mg, 0.047 mmol, 36%) as a yellow solid. ¹H NMR (400 MHz, DMSO-*d₆*) δ 1.20-1.38 (m, 1H), 1.40-1.58 (m, 2H), 1.68-1.80 (m, 3H), 1.80-1.92 (m, 4H), 2.00-2.10 (m, 2H), 3.06-3.09 (m, 1H), 3.48-3.55 (m, 2H), 3.96 (d, J = 10.4 Hz, 2H), 4.27-4.40 (m, 1H), 5.49 (d, J = 1.8 Hz, 1H), 7.62 (d, J = 8.7 Hz, 1H), 7.64 (d, J = 3.4 Hz, 1H), 7.72 (dd, J = 8.5/1.7 Hz, 1H), 8.18 (d, J = 1.5 Hz, 1H), 8.29 (s, 1H), 11.08 (d, J = 3.2 Hz, 1H). MS *m*/*z* ([M+H]⁺) 448.

### Example 18: Synthesis of 7-(1-Cyclohexyl-1H-benzoimidazol-5-yl)-5-((3aS,5R,6aR)-5-hydroxy-hexahydro-cyclopenta[c]pyrrol-2-yl)-3H-thieno[3,4-d]pyrimidin-4-one

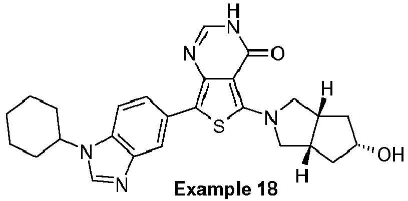

### Step 1: tert-butyl (3aS,5R,6aR)-5-Hydroxy-hexahydro-cyclopenta[c]pyrrole-2-carboxylate (18a)

To a solution of tert-butyl (3aS,6aR)-5-Oxo-hexahydro-cyclopenta[c]pyrrole-2-carboxylate (10.20 g, 45.3 mmol) in dry MeOH (306 mL), NaBH₄ (2.85 g, 45.3 mmol) was added in three portions at 0°C under Argon. After 30 minutes stirring, the reaction was quenched with saturated NH₄Cl solution. The product was extracted with EtOAc. Mixed organic layers were washed with NH₄Cl solution, dried (MgSO₄) and concentrated to give compound **(18a)** (9.66 g, 42.5 mmol, 94%). ¹H NMR (300 MHz, CDCl₃) δ 1.45 (s, 9H), 1.46-1.59 (m, 3H), 2.14-2.17 (m, 2H), 2.57-2.61 (m, 2H), 3.34 (dd, J = 2.2/11.2 Hz, 2H), 3.49 (dd, J = 8.0/11.3 Hz, 2H), 4.26 (bs, 1H).

### Step 2: (3aS,5R,6aR)-Octahydro-cyclopenta[c]pyrrol-5-ol hydrochloride (18b)

Compound **(18a)** (2.40 g, 10.6 mmol) was dissolved in MeOH (36 mL) and 3M HCl in dioxane (50 mL) was added. After 3 hours, middle was concentrated and the residue was triturated with Et₂O to afford compound **(18b)** (1.32 g, 10.4 mmol, 98%). ¹H NMR (300 MHz, DMSO-*d₆*) δ 1.50-1.54 (m, 2H), 1.80-1.84 (m, 2H), 2.74-2.77 (m, 2H), 3.05-3.08 (m, 2H), 3.16-3.20 (m, 2H), 4.10 (bs, 1H), 5.12 (bs, 1H), 8.42 (bs, 1H). MS *m*/*z* ([M+H]⁺) 128.

### Step 3: Methyl 5-(1-Cyclohexyl-1H-benzoimidazol-5-yl)-2-((3aS,5R,6aR)-5-hydroxy-hexahydro-cyclopenta[c]pyrrol-2-yl)-4-nitro-thiophene-3-carboxylate (18c)

According to the procedure described in example 15, step 1, compound **(1c)** (100 mg, 0.238 mmol) was converted, by reaction with compound **(18b)** (58 mg, 0.358 mmol) and after purification by preparative TLC on silica gel (DCM/MeOH 93/7), to compound **(18c)** (52 mg, 0.101 mmol, 43%) as an orange solid. MS *m*/*z* ([M+H]⁺) 511.

### Step 4: Methyl 4-Amino-5-(1-cyclohexyl-1H-benzoimidazol-5-yl)-2-((3aS,5R,6aR)-5-hydroxy-hexahydro-cyclopenta[c]pyrrol-2-yl)-thiophene-3-carboxylate (18d)

According to the procedure described in example 1, step 5, compound **(18c)** (52 mg, 0.101 mmol) was converted to compound **(18d)** (38mg, 0.079 mmol, 78%) as a yellow residue. MS *m*/*z* ([M+H]⁺) 481.

### Step 5: 7-(1-Cyclohexyl-1H-benzoimidazol-5-yl)-5-((3aS,5R,6aR)-5-hydroxy-hexahydro-cyclopenta[c]pyrrol-2-yl)-3H-thieno[3,4-d]pyrimidin-4-one (Example 18)

According to the procedure described in example 1, step 6, compound **(18d)** (38 mg, 0.079 mmol) was converted, after purification by preparative TLC on silica gel (DCM/MeOH 9/1+2%NH₃ 7M in MeOH), to **(Example 18)** (9 mg, 0.019 mmol, 24%) as a yellow solid. ¹H NMR (400 MHz, CD₃OD) δ 1.32-1.48 (m, 1H), 1.52-1.70 (m, 4H), 1.78-2.04 (m, 5H), 2.14-2.28 (m, 4H), 2.80-2.90 (m, 2H), 3.50-3.58 (m, 2H), 3.74 (dd, J = 10.0/2.5 Hz, 2H), 4.14-4.24 (m, 1H), 4.32-4.44 (m, 1H), 7.59 (s, 1H), 7.61 (d, J = 8.5 Hz, 1H), 7.81 (dd, J = 8.5/1.6 Hz, 1H), 8.13 (d, J = 1.3Hz, 1H), 8.25 (s, 1H). MS *m*/*z* ([M+H]⁺) 476.

### Example 19: Synthesis of 7-(1-Cyclohexyl-1H-benzoimidazol-5-yl)-5-((3aS,5R,6aR)-5-hydroxy-hexahydro-cyclopenta[c]pyrrol-2-yl)-3H-thieno[3,4-d]pyrimidin-4-one

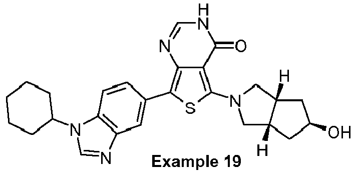

### Step 1: tert-butyl (3aS,5S,6aR)-5-(4-Nitro-benzoyloxy)-hexahydro-cyclopenta[c]pyrrole-2-carboxylate (19a)

Under a nitrogen atmosphere, DIAD (7.5 mL, 19 mmol) was added at -78°C to a solution of compound **(18a)** (4.33 g, 38.1 mmol), triphenylphosphine (10 g, 38.1 mmol) and 4-nitrobenzoic acid (4.77 g, 28.6 mmol) in Et₂O (126 mL). The reaction mixture was stirred at 25°C for 18 hours. The mixture was then quenched with MeOH (10 mL) at 25°C for 15 minutes and evaporated. The crude product was purified by column chromatography on silica gel (cyclohexane/EtOAc 7/3) to give compound **(19a)** as a white powder (5.6 g, 14.9 mmol, 78%). ¹H NMR (300 MHz, CDCl₃) δ 1.46 (s, 9H), 1.88-1.97 (m, 2H), 2.13-2.20 (m, 2H), 2.89-2.93 (m, 2H), 3.24-3.25 (m, 2H), 3.54-3.58 (m, 2H), 5.53-5.59 (m, 1H), 8.16 (d, J = 8.7 Hz, 2H), 8.27 (d, J = 8.7 Hz, 2H). MS *m*/*z* ([M+Na]⁺) 399.

### Step 2: tert-butyl (3aS,5S,6aR)-5-Hydroxy-hexahydro-cyclopenta[c]pyrrole-2-carboxylate (19b)

To a solution of compound **(19a)** (4.96 g, 13.2 mmol) in THF (99 mL) was added potasium trimethylsilanolate (8.45 g, 65.9 mmol). The reaction mixture was stirred at 25°C for 2 hours. The middle was concentrated and purified by column chromatography on silica gel (DCM/MeOH 99/1) to give compound **(19b)** (1.2 g, 0.53 mmol, 40%). ¹H NMR (300 MHz, CDCl₃) δ 1.46 (s, 9H), 1.69-1.72 (m, 2H), 1.84-1.93 (m, 2H), 2.80-2.87 (m, 2H), 3.13-3.16 (m, 2H), 3.46-3.52 (m, 2H), 4.45-4.47 (m, 1H).

### Step 3: (3aS,5S,6aR)-Octahydro-cyclopenta[c]pyrrol-5-ol hydrochloride (19c)

According to the procedure described in example 12, step 4, compound **(19b)** (0.94 g, 4.1 mmol) was converted to compound **(19c)** (520 mg, 4.1 mmol, 100%). ¹H NMR (300 MHz, DMSO-*d₆*) δ 1.55-1.60 (m, 2H), 1.72-1.76 (m, 2H), 2.75-2.93 (m, 4H), 3.13-3.19 (m, 2H), 4.18-4.22 (m, 1H), 4.32 (bs, 1H), 9.35 (bs, 1H). MS *m*/*z* ([M+H]⁺) 128.

### Step 4: Methyl 5-(1-Cyclohexyl-1H-benzoimidazol-5-yl)-2-((3aS,5S,6aR)-5-hydroxy-hexahydro-cyclopenta[c]pyrrol-2-yl)-4-nitro-thiophene-3-carboxylate (19d)

According to the procedure described in example 15, step 1, compound **(1c)** (58 mg, 0.358 mmol) was converted, by reaction with compound **(19c)** (58 mg, 0.358 mmol) and after purification by preparative TLC on silica gel (DCM/MeOH 93/7), to compound **(19d)** (80 mg, 0.156 mmol, 66%) as an orange solid. ¹H NMR (400 MHz, CDCl₃) δ 1.28-1.38 (m, 1H), 1.44-1.56 (m, 2H), 1.74-1.86 (m, 5H), 1.95-2.04 (m, 4H), 2.18-2.26 (m, 2H), 3.01-3.08 (m, 2H), 3.28-3.34 (m, 2H), 3.43-3.50 (m, 2H), 3.78 (s, 3H), 4.12 (q, J = 7.1 Hz, 1H), 4.18 (tt, J = 11.9/3.8 Hz, 1H), 4.55 (bs, 1H), 7.31 (dd, J = 8.5/1.7 Hz, 1H), 7.42 (d, J = 8.5 Hz, 1H), 7.84 (d, J = 1.4 Hz, 1H), 8.02 (s, 1H). MS *m*/*z* ([M+H]⁺) 511.

### Step 5: Methyl 4-Amino-5-(1-cyclohexyl-1H-benzoimidazol-5-yl)-2-((3aS,5S,6aR)-5-hydroxy-hexahydro-cyclopenta[c]pyrrol-2-yl)-thiophene-3-carboxylate (19e)

According to the procedure described in example 1, step 5, compound **(19d)** (80 mg, 0.156 mmol) was converted to compound **(19e)** (58mg, 0.12 mmol, 77%) as a yellow residue without further purification. MS *m*/*z* ([M+H]⁺) 481.

### Step 6: 7-(1-Cyclohexyl-1H-benzoimidazol-5-yl)-5-((3aS,5S,6aR)-5-hydroxy-hexahydro-cyclopenta[c]pyrrol-2-yl)-3H-thieno[3,4-d]pyrimidin-4-one (Example 19)

According to the procedure described in example 1, step 6, compound **(19e)** (58mg, 0.12 mmol) was converted, after purification by preparative TLC on silica gel (DCM/MeOH 9/1+2%NH₃ 7M in MeOH), to **(Example 19)** (12 mg, 0.025 mmol, 21%) as a yellow solid. ¹H NMR (400 MHz, CD₃OD) 6 1.30-1.48 (m, 1H), 1.52-1.69 (m, 2H), 1.76-1.86 (m, 3H), 1.87-2.04 (m, 6H), 2.14-2.24 (m, 2H), 2.97-3.08 (m, 2H), 3.40-3.50 (m, 2H), 3.68 (dd, J = 10.0/2.0 Hz, 2H), 4.32-4.48 (m, 2H), 7.58 (s, 1H), 7.60 (d, J = 8.5 Hz, 1H), 7.80 (dd, J = 8.5/1.5 Hz, 1H), 8.12 (d, J = 1.3 Hz, 1H), 8.25 (s, 1H). MS *m*/*z* ([M+H]⁺) 476.

### Example 20: Synthesis of 1-[7-(1-Cyclohexyl-1H-benzoimidazol-5-yl)-4-oxo-3,4-dihydro-thieno[3,4-d]pyrimidin-5-yl]-pyrrolidine-3-carboxamide

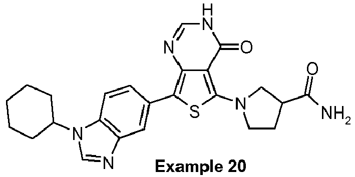

### Step 1: Pyrrolidine-3-carboxamide hydrochloride (20a)

According to the procedure described in example 12, step 4, tert-butyl 3-carbamoyl-pyrrolidine-1-carboxylate (200 mg, 0.93 mmol) was converted to compound **(20a)** (126 mg, 0.84 mmol, 90%) as a white solid. ¹H NMR (400 MHz, DMSO-*d₆*) δ 1.86-1.96 (m, 1H), 2.07-2.17 (m, 1H), 3.01 (quin, J = 7.5 Hz, 1H), 3.08-3.22 (m, 3H), 3.22-3.32 (m, 1H), 7.10 (s, 1H), 7.66 (s, 1H), 9.18 (bs, 1H), 9.49 (bs, 1H).

### Step 2: Methyl 2-(3-Carbamoyl-pyrrolidin-1-yl)-5-(1-cyclohexyl-1H-benzoimidazol-5-yl)-4-nitrothiophene-3-carboxylate (20b)

According to the procedure described in example 15, step 1, compound **(1c)** (100 mg, 0.238 mmol) was converted, by reaction with compound **(20a)** (54 mg, 0.358 mmol) and after purification by preparative TLC on silica gel (DCM/MeOH 92/8), to compound **(20b)** (90 mg, 0.181 mmol, 76%) as an orange solid. ¹H NMR (400 MHz, CDCl₃) δ 1.34 (qt, J = 12.9/3.7 Hz, 1H), 1.51 (qt, J = 13.0/3.6 Hz, 2H), 1.74-1.86 (m, 3H), 1.95-2.03 (m, 2H), 2.18-2.25 (m, 2H), 2.34 (q, J = 7.0 Hz, 2H), 3.11 (quin, J = 7.0 Hz, 1H), 3.50-3.56 (m, 2H), 3.59-3.64 (m, 1H), 3.69-3.74 (m, 1H), 3.78 (s, 3H), 4.18 (tt, J = 11.9/3.8 Hz, 1H), 5.38 (bs, 1H), 5.80 (bs, 1H), 7.32 (dd, J = 8.5/1.4 Hz, 1H), 7.42 (d, J = 8.5 Hz, 1H), 7.84 (d, J = 1.4 Hz, 1H), 8.02 (s, 1H). MS *m*/*z* ([M+H]⁺) 498.

### Step 3: Methyl 4-Amino-2-(3-carbamoyl-pyrrolidin-1-yl)-5-(1-cyclohexyl-1H-benzoimidazol-5-yl)-thiophene-3-carboxylate (20c)

According to the procedure described in example 1, step 5, compound **(20b)** (90 mg, 0.181 mmol) was converted to compound **(20c)** (84 mg, 0.181 mmol, 100%) as a yellow residue. MS *m*/*z* ([M+H]⁺) 468.

### Step 4: 1-[7-(1-Cyclohexyl-1H-benzoimidazol-5-yl)-4-oxo-3,4-dihydro-thieno[3,4-d]pyrimidin-5-yl]-pyrrolidine-3-carbox amide (Example 20)

According to the procedure described in example 1, step 6, compound **(20c)** (84 mg, 0.181 mmol) was converted, after purification by preparative TLC on silica gel (DCM/MeOH 9/1+2% NH₃ 7M in MeOH), to **(Example 20)** (5mg, 0.011 mmol, 6%) as a yellow solid. ¹H NMR (400 MHz, CD₃OD) δ 1.32-1.46 (m, 1H), 1.52-1.67 (m, 2H), 1.75-1.90 (m, 2H), 1.90-2.01 (m, 3H), 2.14-2.24 (m, 2H), 2.25-2.38 (m, 2H), 3.16-3.26 (m, 1H), 3.64-3.76 (m, 2H), 3.84-3.90 (m, 2H), 4.32-4.44 (m, 1H), 7.56 (s, 1H), 7.61 (d, J = 8.6 Hz, 1H), 7.81 (dd, J = 8.6/1.5 Hz, 1H), 8.13 (d, J= 1.1 Hz, 1H), 8.31 (s, 1H). MS *m*/*z* ([M+H]⁺) 463.

### Example 21: Synthesis of 7-(1-Cyclohexyl-1H-benzoimidazol-5-yl)-5-[(3-dimethylamino-propyl)-methyl-amino]-3H-thieno[3,4-d]pyrimidin-4-one

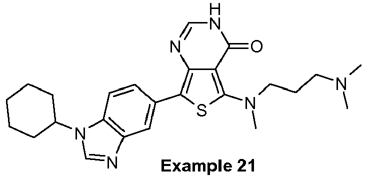

### Step 1: Methyl 5-(1-Cyclohexyl-1H-benzoimidazol-5-yl)-2-[(3-dimethylamino-propyl)-methyl-amino]-4-nitro-thiophene-3-carboxylate (21a)

According to the procedure described in example 15, step 1, compound **(1c)** (100 mg, 0.238 mmol) was converted, by reaction with N,N,N'-Trimethyl-propane-1,3-diamine (42 mg, 0.36 mmol) and after purification by preparative TLC on silica gel (DCM/MeOH 92/8), to compound **(21a)** (75 mg, 0.15 mmol, 64%) as an orange solid. ¹H NMR (400 MHz, CDCl₃) δ 1.31 (qt, J = 12.7/3.4 Hz, 1H), 1.42-1.59 (m, 2H), 1.72-1.90 (m, 5H), 1.92-2.03 (m, 2H), 2.15-2.24 (m, 2H), 2.25 (s, 6H), 2.34 (t, J = 7.2 Hz, 2H), 3.01 (s, 3H), 3.36 (t, J = 7.2 Hz, 2H), 3.78 (s, 3H), 4.17 (tt, J = 11.9/3.7 Hz, 1H), 7.31 (dd, J = 8.5/1.6 Hz, 1H), 7.42 (d, J = 8.5 Hz, 1H), 7.83 (d, J = 1.5 Hz, 1H), 8.01 (s, 1H). MS *m*/*z* ([M+H]⁺) 500.

### Step 2: Methyl 4-Amino-5-(1-cyclohexyl-1H-benzoimidazol-5-yl)-2-[(3-dimethylamino-propyl)-methyl-amino]-thiophene-3-carboxylate (21b)

Compound **(21a)** (75 mg, 0.15 mmol) was dissolved in a mixture of MeOH (4mL) and DCM (4mL). Pd(C) (23 mg, 30% mass) was added and the mixture was stirred under hydrogen atmosphere overnight. The middle was filtered on PTFE filter and concentrated to give compound **(21b)** (70 mg, 0.15 mmol, 100%) as a yellow residue without further purification. MS *m*/*z* ([M+H]⁺) 470.

### Step 3: 7-(1-Cyclohexyl-1H-benzoimidazol-5-yl)-5-[(3-dimethylamino-propyl)-methyl-amino]-3H-thieno[3,4-d]pyrimidin-4-one (Example 21)

According to the procedure described in example 1, step 6, compound **(21b)** (70 mg, 0.15 mmol) was converted, after purification by preparative TLC on silica gel (DCM/MeOH 9/1+2%NH₃ 7M in MeOH), to **(Example 21)** (25 mg, 0.054 mmol, 36%) as a yellow solid. ¹H NMR (400 MHz, DMSO-*d₆*) δ 1.22-1.36 (m, 1H), 1.43-1.57 (m, 2H), 1.68-1.80 (m, 3H), 1.81-1.90 (m, 4H), 2.01-2.08 (m, 2H), 2.10 (s, 6H), 2.20 (t, J = 7.0 Hz, 2H), 3.08 (s, 3H), 3.55-3.61 (m, 2H), 4.29-4.39 (m, 1H), 7.62 (d, J = 8.6 Hz, 1H), 7.65 (d, J = 2.8 Hz, 1H), 7.74 (dd, J = 8.6/1.7 Hz, 1H), 8.21 (d, J = 1.7 Hz, 1H), 8.29 (s, 1H), 11.11 (bs, 1H). (MS *m*/*z* ([M+H]⁺) 465.

### Example 22: Synthesis of 7-(1-Cyclohexyl-1H-benzoimidazol-5-yl)-5-(3-dimethylaminomethyl-pyrrolidin-1-yl)-3H-thieno[3,4-d]pyrimidin-4-one

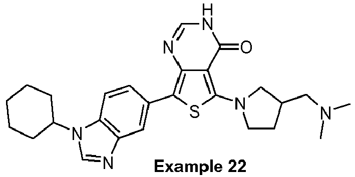

### Step 1: Methyl 5-(1-Cyclohexyl-1H-benzoimidazol-5-yl)-2-(3-dimethylaminomethyl-pyrrolidin-1-yl)-4-nitro-thiophene-3-carboxylate (22a)

According to the procedure described in example 15, step 1, compound **(1c)** (100 mg, 0.238 mmol) was converted, by reaction with dimethyl-pyrrolidin-3-ylmethyl-amine (46 mg, 0.36 mmol) and after purification by preparative TLC on silica gel (DCM/MeOH 92/8), to compound **(22a)** (86 mg, 0.17 mmol, 71%) as an orange solid. ¹H NMR (300 MHz, CDCl₃) δ 1.30 (qt, J = 12.7/3.5 Hz, 1H), 1.41-1.58 (m, 2H), 1.70-1.86 (m, 4H), 1.91-2.04 (m, 3H), 2.10-2.20 (m, 2H), 2.23 (s, 6H), 2.32 (d, J = 7.5 Hz, 2H), 2.48-2.60 (m, 1H), 3.15-3.23 (m, 1H), 3.35-3.54 (m, 3H), 3.76 (s, 3H), 4.16 (tt, J = 11.9/3.7 Hz, 1H), 7.29 (dd, J = 8.5/1.6 Hz, 1H), 7.41 (d, J = 8.5 Hz, 1H), 7.83 (d, J = 1.4 Hz, 1H), 8.00 (s, 1H). MS *m*/*z* ([M+H]⁺) 512.

### Step 2: Methyl 4-Amino-5-(1-cyclohexyl-1H-benzoimidazol-5-yl)-2-(3-dimethylaminomethyl-pyrrolidin-1-yl)-thiophene-3-carboxylate (22b)

According to the procedure described in example 21, step 2, compound **(22a)** (86 mg, 0.17 mmol) was converted to compound **(22b)** (82 mg, 0.17 mmol, 100%) as a yellow residue without further purification. MS *m*/*z* ([M+H]⁺) 482.

### Step 3: 7-(1-Cyclohexyl-1H-benzoimidazol-5-yl)-5-(3-dimethylaminomethyl-pyrrolidin-1-yl)-3H-thieno[3,4-d]pyrimidin-4-one (Example 22)

According to the procedure described in example 1, step 6, compound **(22b)** (82 mg, 0.17 mmol) was converted, after purification by preparative TLC on silica gel (DCM/MeOH 9/1+2%NH₃ 7M in MeOH), to **(Example 22)** (21 mg, 0.044 mmol, 26%) as a yellow solid. ¹H NMR (400 MHz, DMSO-*d₆*) δ 1.22-1.36 (m, 1H), 1.43-1.57 (m, 2H), 1.68-1.78 (m, 2H), 1.79-1.92 (m, 4H), 2.0-2.15 (m, 3H), 2.18 (s, 6H), 2.24-2.38 (m, 2H), 2.52-2.60 (m, 1H), 3.33-3.39 (m, 1H), 3.55-3.69 (m, 3H), 4.28-4.38 (m, 1H), 7.59-7.65 (m, 2H), 7.72-7.77 (m, 1H), 8.18-8.21 (m, 1H), 8.29 (s, 1H), 10.96-11.00 (m, 1H). MS *m*/*z* ([M+H]⁺) 477.

### Example 23: Synthesis of 7-(1-Cyclohexyl-1H-benzoimidazol-5-yl)-5-morpholin-4-yl-3H-thieno[3,4-d]pyrimidin-4-one

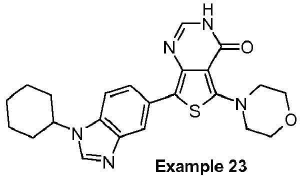

### Step 1: Methyl 5-(1-Cyclohexyl-1H-benzoimidazol-5-yl)-2-morpholin-4-yl-4-nitro-thiophene-3-carboxylate (23a)

According to the procedure described in example 15, step 1, compound **(1c)** (100 mg, 0.238 mmol) was converted, by reaction with morpholine (32 mg, 0.36 mmol) and after purification by preparative TLC on silica gel (DCM/MeOH 92/8), to compound **(23a)** (100 mg, 0.21 mmol, 90%) as an orange solid. ¹H NMR (300 MHz, CDCl₃) δ 1.30 (qt, J = 12.7/3.4 Hz, 1H), 1.41-1.58 (m, 2H), 1.71-1.86 (m, 3H), 1.92-2.02 (m, 2H), 2.15-2.25 (m, 2H), 3.26-3.30 (m, 4H), 3.79 (s, 3H), 3.85-3.89 (m, 4H), 4.17 (tt, J = 11.9/3.7 Hz, 1H), 7.31 (dd, J = 8.5/1.7 Hz, 1H), 7.43 (d, J = 8.5 Hz, 1H), 7.85 (d, J = 1.4 Hz, 1H), 8.02 (s, 1H). MS *m*/*z* ([M+H]⁺) 471.

### Step 2: Methyl 4-Amino-5-(1-cyclohexyl-1H-benzoimidazol-5-yl)-2-morpholin-4-yl-thiophene-3-carboxylate (23b)

According to the procedure described in example 21, step 2, compound **(23a)** (100 mg, 0.21 mmol) was converted to compound **(23b)** (92 mg, 0.21 mmol, 100%) as a yellow residue. MS *m*/*z* ([M+H]⁺) 441.

### Step 3: 7-(1-Cyclohexyl-1H-benzoimidazol-5-yl)-5-morpholin-4-yl-3H-thieno[3,4-d]pyrimidin-4-one (Example 23)

According to the procedure described in example 1, step 6, compound **(23b)** (92 mg, 0.21 mmol) was converted, after purification by preparative TLC on silica gel (DCM/MeOH 9/1+2%NH₃ 7M in MeOH), to **(Example 23)** (10 mg, 0.044 mmol, 11%) as a yellow solid. ¹H NMR (400 MHz, DMSO-*d6*) δ 1.24-1.37 (m, 1H), 1.44-1.58 (m, 2H), 1.70-1.78 (m, 1H), 1.80-1.92 (m, 4H), 2.02-2.10 (m, 2H), 3.36-3.42 (m, 4H), 3.79-3.84 (m, 4H), 4.32-4.41 (m, 1H), 7.67 (d, J = 8.6 Hz, 1H), 7.72 (s, 1H), 7.76 (dd, J = 8.6/1.5 Hz, 1H), 8.26 (d, J = 1.4 Hz, 1H), 8.33 (s, 1H), 11.33 (bs, 1H). MS *m*/*z* ([M+H]⁺) 436.

### Example 24: Synthesis of 7-(1-Cyclohexyl-1H-benzoimidazol-5-yl)-5-(4,4-difluoro-piperidin-1-yl)-3H-thieno[3,4-d]pyrimidin-4-one

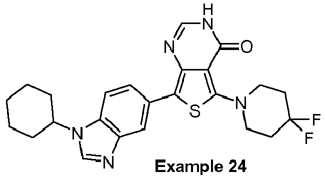

### Step 1: Methyl 5-(1-Cyclohexyl-1H-benzoimidazol-5-yl)-2-(4,4-difluoro-piperidin-1-yl)-4-nitrothiophene-3-carboxylate (24a)

According to the procedure described in example 15, step 1, compound **(1c)** (100 mg, 0.238 mmol) was converted, by reaction with 4,4-Difluoro-piperidine hydrochloride (56 mg, 0.36 mmol) and after purification by preparative TLC on silica gel (DCM/MeOH 92/8), to compound **(24a)** (81 mg, 0.16 mmol, 68%) as an orange solid. ¹H NMR (300 MHz, CDCl₃) δ 1.32 (qt, J = 12.9/3.5 Hz, 1H), 1.32 (qt, J = 13.0/3.4 Hz, 2H), 1.75-1.87 (m, 3H), 1.95-2.03 (m, 2H), 2.15-2.27 (m, 6H), 3.40 (t, J = 5.6 Hz, 4H), 3.82 (s, 3H), 4.19 (tt, J = 11.9/3.7 Hz, 1H), 7.32 (dd, J = 8.5/1.7 Hz, 1H), 7.44 (d, J = 8.5 Hz, 1H), 7.85 (d, J = 1.7 Hz, 1H), 8.04 (s, 1H). MS *m*/*z* ([M+H]⁺) 505.

### Step 2: Methyl 4-Amino-5-(1-cyclohexyl-1H-benzoimidazol-5-yl)-2-(4,4-difluoro-piperidin-1-yl)-thiophene-3-carboxylate (24b)

According to the procedure described in example 21, step 2, compound **(24a)** (81 mg, 0.16 mmol) was converted to compound **(24b)** (76 mg, 0.16 mmol, 100%) as a yellow residue. MS *m*/*z* ([M+H]⁺) 475.

### Step 3: 7-(1-Cyclohexyl-1H-benzoimidazol-5-yl)-5-(4,4-difluoro-piperidin-1-yl)-3H-thieno[3,4-d]pyrimidin-4-one (Example 24)

According to the procedure described in example 1, step 6, compound **(24b)** (76 mg, 0.16 mmol) was converted, after purification by preparative TLC on silica gel (DCM/MeOH 9/1+2%NH3 7M in MeOH in first and DCM/EtOAc/MeOH 45/45/10 in second), to **(Example 24)** (10 mg, 0.021 mmol, 14%) as a yellow solid. ¹H NMR (400 MHz, DMSO-*d₆*) δ 1.27-1.40 (m, 1H), 1.46-1.59 (m, 2H), 1.76-1.88 (m, 3H), 1.95-2.03 (m, 2H), 2.20-2.34 (m, 6H), 3.57 (t, J = 5.6 Hz, 4H), 4.19 (tt, J = 11.98/3.7 Hz, 1H), 7.45 (d, J = 8.5 Hz, 1H), 7.64-7.70 (m, 1H), 7.81-7.86 (m, 1H), 8.01-8.04 (m, 1H), 8.21-8.25 (m, 1H), 9.00 (bs, 1H). (MS *m*/*z* ([M+H]⁺) 470.

### Example 25: Synthesis of 7-(1-Cyclohexyl-1H-benzoimidazol-5-yl)-5-(hexahydro-pyrrolo[3,4-c]pyrrol-2-yl)-3H-thieno[3,4-d]pyrimidin-4-one hydrochloride

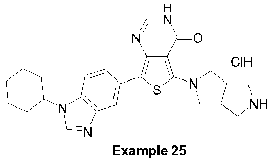

### Step 1: tert-butyl 5-[5-(1-Cyclohexyl-1H-benzoimidazol-5-yl)-3-methoxycarbonyl-4-nitro-thiophen-2-yl]-hexahydro-pyrrolo[3,4-c]pyrrole-2-carboxylate (25a)

According to the procedure described in example 15, step 1, compound **(1c)** (100 mg, 0.238 mmol) was converted, by reaction with tert-butyl hexahydro-pyrrolo[3,4-c]pyrrole-2-carboxylate (76 mg, 0.36 mmol) and after purification by preparative TLC on silica gel (DCM/MeOH 92/8), to compound **(25a)** (105 mg, 0.17 mmol, 75%) as an orange solid. ¹H NMR (300 MHz, CDCl₃) δ 1.24-1.36 (m, 1H), 1.42-1.56 (m, 11H), 1.72-1.85 (m, 3H), 1.92-2.01 (m, 2H), 2.16-2.23 (m, 2H), 2.98-3.06 (m, 2H), 3.22-3.39 (m, 4H), 3.60-3.70 (m, 4H), 3.76 (s, 3H), 4.16 (tt, J = 12.0/3.7 Hz, 1H), 7.30 (dd, J = 8.5/1.7 Hz, 1H), 7.41 (d, J = 8.3 Hz, 1H), 7.83 (d, J = 1.3Hz, 1H), 8.00 (s, 1H). MS *m*/*z* ([M+H]⁺) 596.

### Step 2: tert-butyl 5-[4-Amino-5-(1-cyclohexyl-1H-benzoimidazol-5-yl)-3-methoxycarbonyl-thiophen-2-yl]-hexahydro-pyrrolo[3,4-c]pyrrole-2-carboxylate (25b)

Compound **(25a)** (105 mg, 0.17 mmol) was dissolved in EtOH (4mL). Pd(C) (35 mg, 30% mass) was added and the mixture was stirred under hydrogen atmosphere overnight. The middle was filtered on PTFE filter and concentrated to give compound **(25b)** (96 mg, 0.17 mmol, 100%) as a yellow residue. MS *m*/*z* ([M+H]⁺) 566.

### Step 3: tert-butyl 5-[7-(1-Cyclohexyl-1H-benzoimidazol-5-yl)-4-oxo-3,4-dihydro-thieno[3,4-d]pyrimidin-5-yl]-hexahydro-pyrrolo[3,4-c]pyrrole-2-carboxylate (25c)

According to the procedure described in example 1, step 6, compound **(25b)** (96 mg, 0.17 mmol) was converted, after purification by preparative TLC on silica gel (DCM/MeOH 9/1+2%NH₃ 7M in MeOH in first and DCM/EtOAc/MeOH 45/45/10 in second), to compound **(25c)** (19 mg, 0.037 mmol, 19%) as a yellow solid. ¹H NMR (400 MHz, CDCl₃) δ 1.25-1.40 (m, 1H), 1.46 (s, 9H), 1.46-1.60 (m, 2H), 1.74-1.89 (m, 3H), 1.93-2.03 (m, 2H), 2.18-2.28 (m, 2H), 2.84-2.96 (m, 1H), 3.02-3.12 (m, 1H), 3.28-3.44 (m, 2H), 3.52-3.76 (m, 4H), 3.90-4.00 (m, 2H), 4.12-4.24 (m, 1H), 7.43 (d, J = 8.5 Hz, 1H), 7.58

(d, J = 2.5 Hz, 1H), 7.86 (dd, J = 8.5/1.6 Hz, 1H), 7.99 (s, 1H), 8.16-8.24 (m, 1H), 8.52 (bs, 1H). MS *m*/*z* ([M+H]⁺) 561.

### Step 4: 7-(1-Cyclohexyl-1H-benzoimidazol-5-yl)-5-(hexahydro-pyrrolo[3,4-c]pyrrol-2-yl)-3H-thieno[3,4-d]pyrimidin-4-one hydrochloride (Example 25)

According to the procedure described in example 12, step 4, compound **(25c)** (19 mg, 0.034 mmol) was converted to **(Example 25)** (12 mg, 0.024 mmol, 71%) as a yellow solid. ¹H NMR (400 MHz, CD₃OD) δ 1.42 (qt, J = 13.0/3.5 Hz, 1H), 1.61-1.73 (m, 2H), 1.82-2.08 (m, 5H), 2.31-2.38 (m, 2H), 3.32-3.40 (m, 4H), 3.52-3.59 (m, 2H), 3.62-3.70 (m, 2H), 3.98-4.03 (m, 2H), 4.69-4.78 (m, 1H), 7.98 (dd, J = 8.8/1.4 Hz, 1H), 8.10 (d, J = 8.8 Hz, 1H), 8.23 (s, 1H), 8.33 (d, J = 1.0 Hz, 1H), 9.57 (s, 1H). MS m/z ([M+H]⁺) 461.

### Example 26: Synthesis of N-{(R)-1-[7-(1-cyclohexyl-1H-benzoimidazol-5-yl)-4-oxo-3,4-dihydro-thieno[3,4-d]pyrimidin-5-yl]-pyrrolidin-3-yl}-acetamide

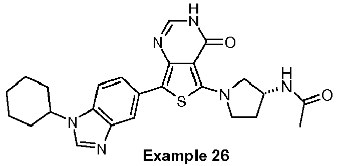

### Step 1: Methyl 2-((R)-3-acetylamino-pyrrolidin-1-yl)-5-(1-cyclohexyl-1H-benzoimidazol-5-yl)-4-nitro-thiophene-3-carboxylate (26a)

According to the procedure described in example 15, step 1, compound **(1c)** (100 mg, 0.238 mmol) was converted, by reaction with (*R*)-N-pyrrolidin-3-yl-acetamide (45.8 mg, 0.36 mmol) and after purification by preparative TLC on silica gel (DCM/MeOH 95/5), to compound **(26a)** as orange oil (75.3 mg, 0.15 mmol, 61%). MS *m*/*z* ([M+H]⁺) 512.

### Step 2: Methyl 2-((R)-3-acetylamino-pyrrolidin-1-yl)-4-amino-5-(1-cyclohexyl-1H-benzoimidazol-5-yl)-thiophene-3-carboxylate (26b)

According to the procedure described in example 1, step 5, compound **(26a)** (75.3 mg 0.15 mmol) was converted to compound **(26b)** as a yellow solid (62.2 mg, 0.13 mmol, 88%) without further purification. MS *m*/*z* ([M+H]⁺) 482.

### Step 3: N-{(R)-1-[7-(1-cyclohexyl-1H-benzoimidazol-5-yl)-4-oxo-3,4-dihydro-thieno[3,4-d]pyrimidin-5-yl]-pyrrolidin-3-yl}-acetamide (Example 26)

According to the procedure described in example 1, step 6, compound **(26b)** (62.2 mg, 0.13 mmol) was converted, after purification by preparative TLC on silica gel (DCM/MeOH 9/1 + 2% NH₃), to **(Example 26)** as a yellow solid (13 mg, 0.03 mmol, 21%). ¹H NMR (400 MHz, DMSO-d₆) δ 1.23-1.55 (m, 1H), 1.45-1.55 (m, 2H), 1.71-1.74 (m, 1H), 1.80-1.98 (m, 8H), 2.03-2.06 (m, 2H), 2.16-2.26 (m, 1H), 3.44 (dd, J = 4.3/11.2 Hz, 1H), 3.53-3.59 (m, 1H), 3.67-3.73 (m, 1H), 3.64-3.88 (m, 1H), 4.30-4.38 (m, 2H), 7.62 (d, J = 8.6 Hz, 1H), 7.65 (d, J = 3.3 Hz, 1H), 7.75 (dd, J = 1.6/8.5 Hz, 1H), 8.19-8.22 (m, 2H), 8.30 (s, 1H), 11.02 (d, J = 3.3 Hz, 1H). MS *m*/*z* ([M+H]⁺) 477.

### Example 27: Synthesis of N-{(S)-1-[7-(1-cyclohexyl-1H-benzoimidazol-5-yl)-4-oxo-3,4-dihydro-thieno[3,4-d]pyrimidin-5-yl]-pyrrolidin-3-yl}-acetamide

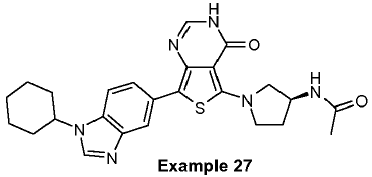

### Step 1: Methyl 2-((S)-3-acetylamino-pyrrolidin-1-yl)-5-(1-cyclohexyl-1H-benzoimidazol-5-yl)-4-nitro-thiophene-3-carboxylate (27a)

According to the procedure described in example 15, step 1, compound (1c) (100 mg, 0.238 mmol) was converted, by reaction with (S)-N-pyrrolidin-3-yl-acetamide (45.8 mg, 0.36 mmol) and after purification by preparative TLC on silica gel (DCM/MeOH 95/5), to compound (27a) as orange oil (109.3 mg, 0.21 mmol, 89%). MS m/z ([M+H]⁺) 512.

### Step 2: Methyl 2-((S)-3-acetylamino-pyrrolidin-l-yl)-4-amino-5-(1-cyclohexyl-1H-benzoimidazol-5-yl)-thiophene-3-carboxylate (27b)

According to the procedure described in example 21, step 2, compound **(27a)** (109 mg 0.15 mmol) was converted to compound **(27b)** as a yellow solid (84.8 mg, 0.18 mmol, 83%). MS *m*/*z* ([M+H]⁺) 482.

### Step 3: N-{(S)-1-[7-(1-cyclohexyl-1H-benzoimidazol-5-yl)-4-oxo-3,4-dihydro-thieno [3,4-d]pyrimidin-5-yl]-pyrrolidin-3-yl}-acetamide (Example 27)

According to the procedure described in example 1, step 6, compound **(27b)** (84.8 mg, 0.18 mmol) was converted, after purification by preparative TLC on silica gel (DCM/MeOH 9/1 + 2% NH₃), to **(Example 27)** as a yellow solid (14.6 mg, 0.03 mmol, 17%). ¹H NMR (400 MHz, DMSO-*d₆*) δ 1.24-1.34 (m, 1H), 1.45-1.55 (m, 2H), 1.71-1.74 (m, 1H), 1.82-1.98 (m, 8H), 2.04-2.06 (m, 2H), 2.18-2.26 (m, 1H), 3.44 (dd, J = 4.3/11.2 Hz, 1H), 3.57 (s, 1H), 3.67-3.73 (m, 1H), 3.86 (dd, J = 6.3/11.2 Hz, 1H), 4.31-4.37 (m, 2H), 7.61-7.65 (m, 2H), 7.75 (dd, J = 1.6/8.6 Hz, 1H), 8.19-8.22 (m, 2H), 8.30 (s, 1H), 11.03 (d, J = 3.2 Hz, 1H). MS *m*/*z* ([M+H]⁺) 477.

### Example 28: Synthesis of 7-(1-cyclohexyl-1H-benzoimidazol-5-yl)-5-piperazin-1-yl-3H-thieno[3,4-d]pyrimidin-4-one hydrochloride

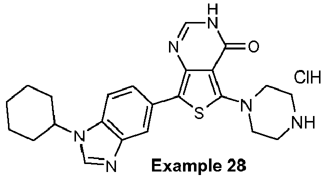

### Step 1: tert-butyl 4-[5-(1-cyclohexyl-1H-benzoimidazol-5-yl)-3-methoxycarbonyl-4-nitro-thiophen-2-yl]-piperazine-1-carboxylate (28a)

According to the procedure described in example 15, step 1, compound **(1c)** (100 mg, 0.238 mmol) was converted, by reaction with piperazine-1-carboxylic acid tert-butyl ester (66.5 mg, 0.36 mmol) and after purification by preparative TLC on silica gel (DCM/MeOH 95/5), to compound **(28a)** (94.8 mg, 0.17 mmol, 84%). MS *m*/*z* ([M+H]⁺) 570.

### Step 2: tert-butyl 4-[4-amino-5-(1-cyclohexyl-1H-benzoimidazol-5-yl)-3-methoxycarbonyl-thiophen-2-yl]-piperazine-1-carboxylate (28b)

According to the procedure described in example 1, step 5, compound **(28a)** (94.8 mg, 0.17 mmol) was converted to compound **(28b)** as a yellow solid (81.4 mg, 0.15 mmol, 90%). MS *m*/*z* ([M+H]⁺) 540.

### Step 3: tert-butyl 4-[7-(1-cyclohexyl-1H-benzoimidazol-5-yl)-4-oxo-3,4-dihydro-thieno[3,4-d]pyrimidin-5-yl]-piperazine-1-carboxylate (28c)

According to the procedure described in example 1, step 6, compound **(28b)** (81.4 mg, 0.15 mmol) was converted, after purification by preparative TLC on silica gel (DCM/MeOH 9/1 + 2% NH₃), to compound **(28c)** as a yellow oil (79 mg, 0.15 mmol, 98%). MS *m*/*z* ([M+H]⁺) 535.

### Step 4: 7-(1-cyclohexyl-1H-benzoimidazol-5-yl)-5-piperazin-1-yl-3H-thieno[3,4-d]pyrimidin-4-one hydrochloride (Example 28)

According to the procedure described in example 12, step 4, compound **(28c)** (81 mg, 0.15 mmol) was converted to **(Example 28)** as a yellow solid (55.4 mg, 0.12 mmol, 86%). ¹H NMR (400 MHz, DMSO-*d₆*) δ 1.24-1.34 (m, 1H), 1.49-1.58 (m, 2H), 1.73-1.76 (m, 1H), 1.76-1.92 (m, 4H), 2.17-2.20 (m, 2H), 3.33 (bs, 4H), 3.64-6.67 (m, 4H), 4.65-4.70 (m, 1H), 7.83 (d, J = 3.2 Hz, 1H), 8.06 (dd, J = 1.6/8.8 Hz, 1H), 8.13 (d, J = 8.8 Hz, 1H), 8.53 (d, J = 1.1 Hz, 1H), 9.36 (bs, 2H) 9.66 (s, 1H), 11.66 (d, J = 3.0 Hz, 1H). MS *m*/*z* ([M+H]⁺) 435.

### Example 29: Synthesis of 7-(1-cyclohexyl-1H-benzoimidazol-5-yl)-5-piperazin-1-yl-3H-thieno[3,4-d]pyrimidin-4-one hydrochloride

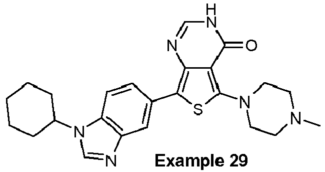

### Step 1: Methyl 5-(1-cyclohexyl-1H-benzoimidazol-5-yl)-2-(4-methyl-piperazin-1-yl)-4-nitrothiophene-3-carboxylate (29a)

According to the procedure described in example 15, step 1, compound **(1c)** (150 mg, 0.36 mmol) was converted, by reaction with 1-methyl-piperazine (51 µg, 0.46 mmol) and after purification by preparative TLC on silica gel (DCM/MeOH 95/5), to compound **(29a)** (112 mg, 0.23 mmol, 58%). MS *m*/*z* ([M+H]⁺) 484.

### Step 2: Methyl 4-amino-5-(1-cyclohexyl-1H-benzoimidazol-5-yl)-2-(4-methyl-piperazin-1-yl)-thiophene-3-carboxylate (29b)

According to the procedure described in example 1, step 5, compound **(29a)** (112 mg, 0.23 mmol) was converted to compound **(29b)** as brown oil (60 mg, 0.13 mmol, 57%). MS *m*/*z* ([M+H]⁺) 454.

### Step 3: 7-(1-cyclohexyl-1H-benzoimidazol-5-yl)-5-(4-methyl-piperazin-1-yl)-3H-thieno[3,4-d]pyrimidin-4-one (Example 29)

According to the procedure described in example 1, step 6, compound **(29b)** (60 mg, 0.13 mmol) was converted, after purification by preparative TLC on silica gel (DCM/MeOH 85/15), to **(Example 29)** as a yellow solid (29.8 mg, 0.07 mmol, 50%). ¹H NMR (400 MHz, DMSO-*d₆*) δ 1.28-1.34 (m, 1H), 1.45-1.55 (m, 2H), 1.71-1.74 (m, 1H), 1.84-1.89 (m 4H), 2.04-2.06 (m, 2H), 2.26 (s, 3H), 2.52 (m, 5H), 3.39 (bs, 3H), 4.35 (tt, J = 3.6/11.6 Hz, 1H) 7.64-7.70 (m, 2H), 7.75 (dd, J = 1.6/8.6 Hz, 1H), 8.24 (d, J = 1.5 Hz 1H), 8.32 (s, 1H), 11.28 (d, J = 3.3 Hz, 1H). MS *m*/*z* ([M+H]⁺) 449.

### Example 30: Synthesis of 7-(1-Cyclohexyl-1H-benzimidazol-5-yl)-5-((1R,5S,6R)-6-hydroxymethyl-3-aza-bicyclo[3.1.0]hex-3-yl)-3H-thieno[3,4-d]pyrimidin-4-one

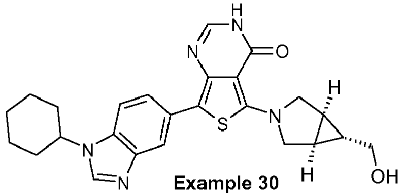

### Step 1: Methyl 5-(1-Cyclohexyl-1H-benzimidazol-5-yl)-2-((1R,5S,6R)-6-hydroxymethyl-3-aza-bicyclo[3.1.0]hex-3-yl)-4-nitro-thiophene-3-carboxylate (30a)

According to the procedure described in example 15, step 1, compound **(1c)** (150 mg, 0.36 mmol) was converted, by reaction with (1R,5S,6R)-3-azabicyclo[3.1.0]hexan-6-ylmethanol (61 mg, 0.54 mmol) and after purification by preparative TLC on silica gel (DCM/MeOH 95/5), to compound **(30a)** as a red oil (104 mg, 0.21 mmol, 58%). MS *m*/*z* ([M+H]⁺) 497.

### Step 2: Methyl 4-Amino-5-(1-cyclohexyl-1H-benzimidazol-5-yl)-2-((1R,5S,6R)-6-hydroxymethyl-3-aza-bicyclo[3.1.0]hex-3-yl)-thiophene-3-carboxylate (30b)

According to the procedure described in example 21, step 2, compound **(30a)** (103 mg, 0.21 mmol) was converted to compound **(30b)** as a yellow solid (90 mg, 0.19 mmol, 93%). MS *m*/*z* ([M+H]⁺) 467.

### Step 3: 7-(1-Cyclohexyl-1H-benzimidazol-5-yl)-5-((1R,5S,6R)-6-hydroxymethyl-3-aza-bicyclo[3.1.0]hex-3-yl)-3H-thieno[3,4-d]pyrimidin-4-one (Example 30)

According to the procedure described in example 1, step 6, compound **(30b)** (90 mg, 0.19 mmol) was converted, after purification by preparative TLC on silica gel (DCM/MeOH 9/1 + 2% NH₃), to **(Example 30)** as an orange solid (24.5 mg, 0.05 mmol, 27%). ¹H NMR (400 MHz, DMSO-*d₆*) δ 0.90-0.95 (m, 1H), 1.26-1.34 (m, 1H), 1.45-1.55 (m, 2H), 1.63 (bs, 2H), 1.71-1.74 (m, 1H), 1.83-1.88 (m, 4H), 2.04-2.06 (m, 2H), 3.33-3.34 (t, J = 6.0 Hz, 2H), 3.47-3.50 (d, J = 10.1 Hz, 2H), 4.01 (d, J = 10.3 Hz, 2H), 4.34 (tt, J = 3.9/11.6 Hz, 1H), 4.56 (t, J = 5.5 Hz, 1H), 7.61-7.64 (m, 2H), 7.73 (dd, J = 1.6/8.6 Hz, 1H), 8.19 (d, J = 1.3 Hz, 1H), 8.30 (s, 1H), 11.08 (d, J = 3.3 Hz, 1H). MS *m*/*z* ([M+H]⁺) 462.

### Example 31: Synthesis of 7-(1-cyclohexyl-1H-benzoimidazol-5-yl)-5-((S)-2-hydroxymethyl-pyrrolidin-1-yl)-3H-thieno[3,4-d]pyrimidin-4-one

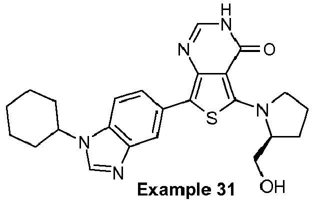

### Step 1: Methyl 5-(1-cyclohexyl-1H-benzoimidazol-5-yl)-2-((S)-2-hydroxymethyl-pyrrolidin-1-yl)-4-nitro-thiophene-3-carboxylate (31a)

According to the procedure described in example 15, step 1, compound **(1c)** (150 mg, 0.36 mmol) was converted, by reaction with (*S*)-2-pyrrolidimethanol (47 mg, 0.46 mmol) and after purification by preparative TLC on silica gel (DCM/MeOH 95/5), to compound **(31a)** as an orange solid (104.8 mg, 0.22 mmol, 60%). MS *m*/*z* ([M+H]⁺) 485.

### Step 2: Methyl 2-[(S)-2-(tert-Butyl-dimethyl-silanyloxymethyl)-pyrrolidin-1-yl]-5-(1-cyclohexyl-1H-benzoimidazol-5-yl)-4-nitro-thiophene-3-carboxylate (31b)

To a solution of compound **(31a)** (96 mg, 0.20 mmol) in anhydrous DCM (300 µL) and MeOH (50 µL), were added imidazole (34 mg, 0.50 mmol), *tert*-butyldimethylsilyl chloride (36 mg, 0.24 mmol). The reaction mixture was stirred at room temperature under nitrogen atmosphere for 24 hours. Imidazole (6.7 mg, 0.10 mmol) and *tert*-butyldimethylsilyl chloride (15 mg, 0.10 mmol) were added maintaining the stirring overnight. DCM and brine were added to the middle and the aqueous layer was extracted twice with DCM. The organic layer was dried over sodium sulfate and concentrated under reduced pressure. The crude was purified by preparative TLC (DCM/MeOH 95/5) to provide compound **(31b)** as a yellow solid (76 mg, 0.13 mmol, 64%). MS *m*/*z* ([M+H]⁺) 599.

### Step 3: Methyl 4-amino-5-(1-cyclohexyl-1H -benzoimidazol-5-yl)-2-((S)-2-hydroxymethyl-pyrrolidin-1-yl)-thiophene-3-carboxylate (31c)

According to the procedure described in example 1, step 5, compound **(31b)** (76 mg, 0.13 mmol) was converted to compound **(31c)** as a yellow solid (72 mg, 0.16 mmol, 100%). MS *m*/*z* ([M+H]⁺) 569.

### Step 4: 7-(1-cyclohexyl-1H-benzoimidazol-5-yl)-5-((S)-2-hydroxymethyl-pyrrolidin-1-yl)-3H-thieno[3,4-d]pyrimidin-4-one (31d)

According to the procedure described in example 1, step 6, compound **(31c)** (72 mg, 0.16 mmol) was converted, after purification by preparative TLC (DCM/MeOH 95/5), to compound **(31d)** as a yellow solid (25 mg, 0.04 mmol, 35%). MS *m*/*z* ([M+H]⁺) 564.

### Step 5: 7-(1-cyclohexyl-1H-benzoimidazol-5-yl)-5-((S)-2-hydroxymethyl-pyrrolidin-1-yl)-3H-thieno[3,4-d]pyrimidin-4-one (Example 31)

To a solution compound **(31d)** (25 mg, 0.04 mmol) in anhydrous THF (300 µL) at 0°C under nitrogen atmosphere, was added TBAF solution 1M in THF (66 µL, 0.07 mmol). The reaction mixture was stirred during 3 hours before concentrated under reduced pressure. The crude was purified by preparative TLC (DCM/MeOH 95/5) to provide **(Example 31)** as a yellow solid (19 mg, 0.04 mmol 95%). ¹H NMR (400 MHz, DMSO-*d₆*) δ 1.25-1.35 (m, 2H), 1.46-1.56 (m, 2H), 1.72-1.76 (m, 1H), 1.84-1.92 (m, 4H), 1.99-2.09 (m 5H), 3.30-3.33 (m, 1H), 3.43-3.49 (m, 2H), 3.92-3.99 (m, 1H), 4.31-4.40 (m, 2H), 4.78 (t, J = 5.3 Hz, 1H), 7.62-7.65 (m, 2H), 7.75 (dd, J = 1.7/8.6 Hz, 1H), 8.20 (d, J = 1.3 Hz, 1H), 8.30 (s, 1H), 11.21 (d, J = 3.3 Hz, 1H). MS *m*/*z* ([M+H]⁺) 450.

### Example 32: Synthesis of 7-(1-Cyclohexyl-1H-benzimidazol-5-yl)-5-[(2-dimethylamino-ethyl)-methyl-amino]-3H-thieno[3,4-d]pyrimidin-4-one

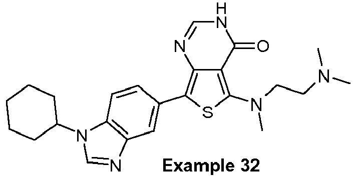

### Step 1: Methyl 5-(1-cyclohexyl-1H-benzoimidazol-5-yl)-2-[(2-dimethylamino-ethyl)-methyl-amino]-4-nitro-thiophene-3-carboxylate (32a)

According to the procedure described in example 15, step 1, compound **(1c)** (150 mg, 0.36 mmol) was converted, by reaction with N,N,N'-trimethyl-ethane-1,2-diamine (70 µL, 0.54 mmol) and after purification by preparative TLC on silica gel (DCM/MeOH 95/5), to compound **(32a)** (165 mg, 0.34 mmol, 100%). MS *m*/*z* ([M+H]⁺) 486.

### Step 2: Methyl 4-amino-5-(1-cyclohexyl-1H-benzoimidazol-5-yl)-2-[(2-dimethylamino-ethyl)-methyl-amino]-thiophene-3-carboxylate (32b)

According to the procedure described in example 21, step 2, compound **(32a)** (165 mg, 0.34 mmol) was converted to compound **(32b)** as yellow oil (155 mg, 0.34 mmol, 100%). MS *m*/*z* ([M+H]⁺) 456.

### Step 3: 7-(1-Cyclohexyl-1H-benzimidazol-5-yl)-5-[(2-dimethylamino-ethyl)-methyl-amino]-3H-thieno[3,4-d]pyrimidin-4-one (Example 32)

According to the procedure described in example 1, step 6, compound **(32b)** (155 mg, 0.34 mmol) was converted, after purification by preparative TLC (DCM/MeOH 9/1 + 0.1% NH₃ 0.5M in 1,4-dioxane), to **(Example 32)** as orange solid (3.9 mg, 0.009 mmol, 2%). ¹H NMR (400 MHz, DMSO-*d6*) δ 1.27-1.34 (m, 1H), 1.45-1.58 (m, 2H), 1.74-1.85 (m, 3H), 1.96-2.0 (m, 2H), 2.20-2.24 (m, 2H), 2.80 (s, 6H), 3.12 (s, 3H), 3.39 (t, J = 6.8 Hz, 2H), 3.96 (t, J = 6.8 Hz, 2H), 4.19 (tt, J = 3.6/11.8 Hz, 1H), 7.43 (d, J = 8.6 Hz, 1H), 7.66 (s, 1H), 7.77 (dd, J = 1.4/8.5 Hz, 1H), 8.01 (s, 1H), 8.22 (s, 1H), 9.54 (bs, 1H). MS *m*/*z* ([M+H]⁺) 451.

### Example 33: Synthesis of 7-(1-cyclohexyl-1H-benzoimidazol-5-yl)-5-((S)-2-hydroxymethyl-pyrrolidin-l-yl)-3H -thienor3_{.}4-dlpyrimidin-4-one

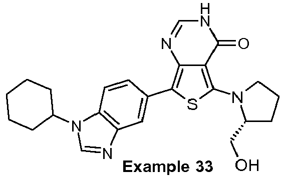

### Step 1: (R)-2-(tert-butyl-dimethyl-silanyloxymethyl)-pyrrolidine (33a)

To a solution of (*R*)-1-pyrrolidin-2-yl-methanol (230 mg, 1.48 mmol) in anhydrous DCM (650 µL) at 0°C under nitrogen atmosphere, were added triethylamine (413 µL, 2.97 mmol) and *tert-*butyldimethylsilyl chloride (223 mg, 1.48 mmol). The reaction mixture was stirred at room temperature overnight. A saturated solution of ammonium chloride was added and the aqueous layer was extracted twice with dichloromethane. The organic layer was dried over sodium sulfate and concentrated under reduced pressure to provide compound **(33a)** as oil (223 mg, 1.03 mmol, 45%).

### Step 2: Methyl 2-[(R)-2-(tert-Butyl-dimethyl-silanyloxymethyl)-pyrrolidin-1-yl]-5-(1-cyclohexyl-1H-benzimidazol-5-yl)-4-nitro-thiophene-3-carboxylate (33b)

According to the procedure described in example 15, step 1, compound **(1c)** (360 mg, 0.86 mmol) was converted, by reaction with compound **(33a)** (212 mg, 1.03 mmol) and after purification by flash chromatography on silica gel (DCM/MeOH 95/5 then cyclohexane/EtOAc 7/3), to compound **(33b)** as an orange solid (217 mg, 0.36 mmol, 42%). MS *m*/*z* ([M+H]⁺) 599.

### Step 3: Methyl 4-Amino-2-[(R)-2-(tert-butyl-dimethyl-silanyloxymethyl)-pyrrolidin-1-yl]-5-(1-cyclohexyl-1H-benzimidazol-5-yl)-thiophene-3-carboxylate (33c)

According to the procedure described in example 1, step 5, compound **(33b)** (217 mg, 0.362 mmol) was converted to compound **(33c)** as a yellow solid (179 mg, 0.32 mmol, 87%). MS *m*/*z* ([M+H]⁺) 569.

### Step 4: 5-[(R)-2-(tert-Butyl-dimethyl-silanyloxymethyl)-pyrrolidin-1-yl]-7-(1-cyclohexyl-1H-benzimidazol-5-yl)-3H-thieno[3,4-d]pyrimidin-4-one (33d)

According to the procedure described in example 1, step 6, compound **(33c)** (178 mg, 0.32 mmol) was converted, after purification by preparative TLC (DCM/MeOH 95/5), to compound **(33d)** as a yellow solid (59 mg, 0.10 mmol, 34%). ¹H RMN (400 MHz, DMSO-*d6*) δ -0.15 (s, 3H), -0.09 (s, 3H), 0.79 (s, 9H), 1.25-1.35 (m, 1H), 1.45-1.55 (m, 2H), 1.72 (d, J = 12.6 Hz, 1H), 1.80-2.06 (m, 9H), 2.11-2.18 (m, 1H), 3.53 (dd, J = 3.2/10.6 Hz, 1H), 3.66-3.80 (m, 2H), 4.30-4.37 (m, 2H), 4.59-4.64 (m, 1H), 7.61-7.64 (m, 2H), 7.72 (dd, J = 1.6/8.6 Hz, 1H), 8.16 (d, J = 1.4 Hz 1H), 8.30 (s, 1H), 11.02 (d, J = 3.2 Hz, 1H). MS *m*/*z* ([M+H]⁺) 564.

### Step 5: 7-(1-Cyclohexyl-1H-benzimidazol-5-yl)-5-((R)-2-hydroxymethyl-pyrrolidin-1-yl)-3H-thieno[3,4-d]pyrimidin-4-one (Example 33)

According to the procedure described in example 31, step 5, compound **(33d)** (58 mg, 0.10 mmol) was converted, after purification by preparative TLC (DCM/MeOH 9/1), to **(Example 33)** as a yellow solid (39 mg, 0.09 mmol, 84%). ¹H RMN (400 MHz, DMSO-*d₆*) δ 1.25-1.35 (m, 1H), 1.46-1.56 (m, 2H), 1.73 (d, J = 11.6 Hz, 1H), 1.80-1.92 (m, 5H), 1.98-2.16 (m, 5H), 3.30-3.33 (m, 1H), 3.42-3.51 (m, 2H), 3.92-3.98 (m, 1H), 4.31-4.40 (m, 2H), 4.78 (t, J = 5.3 Hz, 1H), 7.62-7.65 (m, 2H), 7.75 (dd, J = 1.7/8.6 Hz, 1H), 8.20 (d, J = 1.3 Hz 1H), 8.30 (s, 1H), 11.01 (d, J = 3.3 Hz, 1H). MS m/z ([M+H]+) 450.

### Example 34: Synthesis of 7-(1-cyclohexyl-1H-benzoimidazol-5-yl)-5-hydroxymethyl-hexahydro-pyrrolo[3,4-c]pyrrol-2-yl)-3H-thieno[3,4-d]pyrimidin-4-one hydrochloride

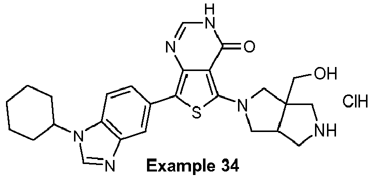

### Step 1: tert-butyl 5-benzyl-3a-hydroxymethyl-hexahydro-pyrrolo[3,4-c]pyrrole-2-carboxylate (34a)

To a solution of (2-benzyl-hexahydro-pyrrolo[3,4-c]pyrrol-3a-yl)-methanol (200 mg, 0.86 mmol) in anhydrous DCM (1 mL) at room temperature under nitrogen atmosphere, was added Boc₂O (281 mg, 1.29 mmol). The reaction mixture was stirred for 2 hours and concentrated under reduced pressure. The residue was purified by flash chromatography on silica gel (EtOAc) to provide compound **(34a)** as uncolored oil (155 mg, 0.47 mmol, 54%). MS m/z ([M+H]⁺) 333.

### Step 2: tert-butyl 3a-hydroxymethyl-hexahydro-pyrrolo[3,4-c]pyrrole-2-carboxylate (34b)

To a suspension of compound **(34a)** (155 mg, 0.47 mmol) in anhydrous MeOH (2.2 mL) and DCM (550 µL) with palladium on carbon (31 mg), was added acetic acid (14 µL, 0.49 mmol). The reaction mixture was stirred overnight at room temperature under hydrogen atmosphere. The mixture was filtered over Millipore and concentrated under reduced pressure to provide compound **(34b)** (113 mg, 0.78 mmol, 100%). ¹H NMR (400 MHz, DMSO-*d₆*) δ 1.38 (s, 9H), 2.38 (bs, 1H), 2.50-2.54 (m, 2H), 2.63 (dd, J = 4.9/11.2 Hz, 1H), 2.70 (d, J = 11.3 Hz, 1H), 2.86 (d, J = 11.3 Hz, 1H), 3.34 (dd, J = 7.6/11.2 Hz, 1H), 3.09-3.17 (m, 2H), 3.31-3.34 (m, 3H), 3.44 (t, J = 9.5 Hz, 1H), 2H under solvent. MS *m*/*z* ([M+H]⁺) 243.

### Step 3: tert-butyl 5-[5-(1-cyclohexyl-1H-benzoimidazol-5-yl)-3-methoxycarbonyl-4-nitro-thiophen-2-yl]-3a-hydroxymethyl-hexahydro-pyrrolo[3,4-c]pyrrole-2-carboxylate (34c)

According to the procedure described in example 16, step 1, compound (1c) (150 mg, 0.58 mmol) was converted, by reaction with compound **(34b)** (113 mg, 0.76 mmol) and after purification by preparative TLC (DCM/MeOH 95/5), to compound **(34c)** as an orange oil (160 mg, 0.26 mmol, 71%). MS m/z ([M+H]⁺) 626.

### Step 4: tert-butyl 5-[4-amino-5-(1-cyclohexyl-1H-benzoimidazol-5-yl)-3-methoxycarbonyl-thiophen-2-yl]-3a-hydroxymethyl-hexahydro-pyrrolo[3,4-c]pyrrole-2-carboxylate (34d)

According to the procedure described in example 1, step 5, compound **(34c)** (160 mg, 0.26 mmol) was converted to compound **(34d)** (125 mg, 0.21 mmol, 82%) without further purification. MS *m*/*z* ([M+H]⁺) 596.

### Step 5: tert-butyl 5-[7-(1-cyclohexyl-1H-benzoimidazol-5-yl)-4-oxo-3,4-dihydro-thieno[3,4-d]pyrimidin-5-yl]-3a-hydroxymethyl-hexahydro-pyrrolo[3,4-c]pyrrole-2-carboxylate (34e)

According to the procedure described in example 1, step 6, compound **(34d)** (125 mg, 0.21 mmol) was converted, after purification by preparative TLC (DCM/MeOH 95/5), to compound **(34e)** as a yellow solid (34 mg, 0.06 mmol, 27%). MS *m*/*z* ([M+H]⁺) 591.

### Step 6: 7-(1-cyclohexyl-1H-benzoimidazol-5-yl)-5-hydroxymethyl-hexahydro-pyrrolo[3,4-c]pyrrol-2-yl)-3H-thieno[3,4-d]pyrimidin-4-one hydrochloride (Example 34)

According to the procedure described in example 12, step 4, compound **(34e)** (34 mg, 0.06 mmol) was converted, after purification by preparative TLC (DCM/MeOH 95/5), to **(Example 34)** as a yellow solid (25 mg, 0.05 mmol, 77%). ¹H NMR (400 MHz, DMSO-*d₆*) δ 1.24-1.34 (m, 1H), 1.49-1.59 (m, 2H), 1.73-1.76 (m, 1H), 1.82-1.92 (m, 4H), 2.17-2.20 (m, 2H), 2.85-2.91 (m, 1H), 3.18-3.24 (m, 1H), 3.46-3.56 (m, 4H), 3.67 (dd, J = 7.5/10.6 Hz, 1H), 3.81-3.89 (m, 4H), 4.64-4.69 (t, J = 11.6 Hz, 1H), 7.79 (d, J = 3.3 Hz, 1H), 8.04 (dd, J = 1.5/8.9 Hz, 1H), 8.10 (d, J = 8.9 Hz, 1H), 8.50 (d, J = 1.2 Hz, 1H), 9.31 (bs, 1H), 9.43 (bs, 1H), 9.64 (s, 1H), 11.46 (d, J = 3.3 Hz, 1H). MS *m*/*z* ([M+H]⁺) 491.

### Example 35: Synthesis of 5-((3R,4R)-3,4-Bis-hydroxymethyl-pyrrolidin-1-yl)-7-(1-cyclohexyl-1H-benzoimidazol-5-yl)-3H-thieno[3,4-d]pyrimidin-4-one

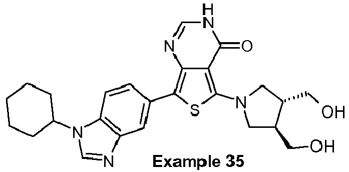

### Step 1: Methyl 2-((3R,4R)-3,4-Bis-hydroxymethyl-pyrrolidin-1-yl)-5-(1-cyclohexyl-1H-benzoimidazol-5-yl)-4-nitro-thiophene-3-carboxylate (35a)

According to the procedure described in example 15, step 1, compound **(1c)** (150 mg, 0.36 mmol) was converted, by reaction with ((*3R,4R*)-4-hydroxymethyl-pyrrolidin-3-yl)-methanol hydrochloride (90 mg, 0.54 mmol) and after purification by preparative TLC (DCM/MeOH 95/5), to compound **(35a)** as a red oil (127 mg, 0.25 mmol, 69%). MS *m*/*z* ([M+H]⁺) 515.

### Step 2: Methyl 4-amino-2-((3R,4R)-3,4-bis-hydroxymethyl-pyrrolidin-1-yl)-5-(1-cyclohexyl-1H-benzoimidazol-5-yl)-thiophene-3-carboxylate (35b)

According to the procedure described in example 1, step 5, compound **(35a)** (127 mg, 0.25 mmol) was converted to compound **(35b)** as yellow solid (109 mg, 0.22 mmol, 92%). MS *m*/*z* ([M+H]⁺) 485.

### Step 3: 5-((3R,4R)-3,4-Bis-hydroxymethyl-pyrrolidin-1-yl)-7-(1-cyclohexyl-1H-benzoimidazol-5-yl)-3H-thieno[3,4-d]pyrimidin-4-one (Example 35)

According to the procedure described in example 1, step 6, compound **(35b)** (109 mg, 0.22 mmol) was converted, after purification by preparative TLC (DCM/MeOH 9/1), to **(Example 35)** as an orange solid (26 mg, 0.05 mmol, 24%). ¹H NMR (400 MHz, DMSO-*d₆*) δ 1.26-1.34 (m 1H), 1.45-1.55 (m, 2H), 1.71-1.74 (m, 1H), 1.82-1.89 (m, 4H), 2.04-2.07 (m, 2H), 2.23-2.29 (m, 2H), 3.39-3.54 (m, 6H), 3.71 (dd, J = 6.8/10.6 Hz, 2H), 4.32-4.38 (m, 1H), 4.80 (t, J = 4.8 Hz, 2H), 7.62-7.65 (m, 2H), 7.75 (dd, J = 1.6/8.6 Hz, 1H), 8.21 (d, J = 1.5 Hz, 1H), 8.34 (s, 1H), 11.0 (d, J = 3.2 Hz, 1H). MS m/z ([M+H]⁺) 480.

### Example 36: Synthesis of 7-(1-cyclohexyl-1H-benzoimidazol-5-yl)-5-(4-hydroxy-hexahydro-cyclopenta[c]pyrrol-2-yl)-3H-thieno[3,4-d]pyrimidin-4-one

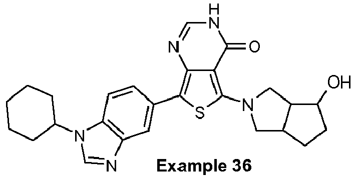

### Step 1: 2-Benzyl-hexahydro-cyclopenta[c]pyrrol-4-one (36a)

To the solution of 2-cyclopenten-1-one (3.06 mL, 36.5 mmol) and N-Benzyl-N-(methoxymethyl)-N-trimethylsilylmethylamine (18.7 mL, 43.8 mmol) in DCM (75 mL) was added 1M TFA in DCM (3.5 mL) at 0°C. Reaction was slowly allowed to obtain room temperature. After 2 hours, saturated NaHCO₃ was added. Organic phase was separated and water phase was extracted twice with DCM. Combined organic phases were dried with sodium sulfate and concentrated to give compound **(36a)** without further purification (7.88 g, 36.5 mmol, 100%). MS *m*/*z* ([M+H]⁺) 216.

### Step 2: Benzyl 4-Oxo-hexahydro-cyclopenta[c]pyrrole-2-carboxylate (36b)

To a solution of compound **(36a)** (7.90 g, 36.7 mmol) in DCM (253 mL) was added benzyl chloroformate (13.1 mL, 55 mmol) at 0°C. After 5 hours, solvents were evaporated and mixture was purified by flash chromatography (Hexane/EtOAc 7/4) to give compound **(36b)** (7.27 g, 22 mmol, 60%). ¹H NMR (300 MHz, CDCl₃) δ 1.79-1.94 (m, 1H), 2.11-2.25 (m, 1H), 2.35 (d, J = 7.4 Hz, 1H), 2.37 (dd, J = 1.1/6.9 Hz, 1H), 2.76 (dt, J = 3.8/8.4 Hz, 1H), 2.99-3.10 (m, 1H), 3.16-3.26 (m, 1H), 3.54-3.67 (m, 1H), 3.70 (dd, J = 3.8/11.4 Hz, 1H), 3.68-3.80 (m, 1H), 5.11 (d, J = 2.3 Hz, 2H), 7.28-7.39 (m, 5H). MS *m*/*z* ([M+Na]⁺) 282.

### Step 3: Benzyl 4-Hydroxy-hexahydro-cyclopenta[c]pyrrole-2-carboxylate (36c)

According to the procedure described in example 18, step 1, compound **(36b)** (7.27 g, 22 mmol) was converted, after purification by flash chromatography (Hexane/EtOAc 8/2), to compound **(36c)** (6.13 g, 23.5 mmol, 84%). ¹H NMR (300 MHz, DMSO-*d₆*) δ 1.38-1.48 (m, 1H), 1.51-1.63 (m, 1H), 1.64-1.80 (m, 2H), 2.53-2.67 (m, 2H), 3.04-3.12 (m, 1H), 3.19-3.31 (m, 1H), 3.45-3.64 (m, 2H), 4.00-4.08 (m, 1H), 4.75 (d, J = 4.0 Hz, 1H), 5.04 (s, 2H), 7.27-7.40 (m, 5H). MS *m*/*z* ([M+Na]⁺) 284.

### Step 4: Octahydro-cyclopenta[c]pyrrol-4-ol (36d)

Pd/C (10%, 0.49 g) was added to a solution of compound **(36c)** (5.27 g, 20.1 mmol) in MeOH (49 mL) and THF (49 mL). The reaction was performed under H₂ balloon overnight. The mixture was filtrated through a Celite pad and the filtrate was concentrated. The product was purified by column chromatography on silica gel (DCM to MeOH) to give compound **(36d)** as orange oil (2.27 g, 17.9 mmol, 89%). ¹H NMR (300 MHz, DMSO-*d₆*) δ 1.25 (ddd, J = 4.3/6.7/18.0 Hz, 1H), 1.51 (dd, J = 6.6/12.9 Hz, 2H), 1.67 (ddd, J = 7.4/12.2/14.1 Hz, 1H), 2.76 (td, J = 6.8/10.6 Hz, 1H), 2.33-2.48 (m, 3H), 2.96 (dd, J = 3.5/11.0 Hz, 1H), 3.60-3.81 (bs, 2H), 4.01 (dd, J = 5.8/11.8 Hz, 1H). MS m/z ([M+H]⁺) 128.

### Step 5: Methyl 5-(1-cyclohexyl-1H-benzoimidazol-5-yl)-2-(4-hydroxy-hexahydro-cyclopenta[c]pyrrol-2-yl)-4-nitro-thiophene-3-carboxylate (36e)

According to the procedure described in example 15, step 1, compound **(1c)** (100 mg, 0.24 mmol) was converted, by reaction with compound **(36d)** (2.27 g, 17.9 mmol) and after purification by preparative TLC (DCM/MeOH 95/5), to compound **(36e)** as red oil (66 mg, 0.13 mmol, 54%). MS *m*/*z* ([M+H]⁺) 511.

### Step 6: Methyl 4-amino-5-(1-cyclohexyl-1H-benzoimidazol-5-yl)-2-(4-hydroxy-hexahydro-cyclopenta[c]pyrrol-2-yl)-thiophene-3-carboxylate (36f)

According to the procedure described in example 1, step 5, compound **(36e)** (66 mg, 0.13 mmol) was converted to compound **(36f)** as red oil (49 mg, 0.10 mmol, 79%). MS *m*/*z* ([M+H]⁺) 481.

### Step 7: 7-(1-cyclohexyl-1H-benzoimidazol-5-yl)-5-(4-hydroxy-hexahydro-cyclopenta[c]pyrrol-2-yl)-3H-thieno[3,4-d]pyrimidin-4-one (Example 36)

According to the procedure described in example 1, step 6, compound **(36e)** (49 mg, 0.10 mmol) was converted, after purification by preparative TLC (DCM/MeOH 9/1 + 2% NH₃ then DCM/MeOH 95/5), to **(Example 36)** as yellow solid (7.4 mg, 0.02 mmol, 12%). ¹H NMR (400 MHz, DMSO-*d₆*) δ 1.25-1.35 (m, 1H), 1.43-1.58 (m, 2H), 1.62-1.78 (m, 5H), 1.83-1.90 (m, 4H), 2.03-2.07 (m, 2H), 2.80 (bs, 2H), 3.44 (dd, J = 4.4/10.2 Hz, 1H), 3.61-3.67 (m, 2H), 3.77-3.83 (m, 1H), 4.08-4.12 (m, 1H), 4.30-4.38 (m, 1H), 4.80 (d, J = 4.1 Hz, 1H), 7.61-7.66 (m, 2H), 7.75 (dd, J = 1.6/8.6 Hz, 1H), 8.21 (d, J = 1.4 Hz, 1H), 8.30 (s, 1H), 11.05 (d, J = 3.1 Hz, 1H). MS *m*/*z* ([M+H]⁺) 476.

### Example 37: Synthesis of 7-(1-cyclohexyl-1H-benzoimidazol-5-yl)-5-(1,2,3,6-tetrahydro-pyridin-4-yl)-3H-thieno[3,4-d]pyrimidin-4-one hydrochloride

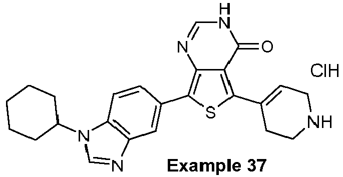

### Step 1: tert-butyl 4-[5-(1-cyclohexyl-1H-benzoimidazol-5-yl)-3-methoxycarbonyl-4-nitro-thiophen-2-yl]-3,6-dihydro-2H-pyridine-1-carboxylate (37a)

According to the procedure described in example 1, step 3, compound **(1c)** (160 mg, 0.38 mmol) was converted, by reaction with tert-butyl 4-(4,4,5,5-tetramethyl-[1,3,2]dioxaborolan-2-yl)-3,6-dihydro-2H-pyridine-1-carboxylate (130 mg, 0.42 mmol) and after purification by preparative TLC (DCM/MeOH 9/1 + 2% NH₃ then DCM/MeOH 95/5), to compound **(37a)** (201 mg, 0.35 mmol, 93%). MS *m*/*z* ([M+H]⁺) 567.

### Step 2: tert-butyl 4-[4-amino-5-(1-cyclohexyl-1H-benzoimidazol-5-yl)-3-methoxycarbonyl-thiophen-2-yl]-3,6-dihydro-2H-pyridine-1-carboxylate (37b)

According to the procedure described in example 1, step 5, compound **(37a)** (200 mg, 0.35 mmol) was converted, after purification by preparative TLC (DCM/MeOH 95/5), to compound **(37b)** (152 mg, 0.28 mmol, 80%). MS *m*/*z* ([M+H]⁺) 537.

### Step 3: tert-butyl 4-[7-(1-cyclohexyl-1H-benzoimidazol-5-yl)-4-oxo-3,4-dihydro-thieno[3,4-d]pyrimidin-5-yl]-3,6-dihydro-2H-pyridine-1-carboxylate (37c)

According to the procedure described in example 1, step 6, compound **(37b)** (152 mg, 0.28 mmol) was converted, after purification by preparative TLC (DCM/MeOH 95/5), to compound **(37c)** (106 mg, 0.20 mmol, 70%). MS *m*/*z* ([M+H]⁺) 532.

### Step 4: 7-(1-cyclohexyl-1H-benzoimidazol-5-yl)-5-(1,2,3,6-tetrahydro-pyridin-4-yl)-3H-thieno[3,4-d]pyrimidin-4-one hydrochloride (Example 37)

According to the procedure described in example 12, step 4, compound **(37c)** (106 mg, 0.20 mmol) was converted to **(Example 37)** as red solid (33 mg, 0.07 mmol, 93%). ¹H NMR (400 MHz, DMSO-*d₆*) δ 1.27-1.35 (m, 1H), 1.49-1.59 (m, 2H), 1.73-1.77 (m, 1H), 1.87-1.92 (m, 4H), 2.17-2.19 (m, 2H), 2.92 (bs, 2H), 3.34 (bs, 2H), 3.82 (bs, 2H), 4.67 (t, J = 11.6 Hz, 1H), 6.27 (s, 1H), 7.95 (d, J = 3.3 Hz, 1H), 8.09 (dd, J = 1.3/8.7 Hz, 1H), 8.15 (d, J = 8.7 Hz, 1H), 8.60 (s, 1H), 9.29 (bs, 2H), 9.56 (bs, 1H), 12.0 (d, J = 3.3 Hz, 1H). MS *m*/*z* ([M+H]⁺) 432.

### Example 38: Synthesis of 7-(1-cyclohexyl-1H-benzoimidazol-5-yl)-5-(1,2,3,6-tetrahydro-pyridin-4-yl)-3H-thieno[3,4-d]pyrimidin-4-one hydrochloride

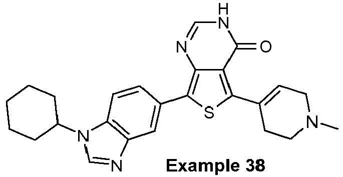

**(Example 37)** (56 mg, 0.12 mmol) was dissolved in MeOH (1 mL). Formaldehyde 37% in water (58 mg, 0.72 mmol) and NaBH₃CN (23 mg, 0.36 mmol) were added and the mixture was stirred at room temperature for 90 minutes. Ammonium chloride solution, water and DCM were added to the middle which was extracted with DCM. Organic layer was dried over sodium sulfate, filtered and concentrated. Residue was purified by preparative TLC on silica gel (DCM/MeOH 9/1). The fraction containing desired product was triturated in CH₃CN to give **(Example 38)** as an orange solid (11 mg, 0.025 mmol, 21%). ¹H NMR (400 MHz, CD₃OD) δ 1.34-1.47 (m, 1H), 1.54-1.68 (m, 2H), 1.79-2.03 (m, 5H), 2.17-2.25 (m, 2H), 2.43 (s, 3H), 2.78-2.87 (m, 4H), 3.21-3.24 (m, 2H), 4.35-4.45 (m, 1H), 6.17-6.21 (m, 1H), 7.67 (d, J = 8.5 Hz, 1H), 7.74 (s, 1H), 7.86 (dd, J = 8.5/1.5 Hz, 1H), 8.30 (d, J = 1.4 Hz, 1H), 8.31 (s, 1H). MS *m*/*z* ([M+H]⁺) 446.

### Example 39: Synthesis of 7-(1-Cyclohexyl-1H-benzoimidazol-5-yl)-5-(cis-4-hydroxymethyl-2-methyl-pyrrolidin-1-yl)-3H-thieno[3,4-d]pyrimidin-4-one

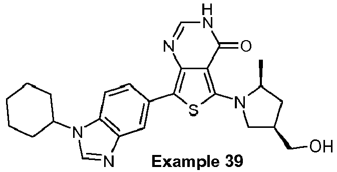

### Step 1: Synthesis of 1-Benzyl-5-methyl-pyrrolidin-2-one (39a)

To a suspension of 5-methylpyrrolidin-2-one (4.6 g, 46.4 mmol) in DMF (30 mL) under a nitrogen atmosphere at 0°C, sodium hydride (60% in oil, 2.8 g, 70 mmol) and benzyl bromide (6.6 mL, 55.7 mmol) were added. After 16 hours of stirring at room temperature, the reaction was quenched with a saturated solution of ammonium chloride. Ethyl acetate was added and the organic layer was extracted twice with ethyl acetate, dried over sodium sulfate, filtered and concentrated under reduced pressure. The crude product was purified by flash chromatography on silica gel (cyclohexane/EtOAc 7/3) to give compound **(39a)** as yellow oil (7.56 g, 40.0 mmol, 88%). ¹H NMR (400 MHz, CDCl₃) δ 1.20 (d, 3H), 1.60 (m, 1H), 2.15 (m, 1H), 2.40 (m, 1H), 2.55 (m, 1H), 3.50 (m, 1H), 4.00 (d, 1H), 5.00 (d, 1H), 7.40-7.20 (m, 5H). MS m/z ([M+H]⁺) 190.

### Step 2: Ethyl 1-Benzyl-5-methyl-2-oxo-pyrrolidine-3-carboxylate (39b)

Under a nitrogen atmosphere at -78°C, a solution of butyl lithium (1.6M in hexane, 41 mL, 71.5 mmol)) was added to a solution of diisopropylamine (13.38 mL, 94 mmol) in THF (50 mL). The mixture was stirred 10 minutes at 0°C and cooled to -78°C. A solution of compound **(39a)** (5 g, 26 mmol) in THF (50 mL) was then added to the mixture. After 90 minutes at -78°C, ethylchloroformate (2.7 mL, 39 mmol) was added and the reaction was stirred for 30 minutes. The middle was warmed to room temperature and stirred for 2 hours. The reaction was quenched with a saturated solution of ammonium chloride. Ethyl acetate was added and the organic layer was extracted twice with ethyl acetate, dried over sodium sulfate, filtered and concentrated under reduced pressure. The crude product was purified by flash chromatography on silica gel (cyclohexane/EtOAc 7/3) to give compound **(39b)** as yellow oil (4.70 g, 18.0 mmol, 68%). MS m/z ([M+H]⁺) 262.

### Step 3: cis-(1-benzyl-5-methylpyrrolidin-3-yl)methanol (39c) and anti-(1-benzyl-5-methylpyrrolidin-3-yl)methanol (39d)

At 0°C, lithium aluminium hydride (2.5 g, 66 mmol) was added to a suspension of compound **(39b)** (4.7 g, 18 mmol) in THF (90 mL). The middle was stirred for 3 hours at room temperature. The reaction was quenched with water and a solution of sodium hydroxide 3M and stirred 1 hour at room temperature. The mixture was filtered and the filtrate was extracted with ethyl acetate. The organic layer was dried over sodium sulfate, filtered and concentrated under reduced pressure. The crude material was purified by flash chromatography on silica gel (DCM/MeOH 95/5 to 90/10 +1% Ammonia) to give both diastereoisomers compound **(39c)** (1.12 g, 5.4 mmol, 30%) and compound **(39d)** (1.12 g, 5.4 mmol, 30%). Compound **(39c)**: ¹H NMR (400 MHz, CDCl₃) δ 1.20 (d, J = 6.1 Hz, 3H), 1.64-1.72 (m, 1H), 1.75-1.81 (m, 1H), 1.99-2.02 (m, 1H), 2.33-2.40 (m, 1H), 2.55-2.62 (m, 1H), 3.05-3.10 (m, 1H), 3.20 (d, J = 12.7 Hz, 1H), 3.48-3.60 (m, 2H), 4.03 (d, J = 12.7 Hz, 1H), 7.26-7.29 (m, 1H), 7.31-7.35 (m, 4H). MS m/z ([M+H]⁺) 206. Compound **(39d):** ¹H NMR (400 MHz, CDCl₃) δ 1.28 (d, J = 6.0Hz, 3H), 1.45-1.51 (m, 1H), 2.19-2.36 (m, 3H), 2.47-2.55 (m, 1H), 2.86 (d, J = 9.4Hz, 1H), 3.08 (d, J = 12.9Hz, 1H), 3.50 (dd, J = 2.9/9.9 Hz, 1H), 3.65 (dd, J = 3.2/9.9 Hz, 1H), 4.10 (d, J= 13.0Hz, 1H), 7.30-7.35 (m, 5H). MS m/z ([M+H]⁺) 206.

### Step 4: cis-(5-methylpyrrolidin-3-yl)-methanol (39e)

A solution of compound **(39c)** (1.12 g, 4.8 mmol) in ethanol (35 mL) was purged with argon. Catalyst palladium hydroxyde on carbon (350 mg) was then added and the reaction was stirred under hydrogen atmosphere (atmospheric pression) for 18 hours. The middle was filtered and concentrated under reduced pressure to give compound **(39e)** as uncolorless oil (630 mg, 4.8 mmol, 100%). ¹H NMR (400 MHz, CD₃OD) δ 1.21-1.25 (m, 3H), 1.47-1.54 (m, 1H), 1.74-1.80 (m, 1H), 2.41-2.48 (m, 1H), 2.60-2.64 (m, 1H), 3.18-3.23 (m, 2H), 3.47-3.59 (m, 2H). MS m/z ([M+H]⁺) 116.

### Step 5: Methyl 5-(1-Cyclohexyl-1H-benzoimidazol-5-yl)-2-(cis-4-hydroxymethyl-2-methylpyrrolidin-1-yl)-4-nitro-thiophene-3-carboxylate (39f)

According to the procedure in example 6, step 1, compound **(1c)** (100 mg, 0.238 mmol) was converted, by reaction with compound **(39e)** (41 mg, 0.358 mmol) and after purification by preparative TLC on silica gel (DCM/MeOH 95/5), to compound **(39f)** as an orange solid (112 mg, 0.224 mmol, 84%). ¹H NMR (400 MHz, CDCl₃) δ 1.25 (d, J = 6.2 Hz, 3H), 1.32 (qt, J = 12.8/3.6 Hz, 1H), 1.51 (qt, J = 13.1/3.2 Hz, 2H), 1.62 (bs, 1H), 1.74-1.88 (m, 4H), 1.94-2.02 (m, 2H), 2.10-2.18 (m, 1H), 2.18-2.26 (m, 2H), 2.55-2.64 (m, 1H), 3.07 (dd, J = 10.7/4.2 Hz, 1H), 3.52-3.62 (m, 2H), 3.76 (s, 3H), 3.89 (dd, J = 10.7/6.9 Hz, 1H), 4.02 (sex, J = 6.4 Hz, 1H), 4.18 (tt, J = 11.9/3.7 Hz, 1H), 7.31 (dd, J = 8.5/1.7 Hz, 1H), 7.41 (d, J = 8.5 Hz, 1H), 7.83 (d, J = 1.5 Hz, 1H), 8.01 (s, 1H). MS *m*/*z* ([M+H]⁺) 499.

### Step 6: Methyl 4-Amino-5-(1-cyclohexyl-1H-benzoimidazol-5-yl)-2-(cis-4-hydroxymethyl-2-methyl-pyrrolidin-1-yl)-thiophene-3-carboxylate (39g)

According to the procedure in example 25, step 2, compound **(39f)** (112 mg, 0.224 mmol) was converted to compound **(39g)** as a yellow residue (104 mg, 0.224 mmol, 100%). MS m/z ([M+H]⁺) 469.

### Step 7: 7-(1-Cyclohexyl-1H-benzoimidazol-5-yl)-5-(cis-4-hydroxymethyl-2-methyl-pyrrolidin-1-yl)-3H-thieno[3,4-d]pyrimidin-4-one (Example 39)

According to the procedure in example 1, step 6, compound **(39g)** (104 mg, 0.224 mmol) was converted, after purification by preparative TLC on silica gel (DCM/MeOH 9/1) and trituration in hot CH₃CN, to **(Example 39)** as a yellow solid (21 mg, 0.045 mmol, 21%). ¹H NMR (400 MHz, DMSO-*d₆*) δ 1.15 (d, J = 6.2 Hz, 3H), 1.23-1.35 (m, 1H), 1.43-1.56 (m, 2H), 1.68-1.80 (m, 2H), 1.81-1.90 (m, 4H), 2.01-2.10 (m, 3H), 2.42-2.48 (m, 1H), 3.15 (dd, J = 11.0/3.9 Hz, 1H), 3.22-3.30 (m, 1H), 3.30-3.38 (m, 1H), 4.04 (dd, J = 10.9/7.0 Hz, 1H), 4.28-4.38 (m, 1H), 4.38-4.44 (m, 1H), 4.71 (t, J = 5.3 Hz, 1H), 7.61 (d, J = 8.6 Hz, 1H), 7.63 (d, J = 3.3 Hz, 1H), 7.73 (dd, J = 8.5/1.6 Hz, 1H), 8.19 (d, J = 1.4 Hz, 1H), 8.28 (s, 1H), 11.00 (d, J = 3.2 Hz, 1H). MS *m*/*z* ([M+H]⁺) 464.

### Example 40: Synthesis of 7-(1-Cyclohexyl-1H-benzoimidazol-5-yl)-5-(trans-4-hydroxymethyl-2-methyl-pyrrolidin-l-yl)-3H-thieno[3,4-d]pyrimidin-4-one

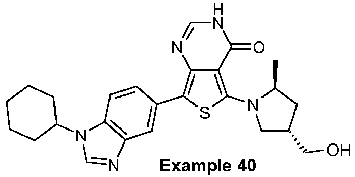

### Step 1: trans-5-methylpyrrolidin-3-yl methanol (40a)

According to the procedure described in example 39, step 4, compound **(39d)** (1.12 g, 4.8 mmol) was converted to compound **(40a)** as yellow oil (630 mg, 4.8 mmol, 100%). ¹H NMR (400 MHz, CD₃OD) δ 1.00-1.08 (m, 1H), 1.22-1.27 (m, 3H), 2.11-2.18 (m, 1H), 2.36-2.47 (m, 1H), 2.86-2.90 (m, 1H), 2.99-3.03 (m, 1H), 3.13-3.21 (m, 1H), 3.49-3.59 (m, 2H). MS *m*/*z* ([M+H]⁺) 116.

### Step 2: Methyl 5-(1-Cyclohexyl-1H-benzoimidazol-5-yl)-2-(trans-4-hydroxymethyl-2-methyl-pyrrolidin-1-yl)-4-nitro-thiophene-3-carboxylate (40b)

According to the procedure in example 6, step 1, compound **(1c)** (100 mg, 0.238 mmol) was converted, by reaction with compound **(40a)** (41 mg, 0.358 mmol) and after purification by preparative TLC on silica gel (DCM/MeOH 95/5), to compound **(40b)** as an orange solid (87 mg, 0.174 mmol, 74%). ¹H NMR (400 MHz, CDCl₃) δ 1.32 (d, J = 6.1Hz, 3H), 1.32-1.40 (m, 1H), 1.44-1.54 (m, 2H), 1.62 (bs, 1H), 1.74-1.86 (m, 4H), 1.94-2.02 (m, 2H), 2.18-2.26 (m, 2H), 2.39-2.48 (m, 2H), 3.22 (dd, J = 10.5/7.5 Hz, 1H), 3.64-3.76 (m, 3H), 3.77 (s, 3H), 3.82-3.90 (m, 1H), 4.18 (tt, J = 11.9/3.7 Hz, 1H), 7.33 (dd, J = 8.5/1.6 Hz, 1H), 7.42 (d, J = 8.5 Hz, 1H), 7.84 (d, J = 1.5 Hz, 1H), 8.02 (s, 1H). MS *m*/*z* ([M+H]⁺) 499.

### Step 3: Methyl 4-Amino-5-(1-cyclohexyl-1H-benzoimidazol-5-yl)-2-(trans-4-hydroxymethyl-2-methyl-pyrrolidin-1-yl)-thiophene-3-carboxylate (40c)

According to the procedure in example 25, step 2, compound **(40b)** (87 mg, 0.174 mmol) was converted to compound **(40c)** as a yellow residue (81 mg, 0.174 mmol, 100%). MS *m*/*z* ([M+H]⁺) 469.

### Step 4: 7-(1-Cyclohexyl-1H-benzoimidazol-5-yl)-5-(trans-4-hydroxymethyl-2-methyl-pyrrolidin-1-yl)-3H-thieno[3,4-d]pyrimidin-4-one (Example 40)

According to the procedure in example 1, step 6, compound **(40c)** (81 mg, 0.174 mmol) was converted, after purification by preparative TLC on silica gel (DCM/MeOH 9/1) and trituration in hot CH₃CN, to **(Example 40)** as a yellow solid (11 mg, 0.024 mmol, 14%). ¹H NMR (400 MHz, DMSO-*d₆*) δ 1.19 (d, J = 6.0 Hz, 3H), 1.24-1.36 (m, 1H), 1.44-1.56 (m, 3H), 1.69-1.76 (m, 1H), 1.82-1.90 (m, 4H), 2.02-2.08 (m, 2H), 2.26-2.36 (m, 2H), 3.31-3.37 (m, 1H), 3.42-3.56 (m, 2H), 3.71 (t, J = 10.3 Hz, 1H), 4.11-4.21 (m, 1H), 4.34 (tt, J = 12.1/3.7 Hz, 1H), 4.68 (t, J = 4.7 Hz, 1H), 7.62 (d, J = 8.6 Hz, 1H), 7.64 (d, J = 3.4 Hz, 1H), 7.75 (dd, J = 8.5/1.7 Hz, 1H), 8.20 (d, J = 1.5 Hz, 1H), 8.31 (s, 1H), 11.02 (d, J = 3.2 Hz, 1H). MS *m*/*z* ([M+H]⁺) 464.

### Example 41: Synthesis of 5-((S)-3-Hydroxymethyl-pyrrolidin-1-yl)-7-(4-methoxy-phenyl)-3H-thieno[3,4-d]pyrimidin-4-one

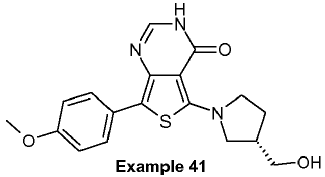

### Step 1: Methyl 2-Chloro-5-(4-methoxy-phenyl)-4-nitro-thiophene-3-carboxylate (41a)

According to the procedure described in example 1, step 3, compound **(1b)** (200 mg, 0.78 mmol) was converted, by reaction with 4-Methoxy-phenyl-boronic acid (142 mg, 0.93 mmol) and after recrystallization in MeOH, to compound **(41a)** (190 mg, 0.58 mmol, 75%) as a tan solid. MS *m*/*z* ([M+H]⁺) 328.

### Step 2: Methyl 2-((S)-3-Hydroxymethyl-pyrrolidin-1-yl)-5-(4-methoxy-phenyl)-4-nitro-thiophene-3-carboxylate (41b)

According to the procedure described in example 15, step 1, compound **(41a)** (100 mg, 0.30 mmol) was converted, by reaction with (S)-1-Pyrrolidin-3-yl-methanol (46 mg, 0.45 mmol) and after purification by preparative TLC on silica gel (DCM/MeOH 9/1), to compound **(41b)** (67 mg, 0.17 mmol, 57%) as an orange solid. MS *m*/*z* ([M+H]⁺) 393.

### Step 3: Methyl 4-Amino-2-((S)-3-hydroxymethyl-pyrrolidin-1-yl)-5-(4-methoxy-phenyl)-thiophene-3-carboxylate (41c)

According to the procedure described in example 21, step 2, compound **(41b)** (67 mg, 0.17 mmol) was converted to compound **(41c)** (61 mg, 0.17 mmol, 100%) as yellow residue. MS *m*/*z* ([M+H]⁺) 363.

### Step 4: 5-((S)-3-Hydroxymethyl-pyrrolidin-1-yl)-7-(4-methoxy-phenyl)-3H-thieno[3,4-d]pyrimidin-4-one (Example 41)

According to the procedure described in example 1, step 6, compound **(41c)** (61 mg, 0.17 mmol) was converted, after purification by preparative TLC on silica gel (DCM/MeOH 9/1+2%NH3 7M in MeOH), to **(Example 41)** (23 mg, 0.064 mmol, 38%) as a yellow solid. ¹H NMR (400 MHz, CD₃OD) δ 1.82-1.94 (m, 1H), 2.12-2.22 (m, 1H), 2.52-2.63 (m, 1H), 3.49 (dd, J = 10.7/6.9 Hz, 1H), 3.55-3.74 (m, 5H), 3.81 (s, 3H), 6.90-6.95 (m, 2H), 7.51 (s, 1H), 7.68-7.73 (m, 2H). MS *m*/*z* ([M+H]⁺) 358.

### Example 42: Synthesis of 5-((S)-3-Hydroxymethyl-pyrrolidin-1-yl)-7-(6-methoxy-pyridin-3-yl)-3H-thieno[3,4-d]pyrimidin-4-one

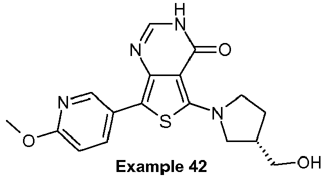

### Step 1: Methyl 2-Chloro-5-(6-methoxy-pyridin-3-yl)-4-nitro-thiophene-3-carboxylate (42a)

According to the procedure described in example 1, step 3, compound **(1b)** (200 mg, 0.78 mmol) was converted, by reaction with 2-Methoxy-pyridine-5-boronic acid (142 mg, 0.93 mmol) and after purification by preparative TLC on silica gel (DCM/MeOH 98/2), to compound **(42a)** (164 mg, 0.5 mmol, 65%). ¹H NMR (400 MHz, CDCl₃) δ 3.92 (s, 3H), 3.98 (s, 3H), 6.80-6.84 (m, 1H), 7.66 (dd, J = 8.6/2.5 Hz, 1H), 8.24-8.27 (m, 1H). MS *m*/*z* ([M+H]⁺) 329.

### Step 2: Methyl 2-((S)-3-Hydroxymethyl-pyrrolidin-1-yl)-5-(6-methoxy-pyridin-3-yl)-4-nitro-thiophene-3-carboxylate (42b)

According to the procedure described in example 15, step 1, compound **(42a)** (100 mg, 0.30 mmol) was converted, by reaction with (S)-1-Pyrrolidin-3-yl-methanol (46 mg, 0.45 mmol) and after purification by preparative TLC on silica gel (DCM/MeOH 94/6), to compound **(42b)** (61 mg, 0.15 mmol, 52%) as an orange solid. ¹H NMR (400 MHz, CDCl₃) δ 1.84-1.95 (m, 1H), 2.12-2.22 (m, 1H), 2.54-2.66 (m, 1H), 3.30 (dd, J = 10.3/6.7 Hz, 1H), 3.40-3.51 (m, 3H), 3.62-3.76 (m, 2H), 3.77 (s, 3H), 3.95 (s, 3H), 6.75 (dd, J = 8.6/0.6 Hz, 1H), 7.60 (dd, J = 8.6/2.5 Hz, 1H), 8.19-8.21 (m, 1H). MS *m*/*z* ([M+H]⁺) 394.

### Step 3: Methyl 4-Amino-2-((S)-3-hydroxymethyl-pyrrolidin-1-yl)-5-(6-methoxy-pyridin-3-yl)-thiophene-3-carboxylate (42c)

According to the procedure described in example 21, step 2, compound **(42b)** (61 mg, 0.15 mmol) was converted to compound **(42c)** (54 mg, 0.15 mmol, 100%) as a yellow residue. MS *m*/*z* ([M+H]⁺) 364.

### Step 4: 5-((S)-3-Hydroxymethyl-pyrrolidin-1-yl)-7-(6-methoxy-pyridin-3-yl)-3H-thieno[3,4-d]pyrimidin-4-one (Example 42)

According to the procedure described in example 1, step 6, compound **(42c)** (54 mg, 0.15 mmol) was converted, after purification by preparative TLC on silica gel (DCM/MeOH 9/1+2%NH3 7M in MeOH), to **(Example 42)** as a yellow solid (11 mg, 0.030 mmol, 20%). ¹H NMR (400 MHz, CD₃OD) δ 1.84-1.94 (m, 1H), 2.14-2.22 (m, 1H), 2.54-2.63 (m, 1H), 3.48-3.55 (m, 1H), 3.56-3.64 (m, 2H), 3.67-3.77 (m, 3H), 3.92 (s, 3H), 6.81 (dd, J = 8.7/0.5 Hz, 1H), 7.56 (s, 1H), 8.17 (dd, J = 8.7/2.5 Hz, 1H), 8.50-8.52 (m, 1H). MS *m*/*z* ([M+H]⁺) 359.

### Example 43: Synthesis of 5-((S)-3-Hydroxymethyl-pyrrolidin-1-yl)-7-(4-methoxy-3-trifluoromethyl-phenyl)-3H-thieno[3,4-d]pyrimidin-4-one

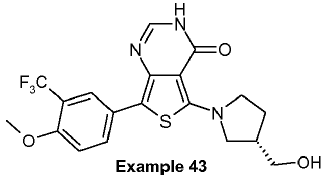

### Step 1: Methyl 2-Chloro-5-(4-methoxy-3-trifluoromethyl-phenyl)-4-nitro-thiophene-3-carboxylate (43a)

According to the procedure described in example 1, step 3, compound **(1b)** (200 mg, 0.78 mmol) was converted, by reaction with 4-Methoxy-3-trifluoromethyl-phenyl-boronic acid (204 mg, 0.93 mmol) and after purification by preparative TLC on silica gel (cyclohexane/EtOAc 75/25), to compound **(43a)** (148 mg, 0.37 mmol, 48%). ¹H NMR (300 MHz, CDCl₃) δ 3.92 (s, 3H), 3.96 (s, 3H), 7.07 (d, J = 8.7Hz, 1H), 7.58-7.66 (m, 2H). MS *m*/*z* ([M+H]⁺) 396.

### Step 2: Methyl 2-((S)-3-Hydroxymethyl-pyrrolidin-1-yl)-5-(4-methoxy-3-trifluoromethyl-phenyl)-4-nitro-thiophene-3-carboxylate (43b)

According to the procedure described in example 15, step 1, compound **(43a)** (100 mg, 0.25 mmol) was converted, by reaction with (S)-1-Pyrrolidin-3-yl-methanol (38 mg, 0.38 mmol) and after purification by preparative TLC on silica gel (DCM/MeOH 94/6), to compound **(43b)** (46 mg, 0.1 mmol, 40%) as an orange solid. ¹H NMR (400 MHz, CDCl₃) δ 1.85-1.96 (m, 1H), 2.13-2.22 (m, 1H), 2.56-2.66 (m, 1H), 3.28-3.35 (m, 1H), 3.40-3.52 (m, 3H), 3.64-3.76 (m, 2H), 3.77 (s, 3H), 3.93 (s, 3H), 7.01 (d, J = 8.7 Hz, 1H), 7.53-7.61 (m, 2H). MS *m*/*z* ([M+H]⁺) 461.

### Step 3: Methyl 4-Amino-2-((S)-3-hydroxymethyl-pyrrolidin-1-yl)-5-(4-methoxy-3-trifluoromethyl-phenyl)-thiophene-3-carboxylate (43c)

According to the procedure described in example 21, step 2, compound **(43b)** (46 mg, 0.1 mmol) was converted to compound **(43c)** (43 mg, 0.1 mmol, 100%) as a yellow residue without further purification. MS *m*/*z* ([M+H]⁺) 431.

### Step 4: 5-((S)-3-Hydroxymethyl-pyrrolidin-1-yl)-7-(4-methoxy-3-trifluoromethyl-phenyl)-3H-thieno[3,4-d]pyrimidin-4-one (Example 43)

According to the procedure described in example 1, step 6, compound **(43c)** (43 mg, 0.1 mmol) was converted, after purification by preparative TLC on silica gel (DCM/MeOH 9/1+2%NH3 7M in MeOH), to **(Example 43)** as a yellow solid (4 mg, 0.009 mmol, 10%). ¹H NMR (400 MHz, CD₃OD) δ 1.84-1.94 (m, 1H), 2.13-2.23 (m, 1H), 2.54-2.63 (m, 1H), 3.48-3.54 (m, 1H), 3.56-3.64 (m, 2H), 3.65-3.77 (m, 3H), 3.91 (s, 3H), 7.16 (d, J = 8.7 Hz, 1H), 7.58 (s, 1H), 7.93 (dd, J = 8.7/2.2 Hz, 1H), 8.16 (d, J = 2.1 Hz, 1H). MS *m*/*z* ([M+H]⁺) 426.

### Example 44: Synthesis of 5-[5-((S)-3-Hydroxymethyl-pyrrolidin-1-yl)-4-oxo-3,4-dihydro-thieno[3,4-d]pyrimidin-7-yl]-2-methoxy-benzonitrile

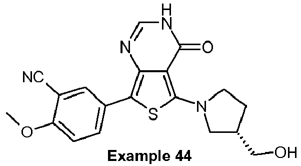

### Step 1: Methyl 2-Chloro-5-(3-cyano-4-methoxy-phenyl)-4-nitro-thiophene-3-carboxylate (44a)

According to the procedure described in example 1, step 3, compound **(1b)** (200 mg, 0.78 mmol) was converted, by reaction with 4-Methoxy-3-cyano-phenyl-boronic acid (164 mg, 0.93 mmol) and after recrystallization in MeOH, to compound **(44a)** (145 mg, 0.41 mmol, 53%). ¹H NMR (400 MHz, DMSO-*d₆*) δ 3.89 (s, 3H), 4.00 (s, 3H), 7.41 (d, J = 8.9 Hz, 1H), 7.87 (dd, J = 8.9/2.3 Hz, 1H), 8.04 (d, J = 2.3 Hz, 1H). MS *m*/*z* ([M+H]⁺) 353.

### Step 2: Methyl 5-(3-Cyano-4-methoxy-phenyl)-2-((S)-3-hydroxymethyl-pyrrolidin-1-yl)-4-nitro-thiophene-3-carboxylate (44b)

According to the procedure described in example 15, step 1, compound **(44a)** (100 mg, 0.28 mmol) was converted, by reaction with (S)-1-Pyrrolidin-3-yl-methanol (43 mg, 0.42 mmol) and after purification by preparative TLC on silica gel (DCM/MeOH 94/6), to compound **(44b)** (60 mg, 0.14 mmol, 51%) as an orange solid. ¹H NMR (400 MHz, CDCl₃) δ 1.59-1.63 (m, 1H), 1.86-1.96 (m, 1H), 2.14-2.23 (m, 1H), 2.55-2.66 (m, 1H), 3.31 (dd, J = 10.2/6.7 Hz, 1H), 3.40-3.51 (m, 3H), 3.63-3.78 (m, 2H), 3.78 (s, 3H), 3.96 (s, 3H), 6.96-7.01 (m, 1H), 7.56-7.60 (m, 2H). MS *m*/*z* ([M+H]⁺) 418.

### Step 3: Methyl 4-Amino-5-(3-cyano-4-methoxy-phenyl)-2-((S)-3-hydroxymethyl-pyrrolidin-1-yl)-thiophene-3-carboxylate (44c)

According to the procedure described in example 21, step 2, compound **(44b)** (60 mg, 0.14 mmol) was converted to compound **(44c)** (54 mg, 0.14 mmol, 100%) as a yellow residue without further purification. MS *m*/*z* ([M+H]⁺) 388.

### Step 4: 5-[5-((S)-3-Hydroxymethyl-pyrrolidin-1-yl)-4-oxo-3,4-dihydro-thieno[3,4-d]pyrimidin-7-yl]-2-methoxy-benzonitrile (Example 44)

According to the procedure described in example 1, step 6, compound **(44c)** (54 mg, 0.14 mmol) was converted, after purification by preparative TLC on silica gel (DCM/MeOH 9/1+2%NH3 7M in MeOH), to **(Example 44)** as a yellow solid (9 mg, 0.023 mmol, 17%). ¹H NMR (400 MHz, CD₃OD) δ 1.84-1.94 (m, 1H), 2.14-2.22 (m, 1H), 2.54-2.63 (m, 1H), 3.48-3.78 (m, 6H), 3.96 (s, 3H), 7.17 (d, J = 8.9 Hz, 1H), 7.60 (s, 1H), 7.97 (dd, J = 8.9/2.3 Hz, 1H), 8.25 (d, J = 2.3 Hz, 1H). MS *m*/*z* ([M+H]⁺) 383.

### Example 45: Synthesis of 7-(1-ethyl-1H-benzoimidazol-5-yl)-5-((S)-3-hydroxymethyl-pyrrolidin-1-yl)-3H-thieno[3,4-d]pyrimidin-4-one

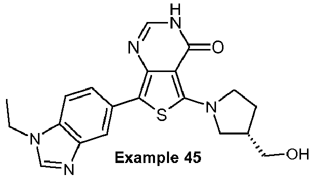

### Step 1: Methyl 2-Chloro-5-(1-ethyl-1H-benzimidazol-5-yl)-4-nitro-thiophene-3-carboxylate (45a)

According to the procedure described in example 1, step 3, compound **(1b)** (736 mg, 2.87 mmol) was converted, by reaction with 1-ethyl-5-(4,4,5,5-tetramethyl-[1,3,2]dioxaborolan-2-yl)-1H-benzoimidazole (860 mg, 3.16 mmol) and after purification by flash chromatography on silica gel (DCM/MeOH 95/5), to compound **(45a)** as a brown oil (977 mg, 2.67 mmol, 90%). MS *m*/*z* ([M+H]⁺) 366.

### Step 2: Methyl 5-(1-ethyl-1H-benzoimidazol-5-yl)-2-((S)-3-hydroxymethyl-pyrrolidin-1-yl)-4-nitro-thiophene-3-carboxylate (45b)

According to the procedure described in example 15, step 1, compound **(45a)** (150 mg, 0.41 mmol) was converted, by reaction with (S)-1-Pyrrolidin-3-yl-methanol (62 mg, 0.61 mmol) and after purification by preparative TLC on silica gel (DCM/MeOH 95/5), to compound **(45b)** as a red oil (120 mg, 0.28 mmol, 68%). MS *m*/*z* ([M+H]⁺) 431.

### Step 3: Methyl 4-amino-5-(1-ethyl-1H-benzoimidazol-5-yl)-2-((S)-3-hydroxymethyl-pyrrolidin-1-yl)-thiophene-3-carboxylate (45c)

According to the procedure described in example 21, step 2, compound **(45b)** (120 mg, 0.28 mmol) was converted to compound **(45c)** as a yellow solid (103 mg, 0.26 mmol, 92%). MS *m*/*z* ([M+H]⁺) 401.

### Step 4: 7-(1-ethyl-1H-benzoimidazol-5-yl)-5-((S)-3-hydroxymethyl-pyrrolidin-1-yl)-3H-thieno[3,4-d]pyrimidin-4-one (Example 45)

According to the procedure described in example 1, step 6, compound **(45c)** (103 mg, 0.26 mmol) was converted, after purification by preparative TLC on silica gel (DCM/MeOH 9/1), to **(Example 45)** as a yellow solid (10 mg, 0.02 mmol, 9%). ¹H NMR (400 MHz, DMSO-*d₆*) δ 1.42 (t, J = 7.3 Hz, 3H), 1.76-1.85 (m, 1H), 2.03-2.11 (m, 1H), 2.44-2.49 (m, 1H), 3.39-3.50 (m, 3H), 3.59-3.68 (m, 3H), 4.26 (t, J = 7.3 Hz, 2H), 4.76 (t, J = 5.3Hz, 1H), 7.56-7.64 (m, 2H), 7.78 (dd, J = 1.6/8.6 Hz, 1H), 8.18 (d, J = 1.6 Hz, 1H), 8.22 (s, 1H), 11.0 (d, J = 3.1 Hz, 1H). MS *m*/*z* ([M+H]⁺) 396.

### Example 46: Synthesis of 5-((S)-3-hydroxymethyl-pyrrolidin-1-yl)-7-[1-(2-methoxy-ethyl)-1H-benzoimidazol-5-yl]-3H-thieno[3,4-d]pyrimidin-4-one

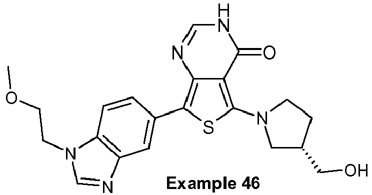

### Step 1: (4-bromo-2-nitro-phenyl)-(2-methoxy-ethyl)-amine (46a)

To a mixture of 4-bromo-1-fluoro-2-nitro-benzene (1.4 g, 6.36 mmol) and 2-methoxyethylamine (0.956 g, 12.73 mmol) in anhydrous CH₃CN (19 mL), was added DIPEA (3.33 mL, 19.09 mmol) under nitrogen atmosphere and the reaction mixture was stirred 16 hours at 85°C. The reaction mixture was partitioned between water and EtOAc and extracted with EtOAc. The combined organic layers were dried over sodium sulfate, filtered and evaporated under reduced pressure to afford compound **(46a)** as an orange solid (1.76 g, 6.36 mmol, 100%) without further purification. MS *m*/*z* ([M+H]⁺) 275/277.

### Step 2: 4-bromo-N*1*-(2-methoxy-ethyl)-benzene-1,2-diamine (46b)

A solution of compound **(46a)** (1.76 g, 6.36 mmol) in a mixture of EtOH/water 2/1 (289 mL) with ammonium chloride (6.46 g, 120.8 mmol) was brought to 60°C until complete dissolution of reagents. Iron (1.07 g, 19.08 mmol) was then added. The mixture was heated at 90°C for 3 hours. After cooling, the solution was filtered through celite and the filtrate was concentrated under reduced pressure. The residue was partitioned between water and EtOAc and extracted with EtOAc. The combined organic phases were dried over sodium sulfate, filtered and evaporated under reduced pressure to afford compound **(46b)** as brownish oil (1.52 g, 6.20 mmol, 98%) which was used in the next step without further purification. MS *m*/*z* ([M+H]⁺) 245/247.

### Step 3: 5-Bromo-1-(2-methoxy-ethyl)-1H-benzoimidazole (46c)

To a mixture of compound **(46b)** (1.52 g, 6.20 mmol) in toluene (32 mL) under nitrogen atmosphere were added trimethyl orthoformate (745 µL, 6.82 mmol) and p-toluenesulfonic acid monohydrate (24 mg, 0.124 mmol) and the resulting solution was refluxed for 3 hours. The mixture was cooled and concentrated under reduced pressure. The residue was partitioned between a saturated solution of sodium hydrogencarbonate and EtOAc and extracted with EtOAc. The combined organic phases were dried over sodium sulfate, filtered and concentrated. The crude residue was purified by column chromatography on silica gel (DCM/MeOH 98/2) to afford compound **(46c)** (1.45 g, 5.68 mmol, 90%) as a brown oil. ¹H NMR (400 MHz, CDCl₃) δ 3.31 (s, 3H), 3.69 (t, J = 5.2 Hz, 2H), 4.29 (t, J = 5.2 Hz, 2H), 7.28 (dd, J = 1.9/8.5 Hz, 1H), 7.39 (d, J = 8.5 Hz, 1H), 7.92-7.95 (m, 2H). MS *m*/*z* ([M+H]⁺) 255/257.

### Step 4: 1-(2-methoxy-ethyl)-5-(4,4,5,5-tetramethyl-[1,3,2]dioxaborolan-2-yl)-1H-benzoimidazole (46d)

A solution of compound **(46c)** (1.45 g, 5.68 mmol), bis(pinacolato)diboron (2.16 g, 8.52 mmol), potassium acetate (1.67 g, 17.05 mmol) in dry 1,4-dioxane (26 mL) was degassed under argon for 10 minutes. Catalyst Pd(dppf)Cl₂·CH₂Cl₂ (232 mg, 0.28 mmol) was added and the reaction mixture was stirred at 85°C overnight. The reaction mixture was filtered through celite and concentrated under reduced pressure. The residue was partitioned between a saturated solution of sodium hydrogencarbonate and DCM and extracted with DCM. The combined organic phases were dried over sodium sulfate, filtered and concentrated. The crude product was triturated in cyclohexane and filtered to afford compound **(46d)** (992 mg, 58%). ¹H NMR (400 MHz, CDCl₃) δ 1.37 (s, 12H), 3.30 (s, 3H), 3.70 (t, J = 5.2 Hz, 2H), 4.32 (t, J = 5.2 Hz, 2H), 7.40 (dd, J = 0.6/8.1 Hz, 1H), 7.75 (dd, J = 0.9/8.1 Hz, 1H), 7.98 (s, 1H), 8.29 (bt, 1H). MS *m*/*z* ([M+H]⁺) 303.

### Step 5: Methyl 2-chloro-5-[1-(2-methoxy-ethyl)-1H-benzoimidazol-5-yl]-4-nitro-thiophene-3-carboxylate (46e)

A solution of compound **(1b)** (230 mg, 0.90 mmol), compound **(46d)** (326 mg, 1.08 mmol) and potassium carbonate (373 mg, 2.69 mmol) in a 5/1 mixture of dioxane (3.4 mL) and water (680 µL) was bubbled with nitrogen for 5 minutes. Palladium tetrakis(triphenylphosphine) (52 mg, 0.05 mmol) was added and the reaction mixture was heated at 90°C overnight. Water was added and the aqueous layer was extracted with ethyl acetate. The organic layer was dried over sodium sulfate and concentrated under reduced pressure. The crude was purified by flash chromatography on silica gel (DCM/MeOH 98/2) then preparative TLC (DCM/MeOH 95/5) to provide compound **(46e)** (201 mg, 0.51 mmol, 56%). MS *m*/*z* ([M+H]⁺) 396.

### Step 6: Methyl 2-((S)-3-hydroxymethyl-pyrrolidin-1-yl)-5-[1-(2-methoxy-ethyl)-1H-benzoimidazol-5-yl]-4-nitro-thiophene-3-carboxylate (46f)

According to the procedure described in example 15, step 1, compound **(46e)** (200 mg, 0.50 mmol) was converted, by reaction with (S)-1-Pyrrolidin-3-yl-methanol (72 mg, 0.71 mmol) and after purification by preparative TLC on silica gel (DCM/MeOH 95/5), to compound **(46f)** (178 mg, 0.39 mmol, 76%). ¹H NMR (400 MHz, DMSO-*d₆*) δ 1.77-1.86 (m, 1H), 2.03-2.11(m, 1H), 2.42-2.45 (m, 1H), 3.16-3.20 (m, 1H), 3.24 (s, 3H), 3.36-3.50 (m, 5H), 3.68-3.70 (m, 5H), 4.45 (t, J = 5.1 Hz, 2H), 4.80 (t, J = 5.1 Hz, 1H), 7.25 (dd, J = 1.7/8.4 Hz, 1H), 7.64 (d, J = 1.2 Hz, 1H), 7.72 (d, J = 8.4 Hz, 1H), 7.29 (s, 1H). MS *m*/*z* ([M+H]⁺) 461.

### Step 7: Methyl 4-amino-2-((S)-3-hydroxymethyl-pyrrolidin-1-yl)-5-[1-(2-methoxy-ethyl)-1H-benzoimidazol-5-yl]-thiophene-3-carboxylate (46g)

According to the procedure described in example 21, step 2, compound **(46f)** (177 mg, 0.38 mmol) was converted to compound **(46g)** as an orange oil (142 mg, 0.33 mmol, 86%) which was used without further purification. MS *m*/*z* ([M+H]⁺) 431.

### Step 8: 5-((S)-3-hydroxymethyl-pyrrolidin-1-yl)-7-[1-(2-methoxy-ethyl)-1H-benzoimidazol-5-yl]-3H-thieno[3,4-d]pyrimidin-4-one (Example 46)

According to the procedure described in example 1, step 6, compound **(46g)** (140 mg, 0.32 mmol) was converted, after purification by preparative TLC on silica gel (DCM/MeOH 95/5), to **(Example 46)** as a yellow solid (16 mg, 0.04 mmol, 11%). ¹H NMR (400 MHz, DMSO-*d₆*) δ 1.76-1.85 (m, 1H), 2.03-2.11 (m, 1H), 2.40-2.45 (m, 1H), 3.23 (s, 3H), 3.37-3.48 (m, 3H), 3.59-3.70 (m, 5H), 4.40 (t, J = 5.1 Hz, 2H), 4.76 (t, J = 5.2 Hz, 1H), 7.58 (d, J = 8.5 Hz, 1H), 7.64 (d, J = 3.3 Hz, 1H), 7.76 (dd, J = 1.6/8.5 Hz, 1H), 8.15 (s, 1H), 8.18 (d, J = 1.3 Hz, 1H), 11.0 (d, J = 3.0 Hz, 1H). MS *m*/*z* ([M+H]⁺) 426.

### Example 47: Synthesis of 7-[1-((1R,2R)-2-fluoro-cyclohexyl)-1H-benzoimidazol-5-yl]-5-((S)-3-hydroxymethyl-pyrrolidin-1-yl)-3H-thieno[3,4-d]pyrimidin-4-one

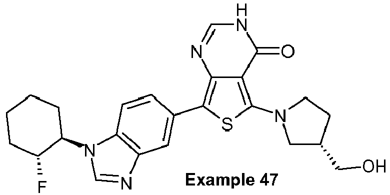

### Step 1: (4-bromo-2-nitro-phenyl)-((1R,2R)-2-fluoro-cyclohexyl)-amine (47a)

To a mixture of 4-bromo-1-fluoro-2-nitro-benzene (1g, 4.55 mmol) and (*1R,2R*)-2-fluoro-cyclohexylamine (0.639 g, 5.45 mmol) in anhydrous n-BuOH (10.1 mL) was added DIPEA (2.39 mL, 13.64 mmol) under nitrogen atmosphere and the reaction mixture was stirred 16 hours at 100°C.

(*1R,2R*)-2-fluoro-cyclohexylamine (266 mg, 2.27 mmol) was added again and stirred for an additional 16 hours. The reaction mixture was concentrated to dryness. The residue was partitioned between water and EtOAc and extracted with EtOAc. The combined organic layers were dried over sodium sulfate, filtered and evaporated under reduced pressure to afford compound **(47a)** as orange oil (1.61 g, 4.55 mmol, 100%) without further purification. MS *m*/*z* ([M+H]⁺) 317/319.

### Step 2: 4-bromo-((1R,2R)-2-fluoro-cyclohexyl)-benzene-1,2-diamine (47b)

According to the procedure described in example 46, step 2, compound **(47a)** (1.61 g, 4.55 mmol) was converted to compound **(47b)** as a brownish oil (1.27 g, 4.42 mmol, 97%) which was used in the next step without further purification. MS *m*/*z* ([M+H]⁺) 287/289.

### Step 3: 5-bromo-1-((1R,2R)-2-fluoro-cyclohexyl)-1H-benzoimidazole (47c)

According to the procedure described in example 46, step 3, compound **(47b)** (1.27 g, 4.42 mmol) was converted, after purification by column chromatography on silica gel (DCM/MeOH 98/2 to 95/5), to compound **(47c)** as a brown oil (1.14 g, 3.84 mmol, 84%). ¹H NMR (400 MHz, CDCl₃) δ 1.43-1.51 (m, 2H), 1.62-1.75 (m, 1H), 1.91-2.03 (m, 3H), 2.21-2.30 (m, 1H), 2.32-2.41 (m, 1H), 4.20-4.29 (m, 1H), 4.62-4.81 (m, 1H), 7.31 (d, J = 8.6 Hz, 1H), 7.39 (dd, J = 1.8/8.6 Hz, 1H), 7.95 (d, J = 1.5 Hz, 1H), 7.98 (s, 1H). MS *m*/*z* ([M+H]⁺) 297/299.

### Step 4: 1-((1R,2R)-2-fluoro-cyclohexyl)-5-(4,4,5,5-tetramethyl-[1,3,2]dioxaborolan-2-yl)-1H-benzoimidazole (47d)

According to the procedure described in example 46, step 4, compound **(47c)** (1.14 g, 3.83 mmol) was converted, after trituration in cyclohexane, to compound **(47d)** as a brown solid (1.09 g, 3.18 mmol, 83%) which was engaged in the following step without additional purification. ¹H NMR (400 MHz, CDCl₃) δ 1.37 (s, 12H), 1.44-1.53 (m, 2H), 1.63-1.76 (m, 1H), 1.92-2.01 (m, 3H), 2.21-2.29 (m, 1H), 2.32-2.40 (m, 1H), 4.25-4.33 (m, 1H), 4.68-4.87 (m, 1H), 7.44 (d, J = 8.2 Hz, 1H), 7.74 (dd, J = 0.9/8.2 Hz, 1H), 8.00 (s, 1H), 8.29 (s, 1H). MS *m*/*z* ([M+H]⁺) 345.

### Step 5: Methyl 2-chloro-5-[1-((1R,2R)-2-fluoro-cyclohexyl)-1H-benzoimidazol-5-yl]-4-nitrothiophene-3-carboxylate (47e)

According to the procedure described in example 46, step 5, compound **(1b)** (230 mg, 0.90 mmol) was converted, by reaction with compound **(47d)** (371 mg, 1.08 mmol) and after purification by flash chromatography on silica gel (DCM/MeOH 98/2), to compound **(47e)** (268 mg, 0.61 mmol, 57%). MS *m*/*z* ([M+H]⁺) 438.

### Step 6: Methyl 5-[1-((1R,2R)-2- fluoro-cyclohexyl)-1H-benzoimidazol-5-yl]-2-((S)-3-hydroxymethyl-pyrrolidin-1-yl)-4-nitro-thiophene-3-carboxylate (47f)

According to the procedure described in example 15, step 1, compound **(47e)** (225 mg, 0.51 mmol) was converted, by reaction with (S)-1-Pyrrolidin-3-yl-methanol (72.8 mg, 0.72 mmol) and after purification by preparative TLC (DCM/MeOH 95/5), to compound **(47f)** as orange oil (233 mg, 0.46 mmol, 90%). ¹H NMR (400 MHz, DMSO-*d₆*) δ 1.46-1.51 (m, 2H), 1.63-1.70 (m, 1H), 1.78-1.86 (m, 3H), 2.03-2.11 (m, 3H), 2.24 (bs, 1H), 2.45-2.50 (m, 1H), 3.16-3.20 (m, 1H), 3.34-3.50 (m, 5H), 3.70 (s, 3H), 4.57-4.66 (m, 1H), 4.79 (t, J = 5.3 Hz, 1H), 4.90-5.10 (m, 1H), 7.25 (dd, J = 1.7/8.5 Hz, 1H), 7.66 (d, J = 1.5 Hz, 1H), 7.80 (d, J = 8.5 Hz 1H), 8.54 (s, 1H). MS *m*/*z* ([M+H]⁺) 503.

### Step 7: Methyl 4-amino-5-[1-((1R,2R)-2-fluoro-cyclohexyl)-1H-benzoimidazol-5-yl]-2-((S)-3-hydroxymethyl-pyrrolidin-1-yl)-thiophene-3-carboxylate (47g)

According to the procedure described in example 1, step 5, compound **(47f)** (230 mg, 0.46 mmol) was converted to compound **(47g)** as an orange solid (187 mg, 0.39 mmol, 86%) which was used without further purification. MS *m*/*z* ([M+H]⁺) 473.

### Step 8: 7-[1-((1R,2R)-2-fluoro-cyclohexyl)-1H-benzoimidazol-5-yl]-5-((S)-3-hydroxymethyl-pyrrolidin-1-yl)-3H-thieno[3,4-d]pyrimidin-4-one (Example 47)

According to the procedure described in example 1, step 6, compound **(47g)** (185 mg, 0.39 mmol) was converted to **(Example 47)** as a yellow solid (38 mg, 0.08 mmol, 20%). ¹H NMR (400 MHz, DMSO-*d₆*) δ 1.48-1.53 (m, 2H), 1.62-1.69 (m, 1H), 1.78-1.85 (m, 3H), 2.03-2.11 (m, 3H), 2.23 (bs, 1H), 2.45-2.50 (m, 1H), 3.39-3.50 (m, 3H), 3.59-3.69 (m, 3H), 3.50-3.58 (m, 1H), 4.76 (t, J = 5.3 Hz, 1H), 4.90-5.09 (m, 1H), 7.64-7.66 (m, 2H), 7.74 (dd, J = 1.6/8.6 Hz, 1H), 8.20 (d, J = 1.3 Hz, 1H), 8.38 (s, 1H), 11.0 (d, J = 3.3 Hz, 1H). MS *m*/*z* ([M+H]⁺) 468.

### Example 48: Synthesis of 5-((S)-3-Hydroxymethyl-pyrrolidin-1-yl)-7-[1-(trans-3-ethylcyclohexyl)-1H-benzoimidazol-5-yl]-3H-thieno[3,4-d]pyrimidin-4-one

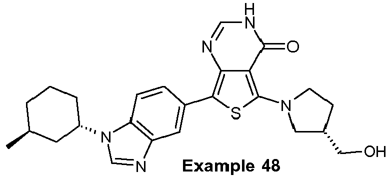

### Step 1: Methyl 2-Chloro-5-[1-(trans-3-methyl-cyclohexyl)-1H-benzoimidazol-5-yl]-4-nitrothiophene-3-carboxylate (48a)

According to the procedure described in example 46, step 5, compound **(1b)** (230 mg, 0.90 mmol) was converted, by reaction with 1-(*trans*-3-methyl-cyclohexyl)-5-(4,4,5,5-tetramethyl-[1,3,2]dioxaborolan-2-yl)-1H-benzoimidazole (336 mg, 0.99 mmol) and after purification by flash chromatography on silica gel (DCM/MeOH 95/5) then preparative TLC (DCM/MeOH 95/5), to compound **(48a)** (265 mg, 0.61 mmol, 68%). MS *m*/*z* ([M+H]⁺) 434.

### Step 2: Methyl 2-((S)-3-hydroxymethyl-pyrrolidin-1-yl)-5-[1-(trans-3-methyl-cyclohexyl)-1H-benzoimidazol-5-yl]-4-nitro-thiophene-3-carboxylate (48b)

According to the procedure described in example 15, step 1, compound **(48a)** (263 mg, 0.61 mmol) was converted, by reaction with (S)-1-pyrrolidin-3-yl-methanol (74 mg, 0.73 mmol) and after purification by preparative TLC (DCM/MeOH 95/5), to compound **(48b)** (267 mg, 0.54 mmol, 88%). ¹H NMR (400 MHz, DMSO-*d₆*) δ 1.07 (s, 3H), 1.09-1.11 (m, 3H), 1.41-1.43 (m, 1H), 1.58-1.66 (m, 2H), 1.76-1.89 (m, 3H), 2.02-2.15 (m, 3H), 2.40-2.50 (m, 1H), 3.35-3.49 (m, 5H), 3.69 (m, 3H), 4.60-4.67 (m, 1H), 4.78 (t, J = 5.2 Hz, 1H), 7.23 (dd, J = 1.7/8.5 Hz, 1H), 7.64 (d J = 1.5 Hz, 1H), 7.74 (d, J = 8.5 Hz, 1H), 8.45 (s, 1H), 1H under solvent. MS *m*/*z* ([M+H]⁺) 499.

### Step 3: Methyl 4-amino-2-((S)-3-hydroxymethyl-pyrrolidin-1-yl)-5-[1-(trans-3-methyl-cyclohexyl)-1H-benzoimidazol-5-yl]-thiophene-3-carboxylate (48c)

According to the procedure described in example 1, step 5, compound **(48b)** (265 mg, 0.21 mmol) was converted to compound **(48c)** as a yellow solid (219 mg, 0.47 mmol, 87%) which was used without further purification. MS *m*/*z* ([M+H]⁺) 469.

### Step 4: 5-((S)-3-Hydroxymethyl-pyrrolidin-1-yl)-7-[1-(trans-3-ethyl-cyclohexyl)-1H-benzoimidazol-5-yl]-3H-thieno[3,4-d]pyrimidin-4-one (Example 48)

According to the procedure described in example 1, step 6, compound **(48c)** (218 mg, 0.46 mmol) was converted to **(Example 48)** as a yellow solid (39 mg, 0.08 mmol, 18%). ¹H NMR (400 MHz, DMSO-*d₆*) δ 1.10 (d, J = 7.0 Hz, 3H), 1.40-1.43 (m, 1H), 1.59-1.66 (m, 2H),1.69-1.91(m, 4H), 1.99-2.15 (m, 4H), 3.37-3.50 (m, 4H), 3.59-3.68 (m, 3H), 7.59 (d, J = 8.6 Hz, 1H), 7.64 (d, J = 3.3 Hz, 1H), 7.74 (dd, J = 1.6/8.6 Hz 1H), 8.19-8.20 (d, J = 1.3 Hz, 1H), 8.31 (s, 1H), 10.99-11.00 (d, J = 3.1 Hz, 1H). MS *m*/*z* ([M+H]⁺) 464.

### Example 49: Synthesis of 5-((S)-3-hydroxymethyl-pyrrolidin-1-yl)-7-[1-(tetrahydro-pyran-3-yl)-1-H-benzoimidazol-5-yl]-3H-thieno[3,4-d]pyrimidin-4-one

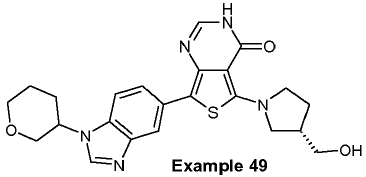

### Step 1: (4-bromo-2-nitro-phenyl)-(tetrahydro-pyran-3-yl)-amine (49a)

According to the procedure described in example 46, step 1, 4-bromo-1-fluoro-2-nitro-benzene (1.5 g, 6.82 mmol) was converted, by reaction with tetrahydro-2H-pyran-3-amine (0.828 g, 8.18 mmol), to compound **(49a)** as an orange solid (2.1 g, 6.82 mmol, 100%) without further purification. MS *m*/*z* ([M+H]⁺) 301/303.

### Step 2: 4-bromo-N*1*-(tetrahydro-pyran-3-yl)-benzene-1,2-diamine (49b)

According to the procedure described in example 46, step 2, compound **(49a)** (2.1 g, 6.82 mmol) was converted to compound **(49b)** as a brownish oil (1.86 g, 6.82 mmol, 100%) which was used in the next step without further purification. MS *m*/*z* ([M+H]⁺) 271/273.

### Step 3: 5-bromo-1-(tetrahydro-pyran-3-yl)-1H-benzoimidazole (49c)

According to the procedure described in example 46, step 3, compound **(49b)** (1.86 g, 6.82 mmol) was converted, after purification by column chromatography on silica gel (DCM/MeOH 98/2 to 95/5), to compound **(49c)** as a brown oil (1.92 g, 6.82 mmol, 100%). ¹H NMR (400 MHz, CDCl₃) δ 1.71-1.89 (m, 2H), 2.09-2.18 (m, 1H), 2.23-2.30 (m, 1H), 3.70-3.76 (m, 1H), 3.83-3.93 (m, 2H), 4.11-4.15 (m, 1H), 4.37-4.42 (m, 1H), 7.28 (d, J = 8.5 Hz, 1H), 7.41 (dd, J = 1.8/8.5 Hz, 1H), 7.96 (d, J = 1.8 Hz, 1H), 8.19 (s, 1H). MS *m*/*z* ([M+H]⁺) 281/283.

### Step 4: 1-(tetrahydro-pyran-3-yl)-5-(4,4,5,5-tetramethyl-[1,3,2]dioxaborolan-2-yl)-1H-benzoimidazole (49d)

According to the procedure described in example 46, step 4, compound **(49c)** (1.92 g, 6.82 mmol) was converted, after trituration in cyclohexane, to compound **(49d)** as a brown solid (1.76 g, 5.35 mmol, 78%) without further purification. ¹H NMR (400 MHz, CDCl₃) δ 1.37 (s, 12H), 1.73-1.89 (m, 2H), 2.11-2.20 (m, 1H), 2.22-2.30 (m, 1H), 3.68-3.74 (m, 1H), 3.80-3.85 (m, 1H), 3.89-3.94 (m, 1H), 4.12-4.16 (m, 1H), 4.41-4.47 (m, 1H), 7.41 (d, J = 8.2 Hz, 1H), 7.75 (dd, J = 0.9/8.2 Hz, 1H), 8.19 (s, 1H), 8.29 (bt, 1H). MS *m*/*z* ([M+H]⁺) 329.

### Step 5: Methyl 2-chloro-4-nitro-5-[1-(tetrahydro-pyran-3-yl)-1H-benzoimidazol-5-yl]-thiophene-3-carboxylate (49e)

According to the procedure described in example 46, step 5, compound **(49d)** (230 mg, 0.90 mmol) was converted, after purification by flash chromatography on silica gel (DCM/MeOH 95/5) then preparative TLC (DCM/MeOH 95/5), to compound (49e) as an orange solid (270 mg, 0.64 mmol, 71%). MS *m*/*z* ([M+H]⁺) 422.

### Step 6: Methyl 2-((S)-3-hydroxymethyl-pyrrolidin-1-yl)-4-nitro-5-[1-(tetrahydro-pyran-3-yl)-1H-benzoimidazol-5-yl]-thiophene-3-carboxylate (49f)

According to the procedure described in example 15, step 1, compound **(49e)** (270 mg, 0.64 mmol) was converted, by reaction with (S)-3-hydroxymethyl-pyrrolidine (78 mg, 0.77 mmol) and after purification by preparative TLC (DCM/MeOH 95/5), to compound **(49f)** as a red oil (300 mg, 0.62 mmol, 96%). ¹H NMR (400 MHz, DMSO-*d₆*) δ 1.73-1.84 (m, 3H), 2.03-2.11 (m, 1H), 2.14-2.19 (m, 2H), 3.36-3.49 (m, 6H), 3.55-3.61 (m, 1H), 3.70 (s, 3H), 3.76-3.88 (m, 3H), 4.00 (dd, J = 3.9/11.1 Hz, 1H), 4.56-4.63 (m, 1H), 4.79 (t, J = 5.2 Hz, 1H), 7.25 (dd, J = 1.7/8.4 Hz, 1H), 7.66 (d, J = 1.4 Hz, 1H), 7.79 (d, J = 8.3 Hz, 1H), 8.49 (s, 1H). MS *m*/*z* ([M+H]⁺) 487.

### Step 7: Methyl 4-Amino-2-((S)-3-hydroxymethyl-pyrrolidin-1-yl)-5-[1-(tetrahydro-pyran-3-yl)-1H-benzoimidazol-5-yl]-thiophene-3-carboxylate (49g)

According to the procedure described in example 1, step 5, compound **(49f)** (300 mg, 0.62 mmol) was converted to compound **(49g)** as a yellow solid (255 mg, 0.56 mmol, 91%) which was used without further purification. MS *m*/*z* ([M+H]⁺) 457.

### Step 8: 5-((S)-3-hydroxymethyl-pyrrolidin-1-yl)-7-[1-(tetrahydro-pyran-3-yl)-1-H-benzoimidazol-5-yl]-3H-thieno[3,4-d]pyrimidin-4-one (Example 49)

According to the procedure described in example 1, step 6, compound **(49g)** (255 mg, 0.56 mmol) was converted, after purification by preparative TLC (DCM/MeOH 95/5), to **(Example 49)** as a yellow solid (28 mg, 0.06 mmol, 11%). ¹H NMR (400 MHz, CD₃OD) δ 1.80-1.92 (m, 3H), 2.15-2.28 (m, 3H) 2.59 (q, J = 7.0 Hz, 1H), 3.50-3.55 (dd, J = 6.8/10.6 Hz, 1H), 3.57-3.66 (m, 2H), 3.69-3.77 (m, 4H), 3.87-3.95 (m, 2H), 4.13 (dd, J = 3.4/11.6 Hz, 1H), 4.58 (sep, J = 3.9 Hz, 1H), 7.57-7.61 (m, 2H), 7.83 (dd, J = 1.6/8.6 Hz, 1H), 8.13 (d, J = 1.3 Hz, 1H), 8.40 (s, 1H). MS *m*/*z* ([M+H]⁺) 452.

### Example 50: Synthesis of 7-(1H-benzoimidazol-5-yl)-5-((S)-3-hydroxymethyl-pyrrolidin-1-yl)-3H-thieno[3,4-d]pyrimidin-4-one

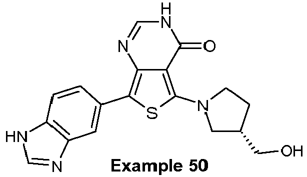

### Step 1: 5-bromo-1-(2-trimethylsilanyl-ethoxymethyl)-1H-benzoimidazole (50a)

At 0°C under nitrogen atmosphere, Sodium hydride 60% in mineral oil (101 mg, 2.54 mmol) washed with pentane was added to a solution of 5-bromo-1H-benzoimidazole (500mg, 2.54 mmol) in anhydrous DMF (5 mL). After 15 minutes, 2-(trimethylsilyl)ethoxymethyl chloride (477 µL, 2.69 mmol) was added and the mixture was stirred for one hour. A saturated solution of ammonium chloride was added to the middle. The aqueous layer was extracted with EtOAc and the combined organic layers were dried over sodium sulfate and concentrated under reduced pressure. The crude was purified by flash chromatography on silica gel (cyclohexane/EtOAc 6/4) to provide compound **(50a)** as uncoloured oil (370 mg, 1.13 mmol, 44%). MS *m*/*z* ([M+H]⁺) 327/329.

### Step 2: 5-(4,4,5,5-tetramethyl-[1,3,2]dioxaborolan-2-yl)-1-(2-trimethylsilanyl-ethoxymethyl)-1H-benzoimidazole (50b)

According to the procedure described in example 46, step 4, compound **(50a)** (119 mg, 0.36 mmol) was converted, after purification by flash chromatography on silica gel (DCM/MeOH 97/3), to compound **(50b)** as a yellow oil (133 mg, 0.35 mmol, 97%). MS *m*/*z* ([M+H]⁺) 375.

### Step 3: Methyl 2-chloro-4-nitro-5-[1-(2-trimethylsilanyl-ethoxymethyl)-1H-benzoimidazol-5-yl]-thiophene-3-carboxylate (50c)

According to the procedure described in example 1, step 3, compound **(1b)** (76 mg, 0.30 mmol) was converted, by reaction with compound **(50b)** (133 mg, 0.36 mmol) and after purification by flash chromatography on silica gel (DCM/MeOH 95/5) then preparative TLC (DCM/MeOH 95/5), to compound **(50c)** as a yellow solid (100 mg, 0.21 mmol, 60%).¹H NMR (400 MHz, DMSO-*d₆*) δ 0.08 (s, 9H), 0.84 (t, J = 8.0 Hz, 2H), 3.50 (t, J = 8.0 Hz, 2H), 3.87 (s, 3H), 5.68 (s, 2H), 7.43 (dd, J = 1.7/8.4 Hz, 1H), 7.79 (d, J = 8.4 Hz, 1H), 7.88 (d, J = 1.3 Hz, 1H), 8.53 (s, 1H). MS *m*/*z* ([M+H]⁺) 468.

### Step 4: Methyl 2-((S)-3-hydroxymethyl-pyrrolidin-1-yl)-4-nitro-5-[1-(2-trimethylsilanyl-ethoxymethyl)-1H-benzoimidazol-5-yl]-thiophene-3-carboxylate (50d)

According to the procedure described in example 15, step 1, compound **(50c)** (100 mg, 0.21 mmol) was converted, by reaction with (S)-1-pyrrolidin-3-yl-methanol (32.4 mg, 0.32 mmol) and after purification by preparative TLC (DCM/MeOH 95/5), to compound **(50d)** as an orange solid (77 mg, 0.14 mmol, 68%). MS *m*/*z* ([M+H]⁺) 533.

### Step 5: Methyl 4-amino-2-((S)-3-hydroxymethyl-pyrrolidin-1-yl)-5-[1-(2-trimethylsilanyl-ethoxymethyl)-1H-benzoimidazol-5-yl]-thiophene-3-carboxylate (50e)

According to the procedure described in example 1, step 5, compound **(50d)** (77 mg, 0.14 mmol) was converted to compound **(50e)** as colored oil (66 mg, 0.13 mmol, 90%) which was used in the next step without further purification. MS *m*/*z* ([M+H]⁺) 503.

### Step 6: 5-((S)-3-hydroxymethyl-pyrrolidin-1-yl)-7-[1-(2-trimethylsilanyl-ethoxymethyl)-1H-benzoimidazol-5-yl]-3H-thieno[3,4-d] pyrimidin-4-one (50f)

According to the procedure described in example 1, step 6, compound **(50e)** (66 mg, 0.13 mmol) was converted, after purification by preparative TLC (DCM/MeOH 95/5), to compound **(50f)** as a yellow solid (17 mg, 0.03 mmol, 26%). MS *m*/*z* ([M+H]⁺) 498.

### Step 7: 7-(1H-benzoimidazol-5-yl)-5-((S)-3-hydroxymethyl-pyrrolidin-1-yl)-3H-thieno[3,4-d]pyrimidin-4-one (Example 50)

To a solution of compound **(50f)** (40 mg, 0.03 mmol) in anhydrous methanol (200 µL) under nitrogen atmosphere was added hydrochloric acid 4N in 1,4-dioxane (88 µL, 0.35 mmol). The mixture was stirred at room temperature for 3 hours. Hydrochloric acid 32% (60 µL) was added and the mixture was stirred at 55°C for 12 hours. The reaction mixture was concentrated under reduced pressure and the crude residue was purified by preparative TLC (DCM/MeOH 8/2) to provide **(Example 50)** (8.5 mg, 0.02 mmol, 65%). ¹H NMR (400 MHz, DMSO-*d₆*) δ 1.24 (s, 2H), 1.78-1.83 (m, 1H), 2.02-2.9 (m, 1H), 3.38-3.49 (m, 3H), 3.62-3.67 (m, 2H), 4.80 (bs, 1H), 7.64-7.72 (m, 3H), 8.20-8.25 (m, 3H), 11.06 (s, 1H). MS *m*/*z* ([M+H]⁺) 368.

### Example 51: Synthesis of 7-(1-Cyclohexyl-1H-benzoimidazol-5-yl)-5-(3-hydroxy-propyl)-3,7-dihydro-pyrrolo[2,3-d]pyrimidin-4-one

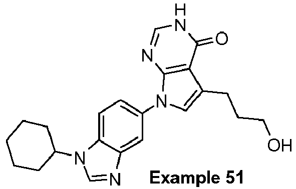

### Step 1: 5-Bromo-4-chloro-7-(1-cyclohexyl-1H-benzoimidazol-5-yl)-7H-pyrrolo[2,3-d]pyrimidine (51a)

To a solution of 7-bromo-6-chloro-7-deazapurine (3.62 g, 15.6 mmol) in MeOH (100 mL) were added (1-cyclohexyl-1H-benzimidazol-5-yl)pinacol boronate (5.01g, 15.60 mmol, prepared as described in patent WO2012/172043), copper (II) acetate (2.83 g, 15.60 mmol) and TMEDA (4.68 mL, 31.29 mmol). The reaction mixture was stirred for 3 days at 50°C. The reaction mixture was cooled to room temperature and *concentrated in vacuo.* Water was added to the residue and the aqueous layer was extracted with EtOAc. The organic layer was dried over sodium sulfate and *concentrated in vacuo.* The residue was purified by flash chromatography on silica gel (cyclohexane/EtOAc 7/3) to provide compound **(51a)** as a white powder (2.85 g, 6.61 mmol, 42%). ¹H NMR (300 MHz, CDCl₃) δ 1.37 (m, 1H), 1.54 (m, 2H), 1.78-1.91 (m, 3H), 2.01 (d, J = 13.6 Hz, 2H), 2.25 (d, J = 12.5 Hz, 2H), 4.25 (tt, J = 3.6/11.9 Hz, 1H), 7.56 (m, 2H), 7.61 (s, 1H), 7.95 (s, 1H), 8.11 (s, 1H), 8.66 (s, 1H). MS *m*/*z* ([M+H]⁺) 430/432.

### Step 2: 4-Benzyloxy-5-bromo-7-(1-cyclohexyl-1H-benzoimidazol-5-yl)-7H-pyrrolo[2,3-d]pyrimidine (51b)

Under a nitrogen atmosphere, sodium hydride 60% in oil (334 mg, 8.36 mmol) was added portion wise to a solution of benzyl alcohol (0.872 mL, 8.36 mmol) in dry THF (50 mL). The mixture was stirred 10 minutes then a solution of compound **(51a)** (2.40 g, 5.57 mmol) in dry THF (25 mL) was added slowly at 0°C. At the end of addition, the mixture was heated at 90°C for 2 hours. The reaction mixture was cooled to room temperature and *concentrated in vacuo.* Water was added to the residue and the aqueous layer was extracted with ethyl acetate. The heterogeneous organic layer was concentrated *in vacuo* and dried by co-distilation with toluene. The residue was purified by flash chromatography on silica gel (DCM/MeOH 98/2) to provide compound **(51b)** as a grey solid (2.62 g, 5.21 mmol, 88%). ¹H NMR (400 MHz, CDCl₃) δ 1.29-1.42 (m, 1H), 1.49-1.61 (m, 2H), 1.79-1.94 (m, 3H), 1.97-2.07 (m, 2H), 2.22-2.32 (m, 2H), 4.20-4.29 (m, 1H), 5.71 (s, 2H), 7.34-7.38 (m, 1H), 7.42-7.46 (m, 3H), 7.59-7.64 (m, 4H), 7.97 (s, 1H), 8.10 (s, 1H), 8.52 (s, 1H). MS *m*/*z* ([M+H]⁺) 502/504.

### Step 3: 4-Benzyloxy-7-(1-cyclohexyl-1H-benzoimidazol-5-yl)-5-iodo-7H-pyrrolo[2,3-d]pyrimidine (51c)

Under nitrogen atmosphere compound **(51b)** (2.60 g, 5.17 mmol), copper (I) iodide (197 mg, 1.03 mmol) and sodium iodide (3.10g, 20.70 mmol) were solubilized in dry 1,4-dioxane (50 mL). To the mixture was added DMEDA (0.291 ml, 2.07 mmol) and the reaction was stirred at 110°C for 4 days. The reaction mixture was cooled to room temperature and concentrated *in vacuo.* The middle was quenched with 10% aqueous ammonia solution and the aqueous layer was extracted with DCM. The organic layer was washed with water, dried over sodium sulfate and *concentrated in vacuo.* The residue was purified by preparative TLC (DCM/MeOH 95/5) to provide intermediate compound **(51c)** as a beige solid (1.38 g, 2.51 mmol, 48%). ¹H NMR (300 MHz, CDCl₃) δ 1.36 (qt, J = 3.4/12.7 Hz, 1H), 1.47-1.62 (m, 2H), 1.74-1.92 (m, 3H), 2.01 (d, J = 13.4 Hz, 2H), 2.25 (d, J = 11.0 Hz, 2H), 4.23 (tt, J = 3.7/11.9 Hz, 1H), 5.68 (s, 2H), 7.31-7.44 (m, 3H), 7.47 (s, 1H), 7.55 (d, J = 1.1 Hz, 2H), 7.64 (d, J = 7.2 Hz, 2H), 7.93 (s, 1H), 8.07 (s, 1H), 8.49 (s, 1H). MS *m*/*z* ([M+H]⁺) 550.

### Step 4: 3-[4-Benzyloxy-7-(1-cyclohexyl-1H-benzoimidazol-5-yl)-7H-pyrrolo[2,3-d]pyrimidin-5-yl]-prop-2-yn-1-ol (51d)

To a solution of compound **(51c)** (48mg, 0.087 mmol) in dry DMF (1mL) under argon atmosphere was added triethylamine (21 µL, 0.174 mmol), copper (I) iodide (5 mg, 0.026 mmol), propargyloxytrimethylsilane (16 µL, 0.104 mmol) and Pd(PPh₃)₄ (10 mg, 0.001 mmol). The mixture was heated at 50°C for 18 hours. The reaction mixture was cooled to room temperature and concentrated *in vacuo.* The residue was purified by preparative TLC (DCM/MeOH 9/1) to provide compound **(51d)** as yellow solid (18 mg, 0.037mmol, 43%). MS *m*/*z* ([M+H]⁺) 478.

### Step 5: 7-(1-Cyclohexyl-1H-benzoimidazol-5-yl)-5-(3-hydroxy-propyl)-3,7-dihydro-pyrrolo[2,3-d]pyrimidin-4-one (Example 51)

A solution of compound **(51d)** (18 mg, 0.037 mmol) in a mixture of ethanol (1 mL) and DCM (0.5 mL) was degassed with argon. The catalyst Pd(OH)₂ (5 mg, 10% on charcoal, 0.003 mmol) was added to the mixture. The resulting reaction mixture was stirred at room temperature under 5 bars of hydrogen atmosphere for 3 days. The resulting mixture was filtered through PTFE isodisc 0.2µm and the filtrate was concentrated under reduced pressure. The residue was purified by preparative TLC (DCM/MeOH 9/1) to provide **(Example 51)** as beige solid (2.8 mg, 0.007 mmol, 19%). ¹H NMR (300 MHz, CD₃OD) δ 1.36-1.47 (m, 1H), 1.53-1.67 (m, 2H), 1.83 (d, J = 12.7 Hz, 1H), 1.89-2.01 (m, 6H), 2.22 (d, J = 11.6 Hz, 2H), 2.95 (t, J = 7.3 Hz, 2H), 3.64 (t, J = 6.4 Hz, 2H), 4.40-4.48 (m, 1H), 7.16 (s, 1H), 7.55 (dd, J = 1.9/8.7 Hz, 1H), 7.76 (d, J = 8.6 Hz, 1H), 7.84 (s, 1H), 7.89 (d, J = 1.6Hz, 1H), 8.38 (s, 1H). MS *m*/*z* ([M+H]⁺) 392.

### Example 52: Synthesis of 7-(1-cyclohexyl-1H-benzoimidazol-5-yl)-5-(1,2,3,6-tetrahydro-pyridin-4-yl)-3,7-dihydro-pyrrolo[2,3-d]pyrimidin-4-one hydrochloride

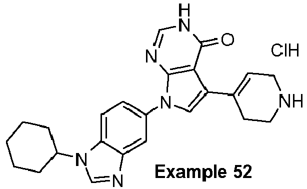

### Step 1: 5-Bromo-7-(1-cyclohexyl-1H-benzoimidazol-5-yl)-3,7-dihydro-pyrrolo[2,3-d]pyrimidin-4-one (52a)

Compound **(51a)** (500 mg, 1.16 mmol) was added to a solution of HCl 1N aqueous (23 mL, 23 mmol) and refluxed with stirring for 24 hours. The mixture was cooled to 0°C and formed precipitate was isolated by filtration, washed with water and dried to provide compound **(52a)** as a white powder (276 mg, 0.669 mmol, 66%). ¹H NMR (300 MHz, DMSO-*d₆*) δ 1.22-1.38 (m, 1H), 1.44-1.62 (m, 2H), 1.68-1.80 (m, 1H), 1.82-1.98 (m, 4H), 2.09-2.22 (m, 2H), 4.57-4.71 (m, 1H), 7.64 (d, J = 9.7 Hz, 1H), 7.74 (s, 1H), 7.95-8.00 (m, 3H), 8.89 (s, 1H), 12.22 (s, 1H). MS *m*/*z* ([M+H]⁺) 412/414.

### Step 2: tert-butyl 4-[7-(1-cyclohexyl-1H-benzoimidazol-5-yl)-4-oxo-4,7-dihydro-3H-pyrrolo[2,3-d]pyrimidin-5-yl]-3,6-dihydro-2H-pyridine-1-carboxylate (52b)

A solution of compound **(52a)** (191 mg, 0.463 mmol), N-Boc-1,2,3,6-tetrahydropyridine-4-boronic acid pinacol ester (143mg, 0.463 mmol) and potassium carbonate (64 mg, 0.926 mmol) in a mixture of 1,4-dioxane (5 mL) and water (1 mL) was degassed with argon. The catalyst Pd(PPh₃)₄ (27 mg, 0.023 mmol) was added to the middle. The sealed reaction vial was placed in the microwave reactor and stirred at 120 °C for 2 hours. The reaction mixture was cooled to room temperature and concentrated *in vacuo*. Water was added to the residue and the aqueous layer was extracted with DCM. The organic layer was dried over sodium sulfate and concentrated *in vacuo.* The residue was purified by preparative TLC (DCM/MeOH 9/1) to provide compound **(52b)** (146 mg, 0.284 mmol, 61%). MS *m*/*z* ([M+H]⁺) 515.

### Step 2: 7-(1-cyclohexyl-1H-benzoimidazol-5-yl)-5-(1,2,3,6-tetrahydro-pyridin-4-yl)-3,7-dihydro-pyrrolo[2,3-d]pyrimidin-4-one hydrochloride (Example 52)

According to the procedure described in example 12, step 4, compound **(52b)** (146 mg, 0.284 mmol) was converted to **(Example 52)** (111 mg, 0.246 mmol, 86%). ¹H NMR (300 MHz, DMSO-*d₆*) δ 1.24-1.37 (m, 1H), 1.48-1.61 (m, 2H), 1.75 (d, J = 11.6 Hz, 1H), 1.84-1.92 (m, 4H), 2.17 (d, J = 9.0 Hz, 2H), 2.78 (bs, 2H), 3.31 (bs, 2H), 3.77 (s, 2H), 4.63-4.71 (m, 1H), 7.25 (s, 1H), 7.70 (s, 1H), 7.84 (dd, J = 1.5/8.8 Hz, 1H), 8.00 (d, J = 3.7 Hz, 1H), 8.15-8.20 (m, 2H), 9.18 (bs, 2H), 9.46 (s, 1H), 12.27 (s, 1H). MS *m*/*z* ([M+H]⁺) 415.

### Example 53: Synthesis of 7-(1-cyclohexyl-1H-benzoimidazol-5-yl)-5-(1-methyl-1,2,3,6-tetrahydro-pyridin-4-yl)-3,7-dihydro-pyrrolo[2,3-d]pyrimidin-4-one

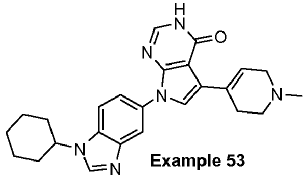

According to the procedure described in example 38, step 1, **(Example 52)** (105 mg, 0.232 mmol) was converted, after purification by preparative TLC (DCM/MeOH 9/1), to **(Example 53)** (48 mg, 0.112 mmol, 48%). ¹H NMR (300 MHz, DMSO-*d₆*) δ 1.28-1.37 (m, 1H), 1.45-1.58 (m, 2H), 1.73 (d, J = 12.6 Hz, 1H), 1.79-1.93 (m, 4H), 2.07 (d, J = 9.0 Hz, 2H), 2.36 (s, 3H), 2.55-2.70 (m, 4H), 3.13 (s, 2H), 4.35-4.50 (m, 1H), 7.08 (s, 1H), 7.45 (s, 1H), 7.51 (dd, J = 2.0/8.7 Hz, 1H), 7.80 (d, J = 8.7 Hz, 1H), 7.88-7.93 (m, 2H), 8.45 (s, 1H), 12.04 (d, J = 3.3 Hz, 1H). MS *m*/*z* ([M+H]⁺) 429.

### Example 54: Synthesis of 7-(1-Cyclohexyl-1H-benzoimidazol-5-yl)-5-(3-hydroxymethyl-pyrrolidin-1-yl)-3,7-dihydro-pyrrolo[2,3-d]pyrimidin-4-one

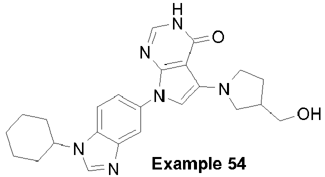

### Step 1: {1-[4-Benzyloxy-7-(1-cyclohexyl-1H-benzoimidazol-5-yl)-7H-pyrrolo[2,3-d]pyrimidin-5-yl]-pyrrolidin-3-yl}-methanol (54a)

A solution of compound **(51c)** (40 mg, 0.089 mmol), 3-pyrrolidine methanol (27 mg, 0.266 mmol), potassium carbonate (37 mg, 0.266 mmol), copper (I) iodide (3 mg, 0.018 mmol) and L-proline (4 mg, 0.036 mmol) was solubilized in dry DMSO (1mL) under argon atmosphere. The mixture was heated to 90°C for 18 hours under argon and then concentrated under reduced pressure. The residue was purified twice by preparative TLC (DCM/MeOH 95/5) then dried *in vacuo* to compound **(54a)** (10 mg, 0.019mmol, 21%). MS *m*/*z* ([M+H]⁺) 523.

### Step 2: 7-(1-Cyclohexyl-1H-benzoimidazol-5-yl)-5-(3-hydroxymethyl-pyrrolidin-1-yl)-3,7-dihydro-pyrrolo[2,3-d]pyrimidin-4-one (Example 54)

According to the procedure described in example 51, step 5, compound **(54a)** (10 mg, 0.019 mmol) was converted, after purification by preparative TLC (DCM/MeOH 9/1), to **(Example 54)** as white solid (3.3 mg, 0.008 mmol, 40%). ¹H NMR (300 MHz, CDCl₃) δ 1.25-1.40 (m, 1H), 1.45-1.56 (m, 2H), 1.76-1.85 (m, 4H), 1.98-2.02 (m, 2H), 2.10-2.26 (m, 3H), 2.55-2.63 (m, 1H), 3.22-3.30 (m, 1H), 3.33-3.39 (m, 1H), 3.42-3.49 (m, 2H), 3.66-3.77 (m, 2H), 4.17-4.28 (m, 1H), 6.53 (s, 1H), 7.52 (s, 2H), 7.80 (s, 1H), 7.93 (s, 1H), 8.06 (s, 1H), 10.66 (s, 1H). MS *m*/*z* ([M+H]⁺) 433.

### Example 55: Synthesis of 7-(1-Cyclohexyl-1H-benzoimidazol-5-yl)-5-((S)-3-hydroxymethyl-pyrrolidin-1-yl)-3,7-dihydro-pyrrolo[2,3-d]pyrimidin-4-one

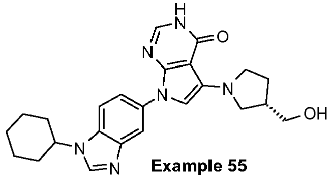

### Step 1: {(S)-1-[4-Benzyloxy-7-(1-cyclohexyl-1H-benzoimidazol-5-yl)-7H-pyrrolo[2,3-d]pyrimidin-5-yl] pyrrolidin-3-yl}-methanol (55a)

According to the procedure described in example 54, step 1, compound **(51c)** (100 mg, 0.182 mmol) was converted, by reaction with 3-(S)-pyrrolidine methanol (55 mg, 0.545 mmol) and after purification by preparative TLC (DCM/MeOH 9/1), to compound **(55a)** as orange oil (43 mg, 0.082mmol, 45%). MS *m*/*z* ([M+H]⁺) 523.

### Step 2: 7-(1-Cyclohexyl-1H-benzoimidazol-5-yl)-5-((S)-3-hydroxymethyl-pyrrolidin-1-yl)-3,7-dihydro-pyrrolo[2,3-d]pyrimidin-4-one (Example 55)

According to the procedure described in example 51, step 5, compound **(55a)** (32 mg, 0.060 mmol) was converted, after purification by preparative TLC (DCM/MeOH 9/1), to **(Example 55)** as yellow solid (3.3 mg, 0.008 mmol, 40%). ¹H NMR (300 MHz, CDCl₃) δ 1.29-1.38 (m, 1H), 1.47-1.57 (m, 2H), 1.78-1.86 (m, 4H), 1.99 (d, J = 13.4 Hz, 2H), 2.15-2.25 (m, 3H), 2.56-2.62 (m, 1H), 3.22-3.29 (m, 1H), 3.34-3.39 (m, 1H), 3.40-3.49 (m, 2H), 3.67-3.76 (m, 2H), 4.18-4.26 (m, 1H), 6.53 (s, 1H), 7.51 (s, 2H), 7.83 (s, 1H), 7.93 (s, 1H), 8.06 (s, 1H), 11.54 (s, 1H). MS *m*/*z* ([M+H]⁺) 433.

### Example 56: Synthesis of 7-(1-Cyclohexyl-1H-benzoimidazol-5-yl)-5-(4-hydroxymethyl-piperidin-1-yl)-3,7-dihydro-pyrrolo[2,3-d]pyrimidin-4-one

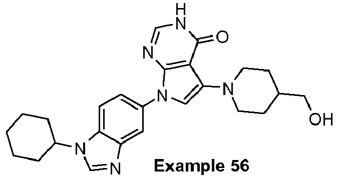

### Step 1: {1-[4-Benzyloxy-7-(1-cyclohexyl-1H-benzoimidazol-5-yl)-7H-pyrrolo[2,3-d]pyrimidin-5-yl]-piperidin-4-yl}-methanol (56a)

According to the procedure described in example 54, step 1, compound **(51c)** (100 mg, 0.182 mmol) was converted, by reaction with 4-piperidine methanol (62.5 mg, 0.543 mmol) and after purification by preparative TLC (DCM/MeOH 95/5), to compound **(56a)** (36 mg, 0.067 mmol, 37%). MS *m*/*z* ([M+H]⁺) 537.

### Step 2: 7-(1-Cyclohexyl-1H-benzoimidazol-5-yl)-5-(4-hydroxymethyl-piperidin-1-yl)-3,7-dihydro-pyrrolo[2,3-d]pyrimidin-4-one (Example 56)

According to the procedure described in example 51, step 5, compound **(56a)** (32 mg, 0.060 mmol) was converted, after purification by preparative TLC (DCM/MeOH 8/2), to **(Example 56)** as yellow solid (9.1 mg, 0.020 mmol, 21%). ¹H NMR (300 MHz, CDCl₃) δ 1.32-1.41 (m, 1H), 1.47-1.59 (m, 2H), 1.59-1.76 (m, 4H), 1.77-1.90 (m, 5H), 2.00 (d, J = 13.2 Hz, 2H), 2.24 (d, J = 11.8 Hz, 2H), 3.48-3.59 (m, 3H), 3.89 (d, J = 10.8 Hz, 2H), 4.23 (tt, J = 3.6/12.0 Hz, 1H), 6.62 (s, 1H), 7.52 (s, 2H), 7.82 (s, 1H), 7.93 (s, 1H), 8.06 (s, 1H), 11.18 (s, 1H). MS *m*/*z* ([M+H]⁺) 447.

### Example 57: Synthesis of 7-(1-Cyclohexyl-1H-benzoimidazol-5-yl)-5-(3-hydroxy-piperidin-1-yl)-3,7-dihydro-pyrrolo[2,3-d]pyrimidin-4-one

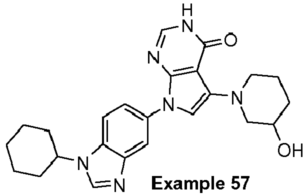

### Step 1: 1-[4-Benzyloxy-7-(1-cyclohexyl-1H-benzoimidazol-5-yl)-7H-pyrrolo[2,3-d]pyrimidin-5-yl]-piperidin-3-ol (57a)

According to the procedure described in example 54, step 1, compound **(51c)** (100 mg, 0.182 mmol) was converted, by reaction with 3-piperidinol (55 mg, 0.543 mmol) and after purification by preparative TLC (DCM/MeOH 95/5), to compound **(57a)** (36 mg, 0.067 mmol, 37%). MS *m*/*z* ([M+H]⁺) 523.

### Step 2: 7-(1-Cyclohexyl-1H-benzoimidazol-5-yl)-5-(3-hydroxy-piperidin-1-yl)-3,7-dihydro-pyrrolo[2,3-d]pyrimidin-4-one (Example 57)

According to the procedure described in example 51, step 5, compound **(57a)** (33 mg, 0.063 mmol) was converted, after purification by preparative TLC (DCM/MeOH 8/2), to **(Example 57)** as yellow oil (10 mg, 0.024 mmol, 38%). ¹H NMR (300 MHz, CDCl₃) δ 1.27-1.38 (m, 1H), 1.45-1.59 (m, 2H), 1.58-1.73 (m, 2H), 1.79-1.88 (m, 3H), 1.96-2.09 (m, 4H), 2.22 (d, J = 11.6 Hz, 2H), 3.00-3.24 (m, 3H), 3.33-3.39 (m, 1H), 4.06 (s, 1H), 4.21(tt, J = 3.6/11.9 Hz, 1H), 6.61 (s, 1H), 7.50 (s, 2H), 7.83 (s, 1H), 7.90 (s, 1H), 8.06 (s, 1H), 11.70 (bs, 1H). MS *m*/*z* ([M+H]⁺) 433.

### Example 58: Synthesis of 7-(1-Cyclohexyl-1H-benzoimidazol-5-yl)-5-(3-hydroxymethyl-cyclopentyl)-3,7-dihydro-pyrrolo[2,3-d]pyrimidin-4-one

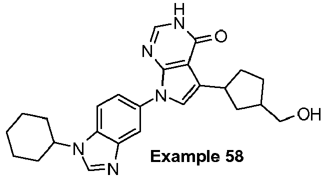

### Step 1: Methyl 3-[4-Benzyloxy-7-(1-cyclohexyl-1H-benzoimidazol-5-yl)-7H-pyrrolo[2,3-d]pyrimidin-5-yl]-cyclopent-3-enecarboxylate (58a)

A solution of compound **(51c)** (100 mg, 0.182 mmol), potassium acetate (27 mg, 0.273 mmol), tetrabutylammonium iodide (33 mg, 0.091 mmol), methyl-3-cyclopentene carboxylate (11 mg, 0.091 mmol) and palladium (II) acetate (1 mg, 0.004 mmol) in dry dimethylformamide (0.5 mL) was heated at 80°C for 3 days. The reaction mixture was cooled to room temperature and concentrated *in vacuo.* Water was added to the residue and the aqueous layer was extracted with DCM. The organic layer was dried over sodium sulfate and concentrated *in vacuo.* The residue was purified by preparative TLC (DCM/MeOH 95/5) to provide compound **(58a)** (29 mg, 0.053 mmol, 29%). MS *m*/*z* ([M+H]⁺) 548.

### Step 2: Methyl 3-[7-(1-Cyclohexyl-1H-benzoimidazol-5-yl)-4-oxo-4,7-dihydro-3H-pyrrolo[2,3-d]pyrimidin-5-yl]-cyclopentanecarboxylate (58b)

According to the procedure described in example 51, step 5, compound **(58a)** (29 mg, 0.053 mmol) was converted, after purification by preparative TLC (DCM/MeOH 9/1), to compound **(58b)** (10 mg, 0.022 mmol, 41%). ¹H NMR (300 MHz, CDCl₃) δ 1.29-1.40 (m, 1H), 1.47-1.61 (m, 2H), 1.70-1.92 (m, 6H), 1.95-2.31 (m, 7H), 2.39-2.62 (m, 1H), 2.96-3.17 (m, 1H), 3.59-3.69 (m, 3H), 4.23 (tt, J = 3.6/11.9 Hz, 1H), 6.96 and 7.03 (2s, 1H), 7.48-7.55 (m, 2H), 7.88 (s, 1H), 7.93 (bs, 1H), 8.08 (s, 1H), 11.54 (bs, 1H). MS *m*/*z* ([M+H]⁺) 460.

### Step 3: 7-(1-Cyclohexyl-1H -benzoimidazol-5-yl)-5-(3-hydroxymethyl-cyclopentyl)-3,7-dihydro-pyrrolo[2,3-d]pyrimidin-4-one (Example 58)

At 0°C, a solution of lithium aluminum hydride (2M in THF,36 µL, 0.072 mmol) was added to a solution of compound **(58b)** (10 mg, 0.022 mmol) in dry THF (0.5 mL). The reaction was stirred 2 hours at room temperature. Water was added to the reaction mixture and the aqueous layer was extracted with DCM. The organic layer was dried over sodium sulfate and concentrated *in vacuo.* The residue was purified by preparative TLC (DCM/MeOH 9/1) to provide **(Example 58)** as a mixture of stereoisomers (5.1mg, 0.012mmol, 53%). ¹H NMR (300 MHz, CDCl₃) δ 1.27-1.41 (m, 1H), 1.47-1.59 (m, 2H), 1.69-2.14 (m, 10H), 2.24 (d, J = 10.7 Hz, 2H), 2.33-2.62 (m, 2H), 3.34-3.72 (m, 3H), 4.17-4.28 (m, 1H), 6.98 (d, J = 13.3 Hz, 1H), 7.48-7.55 (m, 2H), 7.88-7.94 (m, 2H), 8.07 (s, 1H), 11.32 and 11.73 (2s, 1H). MS *m*/*z* ([M+H]⁺) 432.

### Example 59: Synthesis of 7-(1-Cyclohexyl-1H-benzoimidazol-5-yl)-5-((3R,4R)-3-hydroxy-4-hydroxymethyl-piperidin-1-yl)-3,7-dihydro-pyrrolo[2,3-d]pyrimidin-4-one

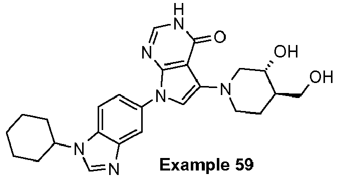

### Step 1: (3R,4R)-1-[4-Benzyloxy-7-(1-cyclohexyl-1H-benzoimidazol-5-yl)-7H-pyrrolo[2,3-d]pyrimidin-5-yl]-4-hydroxymethyl-piperidin-3-ol (59a)

According to the procedure described in example 54, step 1, compound **(51c)** (100 mg, 0.182 mmol) was converted, by reaction with (3R,4R)-4-(hydroxymethyl)-3-piperidinol (71 mg, 0.545 mmol) and after purification by preparative TLC (DCM/MeOH 95/5), to compound **(59a)** as yellow oil (30.5 mg, 0.055 mmol, 30%). ¹H NMR (300 MHz, CDCl₃) δ 1.24-1.41 (m, 1H), 1.47-1.85 (m, 7H), 2.00 (d, J = 13.4 Hz, 2H), 2.24 (d, J = 12.0 Hz, 2H), 2.39 (t, J = 10.1 Hz, 1H), 2.48-2.56 (m, 1H), 2.61 (s, 1H), 2.82-3.20 (m, 2H), 3.54-3.58 (m, 1H), 3.62-3.78 (m, 4H), 4.22 (tt, J = 3.6/11.9 Hz, 1H), 5.58 (dd, J = 11.8/22.0 Hz, 2H), 6.82 (s, 1H), 7.35-7.45 (m, 3H), 7.51-7.57 (m, 3H), 7.66 (dd, J = 1.8/8.7 Hz, 1H), 7.93 (d, J = 1.7 Hz, 1H), 8.06 (s, 1H), 8.45 (s, 1H). MS *m*/*z* ([M+H]⁺) 553.

### Step 5: 7-(1-Cyclohexyl-1H-benzoimidazol-5-yl)-5-((3R,4R)-3-hydroxy-4-hydroxymethyl-piperidin-1-yl)-3,7-dihydro-pyrrolo[2,3-d]pyrimidin-4-one (Example 59)

According to the procedure described in example 51, step 5, compound **(59a)** (30.5 mg, 0.055 mmol) was converted, after purification by preparative TLC (DCM/MeOH 9/1), to **(Example 59)** as yellow solid (7.6 mg, 0.016 mmol, 30%). ¹H NMR (300 MHz, CDCl₃) δ 1.22-1.36 (m, 2H), 1.42-1.55 (m, 2H), 1.69-1.83 (m, 5H), 1.95 (d, J = 13.0 Hz, 3H), 2.19 (d, J = 11.6 Hz, 2H), 2.40-2.57 (m, 2H), 3.59 (d, J = 8.8 Hz, 1H), 3.71-3.80 (m, 2H), 4.02-4.22 (m, 3H), 4.91 (bs, 1H), 6.60 (s, 1H), 7.47 (s, 2H), 7.78 (s, 1H), 7.94 (s, 1H), 8.06 (s, 1H), 11.72 (bs, 1H). MS *m*/*z* ([M+H]⁺) 463.

### Example 60: Synthesis of 7-(1-Cyclohexyl-1H-benzoimidazol-5-yl)-5-[2-(2-hydroxy-ethyl)-pyrrolidin-1-yl]-3,7-dihydro-pyrrolo[2,3-d]pyrimidin-4-one

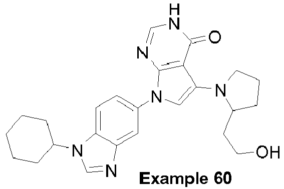

### Step 1: 2-{1-[4-Benzyloxy-7-(1-cyclohexyl-1H-benzoimidazol-5-yl)-7H-pyrrolo[2,3-d]pyrimidin-5-yl]-pyrrolidin-2-yl}-ethanol (60a)

According to the procedure described in example 54, step 1, compound **(51c)** (100 mg, 0.182 mmol) was converted, by reaction with 2-piperidine ethanol (63 mg, 0.545 mmol) and after purification by preparative TLC (DCM/MeOH 95/5), to compound **(60a)** as yellow oil (22.2 mg, 0.041 mmol, 22%). ¹H NMR (300 MHz, CDCl₃) δ 1.31-1.41 (m, 1H), 1.47-1.69 (m, 4H), 1.76-1.93 (m, 5H), 1.95-2.06 (m, 3H), 2.24 (d, J = 10.9 Hz, 2H), 2.95-3.02 (m, 1H), 3.61-3.77 (m, 3H), 3.90-3.98 (m, 2H), 4.22 (tt, J = 3.7/11.9 Hz, 1H), 5.52 (d, J = 12.2 Hz, 1H), 5.70 (d, J = 12.2 Hz, 1H), 7.00 (s, 1H), 7.31-7.43 (m, 3H), 7.51-7.55 (m, 3H), 7.65 (dd, J = 1.9/8.7 Hz, 1H), 7.93 (d, J = 1.8 Hz, 1H), 8.05 (s, 1H), 8.44 (s, 1H). MS *m*/*z* ([M+H]⁺) 537.

### Step 2: 7-(1-Cyclohexyl-1H-benzoimidazol-5-yl)-5-[2-(2-hydroxy-ethyl)-pyrrolidin-1-yl]-3,7-dihydro-pyrrolo[2,3-d]pyrimidin-4-one (Example 60)

According to the procedure described in example 51, step 5, compound **(60a)** (22.2 mg, 0.041 mmol) was converted, after purification by column chromatography on silica gel (DCM/MeOH 9/1 to 8/2), to **(Example 60)** as yellow oily product (1.8 mg, 0.004 mmol, 10%). ¹H NMR (300 MHz, CDCl₃) 1.26-1.43 (m, 1H), 1.47-1.67 (m, 5H), 1.77-1.89 (m, 5H), 1.98-2.06 (m, 3H), 2.15-2.26 (m, 2H), 3.09-3.18 (m, 1H), 3.65-3.79 (m, 2H), 3.85-3.92 (m, 1H), 4.19-4.24 (m, 2H), 6.63 (s, 1H), 7.52 (s, 2H), 7.80 (s, 1H), 7.94 (s, 1H), 8.07 (s, 1H), 10.27 (bs, 1H). MS *m*/*z* ([M+H]⁺) 447.

### Example 61: Synthesis of 7-(1-Cyclohexyl-1H-benzoimidazol-5-yl)-5-((S)-3-hydroxy-pyrrolidin-1-yl)-3,7-dihydro-pyrrolo[2,3-d]pyrimidin-4-one

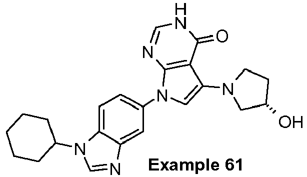

### Step 1: (S)-1-[4-Benzyloxy-7-(1-cyclohexyl-1H-benzoimidazol-5-yl)-7H-pyrrolo[2,3-d]pyrimidin-5-yl]-pyrrolidin-3-ol (61a)

According to the procedure described in example 54, step 1, compound **(51c)** (100 mg, 0.182 mmol) was converted, by reaction with 3-(S)-pyrrolidinol (47 mg, 0.545 mmol) and after purification by preparative TLC (DCM/MeOH 95/5), to compound **(61a)** as yellow oil (39 mg, 0.077 mmol, 41%). ¹H NMR (300 MHz, CDCl₃) δ 1.35 (m, 1H), 1.46-1.60 (m, 2H), 1.75-2.01 (m, 8H), 2.18-2.29 (m, 3H), 3.05-3.13 (m, 1H), 3.28-3.39 (m, 2H), 3.48-3.55 (m, 1H), 4.21 (tt, J = 3.7/11.9 Hz, 1H), 4.45 (bs, 1H), 5.58-5.67 (m, 2H), 6.73 (s, 1H), 7.34-7.43 (m, 3H), 7.51-7.54 (m, 3H), 7.65 (dd, J= 1.8/8.7 Hz, 1H), 7.93 (d, J = 1.7 Hz, 1H), 8.04 (s, 1H), 8.43 (s, 1H). MS *m*/*z* ([M+H]⁺) 509.

### Step 2: 7-(1-Cyclohexyl-1H-benzoimidazol-5-yl)-5-((S)-3-hydroxy-pyrrolidin-1-yl)-3,7-dihydro-pyrrolo[2,3-d]pyrimidin-4-one (Example 61)

According to the procedure described in example 51, step 5, compound **(61a)** (39 mg, 0.077 mmol) was converted, after purification by column chromatography on silica gel (DCM/MeOH 9/1 to 8/2), to **(Example 61)** (20 mg, 0.047 mmol, 64%). ¹H NMR (300 MHz, CDCl₃) δ 1.34 (m, 1H), 1.47-1.56 (m, 2H), 1.76-1.85 (m, 3H), 1.97-2.05 (m, 3H), 2.21-2.34 (m, 3H), 3.16-3.22 (m, 1H), 3.49-3.56 (m, 2H), 3.65 (dd, J = 7.7/16.3 Hz, 1H), 4.21 (tt, J = 3.6/12.0 Hz, 1H), 4.57 (bs, 1H), 6.53 (s, 1H), 7.50 (s, 2H), 7.79 (s, 1H), 7.93 (s, 1H), 8.06 (s, 1H), 11.64 (bs, 1H). MS *m*/*z* ([M+H]⁺) 419.

### Example 62: Synthesis of 7-(1-Cyclohexyl-1H-benzoimidazol-5-yl)-5-((S)-3-hydroxy-pyrrolidin-1-yl)-3,7-dihydro-pyrrolo[2,3-d]pyrimidin-4-one

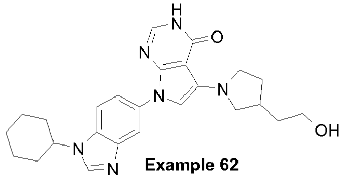

### Step 1: (2-{1-[4-Benzyloxy-7-(1-cyclohexyl-1H-benzoimidazol-5-yl)-7H-pyrrolo[2,3-d]pyrimidin-5-yl]-pyrrolidin-3-yl}-ethanol (62a)

According to the procedure described in example 54, step 1, compound **(51c)** (100 mg, 0.182 mmol) was converted, by reaction with 3-pyrrolidine ethanol (62 mg, 0.545 mmol) and after purification by preparative TLC (DCM/MeOH 95/5), to compound **(62a)** as yellow oil (41 mg, 0.077 mmol, 42%). ¹H NMR (300 MHz, CDCl₃) δ 1.25-1.40 (m, 1H), 1.45-1.69 (m, 4H), 1.74-2.00 (m, 7H), 2.04-2.15 (m, 1H), 2.23 (d, J = 10.9 Hz, 2H), 2.29-2.44 (m, 1H), 2.95 (dd, J = 7.1/9.2 Hz, 1H), 3.17-3.30 (m, 2H), 3.37 (dd, J = 7.7/9.1 Hz, 1H), 3.55 (t, J = 6.6 Hz, 2H), 4.21 (tt, J = 3.7/11.9 Hz, 1H), 5.56 (d, J = 12.0 Hz, 1H), 5.63 (d, J = 12.0 Hz, 1H), 6.70 (s, 1H), 7.34-7.43 (m, 3H), 7.50-7.55 (m, 3H), 7.66 (dd, J = 1.9/8.7Hz, 1H), 7.92 (d, J = 1.8 Hz, 1H), 8.04 (s, 1H), 8.42 (s, 1H). MS *m*/*z* ([M+H]⁺) 537.

### Step 2: 7-(1-Cyclohexyl-1H-benzoimidazol-5-yl)-5-((S)-3-hydroxy-pyrrolidin-1-yl)-3,7-dihydro-pyrrolo[2,3-d]pyrimidin-4-one (Example 62)

A solution of compound **(62a)** (41 mg, 0.077 mmol) in ethanol (2 mL) was degassed with argon. The catalyst Pd 10% on charcoal (17 mg, 0.015 mmol) was then added and the resulting reaction mixture was stirred at room temperature under 1 bar of hydrogen until completion of reaction by TLC (48 hours). The middle was filtered through PTFE isodisc 0.2µm. The filtrate was concentrated under reduced pressure. The residue was purified by chromatography on silica gel (DCM/MeOH 9/1 to 8/2) to provide **(Example 62)** as yellow powder (8.9 mg, 0.020 mmol, 26%). ¹H NMR (300 MHz, CDCl₃) δ 1.25-1.38 (m, 1H), 1.48-1.57 (m, 2H), 1.65-1.86 (m, 7H), 1.99 (d, J = 13.2 Hz, 2H), 2.18-2.25 (m, 2H), 2.46-2.53 (m, 1H), 3.13 (t, J = 8.0 Hz, 1H), 3.34-3.44 (m, 2H), 3.57 (t, J = 8.5 Hz, 1H), 3.69-3.80 (m, 2H), 4.19-4.25 (m, 1H), 6.51 (s, 1H), 7.52 (s, 2H), 7.83 (s, 1H), 7.93 (s, 1H), 8.06 (s, 1H), 11.44 (bs, 1H). MS *m*/*z* ([M+H]⁺) 447.

### Example 63: Synthesis of 7-(1-Cyclohexyl-1H-benzoimidazol-5-yl)-5-((S)-3-hydroxymethyl-pyrrolidin-1-yl)-3H-pyrrolo[2,1-f][1,24]triazin-4-one

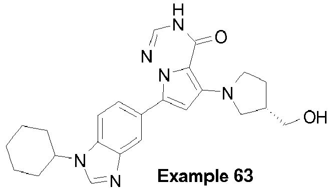

### Step 1: 7-Bromo-pyrrolo[2,1-f][1,2,4]triazin-4-ol (63a)

Under nitrogen atmosphere, trifluoroacetic acid (2 mL) and N-Bromosuccinimide (263 mg, 1.48 mmol) were added to a solution of Pyrrolo[2,1-f][1,2,4]triazin-4-ol (250 mg, 1.85 mmol) in anhydrous DCM (3.2 mL) at 0°C. The middle was stirring for 1 hour at 0°C and then partially concentrated. The middle was poured into a saturated solution of sodium bicarbonate and stirred at room temperature for 20 minutes. The precipitate was filtered and washed with water, cyclohexane then dried under reduced pressure to afford compound **(63a)** as a brown solid (288 mg, 1.35 mmol, 72%). ¹H NMR (400 MHz, DMSO-*d₆*) δ 6.72 (d, J = 4.50 Hz, 1H), 6.98 (d, J = 4.5 Hz, 1H), 7.95 (d, J = 4.09 Hz, 1H), 11.84 (bs, 1H). MS *m*/*z* ([M+H]⁺) 214/216.

### Step 2: 7-(1-cyclohexyl-1H-benzoimidazol-5-yl)-3H-pyrrolo[2,1-f][1,2,4]triazin-4-one (63b)

A solution of compound **(63a)** (30 mg, 0.14 mmol), 1-Cyclohexyl-5-(4,4,5,5-tetramethyl-[1,3,2]dioxaborolan-2-yl)-1H-benzoimidazole (45.7 mg, 0.14 mmol), potassium phosphate (50.52 mg, 0.24 mmol), tricyclohexylphosphine (0.93 mg, 0.003 mmol) and dichlorobis(di-tert-butylphenylphosphine)palladium (II) (1.31 mg, 0.002 mmol) in dioxane (400 µL) was bubbled with nitrogen for 5 minutes and stirred at 85°C overnight. Water was added to the residue and the aqueous layer was extracted with ethyl acetate. The organic layer was dried over sodium sulfate and concentrated under reduced pressure. The crude residue was purified by preparative TLC (DCM/MeOH 95/5) to provide compound **(63b)** as a white solid (16.2 mg, 0.025 mmol, 34%). ¹H NMR (400 MHz, DMSO-*d₆*) δ 1.30 (t = 12.7 Hz, 1H), 1.46-1.56 (t, J = 13.2 Hz, 2H), 1.74 (d, J = 12.8 Hz, 1H), 1.83-1.92 (m, 4H), 2.08-2.06 (m, 2H), 4.39 (tt, J = 3.6/11.7Hz, 1H), 6.91 (d, J = 4.5 Hz, 1H), 7.05 (d, J = 4.5 Hz, 1H), 7.69 (m, 2H), 7.92 (bs, 1H), 8.27 (bs, 1H), 8.37 (s, 1H), 11.70 (bs, 1H). MS *m*/*z* ([M+H]⁺) 334.

### Step 3: 7-(1-Cyclohexyl-1H-benzoimidazol-5-yl)-5-iodo-3H-pyrrolo[2,1-f][1,2,4]triazin-4-one (63c)

At 0°C, N-iodosuccinimide (105 mg, 0.47 mmol) was added to a solution of compound **(63b)** (130 mg, 0.390 mmol) in anhydrous DCM (11 mL). The mixture was stirred at room temperature overnight. The solution was concentrated under reduced pressure. The residue was triturated with EtOAc and DCM. The white solid was filtered off. The filtrate was concentrated under reduced pressure and purified on preparative TLC (DCM/MeOH 95/5) to provide compound **(63c)** as a white solid (37.7 mg, 0.08 mmol, 21%). ¹H NMR (400 MHz, DMSO-*d₆*) δ 1.25-1.35 (m, 1H), 1.45-1.56 (m, 2H), 1.71-1.74 (m, 1H), 1.82-1.90 (m, 4H), 2.04-2.07 (m, 2H), 4.37-4.43 (m, 1H), 7.02-7.10 (m, 1H), 7.68-7.75 (m, 2H), 8.19-8.22 (m, 1H), 8.39-8.40 (m, 2H), 11.74 (d, J = 3.7 Hz, 1H). MS *m*/*z* ([M+H]⁺) 460.

### Step 4: 7-(1-Cyclohexyl-1H-benzoimidazol-5-yl)-5-((S)-3-hydroxymethyl-pyrrolidin-1-yl)-3H-pyrrolo[2,1-f][1,2,4]triazin-4-one (Example 63)

According to the procedure described in example 54, step 1, compound **(63c)** (37 mg, 0.081 mmol) was converted, by reaction with (S)-1-Pyrrolidin-3-yl-methanol (12.2 mg, 0.12 mmol) and after purification by preparative TLC (DCM/MeOH 95/5), to **(Example 63)** as yellow solid (3 mg, 0.007 mmol, 8%). ¹H NMR (400 MHz, CD₃OD) δ 1.41-1.51 (m, 1H), 1.61-1.72 (m, 2H), 1.77-2.06 (m, 6H), 2.13-2.21 (m, 1H), 2.26 (d, J = 12.2 Hz, 2H), 2.58 (sep, J = 7.1 Hz, 1H), 3.40-3.65 (m, 4H), 3.66 (d, J = 6.9 Hz, 2H), 4.47 (tt, J = 11.7/3.7 Hz, 1H), 6.35 (bs, 1H), 7.50 (bs, 1H), 7.73 (d, J = 8.6, 1H), 7.88 (d, J = 8.6 Hz, 1H), 8.33 (s, 1H), 8.43 (s, 1H). MS m/z ([M+H]⁺) 433.

### Example 64: Synthesis of 7-(1-Cyclohexyl-1H-benzoimidazol-5-yl)-5-pyrrolidin-1-yl-3H-pyrrolo[2,1-f][1,2,4]triazin-4-one

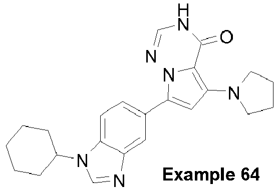

According to the procedure described in example 54, step 1, compound **(63c)** (30 mg, 0.06 mmol) was converted, by reaction with pyrrolidine (14 mg, 0.20 mmol) and after purification by preparative TLC (DCM/MeOH 95/5), to **(Example 64)** as orange solid (10.7 mg, 0.03 mmol, 41%). ¹H NMR (400 MHz, DMSO-*d₆*) δ 1.29-1.35 (m, 1H), 1.46-1.56 (m, 2H), 1.72-1.75 (m, 1H), 1.85-1.91 (m, 8H), 2.05-2.07 (m, 2H), 3.39 (t, J = 6.5 Hz, 4H), 4.36-4.42 (m, 1H), 6.29 (s, 1H), 7.57 (d, J = 3.5 Hz, 1H), 7.72 (s, 2H), 8.22 (bs, 1H), 8.38 (bs, 1H), 10.83 (d, J = 3.5 Hz, 1H). MS m/z ([M+H]⁺) 403.

### Example 65: Synthesis of 7-(1-Cyclohexyl-1H-benzimidazol-5-yl)-5-(3-hydroxy-prop-1-ynyl)-3H-pyrrolo[2,1-f][1,2,4]triazin-4-one

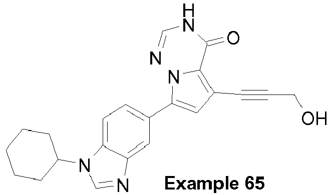

Under nitrogen atmosphere, a mixture of compound **(63c)** (29 mg, 0.06 mmol), tetrakis(triphenylphosphine)palladium (8.2 mg, 0.007 mmol), copper(I) iodide (3.3 mg, 0.02 mmol), triethylamine (18 µL, 0.13 mmol) and trimethyl-prop-2-ynyloxy-silane (94 mg 0.73 mmol) in anhydrous DMF (250 µL) was bubbled with nitrogen for 5 minutes and heated at room temperature overnight. The reaction mixture was concentrated under reduced pressure and the crude was purified twice by preparative TLC (DCM/MeOH 95/5) to provide **(Example 65)** as a beige solid (0.8 mg, 0.002 mmol, 2.7%). ¹H NMR (400 MHz, DMSO-*d₆*) δ 1.23-1.53 (m, 1H), 1.46-1.56 (m, 2H), 11.73 (d, J = 12.8 Hz, 1H), 1.82-1.91 (m, 4H), 2.05-2.07 (m, 2H), 4.33 (d, J = 5.6 Hz, 2H), 4.37-4.42 (m, 1H), 5.31 (t, J = 6 Hz, 1H), 6.99 (s, 1H) 7.73 (s, 2H), 7.92 (s, 1H), 8.23 (s, 1H), 8.38 (s, 1H), 11.81 (s, 1H). MS m/z ([M+H]⁺) 388.

### Example 66: Synthesis of 7-(1-Cyclohexyl-1H-benzimidazol-5-yl)-N,N-dimethyl-4-oxo-3,4-dihydro-pyrrolo[3,2-d]pyrimidine-5-sulfonamide

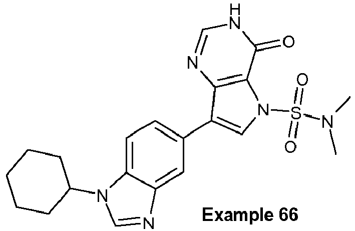

### Step 1: 7-Bromo-4-chloro-5H-pyrrolo[3,2-d]pyrimidine (66a)

Under argon atmosphere, a solution of 7-Bromo-3,5-dihydro-pyrrolo[3,2-d]pyrimidin-4-one (500 mg, 2.33 mmol) in phosphorus(V) oxychoride (6 mL) was refluxed for 2 hours. The reaction mixture was then cooled to room temperature, concentrated and the residue was diluted with ethyl acetate. The organic layer was washed with a solution of sodium bicarbonate, brine, dried over sodium sulfate, filtered and evaporated under reduced pressure to afford compound **(66a)** as a pale solid (490 mg, 2.10 mmol, 90%) without further purification. ¹H NMR (400 MHz, DMSO-*d₆*) δ 8.24 (d, *J* = 3.0 Hz, 1H), 8.72 (s, 1H), 12.95 (bs, 1H). MS *m*/*z* ([M+H]⁺) 232/234.

### Step 2: 4-Benzyloxy-7-bromo-5H-pyrrolo[3,2-d]pyrimidine (66b)

Benzyl alcohol (19.14 mL, 185.6 mmol) was stirred in THF (270 mL) and sodium hydride (60%, 7.42 g, 185.6 mmol) was added. The mixture was stirred for 30 minutes before addition of compound **(66a)** (10.8 g, 46.4 mmol). The reaction was heated to 65°C for 2 hours. The reaction was allowed to cool to room temperature. Water was added and the mixture was stirred for 10 minutes before extracting with DCM. The organic layers were washed with water, dried over sodium sulfate and concentrated under reduced pressure. The solid was triturated with Hexane to give compound **(66b)** (11.3 g, 37.1 mmol, 80%). ¹H NMR (400 MHz, DMSO-*d₆*) δ 2.14 (s, 2H), 7.35-7.43 (m, 3H), 7.54-7.56 (dd, J = 1.5/8.3 Hz, 2H), 7.88 (d, J = 1.5 Hz, 1H), 8.49 (s, 1H), 12.50 (bs, 1H). MS *m*/*z* ([M+H]⁺) 304/306.

### Step 3: 4-(benzyloxy)-7-bromo-N,N-dimethyl-5H-pyrrolo[3,2-d]pyrimidine-5-sulfonamide (66c)

Compound **(66b)** (2 g, 6.6 mol) was stirred in DCM (60 mL). Triethylamine (3.66 mL, 26.4 mmol), 4-dimethylaminopyridine (0.4 g, 3.3 mmol) and *N,N-*dimethylsulfamoyl chloride (1.76 mL, 16.5 mmol) were added. The reaction mixture was heated to reflux for 48 hours. The reaction was left to cool to room temperature before the mixture was washed with water. The organic layer was dried over sodium sulfate and concentrated under vacuum to give the crude product. The crude material was purified by column chromatography (DCM/EtOAc 98/2) to give compound **(66c)** (2.6 g, 6.32 mmol, 96%). ¹H NMR (400 MHz, CDCl₃) δ 2.72 (s, 6H), 5.66 (s, 2H), 7.34-7.42 (m, 3H), 7.51-7.55 (m, 2H), 7.90 (s, 1H), 8.71 (s, 1H). MS *m*/*z* ([M+H]⁺) 411/413.

### Step 4: 4-Benzyloxy-7-(1-cyclohexyl-1H-benzimidazol-5-yl)-N,N-dimethyl-pyrrolo[3,2-d]pyrimidine-5-sulfonamide (66d)

Under argon atmosphere, compound **(66c)** (50 mg, 0.121 mmol), 1-Cyclohexyl-5-(4,4,5,5-tetramethyl-1,3,2-dioxaborolan-2-yl)-1H-benzimidazole (95 mg, 0.292 mmol) and potassium carbonate (100 mg, 0.73 mmol) were dissolved in a mixture of THF (4 mL) and water (0.8 mL). The solution was degassed under argon for 5 minutes and bis(triphenylphosphine)palladium (II) dichloride (34 mg, 0,048 mmol) was added. The reaction was heated at 110°C for 60 minutes under microwave irradiation. The middle was then filtered and diluted with water and ethyl acetate. The organic layer was washed with brine, dried over sodium sulfate, filtered and concentrated under reduced pressure. The crude material was purified by preparative TLC on silica gel (EtOAc/MeOH 97/3) to give compound **(66d)** (45 mg, 0.085 mmol, 70%). ¹H NMR (400 MHz, CDCl₃) δ 1.30-1.41 (m, 1H), 1.46-1.61 (m, 2H), 1.75-1.90 (m, 3H), 1.94-2.04 (m, 2H), 2.20-2.30 (m, 2H), 2.75 (s, 6H), 4.17-4.28 (m, 1H), 5.69 (s, 2H), 7.33-7.43 (m, 3H), 7.51 (d, J = 8.4 Hz, 1H), 7.57 (m, 2H), 7.97 (dd, J = 1.6/8.5 Hz, 1H), 8.02 (s, 1H), 8.08 (s, 1H), 8.38 (d, J = 1.2 Hz, 1H), 8.74 (s, 1H). MS *m*/*z* ([M+H]⁺) 531.

### Step 5: 7-(1-Cyclohexyl-1H-benzimidazol-5-yl)-N,N-dimethyl-4-oxo-3,4-dihydro-pyrrolo[3,2-d]pyrimidine-5-sulfonamide (Example 66)

A solution of compound **(66d)** (45 mg, 0.085 mmol) in HCl 12N (4 mL) was stirred for 1 minute. The middle was neutralized with sodium bicarbonate and diluted with DCM. The organic layer was washed with brine, dried over sodium sulfate, filtered and concentrated under reduced pressure. The crude material was purified by preparative TLC on silica gel (DCM/EtOAc/MeOH 45/45/10) to give **(Example 66)** (25 mg, 0.057 mmol, 67%). ¹H NMR (400 MHz, CDCl₃) δ 1.27-1.41 (m, 1H), 1.45-1.62 (m, 2H), 1.72-1.89 (m, 3H), 1.93-2.06 (m, 2H), 2.19-2.30 (m, 2H), 3.07 (s, 6H), 4.17-4.27 (m, 1H), 7.50 (d, J = 8.5 Hz, 1H), 7.79 (dd, J = 1.5/8.5 Hz, 1H), 8.00 (s, 1H), 8.06 (s, 1H), 8.13 (s, 1H), 8.38 (d, J = 1.5 Hz, 1H), 11.63 (bs, 1H). MS *m*/*z* ([M+H]⁺) 441.

### Example 67: Synthesis of 7-(1-Cyclohexyl-1H-benzimidazol-5-yl)-5-(3-hydroxy-propyl)-3,5-dihydro-pyrrolo[3,2-d]pyrimidin-4-one

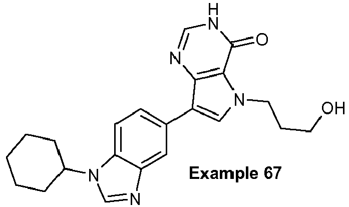

### Step 1: 4-Benzyloxy-7-(1-cyclohexyl-1H-benzimidazol-5-yl)-5H-pyrrolo[3,2-d]pyrimidine (67a)

A solution of tetrabutylammonium fluoride (1N in THF, 4.1 mL, 4.1 mmol) was added to a solution of compound **(66d)** (460 mg, 0.87 mmol) in THF (27 mL). The middle was refluxed for 2 hours and concentrated under reduced pressure. The crude material was purified by chromatography on silica gel (EtOAc/MeOH 95/5) to give compound **(67a)** (200 mg, 0.47 mmol, 54%). ¹H NMR (400 MHz, DMSO-*d₆*) δ 1.22-1.38 (m, 2H), 1.45-1.58 (m, 2H), 1.70-1.77 (m, 1H), 1.81-1.92 (m, 3H), 2.04-2.11 (m, 2H), 4.32-4.41 (m, 1H), 5.66 (s, 2H), 7.34-7.38 (m, 1H), 7.41-7.44 (m, 2H), 7.55-7.60 (m, 2H), 7.66 (d, J = 8.6 Hz, 1H), 8.09 (dd, J = 1.5/8.5 Hz, 1H), 8.18 (d, J = 3.0 Hz, 1H), 8.28 (s, 1H), 8.56 (s, 1H), 8.57 (d, J = 1.5 Hz, 1H), 12.21 (d, J = 1.5 Hz, 1H). MS *m*/*z* ([M+H]⁺) 424.

### Step 2: 4-Benzyloxy-5-[3-(tert-butyl-dimethyl-silanyloxy)-propyl]-7-(1-cyclohexyl-1H-benzimidazol-5-yl)-5H-pyrrolo[3,2-d]pyrimidine (67b)

At room temperature, sodium hydride (60%, 11mg, 0.275 mmol) was added to a solution of compound **(67a)** (60 mg, 0.141 mmol) in DMF (3 mL). After 10 minutes, (3-Bromo-propoxy)-tert-butyldimethyl-silane (60 µl, 0.26 mmol) was added and the reaction was stirred for 45 minutes. The middle was hydrolyzed with water and diluted with EtOAc. The organic layer was washed with water and brine and dried over sodium sulphate and concentrated. The crude material was purified by preparative TLC on silica gel (DCM/MeOH 95/5) to give compound **(67b)** (70 mg, 0.12 mmol, 71%). ¹H NMR (400 MHz, CDCl₃) δ 0.02 (s, 6H), 0.90 (s, 9H), 1.29-1.39 (m, 1H), 1.47-1.59 (m, 2H), 1.76-1.87 (m, 3H), 1.95-2.03 (m, 4H), 2.21-2.26 (m, 2H), 3.51 (t, J = 6.0 Hz, 2H), 4.17-4.25 (m, 1H), 4.46 (t, J = 6 Hz, 2H), 5.63 (s, 2H), 7.34-7.43 (m, 3H), 7.46-7.50 (m, 3H), 7.56 (s, 1H), 8.00 (d, J = 8.1 Hz, 1H), 8.14 (dd, J = 1.0/8.5 Hz, 1H), 8.22 (d, J = 1.0 Hz, 1H), 8.61 (s, 1H). MS *m*/*z* ([M+H]⁺) 596.

### Step 3: 7-(1-Cyclohexyl-1H-benzimidazol-5-yl)-5-(3-hydroxy-propyl)-3,5-dihydro-pyrrolo[3,2-d]pyrimidin-4-one (Example 67)

A solution of compound **(67b)** (60 mg, 0.1 mmol) in THF (3 mL) and HCl 12N (3 mL) was stirred for 10 minutes. The middle was neutralized with sodium hydroxide until pH 7 and diluted with DCM. The organic layer was washed with brine, dried over sodium sulfate, filtered and concentrated under reduced pressure. The crude material was purified by preparative TLC on silica gel (DCM/MeOH 9/1) to give **(Example 67)** (24 mg, 0.061 mmol, 52%). ¹H NMR (400 MHz, CDCl₃) δ 1.28-1.41 (m, 1H), 1.46-1.56 (m, 2H), 1.76-1.89 (m, 3H), 1.95-2.02 (m, 2H), 2.05-2.13 (m, 2H), 2.21-2.29 (m, 2H), 3.58 (bs, 2H), 3.89 (bs, 1H), 4.16-4.26 (m, 1H), 4.68 (m, 2H), 7.48 (s, 1H), 7.49 (d, J = 8.5 Hz, 1H), 7.92 (dd, J = 1.5/8.5 Hz, 1H), 7.94 (m, 1H), 8.01 (s, 1H), 8.24 (d, J = 1.5 Hz, 1H), 1.05 (bs, 1H). MS *m*/*z* ([M+H]⁺) 392.

### Example 68: Synthesis of 7-(1-Cyclohexyl-1H-benzimidazol-5-yl)-5-(5-hydroxy-pentyl)-3,5-dihydro-pyrrolo[3,2-d]pyrimidin-4-one

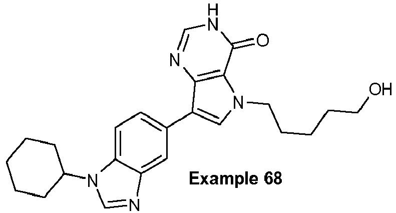

### Step 1: (5-Chloro-pentyloxymethyl)-benzene (68a)

A solution of 5-chloro-1-pentanol (200 mg, 1.63 mmol) and silver oxide (870 mg, 3.75 mmol) in benzyl bromide (1 mL) was vigorously stirred for 24 hours. The middle was then filtrated and concentrated. The crude material was purified by preparative TLC on silica gel (cyclohexane/EtOAc 92/8) to give compound **(68a)** (210 mg, 0.99 mmol, 60%). ¹H NMR (400 MHz, CDCl₃) δ 1.48-1.58 (m, 2H), 1.61-1.70 (m, 2H), 1.75-1.85 (m, 2H), 3.49 (t, J = 6.4 Hz, 2H), 3.54 (t, J= 6.7 Hz, 2H), 4.51 (s, 2H), 7.28-7.38 (m, 5H).

### Step 2: 4-Benzyloxy-5-(5-benzyloxy-pentyl)-7-(1-cyclohexyl-1H-benzimidazol-5-yl)-5H-pyrrolo[3,2-d]pyrimidine (68b)

At room temperature, sodium hydride (60%, 10mg, 0.24 mmol) was added to a solution of compound **(67a)** (40 mg, 0.094 mmol) in DMF (2 mL). After 10 minutes, a solution of compound **(68a)** (46 mg, 0.22 mmol) in DMF (0.5 mL) was added and the reaction was stirred for 39 hours. The middle was hydrolyzed with water and diluted with EtOAc. The organic layer was washed with water, brine, dried over sodium sulfate and concentrated. The crude material was purified by preparative TLC on silica gel (EtOAc/MeOH 95/5) to give compound **(68b)** (40 mg, 0.067 mmol, 71%). ¹H NMR (400 MHz, CDCl₃) δ 1.27-1.40 (m, 3H), 1.47-1.55 (m, 4H), 1.77-1.87 (m, 5H), 1.95-2.03 (m, 2H), 2.22-2.28 (m, 2H), 3.38 (t, J = 6.4 Hz, 2H), 4.18-4.25 (m, 1H), 4.33 (m, 2H), 4.45 (s, 2H), 5.62 (s, 2H), 7.28-7.34 (m, 6H), 7.38 (t, J = 7.6 Hz, 2H), 7.48-7.52 (m, 4H), 7.99 (s, 1H), 8.15 (d, J = 9 Hz, 1H), 8.21 (s, 1H), 8.62 (s, 1H). MS *m*/*z* ([M+H]⁺) 600.

### Step 3: 7-(1-Cyclohexyl-1H-benzimidazol-5-yl)-5-(5-hydroxy-pentyl)-3,5-dihydro-pyrrolo[3,2-d]pyrimidin-4-one (Example 68)

A solution of compound **(68b)** (40 mg, 0.067 mmol) in MeOH (3 mL) and DCM (1 mL) was purged with hydrogen. Catalyst palladium on charcoal 10% (15 mg) was then added and the reaction was stirred under hydrogen atmosphere (1 bar) for 24 hours. The middle was filtrated and concentrated under reduced pressure. The crude material was purified by preparative TLC on silica gel (DCM/MeOH 88/12) to give **(Example 68)** (7.8 mg, 0.0186 mmol, 27%). ¹H NMR (400 MHz, CD₃OD) δ 1.37-1.45 (m, 3H), 1.54-1.65 (m, 4H), 1.80-1.99 (m, 7H), 2.17-2.23 (m, 2H), 3.54 (t, J = 6.5 Hz, 2H), 4.32-4.40 (m, 1H), 4.50 (t, J = 6.5 Hz, 2H), 7.60 (d, J = 8.5 Hz, 1H), 7.72 (s, 1H), 7.86-7.88 (m, 2H), 8.24 (s, 1H), 8.26 (s, 1H). MS *m*/*z* ([M+H]⁺) 420.

### Example 69: Synthesis of 5-(5-Benzyloxy-pentyl)-7-(1-cyclohexyl-1H-benzimidazol-5-yl)-3,5-dihydro-pyrrolo[3,2-d]pyrimidin-4-one

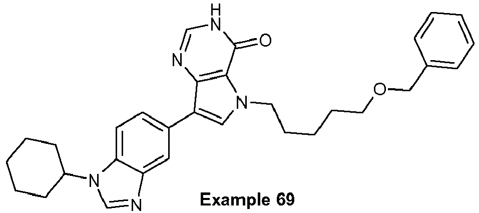

A solution of compound **(68b)** (40 mg, 0.067 mmol) in MeOH (3 mL) and DCM (1 mL) was purged with hydrogen. Catalyst palladium on charcoal 10% (15 mg) was then added and the reaction was stirred under hydrogen atmosphere (1 bar) for 24 hours. The middle was filtrated and concentrated under reduced pressure. The crude material was purified by preparative TLC on silica gel (DCM/MeOH 88/12) to give **(Example 69)** as a byproduct of the reaction (6.2 mg, 0.0012 mmol, 18%). ¹H NMR (400 MHz, CD₃OD) δ 1.37-1.46 (m, 3H), 1.55-1.67 (m, 4H), 1.78-2.03 (m, 7H), 2.16-2.24 (m, 2H), 3.47 (t, J = 6.3 Hz, 2H), 4.33-4.42 (m, 1H), 4.43 (s, 2H), 4.50 (t, J = 7 Hz, 2H), 7.18 (m, 1H), 7.24-7.26 (m, 4H), 7.61 (d, J = 8.5 Hz, 1H), 7.71 (s, 1H), 7.86 (s, 1H), 7.87 (dd, J = 1.5/8.5 Hz, 1H), 8.25 (s, 1H), 8.28 (d, J = 1.5 Hz, 1H). MS *m*/*z* ([M+H]⁺) 510.

### Example 70A and Example 70B: Synthesis of 7-(1-Cyclohexyl-1H-benzimidazol-5-yl)-5-(3-hydroxy-cyclohexyl)-3,5-dihydro-pyrrolo[3,2-d]pyrimidin-4-one

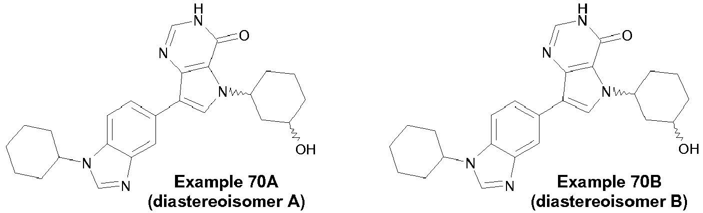

### Step 1: 3-[4-Benzyloxy-7-(1-cyclohexyl-1H-benzimidazol-5-yl)-pyrrolo[3,2-d]pyrimidin-5-yl]-cyclohexanol (70a/70b)

At room temperature, a solution of compound **(67a)** (30 mg, 0.071 mmol), cyclohexenone (100 µL, 1.07 mmol) and cesium carbonate (45 mg, 0.14 mmol) in tetrahydrofuran (5 mL) was stirred for 72 hours. Methanol (0.5 mL) was then added followed by addition of sodium borohydride (30 mg, 0.8 mmol). After 3 hours, the mixture was hydrolyzed with water and diluted with ethyl acetate. The organic layer was washed with water, brine, dried over sodium sulfate, filtered and concentrated under reduced pressure. The crude material was purified by preparative TLC on silica gel (EtOAc/MeOH (NH₃ 7M) 9/1) to give both diastereoisomers **(70a)** (5 mg, 0.01 mmol, 13%) and **(70b)** (15 mg, 0.029 mmol, 40%) as racemic mixture. Compound **(70a)**: ¹H NMR (400 MHz, CDCl₃) δ 1.20-2.02 (m, 13H), 2.14-2.30 (m, 2H), 3.36-3.67 (m, 2H), 4.12-4.25 (m, 3H), 5.19-5.27 (m, 1H), 5.57 (d, J = 12 Hz, 1H), 5.70 (d, J = 12 Hz, 1H), 7.37-7.46 (m, 3H), 7.50 (d, J = 8.4 Hz, 1H), 7.57-7.59 (m, 2H), 7.69 (s, 1H), 8.01 (s, 1H), 8.15 (d, J = 8.5 Hz, 1H), 8.20 (s, 1H), 8.62 (s, 1H). MS *m*/*z* ([M+H]⁺) 522. Compound **(70b)**: ¹H NMR (400 MHz, CDCl₃) δ 1.21-1.38 (m, 3H), 1.46-1.63 (m, 3H), 1.66-1.87 (m, 5H), 1.92-2.04 (m, 3H), 206-2.11 (m, 1H), 2.18-2.27 (m, 2H), 2.41-2.50 (m, 1H), 3.55-3.64 (m, 1H), 4.15-4.23 (m, 1H), 4.70-4.80 (m, 1H), 5.56-5.62 (m, 2H), 7.34-7.43 (m, 3H), 7.47-7.50 (m, 3H), 7.67 (s, 1H), 7.98 (s, 1H), 8.10 (d, J = 1.5/8.5 Hz, 1H), 8.23 (d, J = 1.5 Hz, 1H), 8.61 (s, 1H). MS *m*/*z* ([M+H]⁺) 522.

### Step 2: 7-(1-Cyclohexyl-1H-benzimidazol-5-yl)-5-(3-hydroxy-cyclohexyl)-3,5-dihydro-pyrrolo[3,2-d]pyrimidin-4-one (Example 70A)

A solution of compound **(70a)** (5 mg, 0.01 mmol) in methanol (5 mL) was purged with hydrogen. Catalyst palladium on charcoal 10% (5 mg) was then added and the reaction was stirred under hydrogen atmosphere (1 bar) for 2 hours. The middle was filtrated and concentrated under reduced pressure. The crude material was purified by preparative TLC on silica gel (EtOAc/MeOH (NH₃ 7M) 9/1) to give **(Example 70A)** (1.2 mg, 0.0028 mmol, 27%) as racemic mixture of one diastereoisomer. ¹H NMR (400 MHz, CD₃OD) δ 1.40-1.45 (m, 1H), 1.54-1.73 (m, 4H), 1.78-2.02 (m, 9H), 2.12-2.24 (m, 4H), 4.26-4.30 (m, 1H), 4.32-4.42 (m, 1H), 5.46-5.55 (m, 1H), 7.61 (d, J = 8.5 Hz, 1H), 7.85-7.87 (m, 3H), 8.23-8.24 (m, 2H). MS *m*/*z* ([M+H]⁺) 432.

### Step 3: 7-(1-Cyclohexyl-1H-benzimidazol-5-yl)-5-(3-hydroxy-cyclohexyl)-3,5-dihydro-pyrrolo[3,2-d]pyrimidin-4-one (Example 70B)

According to the procedure described in example 70, step 2, compound **(70b)** (15 mg, 0.029 mmol) was converted, after purification by preparative TLC on silica gel (EtOAc/MeOH (NH₃ 7M) 9/1), to **(Example 70B)** (11 mg, 0.027 mmol, 93%) as racemic mixture of one diastereoisomer. ¹H NMR (400 MHz, CD₃OD) δ 1.30-1.42 (m, 1H), 1.44-1.62 (m, 3H), 1.71-2.08 (m, 11H), 2.15-2.18 (m, 2H), 2.37-2.40 (m, 1H), 3.70-3.78 (m, 1H), 4.29-4.37 (m, 1H), 5.10-5.18 (m, 1H), 7.58 (d, J = 8.5 Hz, 1H), 7.84 (dd, J = 1.5/8.5 Hz, 1H), 7.85 (s, 1H), 7.88 (s, 1H), 8.20 (s, 1H), 8.25 (d, J = 1.5 Hz, 1H). MS *m*/*z* ([M+H]⁺) 432.

### Example 71A and Example 71B: Synthesis of 7-(1-Cyclohexyl-1H-benzimidazol-5-yl)-5-(3-hydroxy-cyclopentyl)-3,5-dihydro-pyrrolo[3,2-d]pyrimidin-4-one

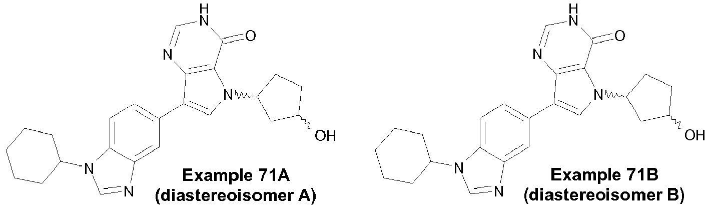

### Step 1: 3-[4-Benzyloxy-7-(1-cyclohexyl-1H-benzimidazol-5-yl)-pyrrolo[3,2-d]pyrimidin-5-yl]-cyclopentanol (71a)

According to the procedure described in example 70, step 1, compound **(67a)** (10 mg, 0.024 mmol) was converted, by reaction with cyclopentenone (45 µL, 0.54 mmol) and after purification by preparative TLC on silica gel (EtOAc/MeOH (NH₃ 7M) 9/1), to compound **(71a)** (9 mg, 0.018 mmol, 74%) as racemic mixture of both diastereoisomers. MS m/z ([M+H]⁺) 508.

### Step 2: 7-(1-Cyclohexyl-1H-benzimidazol-5-yl)-5-(3-hydroxy-cyclopentyl)-3,5-dihydro-pyrrolo[3,2-d]pyrimidin-4-one (Example 71A and Example 71B)

According to the procedure described in example 70, step 2, compound **(71a)** (9 mg, 0.018 mmol) was converted, after purification by preparative TLC on silica gel (EtOAc/MeOH (NH₃ 7M) 9/1), to **(Example 71A)** (2 mg, 0.0048 mmol, 26%) as racemic mixture of one diastereoisomer and to **(Example 71B)** (3.1 mg, 0.0074 mmol, 41%) as racemic mixture of the other diastereoisomer. **(Example 71A):** ¹H NMR (400 MHz, DMSO-*d₆*) δ 1.20-1.37 (m, 1H), 1.40-1.45 (m, 1H), 1.46-1.57 (m, 2H), 1.69-1.92 (m, 7H), 2.00-2.10 (m, 2H), 2.12-2.21 (m, 1H), 3.35-3.42 (m, 1H), 4.23-4.29 (m, 1H), 4.31-4.45 (m, 2H), 4.95 (d, J = 3.6 Hz, 1H), 5.62-5.70 (m, 1H), 7.64 (d, J = 8.6 Hz, 1H), 7.88-7.93 (m, 2H), 8.16 (s, 1H), 8.29 (s, 1H), 8.40 (s, 1H), 12.00 (d, J = 3.0 Hz, 1H). MS *m*/*z* ([M+H]⁺) 418. **(Example 71B):** ¹H NMR (400 MHz, CD₃OD) δ 1.27-1.46 (m, 1H), 1.54-1.66 (m, 2H), 1.72-1.84 (m, 2H), 1.87-2.04 (m, 5H), 2.16-2.22 (m, 2H), 2.26-2.34 (m, 3H), 2.42-2.51 (m, 1H), 4.34-4.42 (m, 1H), 4.52-4.57 (m, 1H), 5.80-5.88 (m, 1H), 7.62 (d, J = 8.5 Hz, 1H), 7.86-7.89 (m, 3H), 8.24-8.27 (m, 2H). MS *m*/*z* ([M+H]⁺) 418.

### Example 72: Synthesis of 7-(1-Cyclohexyl-1H-benzimidazol-5-yl)-5-(1-methyl-piperidin-4-yl)-3,5-dihydro-pyrrolo[3,2-d]pyrimidin-4-one

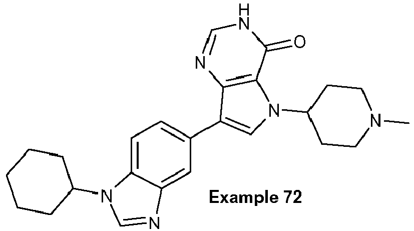

### Step 1: 4-Benzyloxy-7-(1-cyclohexyl-1H-benzimidazol-5-yl)-5-(1-methyl-piperidin-4-yl)-5H-pyrrolo[3,2-d]pyrimidine (72a)

At room temperature, a solution of 1-Methyl-piperidin-4-ol (82 mg, 0.71 mmol) in THF (1 mL) was slowly added to a solution of compound **(67a)** (100 mg, 0.236 mmol), triphenylphosphine (185 mg, 0.71 mmol) and DTAD (163 mg, 0.71 mmol) in THF (4 mL). The middle was stirred for 2 hours and then diluted with ethyl acetate. The organic layer was washed with water, brine, dried over sodium sulfate, filtered and concentrated under reduced pressure to give compound **(72a)** as a crude material which was used without further purification. MS *m*/*z* ([M+H]⁺) 521.

### Step 2: 7-(1-Cyclohexyl-1H-benzimidazol-5-yl)-5-(1-methyl-piperidin-4-yl)-3,5-dihydro-pyrrolo[3,2-d]pyrimidin-4-one (Example 72)

At room temperature, compound **(72a)** was diluted in hydrochloric acid 12N (2 mL) and stirred for 1 hour. The middle was then neutralized to pH 7 with sodium hydroxide 1N and concentrated under reduced pressure. The residue was triturated with ethanol and filtered. The filtrate was concentrated to give a residue which was purified by preparative TLC on silica gel (DCM/MeOH (NH₃ 7M) 9/1) to give **(Example 72)** (53 mg, 0.123 mmol, 52%). ¹H NMR (400 MHz, CD₃OD) δ 1.23-1.44 (m, 1H), 1.54-1.64 (m, 2H), 1.76-1.84 (m, 1H), 1.85-2.00 (m, 4H), 2.07-2.22 (m, 6H), 2.25-2.32 (m, 2H), 2.36 (s, 3H), 3.01-3.09 (m, 2H), 4.32-4.40 (m, 1H), 5.13-5.21 (m, 1H), 7.60 (d, J = 8.5 Hz, 1H), 7.84 (dd, J = 1.5/8.5 Hz, 1H), 7.86 (s, 1H), 7.90 (s, 1H), 8.22 (s, 1H), 8.28 (d, J = 1.5 Hz, 1H). MS *m*/*z* ([M+H]⁺) 431.

### Example 73: Synthesis of 7-(1-Cyclohexyl-1H-benzimidazol-5-yl)-5-piperidin-4-yl-3,5-dihydro-pyrrolo[3,2-d]pyrimidin-4-one

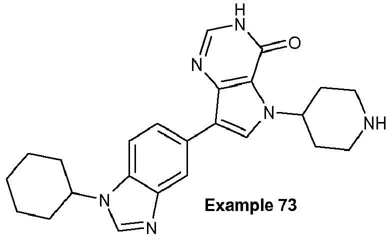

### Step 1: Benzyl 4-[4-Benzyloxy-7-(1-cyclohexyl-1H-benzimidazol-5-yl)-pyrrolo[3,2-d]pyrimidin-5-yl]-piperidine-1-carboxylate (73a)

According to the procedure described in example 72, step 1, compound **(67a)** (20 mg, 0.047 mmol) was converted, by reaction with benzyl 4-Hydroxy-piperidine-1-carboxylate (60 mg, 0.25 mmol) and after purification by preparative TLC on silica gel (a first one with DCM/MeOH 95/5 and a second one with EtOAc/MeOH 98/2), to compound **(73a)** (20 mg, 0.031 mmol, 66%). MS *m*/*z* ([M+H]⁺) 641.

### Step 2: 7-(1-Cyclohexyl-1H-benzimidazol-5-yl)-5-piperidin-4-yl-3,5-dihydro-pyrrolo[3,2-d]pyrimidin-4-one (Example 73)

According to the procedure described in example 70, step 2, compound (73a) (20 mg, 0.031 mmol) was converted, after purification by preparative TLC on silica gel (DCM/MeOH (NH₃ 7M) 9/1), to **(Example 73)** (3.8 mg, 0.009 mmol, 29%). ¹H NMR (400 MHz, CD₃OD) δ 1.33-1.45 (m, 1H), 1.53-1.65 (m, 2H), 1.77-1.84 (m, 1H), 1.85-2.00 (m, 4H), 2.05-2.24 (m, 6H), 2.92-2.99 (m, 2H), 3.31-3.37 (m, 2H), 4.32-4.40 (m, 1H), 5.22-5.31 (m, 1H), 7.61 (d, J = 8.5 Hz, 1H), 7.86-7.89 (m, 3H), 8.23 (s, 1H), 8.25 (d, J = 1.5 Hz, 1H). MS *m*/*z* ([M+H]⁺) 417.

### Example 74: Synthesis of 7-(1-Cyclohexyl-1H-benzimidazol-5-yl)-5-[cis-(4-hydroxymethyl-cyclohexyl)]-3,5-dihydro-pyrrolo[3,2-d]pyrimidin-4-one

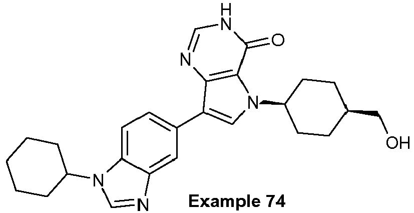

### Step 1: 4-Benzyloxymethyl-cyclohexanone (74a)

At room temperature, a solution of 4-Hydroxymethyl-cyclohexanone (300 mg, 2.3 mmol) and silver (I) oxide (870 mg, 3.75 mmol) in benzyl bromide (1.5 mL) was vigorously stirred for 48 hours. The middle was then filtered and concentrated under reduced pressure. The crude material was purified by column chromatography on silica gel (cyclohexane/EtOAc 10/0 to 9/1) to give compound **(74a)** (390 mg, 1.78 mmol, 78%). ¹H NMR (400 MHz, CD₃OD) δ 1.42-1.52 (m, 2H), 2.03-2.19 (m, 3H), 2.29-2.42 (m, 4H), 3.39 (d, J = 6.2 Hz, 2H), 4.53 (s, 2H), 7.27-7.37 (m, 5H). MS *m*/*z* ([M+H]⁺) 219.

### Step 2: trans-4-Benzyloxymethyl-cyclohexanol (84b) and cis-4-Benzyloxymethyl-cyclohexanol (74c)

According to the procedure described in example 18, step 1, compound **(74a)** (390 mg, 1.78 mmol) was converted, after purification by preparative TLC on silica gel (cyclohexane/EtOAc 4/6), to diastereoisomer *trans*-**(74b)** (280 mg, 1.27 mmol, 71%) and diastereoisomer *cis-***(74c)** (80 mg, 0.36 mmol, 20%). Compound **(74b):** ¹H NMR (400 MHz, CDCl₃) δ 0.98-1.08 (m, 2H), 1.21-1.31 (m, 2H), 1.35 (d, J = 4.6 Hz, 1H), 1.55-1.65 (m, 1H), 1.82-1.90 (m, 2H), 1.96-2.04 (m, 2H), 3.28 (d, J = 6.4 Hz, 2H), 3.51-3.60 (m, 1H), 4.49 (s, 2H), 7.27-7.36 (m, 5H). Compound **(74c):** ¹H NMR (400 MHz, CDCl₃) δ 1.25 (d, J = 4.0 Hz, 1H), 1.38-1.49 (m, 2H), 1.55-1.62 (m, 4H), 1.66-1.75 (m, 3H), 3.33 (d, J = 6.7 Hz, 2H), 3.97-4.02 (m, 1H), 4.51 (s, 2H), 7.27-7.38 (m, 5H).

### Step 3: 4-Benzyloxy-5-(4-benzyloxymethyl-cyclohexyl)-7-[cis-(1-cyclohexyl-1H-benzimidazol-5-yl)]-5H-pyrrolo[3,2-d]pyrimidine (74d)

According to the procedure described in example 72, step 1, compound **(67a)** (20 mg, 0.047 mmol) was converted, by reaction with compound **(74b)** (67 mg, 0.33 mmol) and after purification by preparative TLC on silica gel (DCM/MeOH 95/5), to compound **(74d)** (25 mg, 0.04 mmol, 85%). MS *m*/*z* ([M+H]⁺) 626.

### Step 4: 7-(1-Cyclohexyl-1H-benzimidazol-5-yl)-5-[cis-(4-hydroxymethyl-cyclohexyl)]-3,5-dihydro-pyrrolo[3,2-d]pyrimidin-4-one (Example 74)

According to the procedure described in example 69, compound **(74d)** (25 mg, 0.04 mmol) was converted, after purification by preparative TLC on silica gel (DCM/MeOH (NH3 7M) 9/1) and trituration in EtOAc, to **(Example 74)** (1.3 mg, 0.003 mmol, 7%). ¹H NMR (400 MHz, CD₃OD) δ 1.32-1.48 (m, 1H), 1.51-1.61 (m, 2H), 1.68-1.80 (m, 3H), 1.83-2.06 (m, 6H), 2.12-2.18 (m, 2H), 2.40 (dd, J = 8.5/12.1 Hz, 1H), 2.47-2.53 (m, 1H), 2.76-2.81 (m, 1H), 2.95-2.99 (m, 1H), 3.53-3.59 (m, 1H), 3.71 (d, J = 7.4 Hz, 2H), 4.29-4.36 (m, 1H), 5.01-5.09 (m, 1H), 7.57 (d, J = 8.5 Hz, 1H), 7.82-7.86 (m, 3H), 8.20 (s, 1H), 8.23 (d, J = 1.1 Hz, 1H). MS *m*/*z* ([M+H]⁺) 446.

### Example 75: Synthesis of 7-(1-Cyclohexyl-1H-benzimidazol-5-yl)-5-(R)-piperidin-3-yl-3,5-dihydro-pyrrolo[3,2-d]pyrimidin-4-one

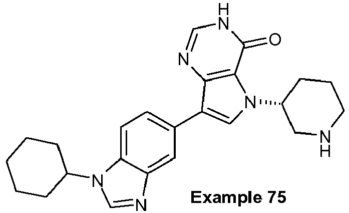

### Step 1: tert-butyl (R)-3-[4-Benzyloxy-7-(1-cyclohexyl-1H-benzimidazol-5-yl)-pyrrolo[3,2-d]pyrimidin-5-yl]-piperidine-1-carboxylate (75a)

According to the procedure described in example 72, step 1, compound **(67a)** (20 mg, 0.047 mmol) was converted, by reaction with tert-butyl (S)-3-Hydroxy-piperidine-1-carboxylate (67 mg, 0.33 mmol), to compound **(75a)** which was used without further purification. MS *m*/*z* ([M+H]⁺) 607.

### Step 2: 7-(1-Cyclohexyl-1H-benzimidazol-5-yl)-5-(R)-piperidin-3-yl-3,5-dihydro-pyrrolo[3,2-d]pyrimidin-4-one (Example 85)

According to the procedure described in example 72, step 2, crude compound **(75a)** was converted, after purification by preparative TLC on silica gel (EtOAc/MeOH (NH₃ 7M) 8/2), to **(Example 75)** (4.3 mg, 0.01 mmol, 22% yield over two steps). ¹H NMR (400 MHz, CD₃OD) δ 1.33-1.44 (m, 1H), 1.54-1.64 (m, 2H), 1.67-2.01 (m, 8H), 2.08-2.29 (m, 3H), 2.65-2.78 (m, 1H), 2.78-2.95 (m, 1H), 3.00-3.09 (m, 1H), 3.32-3.38 and 3.74-3.81 (2m, 1H), 4.32-4.40 (m, 1H), 5.15-5.23 (m, 1H), 7.60 (d, J = 8.5 Hz, 1H), 7.86 (dd, J = 1.5/8.5 Hz, 1H), 7.87 (s, 1H), 7.89 (s, 1H), 8.22 (s, 1H), 8.25 (d, J = 1.5 Hz, 1H). MS *m*/*z* ([M+H]⁺) 417.

### Example 76: Synthesis of 7-(1-Cyclohexyl-1H-benzimidazol-5-yl)-5-(S)-piperidin-3-yl-3,5-dihydro-pyrrolo[3,2-d]pyrimidin-4-one

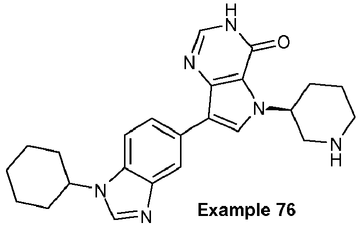

### Step 1: tert-butyl (S)-3-[4-Benzyloxy-7-(1-cyclohexyl-1H-benzimidazol-5-yl)-pyrrolo[3,2-d]pyrimidin-5-yl]-piperidine-1-carboxylate (76a)

According to the procedure described in example 72, step 1, compound **(67a)** (20 mg, 0.047 mmol) was converted, by reaction with tert-butyl (R)-3-Hydroxy-piperidine-1-carboxylate (67 mg, 0.33 mmol), to compound **(76a)** which was used without further purification. MS *m*/*z* ([M+H]⁺) 607.

### Step 2: 7-(1-Cyclohexyl-1H-benzimidazol-5-yl)-5-(S)-piperidin-3-yl-3,5-dihydro-pyrrolo[3,2-d]pyrimidin-4-one (Example 76)

According to the procedure described in example 72, step 2, crude compound **(76a)** was converted, after purification by preparative TLC on silica gel (EtOAc/MeOH (NH₃ 7M) 8/2), to **(Example 76)** (7 mg, 0.017 mmol, 36% yield over two steps). ¹H NMR (400 MHz, CD₃OD) δ 1.31-1.43 (m, 1H), 1.49-1.63 (m, 2H), 1.67-1.83 (m, 1H), 1.84-1.99 (m, 5H), 2.05-2.28 (m, 3H), 2.60-2.78 (m, 2H), 2.85-3.06 (m, 3H), 3.30-3.33 and 3.69-3.76 (2m, 1H), 4.31-4.38 (m, 1H), 5.13-5.21 (m, 1H), 7.60 (d, J = 8.5 Hz, 1H), 7.84 (dd, J = 1.5/8.5 Hz, 1H), 7.86 (s, 1H), 7.88 (s, 1H), 8.21 (s, 1H), 8.24 (d, J = 1.5 Hz, 1H). MS *m*/*z* ([M+H]⁺) 417.

### Example 77: Synthesis of 7-(1-Cyclohexyl-1H-benzimidazol-5-yl)-5-pyrrolidin-3-yl-3,5-dihydro-pyrrolo[3,2-d]pyrimidin-4-one

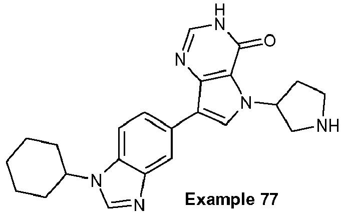

### Step 1: Benzyl 3-[4-Benzyloxy-7-(1-cyclohexyl-1H-benzimidazol-5-yl)-pyrrolo[3,2-d]pyrimidin-5-yl]-pyrrolidine-1-carboxylate (77a)

According to the procedure described in example 72, step 1, compound **(67a)** (50 mg, 0.118 mmol) was converted, by reaction with benzyl 3-Hydroxy-pyrrolidine-1-carboxylate (157 mg, 0.71 mmol), to compound **(77a)** which was used without further purification. MS *m*/*z* ([M+H]⁺) 627.

### Step 2: 7-(1-Cyclohexyl-1H-benzimidazol-5-yl)-5-pyrrolidin-3-yl-3,5-dihydro-pyrrolo[3,2-d]pyrimidin-4-one (Example 77)

According to the procedure described in example 70, step 2, crude compound **(77a)** was converted, after purification by preparative TLC on silica gel (DCM/MeOH (NH₃ 7M) 9/1), to **(Example 77)** (14.5 mg, 0.036 mmol, 30% yield over two steps). ¹H NMR (400 MHz, CD₃OD) δ 1.32-1.41 (m, 1H), 1.51-1.61 (m, 2H), 1.73-1.98 (m, 5H), 2.10-2.25 (m, 3H), 2.39-2.49 (m, 1H), 3.00-3.07 (m, 1H), 3.15-3.20 (m, 1H), 3.28-3.34 (m, 1H), 3.38-3.45 (m, 1H), 4.29-4.38 (m, 1H), 5.70-5.77 (m, 1H), 7.57 (d, J = 8.5 Hz, 1H), 7.84-7.86 (m, 3H), 8.20 (s, 1H), 8.25 (d, J = 1.5 Hz, 1H). MS *m*/*z* ([M+H]⁺) 403.

### Example 78: Synthesis of 7-(1-Cyclohexyl-1H-benzimidazol-5-yl)-5-(2-dimethylamino-ethyl)-3,5-dihydro-pyrrolo[3,2-d]pyrimidin-4-one

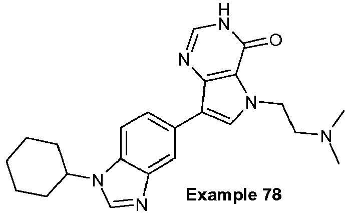

### Step 1: {2-[4-Benzyloxy-7-(1-cyclohexyl-1H-benzimidazol-5-yl)-pyrrolo[3,2-d]pyrimidin-5-yl]-ethyl}-dimethyl-amine (78a)

According to the procedure described in example 72, step 1, compound **(67a)** (50 mg, 0.118 mmol) was converted, by reaction with N,N-dimethylethanolamine (71 µL, 0.71 mmol), to compound **(78a)** which was used without further purification. MS *m*/*z* ([M+H]⁺) 495.

### Step 2: 7-(1-Cyclohexyl-1H-benzimidazol-5-yl)-5-(2-dimethylamino-ethyl)-3,5-dihydro-pyrrolo[3,2-d]pyrimidin-4-one (Example 78)

According to the procedure described in example 72, step 2, crude compound **(78a)** was converted, after purification by preparative TLC on silica gel (EtOAc/MeOH (NH₃ 7M) 9/1), to **(Example 78)** (32 mg, 0.079 mmol, 67% yield over two steps). ¹H NMR (400 MHz, CDCl₃) δ 1.25-1.37 (m, 1H), 1.45-1.56 (m, 2H), 1.75-1.86 (m, 3H), 1.91-2.01 (m, 2H), 2.17-2.27 (m, 2H), 2.33 (s, 6H), 2.82 (t, J = 6.6 Hz, 2H), 4.13-4.22 (m, 1H), 4.63 (t, J = 6.6 Hz, 2H), 7.45 (d, J = 8.5 Hz, 1H), 7.51 (s, 1H), 7.91 (dd, J = 1.5/8.5 Hz, 1H), 7.93 (s, 1H), 8.00 (s, 1H), 8.22 (d, J = 1.5 Hz, 1H), 11.94 (bs, 1H). MS *m*/*z* ([M+H]⁺) 405.

### Example 79: Synthesis of 7-(1-Cyclohexyl-1H-benzimidazol-5-yl)-5-(1-methyl-piperidin-3-yl)-3,5-dihydro-pyrrolo[3,2-d]pyrimidin-4-one

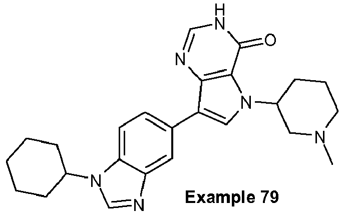

### Step 1: 4-Benzyloxy-7-(1-cyclohexyl-1H-benzimidazol-5-yl)-5-(1-methyl-piperidin-3-yl)-5H-pyrrolo[3,2-d]pyrimidine (79a)

According to the procedure described in example 72, step 1, compound **(67a)** (50 mg, 0.118 mmol) was converted, by reaction with 1-Methyl-piperidin-3-ol (82 µL, 0.71 mmol), to compound **(79a)** which was used without further purification. MS *m*/*z* ([M+H]⁺) 521.

### Step 2: 7-(1-Cyclohexyl-1H-benzimidazol-5-yl)-5-(1-methyl-piperidin-3-yl)-3,5-dihydro-pyrrolo[3,2-d]pyrimidin-4-one (Example 79)

According to the procedure described in example 72, step 2, crude compound **(79a)** was converted, after purification by preparative TLC on silica gel (EtOAc/MeOH (NH₃ 7M) 9/1), to **(Example 79)** (29 mg, 0.067 mmol, 57% yield over two steps). ¹H NMR (400 MHz, CDCl₃) δ 1.30-1.39 (m, 1H), 1.47-1.55 (m, 2H), 1.62-1.74 (m, 3H), 1.77-1.92 (m, 4H), 1.96-2.02 (m, 2H), 2.20-2.29 (m, 3H), 2.37 (s, 3H), 2.76 (bs, 1H), 3.10 (bs, 1H), 4.17-4.23 (m, 1H), 4.24-4.30 (m, 1H), 4.74-4.79 (dd, J = 4.8/13.2 Hz, 1H), 7.47 (d, J = 8.5 Hz, 1H), 7.55 (s, 1H), 7.87 (d, J = 1.5 Hz, 1H), 7.93 (dd, J = 1.5/8.5 Hz, 1H), 7.99 (s, 1H), 8.22 (d, J = 1.5 Hz, 1H), 9.45 (bs, 1H). MS *m*/*z* ([M+H]⁺) 431.

### Example 80: Synthesis of 7-(1-Cyclohexyl-1H-benzimidazol-5-yl)-5-(3-dimethylamino-propyl)-3,5-dihydro-pyrrolo[3,2-d]pyrimidin-4-one

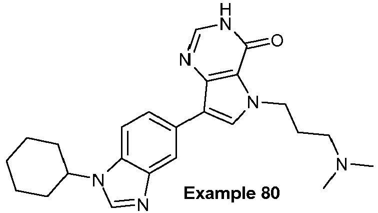

### Step 1: {3-[4-Benzyloxy-7-(1-cyclohexyl-1H-benzimidazol-5-yl)-pyrrolo[3,2-d]pyrimidin-5-yl]-propyl}-dimethyl-amine (80a)

According to the procedure described in example 72, step 1, compound **(67a)** (50 mg, 0.118 mmol) was converted, by reaction with N,N-dimethylpropanolamine (84 µL, 0.71 mmol), to compound **(80a)** which was used without further purification. MS *m*/*z* ([M+H]⁺) 509.

### Step 2: 7-(1-Cyclohexyl-1H-benzimidazol-5-yl)-5-(3-dimethylamino-propyl)-3,5-dihydro-pyrrolo[3,2-d]pyrimidin-4-one (Example 80)

According to the procedure described in example 72, step 2, crude compound (80a) was converted, after purification by preparative TLC on silica gel (DCM/MeOH (NH₃ 7M) 9/1), to (Example 80) (20 mg, 0.048 mmol, 40% yield over two steps). ¹H NMR (400 MHz, CDCl₃) δ 1.31-1.39 (m, 1H), 1.47-1.58 (m, 2H), 1.77-1.87 (m, 3H), 1.95-2.03 (m, 2H), 2.08 (q, J = 6.8 Hz, 2H), 2.20-2.24 (m, 2H), 2.25 (s, 6H), 2.30 (t, J = 6.8 Hz, 2H), 4.17-4.24 (m, 1H), 4.55 (t, J = 6.8 Hz, 2H), 7.47 (s, 1H), 7.48 (d, J = 8.5 Hz, 1H), 7.90 (s, 1H), 7.94 (dd, J = 1.5/8.5 Hz, 1H), 7.99 (s, 1H), 8.21 (d, J = 1.5 Hz, 1H), 10.14 (bs, 1H). MS *m*/*z* ([M+H]⁺) 419.

### Example 81: Synthesis of 7-(1-Cyclohexyl-1H-benzimidazol-5-yl)-5-(tetrahydro-pyran-4-yl)-3,5-dihydro-pyrrolo[3,2-d]pyrimidin-4-one

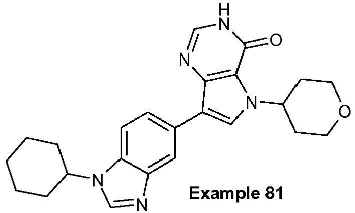

### Step 1: 4-Benzyloxy-7-(1-cyclohexyl-1H-benzimidazol-5-yl)-5-(tetrahydro-pyran-4-yl)-5H-pyrrolo[3,2-d]pyrimidine (81a)

According to the procedure described in example 72, step 1, compound **(67a)** (50 mg, 0.118 mmol) was converted, by reaction with 4-hydroxytetrahydropyrane (70 µL, 0.71 mmol), to compound **(81a)** which was used without further purification. MS *m*/*z* ([M+H]⁺) 508.

### Step 2: 7-(1-Cyclohexyl-1H-benzimidazol-5-yl)-5-(tetrahydro-pyran-4-yl)-3,5-dihydro-pyrrolo[3,2-d]pyrimidin-4-one (Example 81)

According to the procedure described in example 72, step 2, crude compound (81a) was converted, after purification by preparative TLC on silica gel (DCM/MeOH (NH₃ 7M) 9/1), to **(Example 81)** (17 mg, 0.041 mmol, 35% yield over two steps). ¹H NMR (400 MHz, CDCl₃) δ 1.28-1.40 (m, 1H), 1.48-1.59 (m, 3H), 1.78-1.88 (m, 3H), 1.95-2.03 (m, 2H), 2.06-2.14 (m, 1H), 2.15-2.19 (m, 2H), 2.21-2.29 (m, 2H), 3.67 (dt, J = 2.2/11.8 Hz, 2H), 4.15 (dd, J = 4.0/11.8 Hz, 2H), 4.19-4.25 (m, 1H), 5.38-5.46 (m, 1H), 7.49 (d, J = 8.5 Hz, 1H), 7.59 (s, 1H), 7.89 (dd, J = 1.6/6.6 Hz, 1H), 7.91 (s, 1H), 8.02 (s, 1H), 8.24 (d, J = 1.6 Hz, 1H), 9.61 (bs, 1H). MS *m*/*z* ([M+H]⁺) 418.

### Example 82: Synthesis of 7-(1-Cyclohexyl-1H-benzimidazol-5-yl)-5-(1-isopropyl-piperidin-4-yl)-3,5-dihydro-pyrrolo[3,2-d]pyrimidin-4-one

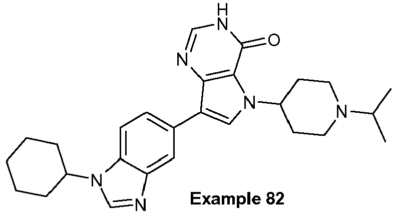

### Step 1: 4-Benzyloxy-7-(1-cyclohexyl-1H-benzimidazol-5-yl)-5-(1-isopropyl-piperidin-4-yl)-5H-pyrrolo[3,2-d]pyrimidine (82a)

According to the procedure described in example 72, step 1, compound **(67a)** (50 mg, 0.118 mmol) was converted, by reaction with 1-Isopropyl-piperidin-4-ol (100 mg, 0.71 mmol) and after trituration in EtOH, to crude compound **(82a)** which was used without further purification. MS *m*/*z* ([M+H]⁺) 549.

### Step 2: 7-(1-Cyclohexyl-1H-benzimidazol-5-yl)-5-(1-isopropyl-piperidin-4-yl)-3,5-dihydro-pyrrolo[3,2-d]pyrimidin-4-one (Example 82)

According to the procedure described in example 72, step 2, crude compound **(82a)** was converted, after purification by preparative TLC on silica gel (DCM/MeOH (NH₃ 7M) 9/1), to **(Example 82)** (14 mg, 0.03 mmol, 26% yield over two steps). ¹H NMR (400 MHz, DMSO-*d₆*) δ 0.99 (bs, 6H), 1.24-1.35 (m, 1H), 1.45-1.56 (m, 2H), 1.70-1.77 (m, 1H), 1.80-1.93 (m, 4H), 1.96-2.12 (m, 6H), 2.22-2.35 (m, 2H), 2.73-2.85 (m, 1H), 2.90-3.01 (m, 2H), 4.31-4.39 (m, 1H), 5.00-5.13 (m, 1H), 7.62 (d, J = 8.5 Hz, 1H), 7.90 (d, J = 2.8 Hz, 1H), 7.96 (dd, J = 1.5/8.5 Hz, 1H), 8.14 (bs, 1H), 8.27 (s, 1H), 8.46 (d, J = 1.5 Hz, 1H), 12.00 (bs, 1H). MS *m*/*z* ([M+H]⁺) 459.

### B-Biological activity

### ○ Examples of pharmaceutical compositions:

Solutions for IV administration have been prepared by diluting the compounds of example 13, 28, 51, 63, 73 or 81 at 1-20 mg/mL in a 10% solution of Hydroxypropyl-beta-cyclodextrine in water for injection, with or without addition of vancomycin (dose 5 mg/mL) or colistin (dose 75mg/ml). Solutions for IV administration have been prepared by diluting 500 mg of compounds of example 13, 28, 51, 63, 73 or 81 in 100 mL of a 10% solution of Hydroxypropyl-beta-cyclodextrine in water for injection.

### ○ Pharmacological study of the compounds of the invention

The effect of the compounds of the invention on DltA enzymes has been based on the measure of their inhibition of DltA activity *in vitro.* To this end, recombinant proteins DltA were expressed and purified after cloning of the genes of *S. aureus, E. faecalis, E. faecium* and *S*. *agalactiae* as described thereafter. The capacity of the compounds of the invention to inhibit DltA enzymatic activity was assessed using the DltA assays as described below:
The dltA gene was amplified from chromosomal DNA of *S. aureus, E. faecalis, E. faecium* and *S*. *agalactiae* and introduced into *E. coli* BL21 (Invitrogen, Carlsbad, CA) following manufacturer's instruction. Bacteria were grown at 37°C under aerobic conditions and isopropyl-beta-D-thiogalactopyranoside (IPTG) was added to 0.5-1 mM when OD₆₀₀ of the bacterial culture had reached 0.5. After a further incubation for 3 hours, cells were harvested by centrifugation. Purification of the recombinant DltA protein was performed by affinity chromatography on nickel-nitrilotriacetic acid (Ni-NTA) column following manufacturer's instructions (Qiagen). Fractions containing recombinant DltA protein were adjusted to 50% glycerol and stored at -20°C until use.

The binding potency of the inhibitors against DltA enzyme was assessed by IC₅₀ determination in an *in vitro* DltA activity assay. The assay buffer "AB" contained 50 mM Hepes pH8.0, 10mM MgCl₂, 50mM KCI, 0.012% Triton-X100, 10mM DTT and 100nM Myelin-basic protein. The following components were added in a white polystyrene Costar plate up to a final volume of 30µL: 3µL DMSO, or inhibitor dissolved in DMSO and 27µL DltA enzyme in AB. After 30min of preincubation at room temperature, 30µL of Substrates mix in AB were added in each well to a final volume of 60µL. This reaction mixture was then composed of DltA (produced in house from S. *aureus, E. faecalis, E. faecium* and *S. agalactiae*), 0.5mM D-Alanine (Sigma), 0.5-5µM ATP (Sigma) and 0.05 u/ml inorganic pyrophosphatase (Sigma) in assay buffer. After typically 1-2hours of incubation at room temperature (conversion rate around 30%), 30µL of the revelation mix were added to a final volume of 90µL, including the following constituents at the respective final concentrations: 10000 u/ml luciferase (Sigma), 30µM D-luciferin (Sigma), 100µM N-acetylcysteamine (Aldrich). Luminescence intensity was immediately measured on a Fluostar Optima (BMG) (excitation 360nm, emission 520nm) and converted into inhibition percentages. For IC₅₀ determinations (Inhibitory Concentration 50%) the inhibitor was added at 6 to 10 different concentrations and the related inhibitions were fitted to a classical Langmuir equilibrium model using XLFIT (IDBS).

For compounds interfering with the luminescent signal (quenching), the same experiment without DltA enzyme was performed to allow quenching determination and correction.

The effect of the compounds of the invention on controlling the bacterial D-alanylation of the wall teichoic acids (WTA) and lipoteichoic acids (LTA) has been based on the measure of their inhibition of bacterial D-alanylation *in vitro.* The strains used in this study were: *S. aureus* SA113, *E*. *faecalis* ATCC 29212, *E. faecium* (VRE) SF090703 and *S. agalactiae* NEM316.

To this end, the compounds to be tested were prepared in deionised water / DMSO (60/40) solutions and distributed (75µl) in a sterile deep-well culture microplate.

The bacteria were grown on tryptic soy agar (TSA) over-night. Isolated colonies were cultured in 10ml of cation-adjusted Mueller-Hinton broth (ca-MHB) for the staphylococci and Todd-Hewitt + 0.5% yeast extracts (THYE) for the streptococci and the enterococci up to an optical density (OD) typically of 0.15. These exponentially growing bacteria were finally diluted to 5e5 cfu/ml and added in each well (1.425ml) for incubation with the compounds at 37°C, with 5% CO2 for streptococci and enterococci. After 16-20H culture, bacteria were pelleted, heat inactivated in 0.5ml MES 0.1M pH6.0 + TritonX100 0.1% for 30min at 100°C. Resulting mixture was centrifuged/washed 3 times in 0.25ml of MES 0.1M pH6.0. The resulting pellet of cell-wall extracts was resuspended in 0.5ml of a mild alkaline buffer TRIS 0.2M pH8.5 + 200µM TritonX100 and heated for 3H at 60°C in an air oven to allow D-Alanine (D-Ala) hydrolysis from the teichoic acids. The mixture was finally centrifuged to perform D-Ala titration in the supernatant, and phosphate titration in the pellet.

D-Ala content was measured with a coupled enzymatic assay using D-amino acid oxidase (DAAO) and peroxidase (HRP) associated with Amplex-Red® as fluorophore (method modified from Kontinen et al., J. Biol. Chem 2000). Dosages were carried out in black polystyrene 96-well microplates in 100 µl final volume. Assay buffer was TRIS 0.2M pH8.5 + 200µM TritonX100. Reagents were added in the microplate in the following order:

D-Ala samples (50µl) or DAla scale for reference, Revelation mix (40µl) containing FAD 5µM, HRP 1u/ml, 5µM Amplex Red (final concentrations). Background signal was read at this point, followed by addition of 0.15u/ml DAAO (10µl). Plate was finally read approximately 90min later (Fluostar Optima from BMG, excitation at 544nm, emission at 590nm). Data treatment: background was subtracted from 90min-signal, then a D-Ala standard curve was established with the references, and used to convert signals into D-Ala concentrations for each sample. Finally, results were expressed in nmol DAla/sample.

Phosphate (Pi) titration was adapted from Zhou et al. (J. Lipid Res., 1992, 33, 1233) and started by Pi hydrolysis from cell wall extracts: pellets coming from DAla hydrolysis were incubated for 2.5 hours in 1ml 0.9M H₂SO₄/0.16M HClO₄. Titrations were carried out in clear polystyrene plates in 200µl final volume. Reagents were added in the microplate in the following order: Pi hydrolysed samples or KH2PO4 scale for reference (100µl), Ammonium molybdate 6mM (50µl), and Malachite green 0.12mM + Tween20 0.06% (50µl). The OD at 620nm was read 20min later (Fluostar Optima from BMG). A Pi standard curve was established with the references, and used to convert samples signals into Pi concentrations. Finally, results were expressed in nmol Pi/sample.

Final treatment: for each sample (treated and untreated), the normalised ratio DAla/Pi was calculated and used to calculate the inhibition percentages of D-Alanylation in the treated samples compared to the untreated ones. The EC₅₀ was defined as the dose of DltA inhibitor leading to 50% inhibition of teichoic acids D-Alanylation for the considered strain.

### Inhibitory activities of the compounds of formula (I)

**IC₅₀ of the compounds of formula (I) on *S*. *aureus* DltA, *E. feacalis* DltA, *E. feacium* DltA and *S***. ***Agalactiae* DltA**

| **Examples** | ***S. aureus* DltA** IC₅₀ (µM) | ***E. feacalis* DltA** IC₅₀ (µM) | ***E. feacium* DltA** IC₅₀ (µM) | ***S. Agalactiae* DltA** IC₅₀ (µM) |
|---|---|---|---|---|
| **7** | 0.07 | 0.037 | 0.12 | 3.2 |
| **11** | 0.14 | 0.12 | 0.19 | 5.4 |
| **13** | 0.044 | 0.041 | 0.14 | 0.98 |
| **17** | 0.22 | 0.085 | 0.19 | 2.2 |
| **25** | 0.039 | 0.022 | 0.088 | 0.63 |
| **28** | 0.15 | 0.048 | 0.16 | 1.2 |
| **30** | 0.22 | 0.16 | 0.32 | 2.0 |
| **34** | 0.027 | 0.014 | 0.061 | 0.4 |
| **35** | 0.054 | 0.039 | 0.093 | 0.87 |
| **37** | 0.068 | 0.025 | 0.042 | 0.66 |
| **38** | 0.13 | 0.042 | 0.13 | 0.91 |
| **45** | 0.13 | 0.070 | 0.39 | 3.4 |
| **46** | 0.15 | 0.15 | 0.44 | 5.1 |
| **47** | 0.077 | 0.046 | 0.11 | 2.6 |
| **48** | 0.068 | 0.046 | 0.11 | 3.7 |
| **49** | 0.097 | 0.082 | 0.16 | 3.3 |
| **51** | 1.1 | 0.33 | 1.2 | 4.5 |
| **52** | 0.15 | 0.026 | 0.12 | 0.35 |
| **53** | 0.15 | 0.013 | 0.094 | 0.59 |
| **58** | 0.81 | 0.19 | 0.48 | 6.1 |
| **63** | 0.46 | 0.35 | 1.1 | 25 |
| **67** | 0.15 | 0.18 | 0.93 | 3.8 |
| **68** | 0.29 | 0.32 | 0.73 | 4.5 |
| **70A** | 0.66 | 0.57 | 0.92 | 2.4 |
| **71A** | 0.36 | 0.36 | 0.89 | 2.7 |
| **73** | 0.072 | 0.061 | 0.28 | 1.0 |
| **74** | 0.15 | 0.093 | 0.14 | 1.1 |
| **76** | 0.067 | 0.069 | 0.46 | 1.2 |
| **77** | 0.093 | 0.13 | 0.51 | 1.6 |
| **80** | 0.27 | 0.055 | 0.62 | 1.5 |
| **81** | 0.076 | 0.17 | 0.3 | 2.5 |
| **82** | 0.056 | 0.1 | 0.38 | 1.9 |

## Claims

**1.** A compound of general formula (I) and their addition salts thereof with acids and bases: 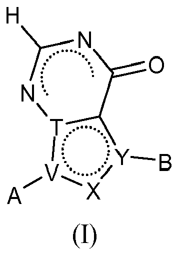 wherein:
the dotted line in the 6-membered heterocycle indicates a double bond in either of the possible positions between one C and one N;
the dotted line in the 5-membered cycle indicates a cycle with an aromatic character;
T is C or N,
VisCorN,
Y is C or N,
X is CH or S with the proviso that when X is S then T is C, V is C and Y is C,
A is selected from the group consisting of phenyl, naphthyl and (5-11) membered monocyclic or bicyclic unsaturated cycle or heterocycle linked by a carbon atom and containing 1-4 heteroatoms selected from N, O and S, all the above members of the group representing A being possibly substituted on carbon or on nitrogen atoms by 1-4 identical or different groups R₁; and
A can also comprise either a further (4-7) membered heterocycle formed with R₁, said heterocyle being a monocycle fused, saturated or unsaturated, and closed with R₁ by a simple bond or a double bond, or a further (4-7) membered monocycle formed by two substituents R₁ one with the other, said monocycle being then fused, saturated or unsaturated, and the two R₁ being linked together by a simple bond or a double bond, the polycyclic system representing A with R₁ then comprising up to 14 members and up to 5 heteroatoms selected from N, O and S;
B is selected from the group consisting of Halogen, NR₂R₃, SO₂NRₐR_{b}, (C₂-C₆)Alkyl, (C₂-C₆)Alkenyl, (C₂-C₆)Alkynyl, (C₃-C₁₂)Cycloalkyl, (C₅-C₈)Cycloalkenyl, O-(C₁-C₆)Alkyl, O-(C₃-C₁₂)Cycloalkyl, S-(C₁-C₆)Alkyl and S-(C₃-C₁₂)Cycloalkyl, the Alkyl, Alkenyl, Cycloalkenyl, Alkynyl and Cycloalkyl groups being possibly substituted by 1 to 3 identical or different substituents selected from the groups consisting of (C₁-C₆)Alkyl, (C₁-C₆)Alkyl-O-Rₐ, (C₁-C₆)Alkyl-S-Rₐ, (C₁-C₆)Alkyl-NRₐR_{b}, C(O)-O-Rₐ, C(O)-NRₐR_{b}, O-Rₐ, NR₂R₃, O-(C₇-C₁₂)Aralkyl and NRₐR_{b};
or B is a (4-10) membered monocyclic or bicyclic saturated or unsaturated heterocycle containing 1 to 3 heteroatoms selected from N, O and S, and possibly substituted by 1 to 3 identical or different substituents R₄;
R₁ is selected from the group consisting of Hydrogen, Halogen, CN, C(Halogen)ₙ with n an integer equal to 1, 2 or 3, NO₂, (C₁-C₆)Alkyl, (C₃-C₁₂)Cycloalkyl, =O, =N-O-(C₁-C₆)Alkyl, =N-O-(C₃-C₁₂)Cycloalkyl, C(O)-NRₐR_{b}, O-Rₐ, NR₂R₃ and NRₐC(O)R_{b}; the Alkyl and Cycloalkyl groups being possibly substituted by 1 to 3 identical or different substituents selected from the group consisting of Halogen, (C₁-C₆)Alkyl, (C₃-C₁₂)Cycloalkyl, O-Rₐ and NR₂R₃;
or R₁ is a (4-8) membered monocyclic saturated or unsaturated heterocycle containing 1-3 heteroatoms selected from N, O and S, and possibly substituted by 1 to 3 identical or different substituents selected from the group consisting of Halogen, (C₁-C₆)Alkyl, (C₃-C₁₂)Cycloalkyl, O-Rₐ and NR₂R₃;
or R₁ forms with A, or forms within A with another R₁, a cycle defined above;
R₂ and R₃, identical or different, are selected from the group consisting of Hydrogen, (C₁-C₆)Alkyl, (C₃-C₁₂)Cycloalkyl, C(O)-Rₐ, C(O)-NRₐR_{b}, C(O)-ORₐ, SO₂(C₁-C₆)Alkyl, SO₂(C₃-C₁₂)Cycloalkyl and O-Rₐ, the Alkyl and Cycloalkyl groups being possibly substituted by 1 to 3 identical or different substituents selected from the group consisting of Halogen, O-Rₐ, NRₐR_{b}, NRₐC(O)-R_{b}, C(O)-Rₐ, SO₂(C₁-C₆)Alkyl, SO₂(C₃-C₁₂)Cycloalkyl, C(O)-O-Rₐ and C(O)-NRₐR_{b};
or R₂ and R₃ are selected from the group consisting of (C₁-C₆)Alkyl and (C₃-C₁₂)Cycloalkyl, which are substituted by a (3-10) membered monocyclic or bicyclic saturated or unsaturated heterocycle possibly containing 1-3 heteroatoms selected from N, O and S, and possibly substituted by 1 to 3 identical or different substituents selected from the group consisting of (C₁-C₆)Alkyl, (C₃-C₁₂)Cycloalkyl and C(O)Rₐ;
or R₂ and R₃ form together with N a (4-10) membered saturated or unsaturated monocyclic or bicyclic heterocycle possibly containing another heteroatom selected from N, O, and S and possibly substituted by 1 to 3 identical or different groups R₄;
R₄ is selected from the group consisting of Halogen, =O, CN, (C₁-C₆)Alkyl, (C₃-C₁₂)Cycloalkyl, C(O)-Rₐ, C(O)-O-Rₐ, C(O)-NRₐR_{b}, O-Rₐ, N(Rₐ)-C(O)-R_{b} and NRₐR_{b}, the Alkyl and Cycloalkyl groups being possibly substituted by 1 to 3 identical or different substituents selected from the group consisting of O-Rₐ and NRₐR_{b};
Rₐ and R_{b}, identical or different, are selected from the group consisting of Hydrogen, (C₁-C₆)Alkyl and (C₃-C₁₂)Cycloalkyl, the Alkyl and Cycloalkyl groups being possibly substituted by 1 to 3 substituents selected from the group consiting of OH, O-(C₁-C₆)Alkyl and O-(C₃-C₁₂)Cycloalkyl; their tautomeric forms, the racemic forms, the pure enantiomers and the non-racemic (scalemic) mixture of enantiomers and diastereoisomers, in case the compounds of formula (I) have one or more chiral centers, as well as the cis (Z) and trans (E) isomers and their mixtures, in case the compounds of formula (I) have unsaturated carbon carbon double bonds.

**2.** Compounds of general formula (I) as defined in claim 1, wherein:
T is C;
V is C or N;
Y is C or N; and
X is S or CH with the proviso that when X is S then T is C, V is C and Y is C;
A is defined as in claim 1;
B is defined as in claim 1;
their tautomeric forms, the racemic forms, the pure enantiomers and the non-racemic (scalemic) mixture of enantiomers and diastereoisomers, in case the compounds of formula (I) have one or more chiral centers, as well as the cis (Z) and trans (E) isomers and their mixtures, in case the compounds of formula (I) have unsaturated carbon carbon double bonds.

**3.** Compounds of general formula (I) as defined in claim 1, wherein:
T is C;
V is C;
Y is C or N; and
X is S or CH with the proviso that when X is S then T is C, V is C and Y is C;
A is defined as in claim 1;
B is defined as in claim 1;
their tautomeric forms, the racemic forms, the pure enantiomers and the non-racemic (scalemic) mixture of enantiomers and diastereoisomers, in case the compounds of formula (I) have one or more chiral centers, as well as the cis (Z) and trans (E) isomers and their mixtures, in case the compounds of formula (I) have unsaturated carbon carbon double bonds.

**4.** Compounds of general formula (I) as defined in claim 1, wherein:
T is C;
V is C;
Y is C;
X is S;
A is defined as in claim 1,
B is selected from the group consisting of NR₂R₃, (C₂-C₆)Alkyl, (C₂-C₆)Alkenyl, (C₂-C₆)Alkynyl, (C₃-C₁₂)Cycloalkyl, the Alkyl, Alkenyl, Alkynyl and Cycloalkyl groups being possibly substituted by 1 to 3 identical or different substituents selected from the groups consisting of ORₐ, NR₂R₃, (C₁-C₆)Alkyl, O-(C₇-C₁₂)Aralkyl, (C₁-C₆)Alkyl-ORₐ and (C₁-C₆)Alkyl-NRaRb;
or B is a (4-10) membered monocyclic or bicyclic saturated or unsaturated heterocycle containing 1 to 3 heteroatoms selected from N, O and S, and possibly substituted by 1 to 3 identical or different substituents R₄;
all the other definitions being as in claim 1;
their tautomeric forms, the racemic forms, the pure enantiomers and the non-racemic (scalemic) mixture of enantiomers and diastereoisomers, in case the compounds of formula (I) have one or more chiral centers, as well as the cis (Z) and trans (E) isomers and their mixtures, in case the compounds of formula (I) have unsaturated carbon carbon double bonds.

**5.** Compounds of general formula (I) as defined in claim 1, wherein:
T is C;
V is C;
YisN;
X is CH,
A is defined as in claim 1;
B is selected from the group consisting of SO₂NRₐR_{b}, (C₂-C₆)Alkyl, (C₃-C₁₂)Cycloalkyl; the Alkyl and Cycloalkyl groups being possibly substituted by 1 to 3 identical or different substituents selected from the groups consisting of ORₐ, NR₂R₃, (C₁-C₆)Alkyl, O-(C₇-C₁₂)Aralkyl, (C₁-C₆)Alkyl-ORₐ and (C₁-C₆)Alkyl-NRₐR_{b};
or B is a (4-10) membered monocyclic or bicyclic saturated or unsaturated heterocycle containing 1 to 3 heteroatoms selected from N, O and S, and possibly substituted by 1 to 3 identical or different substituents R₄;
all the other definitions being as in claim 1;
their tautomeric forms, the racemic forms, the pure enantiomers and the non-racemic (scalemic) mixture of enantiomers and diastereoisomers, in case the compounds of formula (I) have one or more chiral centers, as well as the cis (Z) and trans (E) isomers and their mixtures, in case the compounds of formula (I) have unsaturated carbon carbon double bonds.

**6.** Compounds of general formula (I) as defined in any one of the claim 1 to 5, wherein A is selected from the group consisting of phenyl and (5-11) membered monocyclic or bicyclic unsaturated cycle or heterocycle linked by a carbon atom and containing 1-4 heteroatoms selected from N, O and S; all the above members of the group representing A being substituted on a carbon or a nitrogen atoms by 1-4 identical or different groups R1; and R1 being defined as in claim 1.

**7.** The compounds of general formula (I) as defined in claims 1, which names follow:
- 7-(1-Cyclohexyl-1H-benzoimidazol-5-yl)-5-((S)-3-hydroxymethyl-pyrrolidin-1-yl)-3H-thieno[3,4-d]pyrimidin-4-one,
- 5-(3,3-Bis-hydroxymethyl-pyrrolidin-1-yl)-7-(1-cyclohexyl-1H-benzoimidazol-5-yl)-3H-thieno[3,4-d]pyrimidin-4-one,
- 7-(1-Cyclohexyl-1H-benzoimidazol-5-yl)-5-[(3-dimethylamino-propyl)-methyl-amino]-3H-thieno[3,4-d]pyrimidin-4-one,
- 7-(1-Cyclohexyl-1H-benzoimidazol-5-yl)-5-(hexahydro-pyrrolo[3,4-c]pyrrol-2-yl)-3H-thieno[3,4-d]pyrimidin-4-one hydrochloride,
- 7-(1-cyclohexyl-1H-benzoimidazol-5-yl)-5-piperazin-1-yl-3H-thieno[3,4-d]pyrimidin-4-one hydrochloride,
- 7-(1-cyclohexyl-1H-benzoimidazol-5-yl)-5-hydroxymethyl-hexahydro-pyrrolo[3,4-c]pyrrol-2-yl)-3H-thieno[3,4-d]pyrimidin-4-one hydrochloride,
- 5-((*3R,4R*)-3,4-Bis-hydroxymethyl-pyrrolidin-1-yl)-7-(1-cyclohexyl-1*H*-benzoimidazol-5-yl)-3*H-*thieno[3,4-d]pyrimidin-4-one,
- 7-(1-cyclohexyl-1H-benzoimidazol-5-yl)-5-(1,2,3,6-tetrahydro-pyridin-4-yl)-3H-thieno[3,4-d]pyrimidin-4-one hydrochloride,
- 7-(1-cyclohexyl-1H-benzoimidazol-5-yl)-5-(1,2,3,6-tetrahydro-pyridin-4-yl)-3H-thieno[3,4-d]pyrimidin-4-one hydrochloride,
- 7-(1-ethyl-1H-benzoimidazol-5-yl)-5-((S)-3-hydroxymethyl-pyrrolidin-1-yl)-3H-thieno[3,4-d]pyrimidin-4-one,
- 5-((*S*)-3-hydroxymethyl-pyrrolidin-1-yl)-7-[1-(2-methoxy-ethyl)-1H-benzoimidazol-5-yl]-3H-thieno[3,4-d]pyrimidin-4-one,
- 7-[1-((*1R*,*2R*)-2-fluoro-cyclohexyl)-1H-benzoimidazol-5-yl]-5-((*S*)-3-hydroxymethyl-pyrrolidin-1-yl)-3H-thieno[3,4-d]pyrimidin-4-one,
- 5-((*S*)-3-Hydroxymethyl-pyrrolidin-1-yl)-7-[1-(*trans*-3-ethyl-cyclohexyl)-1H-benzoimidazol-5-yl]-3H-thieno[3,4-d]pyrimidin-4-one,
- 5-((*S*)-3-hydroxymethyl-pyrrolidin-1-yl)-7-[1-(tetrahydro-pyran-3-yl)-1-H-benzoimidazol-5-yl]-3H-thieno[3,4-d]pyrimidin-4-one,
- 7-(1-Cyclohexyl-1H-benzoimidazol-5-yl)-5-(3-hydroxy-propyl)-3,7-dihydro-pyrrolo[2,3-d]pyrimidin-4-one,
- 7-(1-cyclohexyl-1*H*-benzoimidazol-5-yl)-5-(1,2,3,6-tetrahydro-pyridin-4-yl)-3,7-dihydro-pyrrolo[2,3-d]pyrimidin-4-one hydrochloride,
- 7-(1-cyclohexyl-1*H*-benzoimidazol-5-yl)-5-(1-methyl-1,2,3,6-tetrahydro-pyridin-4-yl)-3,7-dihydro-pyrrolo[2,3-d]pyrimidin-4-one,
- 7-(1-Cyclohexyl-1H-benzoimidazol-5-yl)-5-((S)-3-hydroxymethyl-pyrrolidin-1-yl)-3,7-dihydro-pyrrolo[2,3-d]pyrimidin-4-one,
- 7-(1-Cyclohexyl-1H-benzoimidazol-5-yl)-5-(3-hydroxymethyl-cyclopentyl)-3,7-dihydro-pyrrolo[2,3-d]pyrimidin-4-one,
- 7-(1-Cyclohexyl-1H-benzoimidazol-5-yl)-5-((S)-3-hydroxymethyl-pyrrolidin-1-yl)-3H-pyrrolo[2,1-f][1,2,4]triazin-4-one,
- 7-(1-Cyclohexyl-1H-benzimidazol-5-yl)-5-(3-hydroxy-prop-1-ynyl)-3H-pyrrolo[2,1-f][1,2,4]triazin-4-one,
- 7-(1-Cyclohexyl-1H-benzimidazol-5-yl)-5-(3-hydroxy-propyl)-3,5-dihydro-pyrrolo[3,2-d]pyrimidin-4-one,
- 7-(1-Cyclohexyl-1H-benzimidazol-5-yl)-5-(5-hydroxy-pentyl)-3,5-dihydro-pyrrolo[3,2-d]pyrimidin-4-one,
- 7-(1-Cyclohexyl-1H-benzimidazol-5-yl)-5-(1-methyl-piperidin-4-yl)-3,5-dihydro-pyrrolo[3,2-d]pyrimidin-4-one,
- 7-(1-Cyclohexyl-1H-benzimidazol-5-yl)-5-piperidin-4-yl-3,5-dihydro-pyrrolo[3,2-d]pyrimidin-4-one,
- 7-(1-Cyclohexyl-1H-benzimidazol-5-yl)-5-[*cis*-(4-hydroxymethyl-cyclohexyl)]-3,5-dihydro-pyrrolo[3,2-d]pyrimidin-4-one,
- 7-(1-Cyclohexyl-1H-benzimidazol-5-yl)-5-(S)-piperidin-3-yl-3,5-dihydro-pyrrolo[3,2-d]pyrimidin-4-one,
- 7-(1-Cyclohexyl-1H-benzimidazol-5-yl)-5-(3-dimethylamino-propyl)-3,5-dihydro-pyrrolo[3,2-d]pyrimidin-4-one,
- 7-(1-Cyclohexyl-1H-benzimidazol-5-yl)-5-(tetrahydro-pyran-4-yl)-3,5-dihydro-pyrrolo[3,2-d]pyrimidin-4-one,
- 7-(1-Cyclohexyl-1H-benzimidazol-5-yl)-5-(1-isopropyl-piperidin-4-yl)-3,5-dihydro-pyrrolo[3,2-d]pyrimidin-4-one.

**8.** The compound of general formula (I) and pharmaceutically acceptable addition salts thereof with acids and bases, as defined in any one of claim 1 to 7 for use as drugs.

**9.** The compounds of general formula (I) and pharmaceutically acceptable addition salts thereof with acids and bases, as defined in any of claims 1 to 7, for use as drugs for the prevention and/or therapeutical treatment of human or animal infections by microbial pathogens including Gram-positive bacteria.

**10.** The compounds of general formula (I) and pharmaceutically acceptable addition salts thereof with acids and bases, as defined in any of claims 1 to 7, for use as drugs, in association with an antibacterial, an antivirulence agent, a drug reinforcing the host innate immunity and combination thereof.

**14.** The compounds of general formula (I) and pharmaceutically acceptable addition salts thereof with acids and bases, as defined in any of claims 1 to 7, for use as drugs, in association with an antibacterial targeting the bacterial cell wall and/or membrane, an antibacterial of the CAMP type, an antibacterial of the glycopeptides type, an antibacterial of the lipopeptides type, and/or in association with immunomodulatory peptides, and/or in association with GM-CSF.

**15.** A pharmaceutical composition containing, as active principle, a therapeutically effective amount of at least one compound of general formula (I) or a pharmaceutically acceptable addition salt thereof with an acid or a base, as defined in any of claims 1 to 7 and in any of claims 8 to 14, and a pharmaceutically acceptable carrier and/or excipient.

**16.** A mixture or a pharmaceutical association comprising, as active principles, at least one compound of formula (I) or a pharmaceutically acceptable addition salt thereof with an acid or a base, as defined in any of claims 1 to 8, and at least an antibacterial, an antivirulence agent, a drug reinforcing the host innate immunity or a combination of any of them.

**17.** A mixture or pharmaceutical association comprising, as active principles, at least one compound of formula (I) or a pharmaceutically acceptable addition salt thereof with an acid or a base, as defined in any of claims 1 to 8, and at least one antibacterial targeting the bacterial cell wall and/or membrane, an antibacterial of the CAMP type, of the glycopeptides type, or of the lipopeptides type, and/or an immunomodulatory peptide, and/or a GM-CSF.
